# EUROPEAN PATENT APPLICATION

(11) **EP 4 242 196 A1**
(43) Date of publication of application: **13.09.2023**
(21) Application number: 22742879.4
(22) Date of filing: 21.01.2022
(51) Int. Cl.: C07C 17/013, C07C 2/64, C07C 22/04, C07C 15/27, C07C 15/24, C07D 307/91, C07D 209/82, C07D 213/16, C07D 493/04

(54) **METHOD FOR PREPARING DEUTERATED ANTHRACENE COMPOUND, REACTION COMPOSITION, DEUTERATED ANTHRACENE COMPOUND AND COMPOSITION**

(30) Priority: 22.01.2021 KR 20210009589
(71) Applicant: Lg Chem, Ltd., Seoul 07336 (KR)
(72) Inventor: KIM, Sunmin, Daejeon 34122 (KR); JEONG, Kyung Seok, Daejeon 34122 (KR); KIM, Woo Han, Daejeon 34122 (KR); JUNG, Changyoung, Daejeon 34122 (KR)
(74) Representative: Hoffmann Eitle
(86) International application number: PCT/KR2022/001132
(87) International publication number: WO 2022/158903

(57) **Abstract**

The present specification relates to a method for preparing a deuterated anthracene compound comprising the step of reacting halogenated benzene having at least one deuterium with the compound of chemical formula 1:
to synthesize a deuterated anthracene compound, a reaction composition, a deuterated anthracene compound, and a composition.

## Description

### Technical Field

This application claims priority to and the benefit of Korean Patent Application No. 10-2021-0009589 filed in the Korean Intellectual Property Office on January 22, 2021, the entire contents of which are incorporated herein by reference.

The present specification relates to a method for preparing a deuterated anthracene compound, a reaction composition, a deuterated anthracene compound and a composition.

### Background Art

Compounds including deuterium are used for various purposes. For example, compounds including deuterium can be frequently used not only as labeling compounds for elucidating the mechanism of a chemical reaction or elucidating a material metabolism, but also for drugs, pesticides, organic EL materials and other purposes.

A method of deuterium substitution of an aromatic compound is known in order to improve the lifespan of an organic light emitting device (OLED) material. The principle of such an effect is that while the LUMO energy of C-D bond is lower than that of C-H bond during deuterium substitution, the life characteristics of the OLED material are improved.

In the case of an anthracene derivative used as a material for an organic light emitting device, HOMO and LUMO are distributed in the anthracene, so that when the hydrogen of the anthracene is deuterated, the intramolecular vibrational energy of a C-D bond compared to a C-H bond can be reduced and the interaction between molecules can be reduced, thereby improving the service life of the organic light emitting device.

A hydrogen-deuterium exchange method is usually used as a deuteration technique of aromatic compounds. The hydrogen-deuterium exchange method is performed by the use of an organic solvent such as acetone-d6, DMSO-d6, DMF-d7, MeOH-d4, toluene-d5, and benzene-d6 or heavy water (D₂O) as a deuterium source, the use of an acid/base catalyst, supercritical heating, the use of a transition metal catalyst, and the like. The hydrogen-deuterium exchange method can be inefficient in terms of cost and time because a material to be deuterated needs to be able to withstand reaction conditions such as high temperature and high pressure, multiple treatments are required to achieve a high level of deuteration, and the amount of relatively expensive deuterated reagent used is large.

In order to universally use a deuterated aromatic compound as a material for an organic light emitting device, there is a need for a preparation method capable of reducing costs using a small amount of deuterium source.

### Detailed Description of the Invention

### Technical Problem

The present specification has been made in an effort to provide a method for preparing a deuterated anthracene compound, a reaction composition, a deuterated anthracene compound and a composition.

### Technical Solution

An exemplary embodiment of the present specification provides a method for preparing a deuterated anthracene compound, the method including: synthesizing a deuterated anthracene compound by reacting a halogenated benzene having at least one deuterium with a compound of Chemical Formula 1:

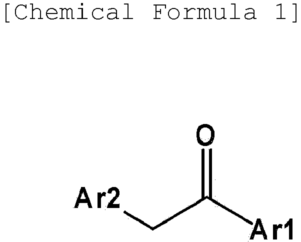

wherein in Chemical Formula 1:
Ar1 is deuterium, a substituted or unsubstituted aryl group, or a substituted or unsubstituted heterocyclic group; and
Ar2 is hydrogen, deuterium, a substituted or unsubstituted aryl group, or a substituted or unsubstituted heterocyclic group.

Further, another exemplary embodiment of the present specification provides a method for preparing a deuterated anthracene compound, the method including: synthesizing a compound of the following Chemical Formula 2 by reacting halogenated benzene-d5 with a compound of the following Chemical Formula 1:

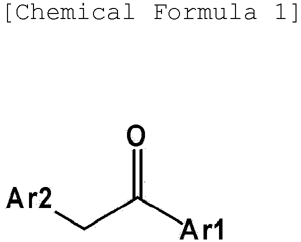

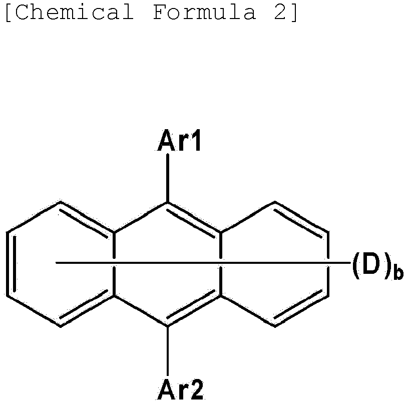

wherein in Chemical Formulae 1 and 2:
Ar1 is deuterium, a substituted or unsubstituted aryl group, or a substituted or unsubstituted heterocyclic group;
Ar2 is hydrogen, deuterium, a substituted or unsubstituted aryl group, or a substituted or unsubstituted heterocyclic group; and
b is an integer from 1 to 8.

In addition, still another exemplary embodiment of the present specification provides a reaction composition including a halogenated benzene having at least one deuterium, and a compound of Chemical Formula 1:

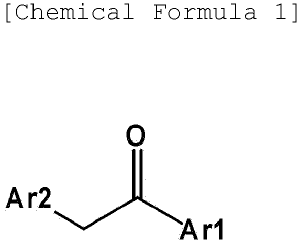

wherein in Chemical Formula 1, Ar1 is deuterium, a substituted or unsubstituted aryl group, or a substituted or unsubstituted heterocyclic group; and
Ar2 is hydrogen, deuterium, a substituted or unsubstituted aryl group, or a substituted or unsubstituted heterocyclic group.

Furthermore, yet another exemplary embodiment of the present specification provides a deuterated anthracene compound prepared by the above-described preparation method.

Further, yet another exemplary embodiment of the present specification provides a composition including an intermediate of Chemical Formula 5:

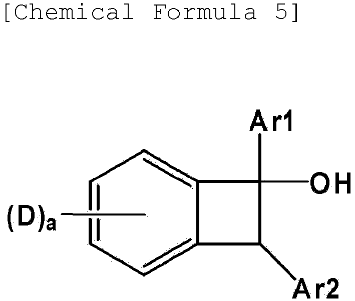

wherein in Chemical Formula 5:
Ar1 is deuterium, a substituted or unsubstituted aryl group, or a substituted or unsubstituted heterocyclic group;
Ar2 is hydrogen, deuterium, a substituted or unsubstituted aryl group, or a substituted or unsubstituted heterocyclic group; and
a is an integer from 1 to 4.

### Advantageous Effects

The preparation method of an exemplary embodiment of the present specification has an advantage in that costs are reduced using a small amount of deuterium source.

The preparation method of another exemplary embodiment of the present specification has an advantage in that the total process time for preparing a deuterated anthracene compound is reduced.

### Detailed Description

Hereinafter, the present specification will be described in detail.

A hydrogen-deuterium exchange method is usually used as a deuteration technique of aromatic compounds. The hydrogen-deuterium exchange method is inefficient in terms of cost and time because a material to be deuterated needs to be able to withstand reaction conditions such as high temperature and high pressure, multiple treatments are required to achieve a high level of deuterium substitution rate, and the amount of relatively expensive deuterium source used is large.

The present specification can dramatically reduce an amount of benzene-d6 or halogenated benzene-d5 used by directly synthesizing deuterated anthracene from benzene-d6 or halogenated benzene-d5 to utilize the deuterated anthracene as an intermediate of an organic light emitting material instead of the hydrogen-deuterium exchange method.

Further, in the case of an anthracene derivative used as a material for an organic light emitting device, a conversion rate for substituting hydrogen of anthracene with deuterium is important because HOMO and LUMO are distributed in anthracene. However, when an anthracene derivative in a final product state in which a substituent is applied to the positions of Nos. 9, 10, and the like of the anthracene is deuterated, substituents substituted in the anthracene, which are located relatively outside, are easily deuterated, but it is difficult to substitute the hydrogen of the anthracene, whose substituent is not substituted, with deuterium. Therefore, it is no exaggeration to say that the deuterium substitution rate of the anthracene derivative depends on how much deuterium is substituted for the hydrogen of the anthracene located inside.

However, since a method for preparing the deuterated anthracene compound of the present specification is a bottom-up method of directly synthesizing deuterated anthracene from benzene-d6 or halogenated benzene-d5, hydrogens at the other positions except for Nos. 9 and 10 positions in the anthracene are substituted with deuteriums before the substituent at No. 9 or 10 is substituted.

Therefore, in the deuterated anthracene compound prepared by the method for preparing a deuterated anthracene compound of the present specification, most of the hydrogens of the anthracene that has not been substituted with the substituent are substituted with deuteriums, so that an anthracene compound having a high deuterium substitution rate can be more easily prepared.

The present specification provides a method for preparing a deuterated anthracene compound, the method including: synthesizing a deuterated anthracene compound from benzene-d6 or a halogenated benzene having at least one deuterium.

The present specification provides a method for preparing a deuterated anthracene compound, the method including: synthesizing a deuterated anthracene compound by reacting a halogenated benzene having at least one deuterium with a compound of the following Chemical Formula 1.

In an exemplary embodiment of the present specification, the halogenated benzene having at least one deuterium can be halogenated benzene-d5.

In an exemplary embodiment of the present specification, the deuterated anthracene compound can be a compound of the following Chemical Formula 2.

The present specification provides a method for preparing a deuterated anthracene compound, the method including: synthesizing a compound of the following Chemical Formula 2 by reacting halogenated benzene-d5 with a compound of the following Chemical Formula 1.

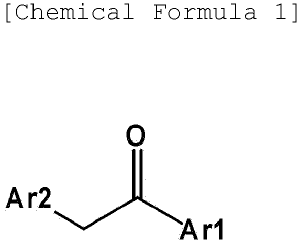

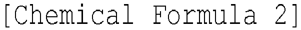

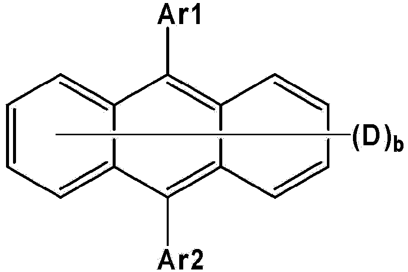

In Chemical Formulae 1 and 2,
Ar1 is deuterium, a substituted or unsubstituted aryl group, or a substituted or unsubstituted heterocyclic group,
Ar2 is hydrogen, deuterium, a substituted or unsubstituted aryl group, or a substituted or unsubstituted heterocyclic group, and
b is an integer from 1 to 8.

In an exemplary embodiment of the present specification, b is the number of deuteriums, and can be 1 to 8 depending on the number of deuteriums to be substituted.

In an exemplary embodiment of the present specification, b is 1.

In an exemplary embodiment of the present specification, b is 2.

In an exemplary embodiment of the present specification, b is 3.

In an exemplary embodiment of the present specification, b is 4.

In an exemplary embodiment of the present specification, b is 5.

In an exemplary embodiment of the present specification, b is 6.

In an exemplary embodiment of the present specification, b is 7.

In an exemplary embodiment of the present specification, b is 8. When b is 8, Chemical Formula 2 can be the following Chemical Formula 2-1:

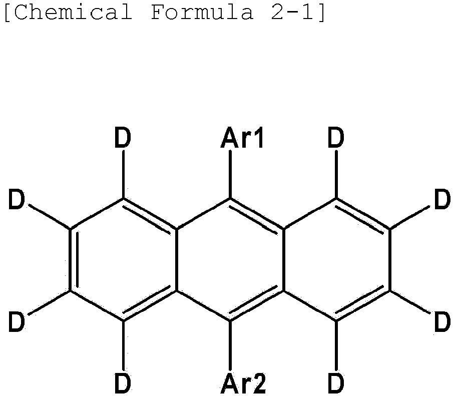

wherein in Chemical Formula 2-1, Ar1 and Ar2 are the same as those defined in Chemical Formula 2.

In an exemplary embodiment of the present specification, Ar1 is deuterium, a substituted or unsubstituted aryl group having 6 to 60 carbon atoms; or a substituted or unsubstituted heterocyclic group having 2 to 60 carbon atoms.

In an exemplary embodiment of the present specification, Ar1 is deuterium, a substituted or unsubstituted aryl group having 6 to 30 carbon atoms; or a substituted or unsubstituted heterocyclic group having 2 to 30 carbon atoms.

In an exemplary embodiment of the present specification, Ar1 is deuterium, a substituted or unsubstituted aryl group having 6 to 20 carbon atoms; or a substituted or unsubstituted heterocyclic group having 2 to 20 carbon atoms.

In an exemplary embodiment of the present specification, Ar1 is deuterium, a substituted or unsubstituted phenyl group, a substituted or unsubstituted biphenyl group, a substituted or unsubstituted terphenyl group, a substituted or unsubstituted naphthyl group, a substituted or unsubstituted phenanthrenyl group, a substituted or unsubstituted triphenylenyl group, a substituted or unsubstituted pyrenyl group, a substituted or unsubstituted fluorenyl group, a substituted or unsubstituted spirobifluorenyl group, a substituted or unsubstituted xanthenyl group, a substituted or unsubstituted thioxanthenyl group, a substituted or unsubstituted benzofuranyl group, a substituted or unsubstituted benzothiophenyl group, a substituted or unsubstituted dibenzofuranyl group, a substituted or unsubstituted dibenzothiophenyl group, a substituted or unsubstituted carbazolyl group, a substituted or unsubstituted benzonaphthofuranyl group, a substituted or unsubstituted benzonaphthothiophenyl group, a substituted or unsubstituted benzocarbazolyl group, or a substituted or unsubstituted dibenzocarbazolyl group.

In an exemplary embodiment of the present specification, Ar1 is deuterium, a substituted or unsubstituted phenyl group, a substituted or unsubstituted naphthyl group, a substituted or unsubstituted phenanthrenyl group, a substituted or unsubstituted triphenylenyl group, a substituted or unsubstituted pyrenyl group, a substituted or unsubstituted fluorenyl group, a substituted or unsubstituted dibenzofuranyl group, a substituted or unsubstituted dibenzothiophenyl group, or a substituted or unsubstituted carbazolyl group.

In an exemplary embodiment of the present specification, Ar1 is deuterium, a substituted or unsubstituted phenyl group, a substituted or unsubstituted naphthyl group, a substituted or unsubstituted dibenzofuranyl group, a substituted or unsubstituted dibenzothiophenyl group, or a substituted or unsubstituted carbazolyl group.

In an exemplary embodiment of the present specification, Ar1 is deuterium, a substituted or unsubstituted phenyl group, a substituted or unsubstituted naphthyl group, a substituted or unsubstituted dibenzofuranyl group, or a substituted or unsubstituted carbazolyl group.

In an exemplary embodiment of the present specification, Ar2 of Chemical Formula 1 or 2 is deuterium, a substituted or unsubstituted aryl group having 6 to 60 carbon atoms; or a substituted or unsubstituted heterocyclic group having 2 to 60 carbon atoms.

In an exemplary embodiment of the present specification, Ar2 of Chemical Formula 1 or 2 is deuterium, a substituted or unsubstituted aryl group having 6 to 30 carbon atoms; or a substituted or unsubstituted heterocyclic group having 2 to 30 carbon atoms.

In an exemplary embodiment of the present specification, Ar2 of Chemical Formula 1 or 2 is deuterium, a substituted or unsubstituted aryl group having 6 to 20 carbon atoms; or a substituted or unsubstituted heterocyclic group having 2 to 20 carbon atoms.

In an exemplary embodiment of the present specification, Ar2 of Chemical Formula 1 or 2 is deuterium, a substituted or unsubstituted phenyl group, a substituted or unsubstituted biphenyl group, a substituted or unsubstituted terphenyl group, a substituted or unsubstituted naphthyl group, a substituted or unsubstituted phenanthrenyl group, a substituted or unsubstituted triphenylenyl group, a substituted or unsubstituted pyrenyl group, a substituted or unsubstituted fluorenyl group, a substituted or unsubstituted spirobifluorenyl group, a substituted or unsubstituted xanthenyl group, a substituted or unsubstituted thioxanthenyl group, a substituted or unsubstituted benzofuranyl group, a substituted or unsubstituted benzothiophenyl group, a substituted or unsubstituted dibenzofuranyl group, a substituted or unsubstituted dibenzothiophenyl group, a substituted or unsubstituted carbazolyl group, a substituted or unsubstituted benzonaphthofuranyl group, a substituted or unsubstituted benzonaphthothiophenyl group, a substituted or unsubstituted benzocarbazolyl group, or a substituted or unsubstituted dibenzocarbazolyl group.

In an exemplary embodiment of the present specification, Ar2 of Chemical Formula 1 or 2 is deuterium, a substituted or unsubstituted phenyl group, a substituted or unsubstituted naphthyl group, a substituted or unsubstituted phenanthrenyl group, a substituted or unsubstituted triphenylenyl group, a substituted or unsubstituted pyrenyl group, a substituted or unsubstituted fluorenyl group, a substituted or unsubstituted dibenzofuranyl group, a substituted or unsubstituted dibenzothiophenyl group, or a substituted or unsubstituted carbazolyl group.

In an exemplary embodiment of the present specification, Ar2 of Chemical Formula 1 or 2 is deuterium, a substituted or unsubstituted phenyl group, a substituted or unsubstituted naphthyl group, a substituted or unsubstituted dibenzofuranyl group, a substituted or unsubstituted dibenzothiophenyl group, or a substituted or unsubstituted carbazolyl group.

In an exemplary embodiment of the present specification, Ar2 of Chemical Formula 1 or 2 is deuterium, a substituted or unsubstituted phenyl group, a substituted or unsubstituted naphthyl group, a substituted or unsubstituted dibenzofuranyl group, or a substituted or unsubstituted carbazolyl group.

In an exemplary embodiment of the present specification, Ar2 of Chemical Formula 1 is hydrogen or deuterium.

In an exemplary embodiment of the present specification, Ar2 of Chemical Formula 2 is hydrogen or deuterium.

In an exemplary embodiment of the present specification, Chemical Formula 1 can be any one of the following Chemical Formulae 1-1 to 1-3: 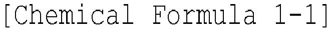 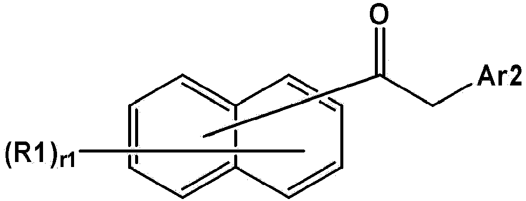 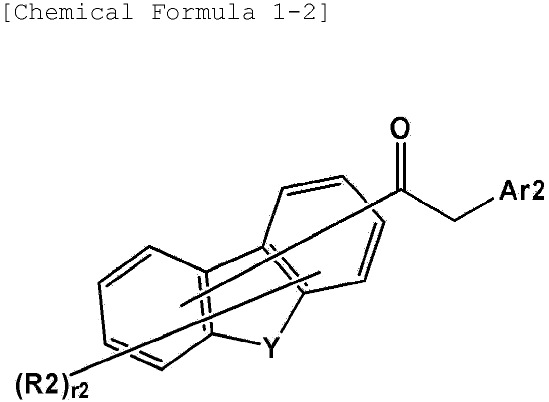 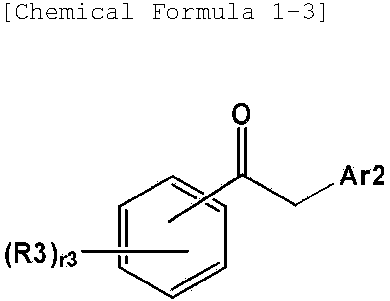 wherein in Formulae 1-1 to 1-3:
Y is CRaRb, NRc, O or S;
Ar2 is hydrogen, deuterium, a substituted or unsubstituted aryl group, or a substituted or unsubstituted heterocyclic group;
Ra, Rb, Rc and R1 to R3 are the same as or different from each other, and are each independently hydrogen, deuterium, a substituted or unsubstituted alkyl group, a substituted or unsubstituted aryl group, or a substituted or unsubstituted heterocyclic group, or are bonded to an adjacent group to form a substituted or unsubstituted ring;
r1 and r2 are each an integer from 1 to 7, and r3 is an integer from 1 to 5,
when r1 is 2 or higher, the R1s are the same as or different from each other,
when r2 is 2 or higher, the R2s are the same as or different from each other, and
when r3 is 2 or higher, the R3s are the same as or different from each other.

In an exemplary embodiment of the present specification, Y is CRaRb.

In an exemplary embodiment of the present specification, Y is NRc.

In an exemplary embodiment of the present specification, Y is O.

In an exemplary embodiment of the present specification, Y is S.

In an exemplary embodiment of the present specification, Ra and Rb are the same as or different from each other, and are each independently hydrogen, deuterium, a substituted or unsubstituted alkyl group, or a substituted or unsubstituted aryl group.

In an exemplary embodiment of the present specification, Ra and Rb are the same as or different from each other, and are each independently a substituted or unsubstituted alkyl group, or a substituted or unsubstituted aryl group.

In an exemplary embodiment of the present specification, Ra and Rb are the same as or different from each other, and are each independently a substituted or unsubstituted aryl group.

In an exemplary embodiment of the present specification, Ra and Rb are the same as or different from each other, and are each independently a substituted or unsubstituted phenyl group.

In an exemplary embodiment of the present specification, Ra and Rb are a phenyl group.

In an exemplary embodiment of the present specification, Rc is hydrogen, deuterium, a substituted or unsubstituted alkyl group, a substituted or unsubstituted aryl group, or a substituted or unsubstituted heterocyclic group.

In an exemplary embodiment of the present specification, Rc is hydrogen, deuterium, or a substituted or unsubstituted aryl group.

In an exemplary embodiment of the present specification, Rc is a substituted or unsubstituted aryl group.

In an exemplary embodiment of the present specification, Rc is a substituted or unsubstituted phenyl group.

In an exemplary embodiment of the present specification, Rc is a phenyl group.

In the present specification, the "adjacent" group can mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring can be interpreted as groups which are "adjacent" to each other. In addition, substituents (four in total) linked to two consecutive carbons in an aliphatic ring can be interpreted as "adjacent" groups.

In the present specification, the "adjacent groups are bonded to each other to form a ring" among the substituents means that a substituent is bonded to an adjacent group to form a substituted or unsubstituted hydrocarbon ring; or a substituted or unsubstituted hetero ring.

In the present specification, "a five-membered or six-membered ring formed by bonding adjacent groups" means that a ring including a substituent participating in the ring formation is five-membered or six-membered. It is possible to include an additional ring fused to the ring including the substituent participating in the ring formation.

In the present specification, an aromatic hydrocarbon ring means a hydrocarbon ring in which pi electrons are completely conjugated and are planar, and a description on an aryl group to be described below can be applied to an aromatic hydrocarbon ring except for a divalent aromatic hydrocarbon ring.

In the present specification, an aliphatic hydrocarbon ring has a cyclically bonded structure, and means a non-aromatic ring. Examples of the aliphatic hydrocarbon ring include cycloalkyl or cycloalkene, a description on a cycloalkyl group to be described below can be applied to cycloalkyl except for a divalent cycloalkyl, and the cycloalkene means that one or more of the single bonds in cycloalkyl have been substituted with double bonds. Further, a substituted aliphatic hydrocarbon ring also includes an aliphatic hydrocarbon ring in which aromatic rings are fused.

In the present specification, a fused ring of an aromatic hydrocarbon ring and an aliphatic hydrocarbon ring means that an aromatic hydrocarbon ring and an aliphatic hydrocarbon ring form a fused ring. Examples of the fused ring of the aromatic ring and the aliphatic ring include a 1,2,3,4-tetrahydronaphthalene group, a 2,3-dihydro-1H-indene group, and the like, but are not limited thereto.

In the present specification, the description on the heterocyclic group to be described below can be applied to a hetero ring except for a divalent hetero ring.

In an exemplary embodiment of the present specification, Ra, Rb, Rc and R1 to R3 can be each independently bonded to an adjacent group to form a substituted or unsubstituted ring.

In an exemplary embodiment of the present specification, Ra, Rb, Rc and R1 to R3 can be each independently bonded to an adjacent group to form a direct bond; a substituted or unsubstituted hydrocarbon ring; or a substituted or unsubstituted hetero ring.

In an exemplary embodiment of the present specification, Ra, Rb, Rc and R1 to R3 can be each independently bonded to an adjacent group to form a direct bond; a substituted or unsubstituted hydrocarbon ring; or a substituted or unsubstituted hetero ring.

In an exemplary embodiment of the present specification, Ra, Rb, Rc and R1 to R3 can be each independently bonded to an adjacent group to form a direct bond; a substituted or unsubstituted benzene ring; a substituted or unsubstituted naphthalene ring; a substituted or unsubstituted indene ring; a substituted or unsubstituted benzofuran ring; or a substituted or unsubstituted benzothiophene ring.

In an exemplary embodiment of the present specification, Ra, Rb, Rc and R1 to R3 can be each independently bonded to an adjacent group to form a direct bond or a substituted or unsubstituted benzene ring.

In an exemplary embodiment of the present specification, Ra, Rb, Rc and R1 to R3 can be each independently bonded to an adjacent group to form a substituted or unsubstituted benzene ring.

In an exemplary embodiment of the present specification, R1 can be each independently bonded to an adjacent group to form a substituted or unsubstituted benzene ring.

In an exemplary embodiment of the present specification, R2 can be each independently bonded to an adjacent group to form a substituted or unsubstituted benzene ring.

In an exemplary embodiment of the present specification, R3 can be each independently bonded to an adjacent group to form a substituted or unsubstituted benzene ring.

In an exemplary embodiment of the present specification, Chemical Formula 1 is any one of the following structures.

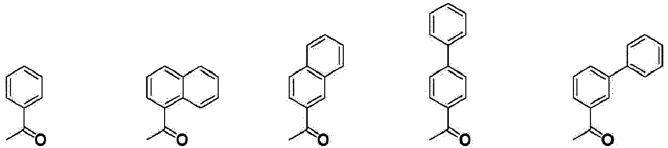

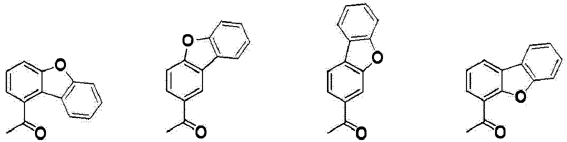

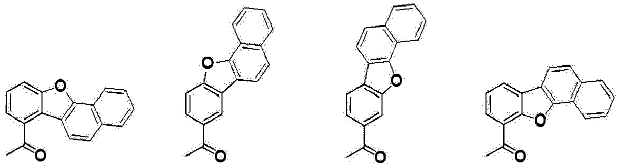

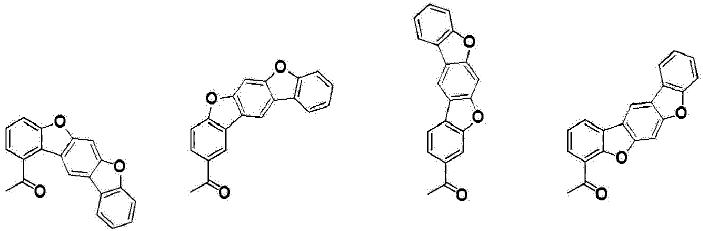

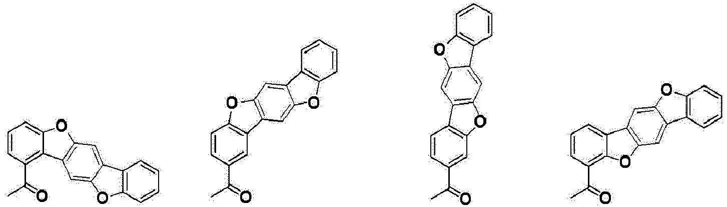

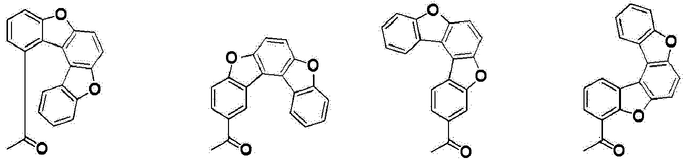

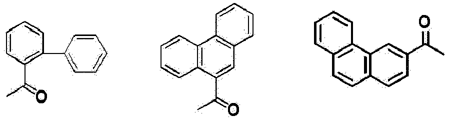

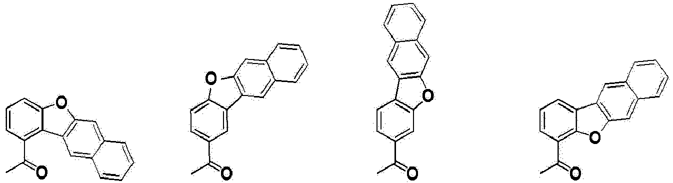

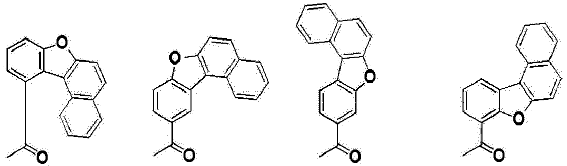

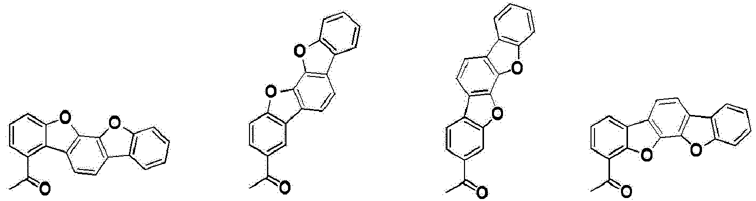

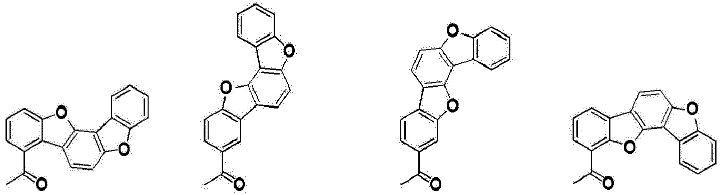

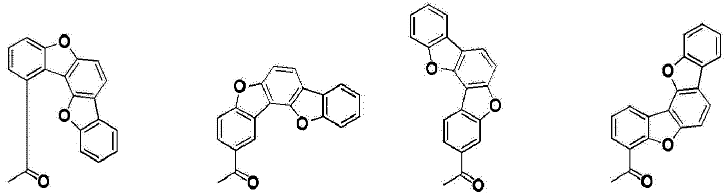

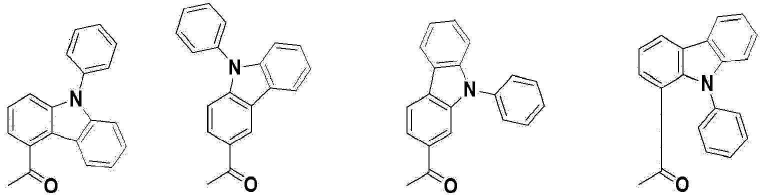

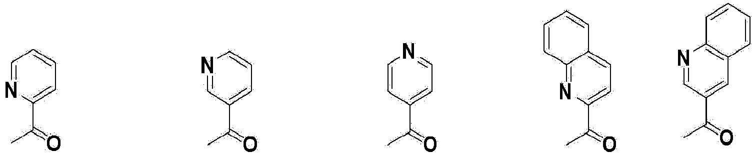

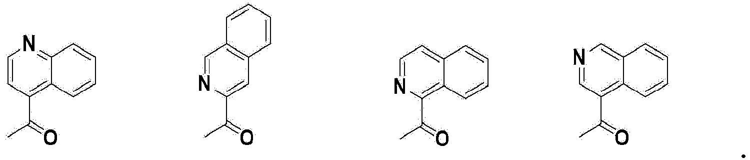

In an exemplary embodiment of the present specification, the synthesizing of the compound of Chemical Formula 2 can synthesize the compound of Chemical Formula 2 using a solution including halogenated benzene-d5, the compound of Chemical Formula 1, 2,2,6,6-tetramethylpiperidine, an alkyllithium and an ether-based solvent.

In an exemplary embodiment of the present specification, the alkyllithium can be, for example, butyllithium.

In an exemplary embodiment of the present specification, the ether-based solvent can be, for example, tetrahydropyran.

In an exemplary embodiment of the present specification, Chemical Formula 2 is any one of the following structures.

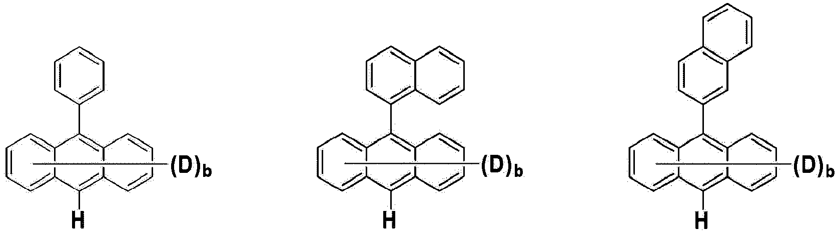

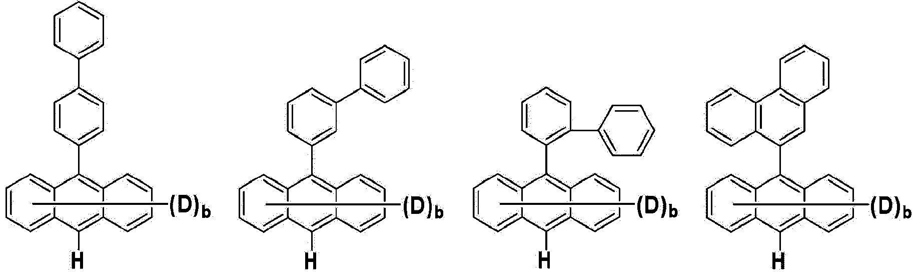

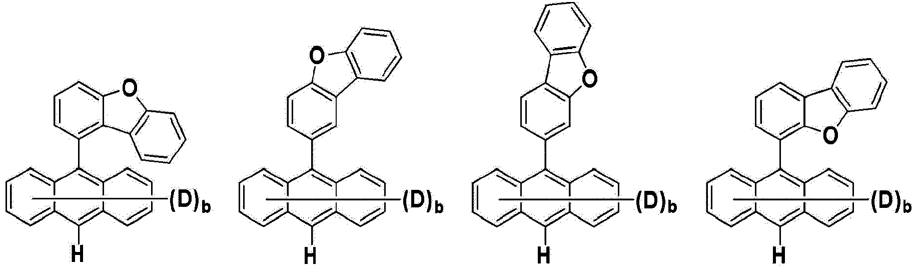

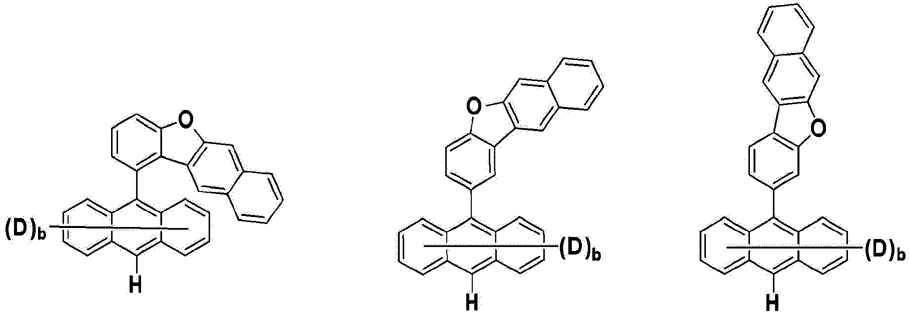

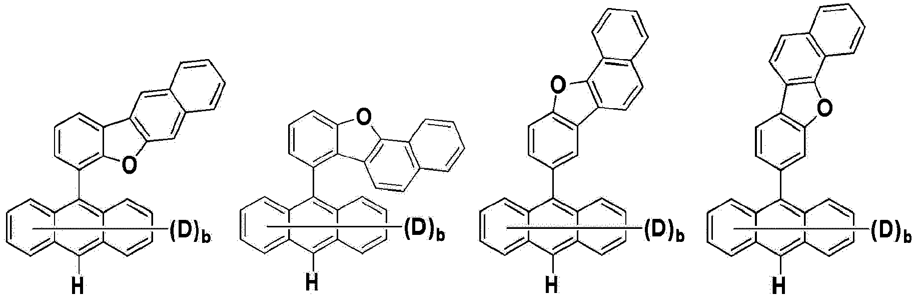

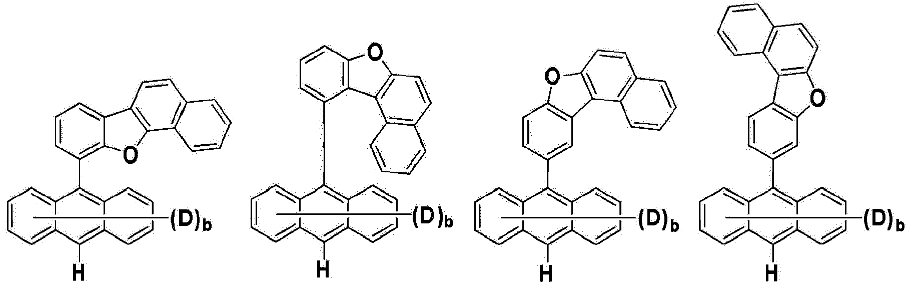

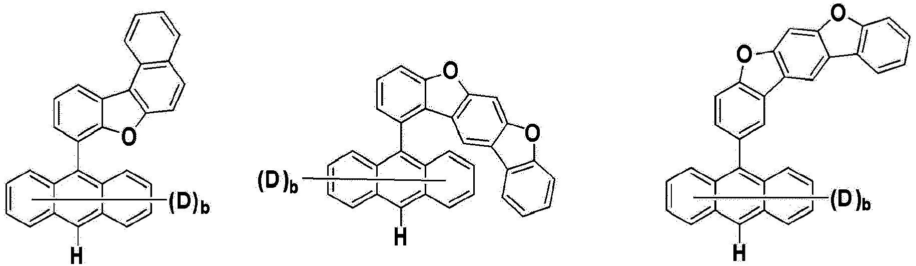

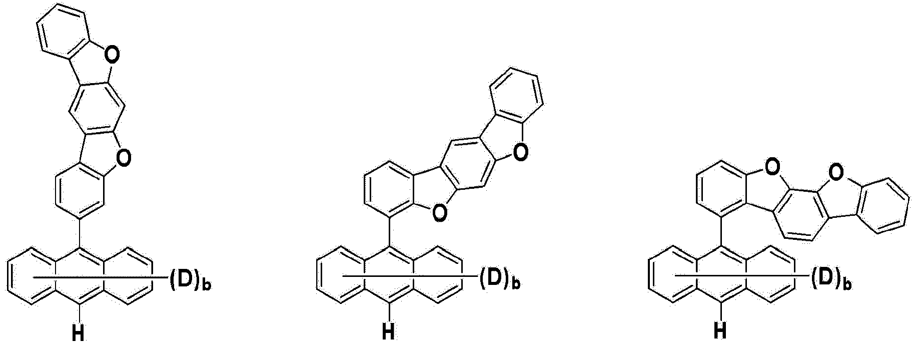

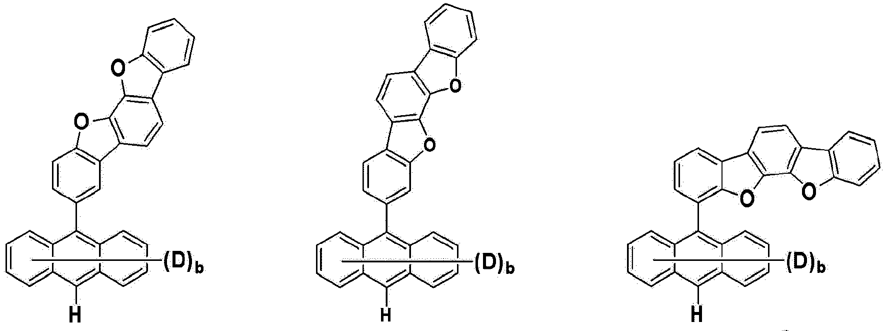

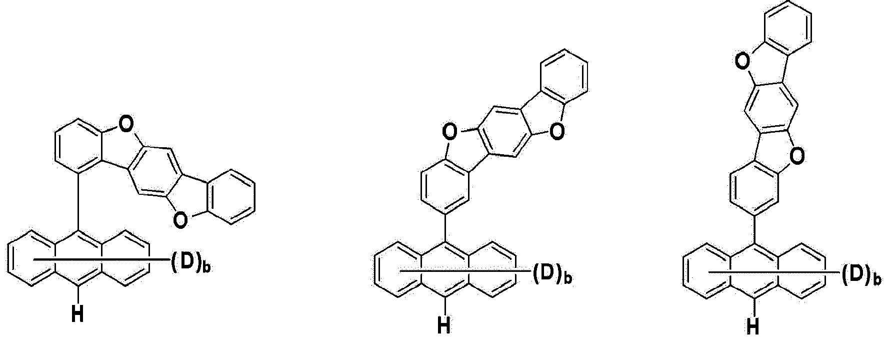

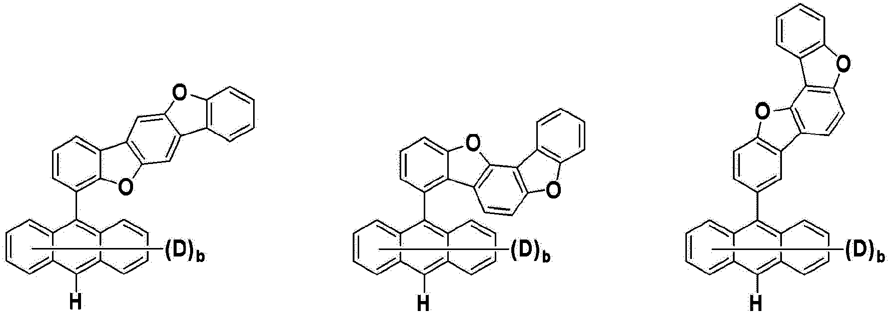

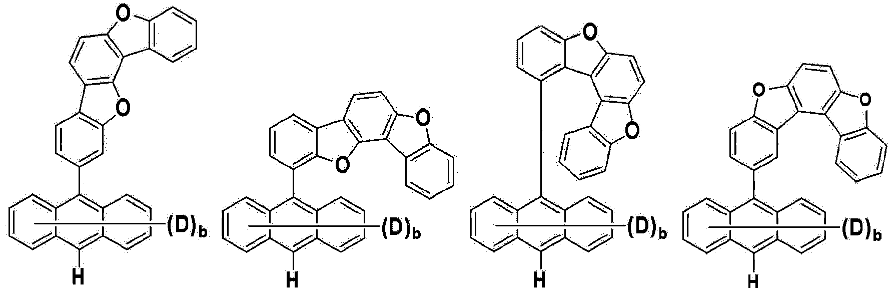

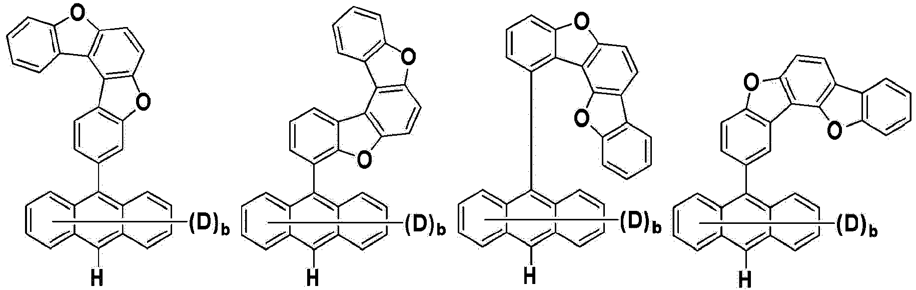

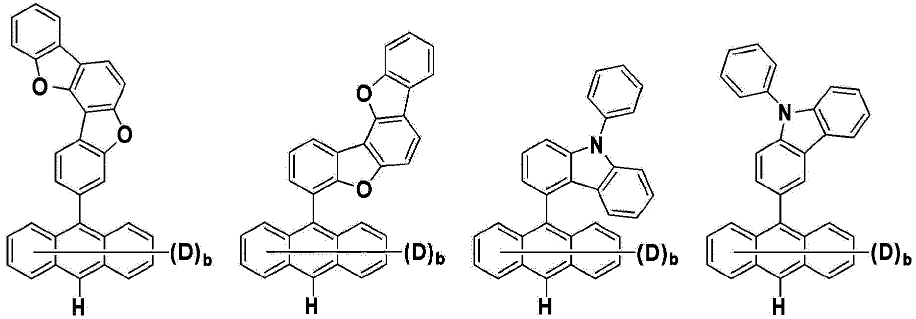

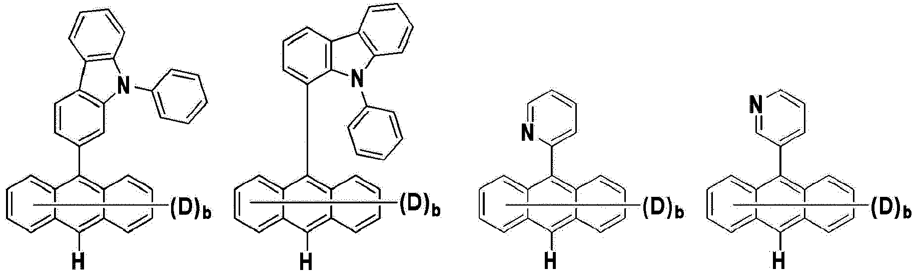

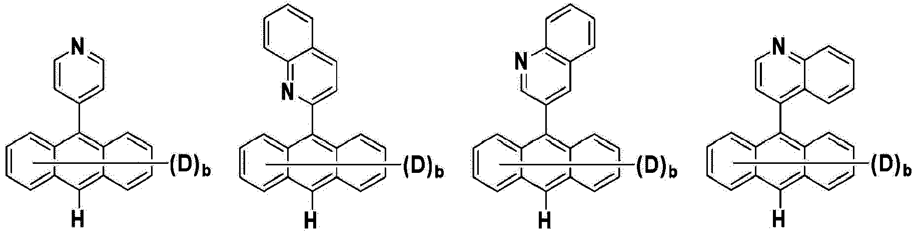

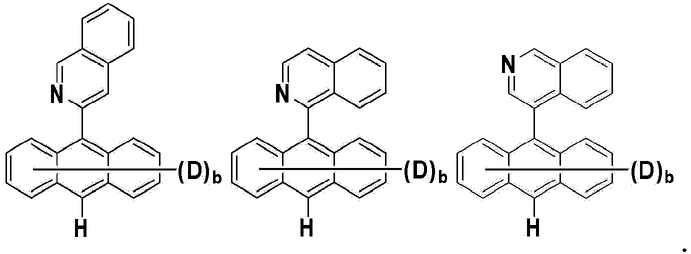

In the structures above, b is the number of deuterium substitutions, and is an integer from 1 to 8. b is determined by the deuterium conversion rate of deuterium substituted according to the synthesis method and conditions, the number of deuteriums substituted with an individual compound, and the substitution position of the deuterium substituted with the individual compound. Even though compounds having the same backbone are prepared, compounds having different numbers and substitution positions of deuteriums can be present in the individual compound.

In an exemplary embodiment of the present specification, the method for preparing a deuterated anthracene compound can include synthesizing a compound of the following Chemical Formula 3 using a solution including a compound in which Ar2 is hydrogen among the compounds of Chemical Formula 2 and a halogen supplying agent.

In an exemplary embodiment of the present specification, the method for preparing a deuterated anthracene compound can include: synthesizing a compound of the following Chemical Formula 3 using a solution including a compound in which Ar2 is hydrogen among the compounds of Chemical Formula 2 and a halogen supplying agent; and synthesizing a compound of the following Chemical Formula 4 by reacting Chemical Formula 3 with L-Ar2.

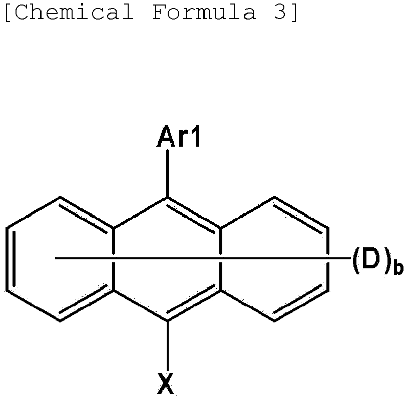

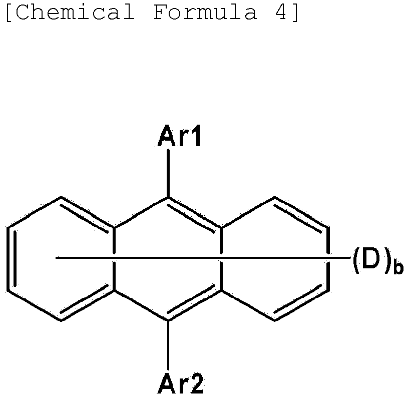

In the structures above, Ar1 and Ar2 are the same as or different from each other, and are each independently a substituted or unsubstituted aryl group; or a substituted or unsubstituted heterocyclic group, X is a halogen group, L is a leaving group, and b is an integer from 1 to 8.

In Chemical Formula 3, b is the number of deuteriums, and can be 1 to 8 depending on the number of deuteriums to be substituted.

In Chemical Formula 3, b is 1.

In Chemical Formula 3, b is 2.

In Chemical Formula 3, b is 3.

In Chemical Formula 3, b is 4.

In Chemical Formula 3, b is 5.

In Chemical Formula 3, b is 6.

In Chemical Formula 3, b is 7.

In Chemical Formula 3, b is 8. When b is 8, Chemical Formula 3 can be the following Chemical Formula 3-1.

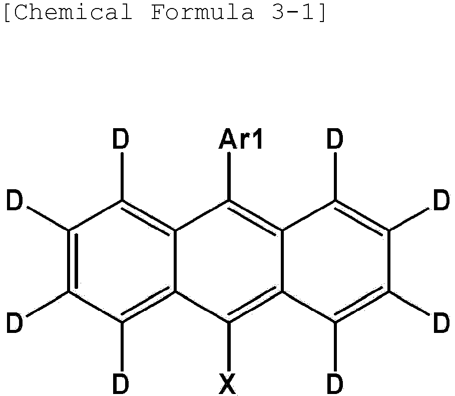

In Chemical Formula 3-1, Ar1 and X are the same as those defined in Chemical Formula 3.

In Chemical Formula 4, b is the number of deuteriums, and can be 1 to 8 depending on the number of deuteriums to be substituted.

In Chemical Formula 4, b is 1.

In Chemical Formula 4, b is 2.

In Chemical Formula 4, b is 3.

In Chemical Formula 4, b is 4.

In Chemical Formula 4, b is 5.

In Chemical Formula 4, b is 6.

In Chemical Formula 4, b is 7.

In Chemical Formula 4, b is 8. When b is 8, Chemical Formula 4 can be the following Chemical Formula 4-1.

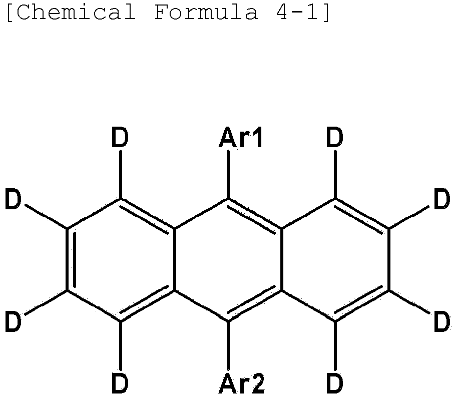

In Chemical Formula 4-1, Ar1 and Ar2 are the same as those defined in Chemical Formula 4.

In an exemplary embodiment of the present specification, X is fluorine (-F), chlorine (-Cl), bromine (-Br) or iodine (-I).

In an exemplary embodiment of the present specification, X is chlorine (-Cl), bromine (-Br) or iodine (-I).

In an exemplary embodiment of the present specification, X is bromine (-Br).

In an exemplary embodiment of the present specification, Ar2 of Chemical Formula 4 is a substituted or unsubstituted aryl group having 6 to 60 carbon atoms; or a substituted or unsubstituted heterocyclic group having 2 to 60 carbon atoms.

In an exemplary embodiment of the present specification, Ar2 of Chemical Formula 4 is a substituted or unsubstituted aryl group having 6 to 30 carbon atoms; or a substituted or unsubstituted heterocyclic group having 2 to 30 carbon atoms.

In an exemplary embodiment of the present specification, Ar2 of Chemical Formula 4 is a substituted or unsubstituted aryl group having 6 to 20 carbon atoms; or a substituted or unsubstituted heterocyclic group having 2 to 20 carbon atoms.

In an exemplary embodiment of the present specification, Ar2 of Chemical Formula 4 is a substituted or unsubstituted phenyl group, a substituted or unsubstituted biphenyl group, a substituted or unsubstituted terphenyl group, a substituted or unsubstituted naphthyl group, a substituted or unsubstituted phenanthrenyl group, a substituted or unsubstituted triphenylenyl group, a substituted or unsubstituted pyrenyl group, a substituted or unsubstituted fluorenyl group, a substituted or unsubstituted spirobifluorenyl group, a substituted or unsubstituted xanthenyl group, a substituted or unsubstituted thioxanthenyl group, a substituted or unsubstituted benzofuranyl group, a substituted or unsubstituted benzothiophenyl group, a substituted or unsubstituted dibenzofuranyl group, a substituted or unsubstituted dibenzothiophenyl group, a substituted or unsubstituted carbazolyl group, a substituted or unsubstituted benzonaphthofuranyl group, a substituted or unsubstituted benzonaphthothiophenyl group, a substituted or unsubstituted benzocarbazolyl group, or a substituted or unsubstituted dibenzocarbazolyl group.

In an exemplary embodiment of the present specification, Ar2 of Chemical Formula 4 is a substituted or unsubstituted phenyl group, a substituted or unsubstituted naphthyl group, a substituted or unsubstituted phenanthrenyl group, a substituted or unsubstituted triphenylenyl group, a substituted or unsubstituted pyrenyl group, a substituted or unsubstituted fluorenyl group, a substituted or unsubstituted dibenzofuranyl group, a substituted or unsubstituted dibenzothiophenyl group, or a substituted or unsubstituted carbazolyl group.

In an exemplary embodiment of the present specification, Ar2 of Chemical Formula 4 is a substituted or unsubstituted phenyl group, a substituted or unsubstituted naphthyl group, a substituted or unsubstituted dibenzofuranyl group, a substituted or unsubstituted dibenzothiophenyl group, or a substituted or unsubstituted carbazolyl group.

In an exemplary embodiment of the present specification, Ar2 of Chemical Formula 4 is a substituted or unsubstituted phenyl group, a substituted or unsubstituted naphthyl group, a substituted or unsubstituted dibenzofuranyl group, or a substituted or unsubstituted carbazolyl group.

In an exemplary embodiment of the present specification, Ar2 of Chemical Formula 4 is a substituted or unsubstituted phenyl group, a substituted or unsubstituted naphthyl group, or a substituted or unsubstituted dibenzofuranyl group.

In an exemplary embodiment of the present specification, L is -B(OH)₂.

In an exemplary embodiment of the present specification, L-Ar2 is any one of the following structures.

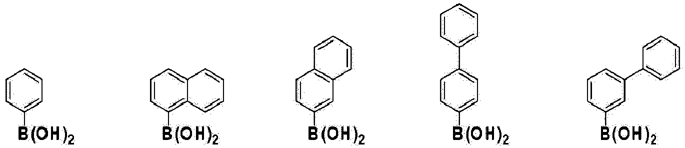

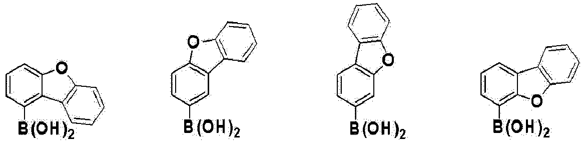

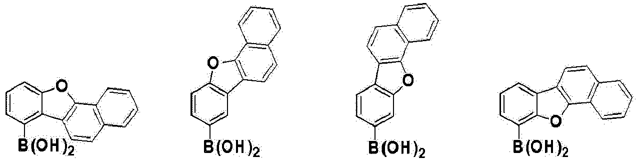

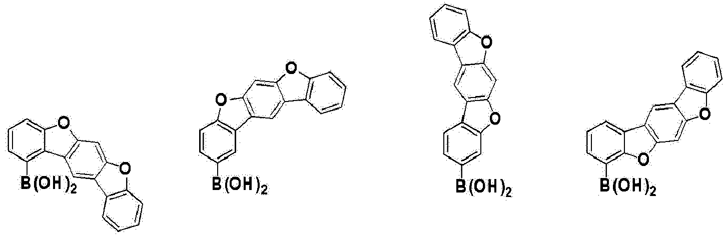

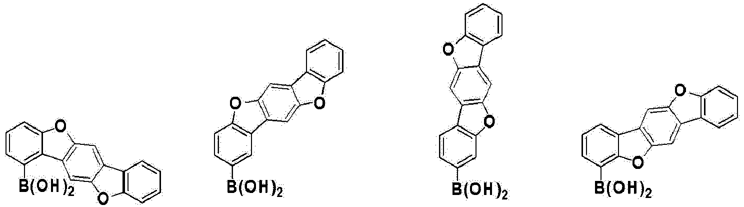

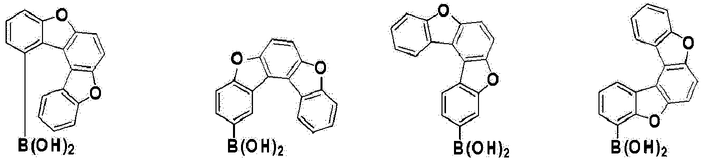

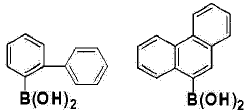

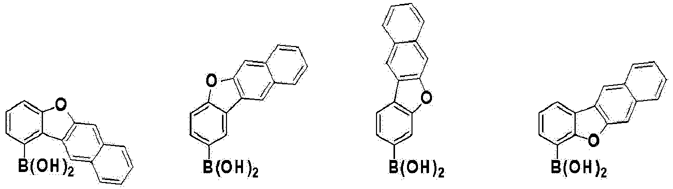

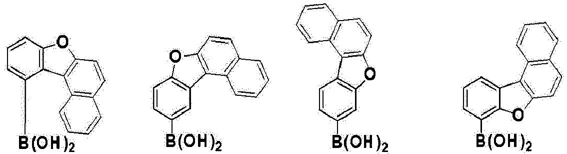

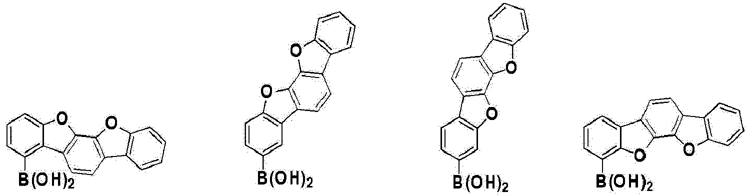

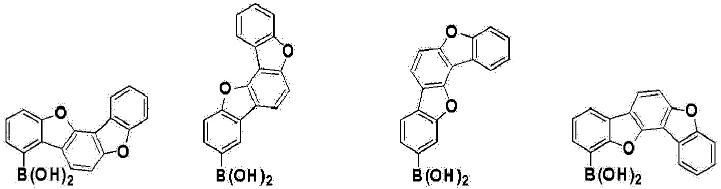

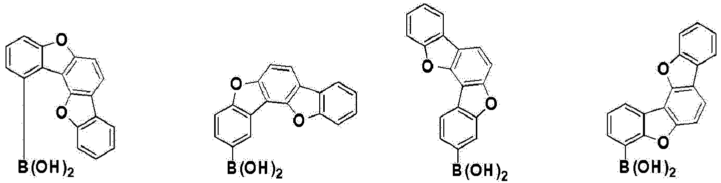

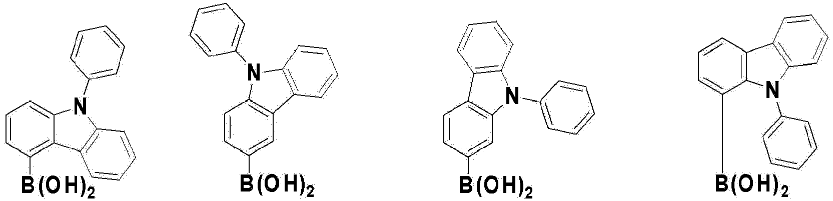

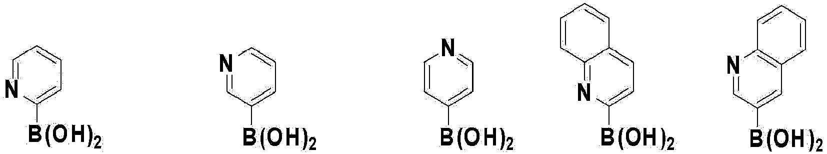

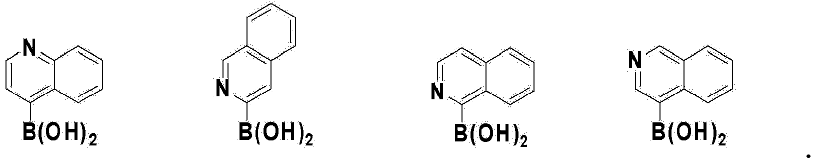

In an exemplary embodiment of the present specification, Chemical Formula 3 is any one of the following structures.

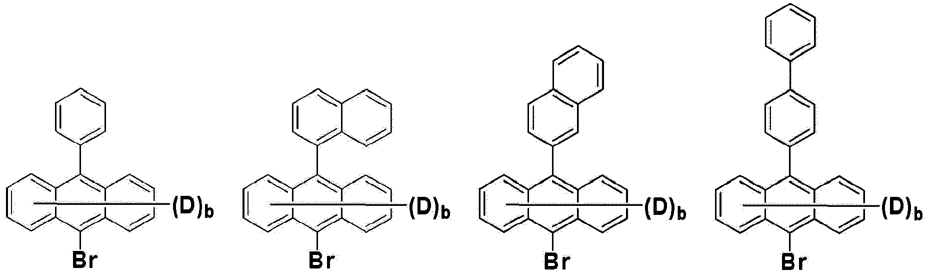

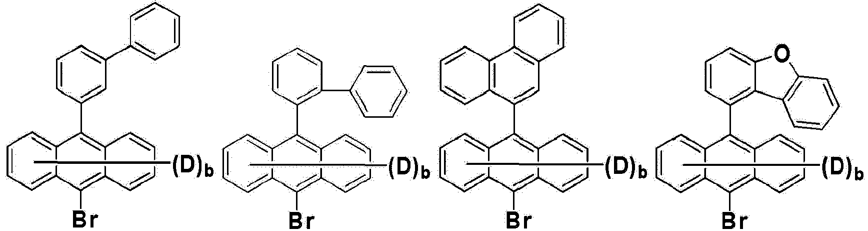

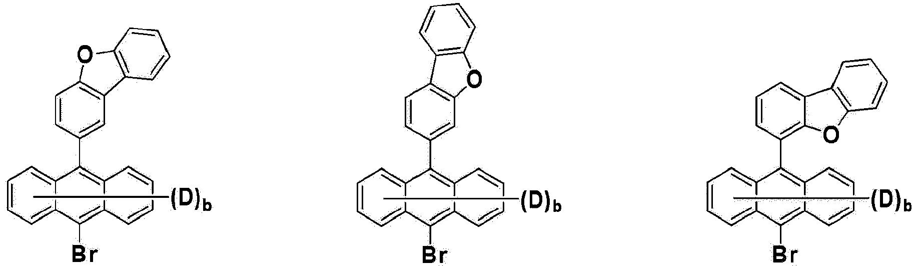

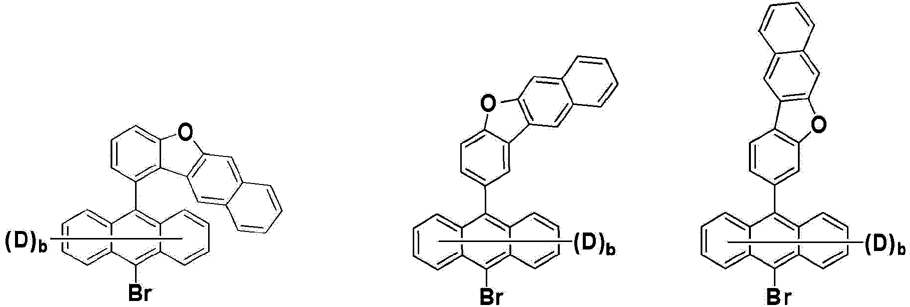

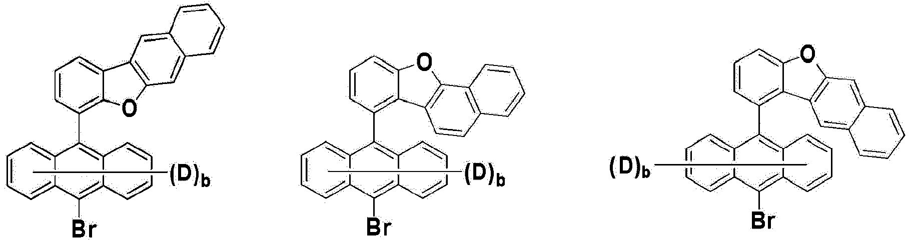

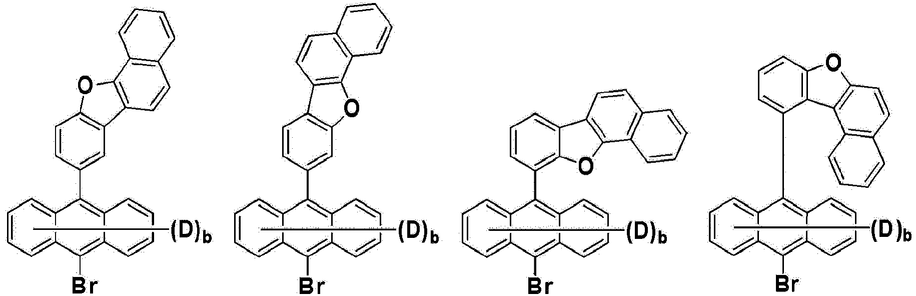

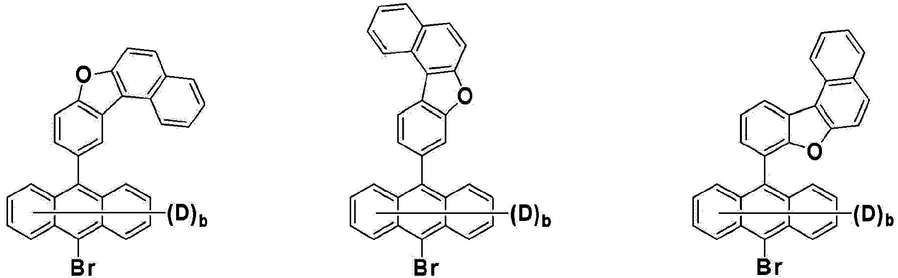

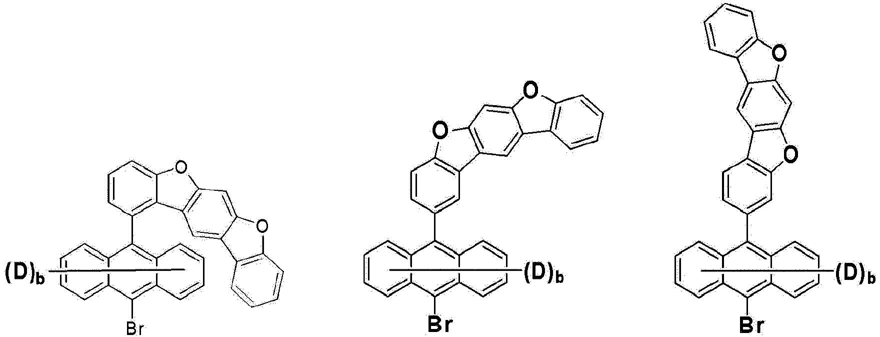

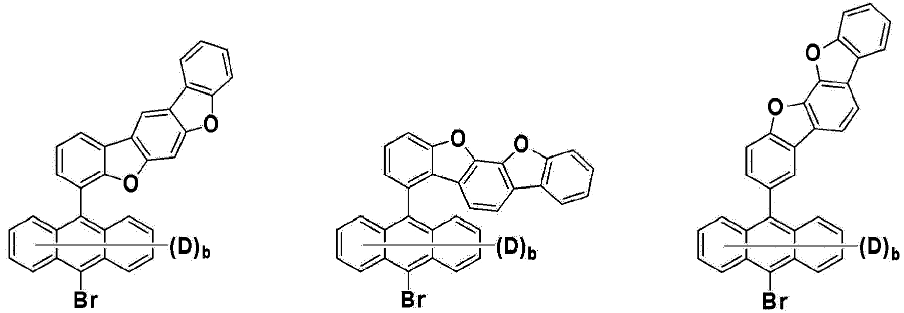

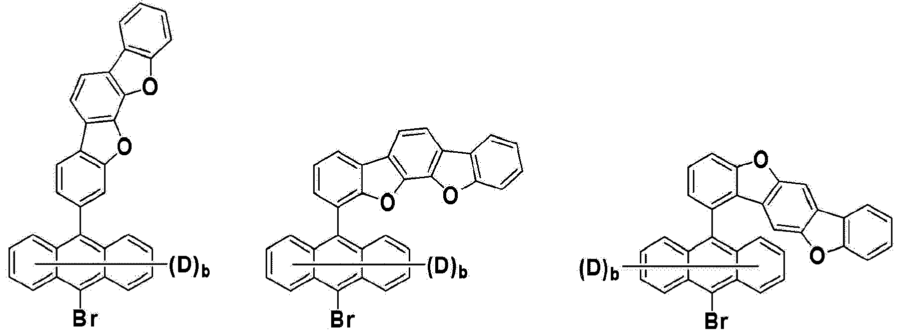

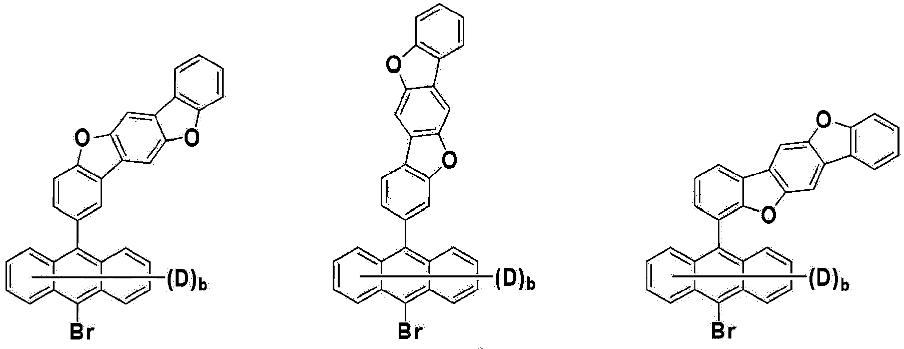

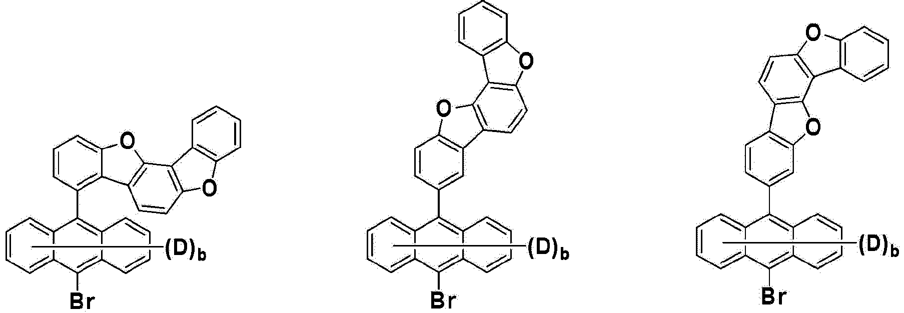

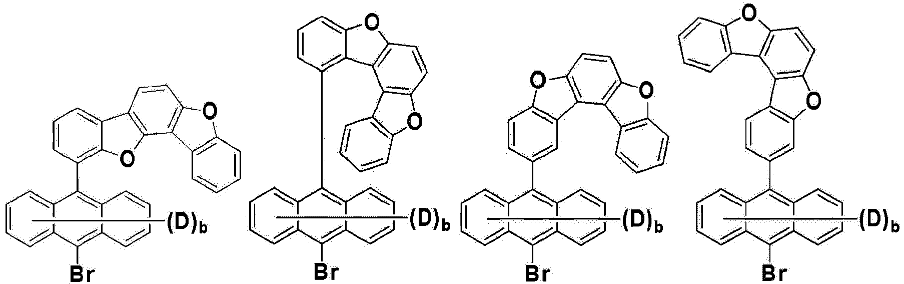

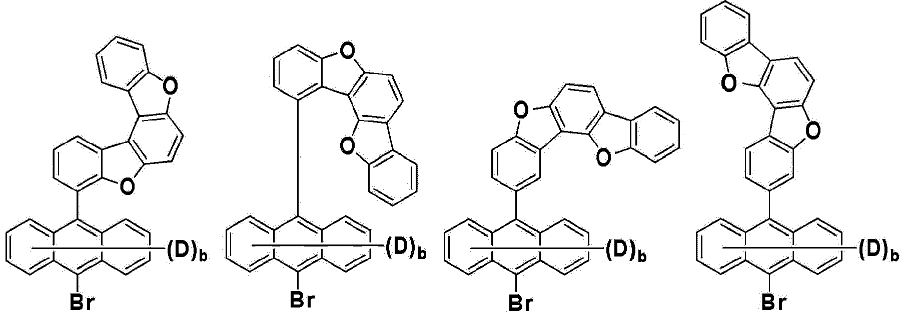

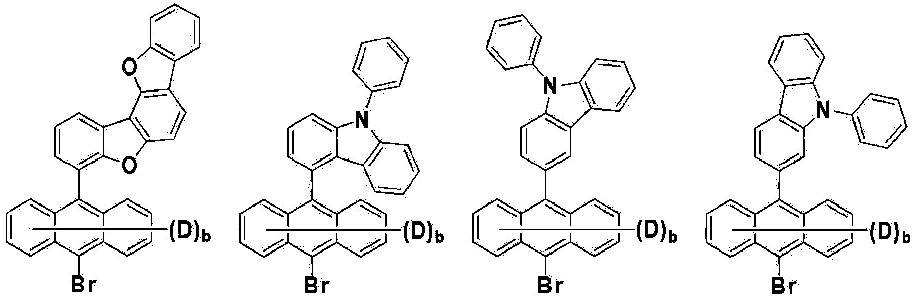

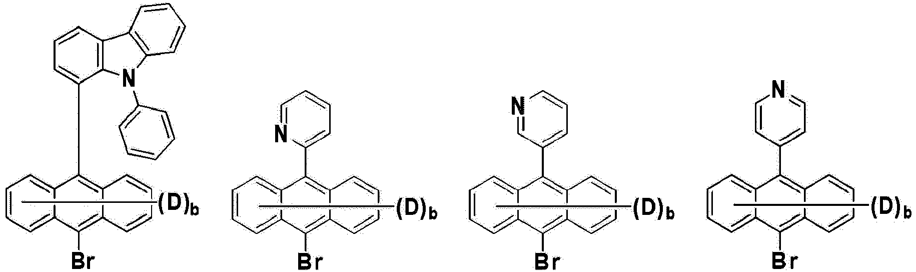

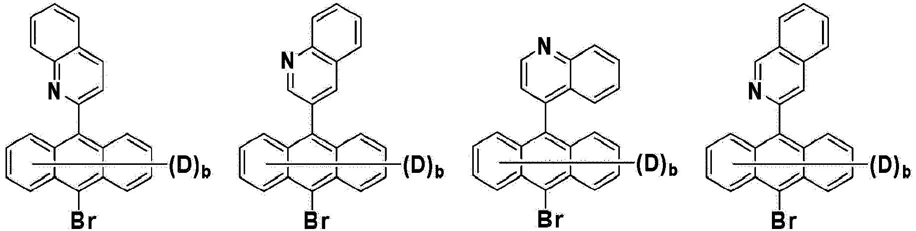

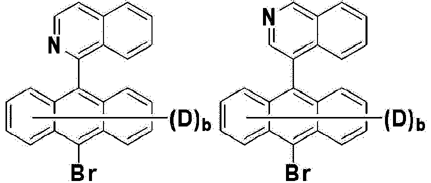

In the structures above, b is the number of deuterium substitutions, and is an integer from 1 to 8. b is determined by the deuterium conversion rate of deuterium substituted according to the synthesis method and conditions, the number of deuteriums substituted with an individual compound, and the substitution position of the deuterium substituted with the individual compound. Even though compounds having the same backbone are prepared, compounds having different numbers and substitution positions of deuteriums can be present in the individual compound.

In an exemplary embodiment of the present specification, Chemical Formula 4 is any one of the following structures.

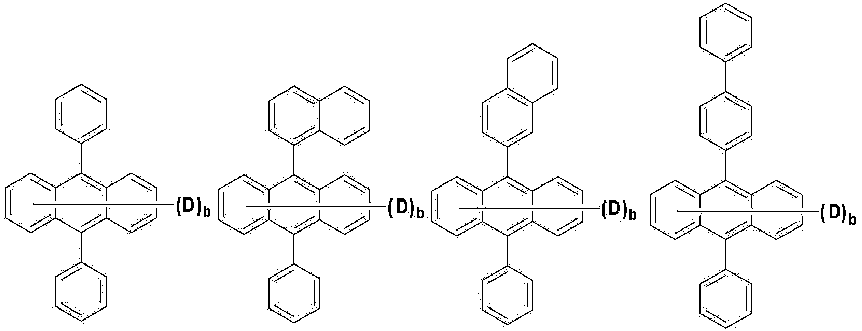

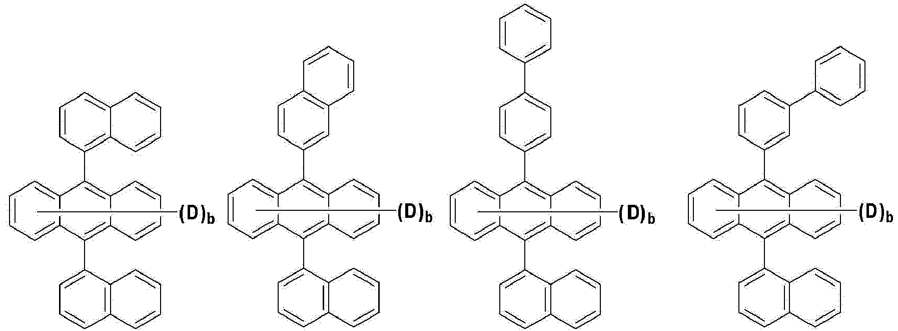

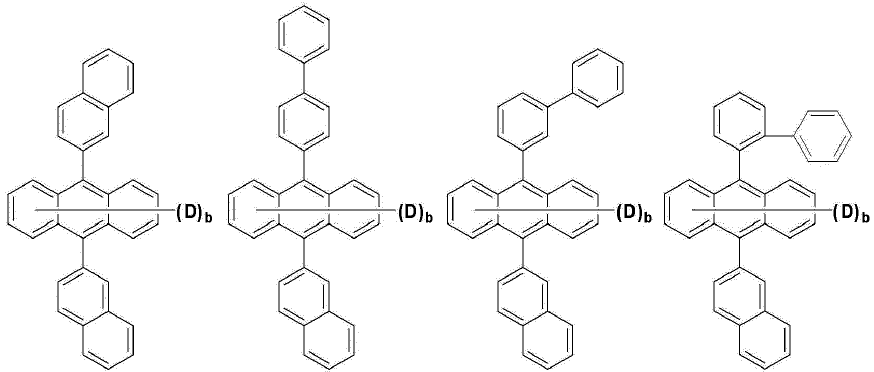

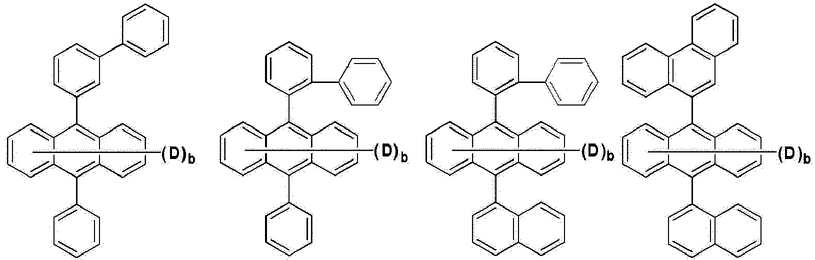

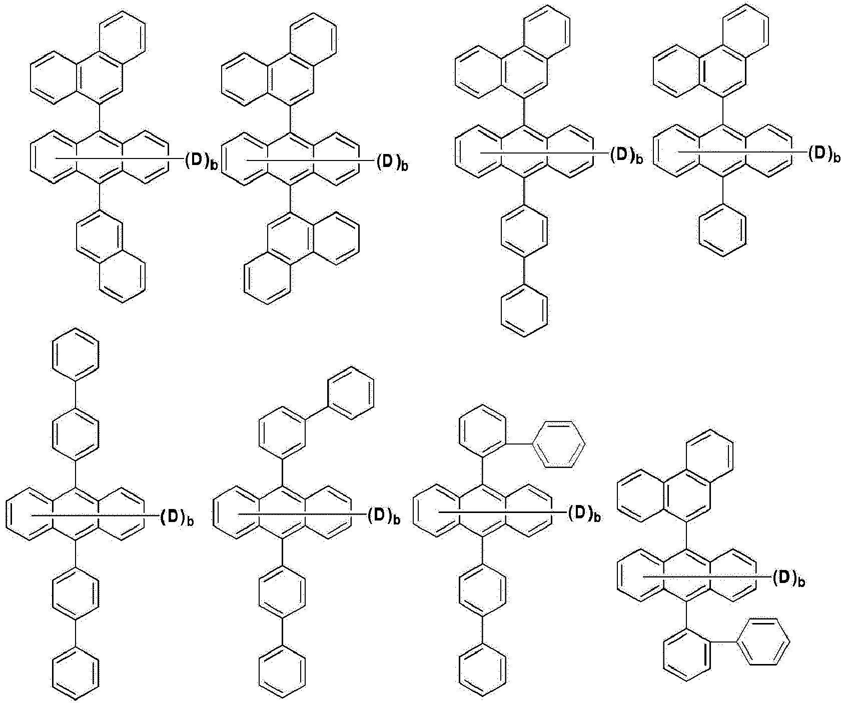

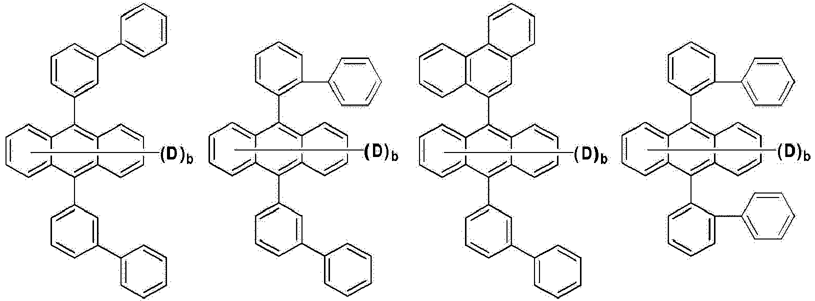

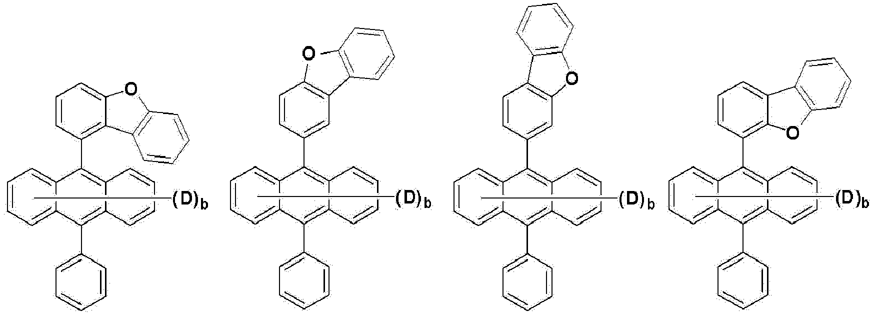

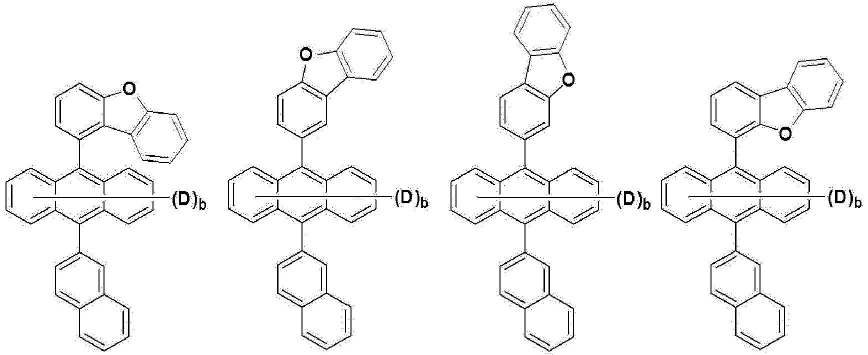

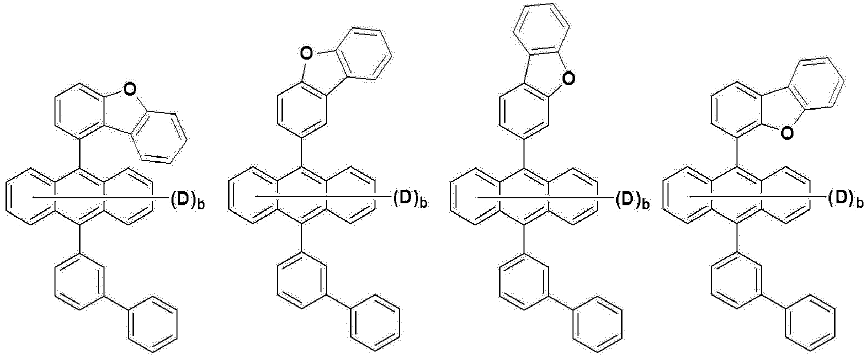

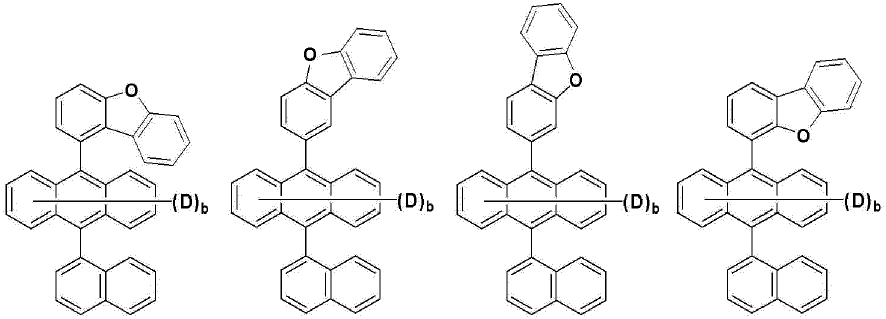

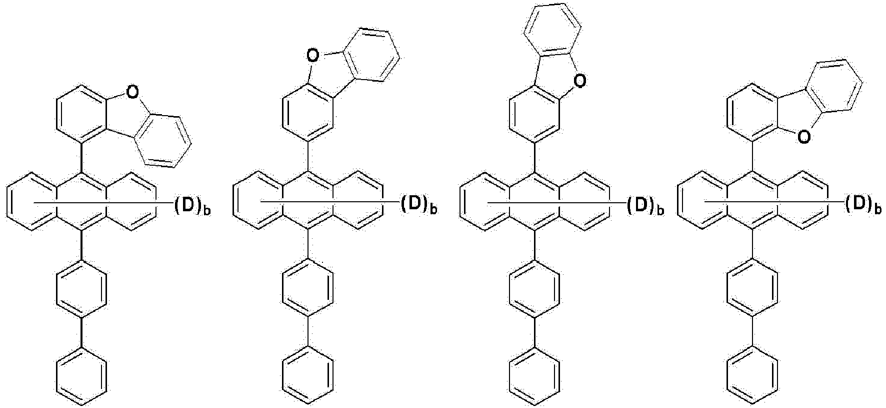

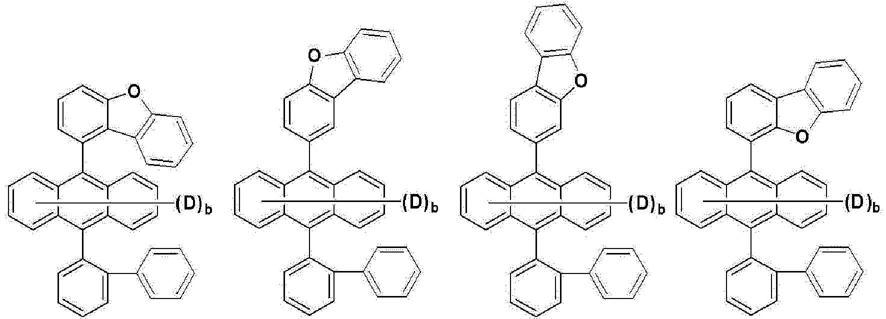

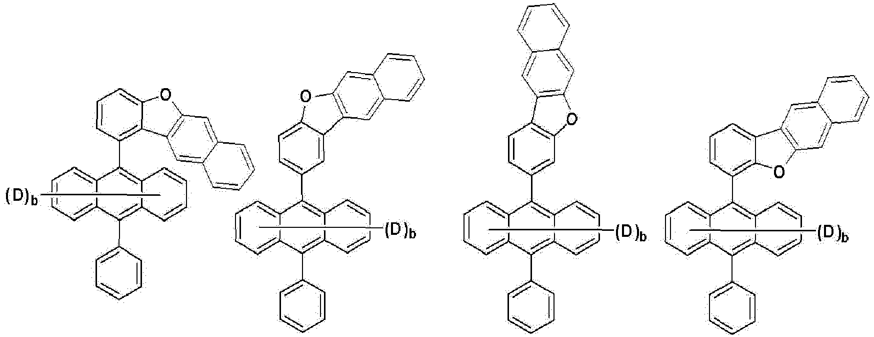

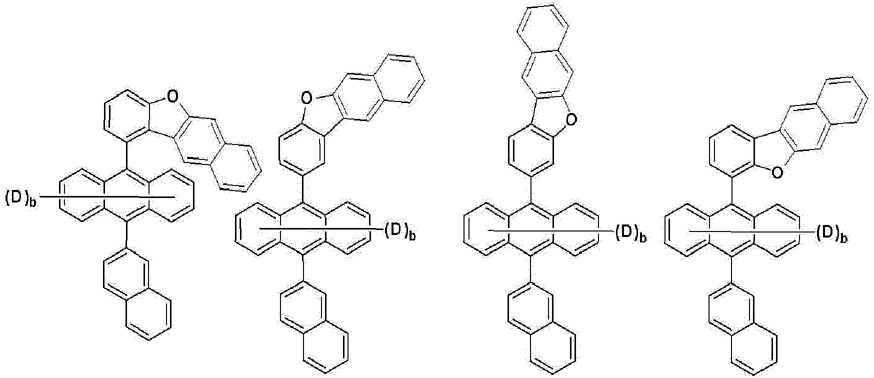

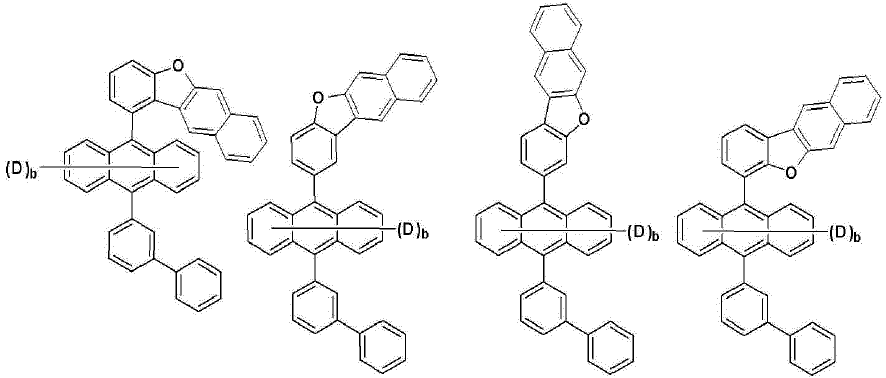

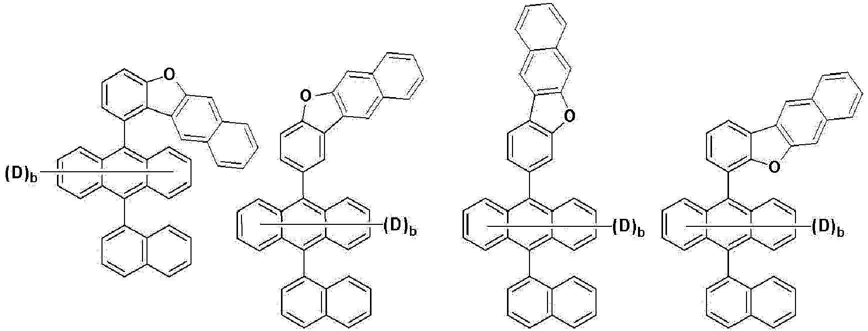

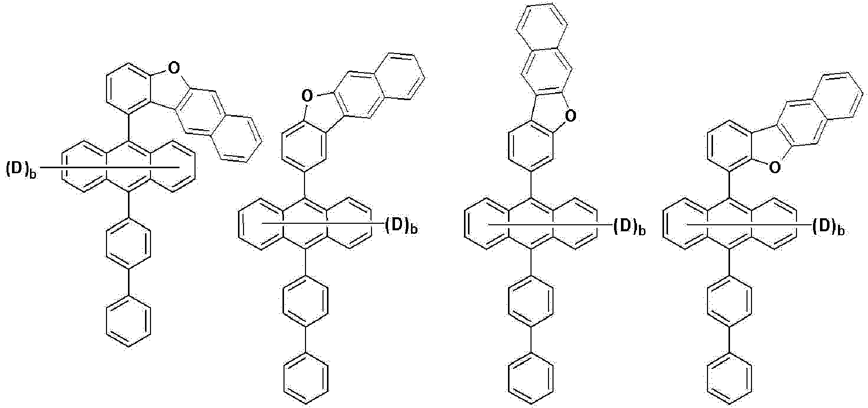

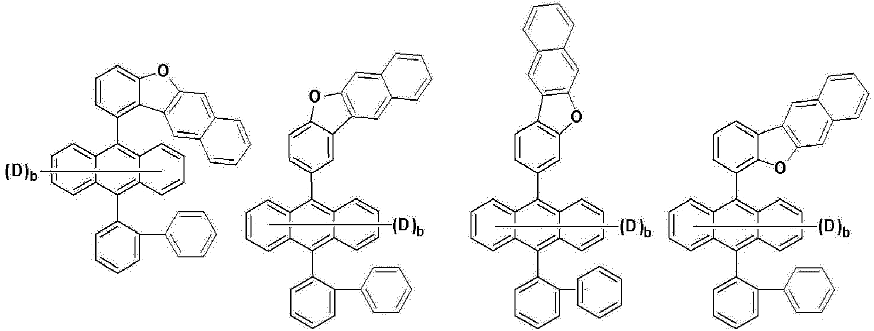

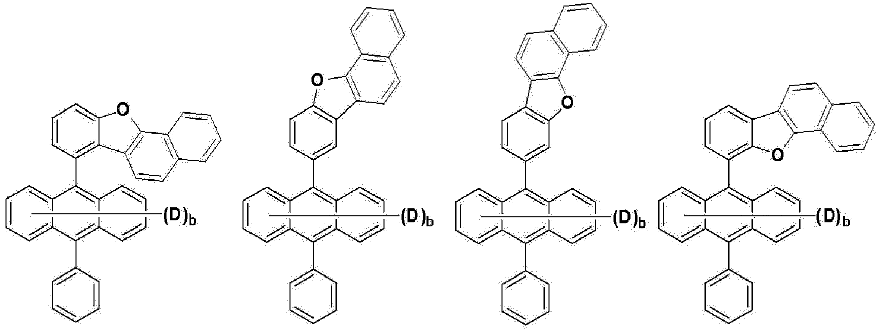

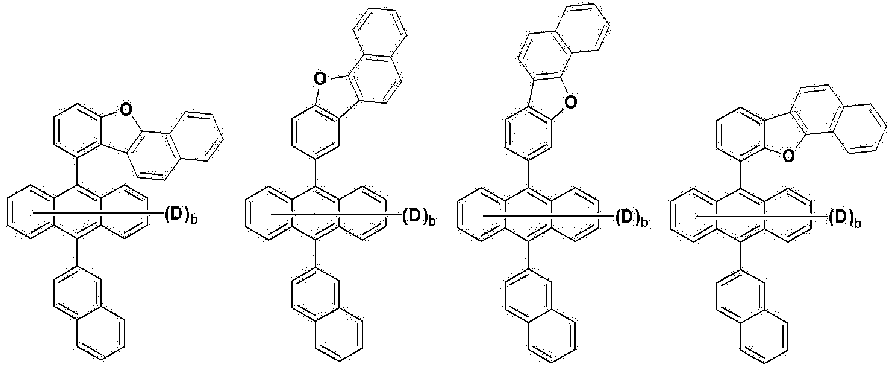

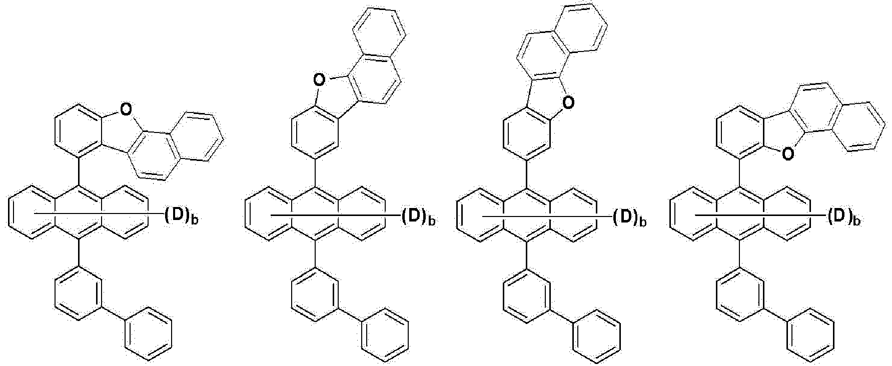

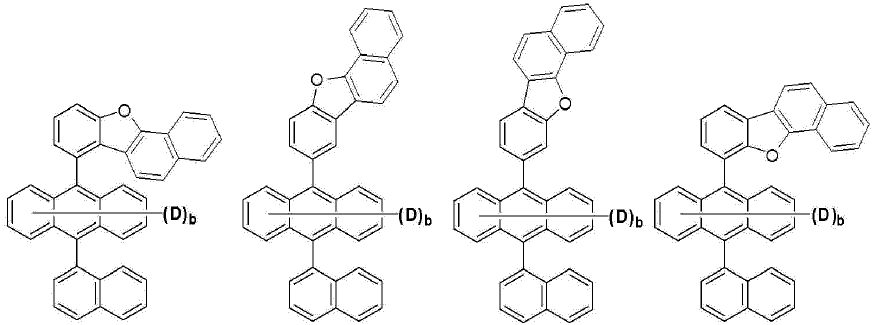

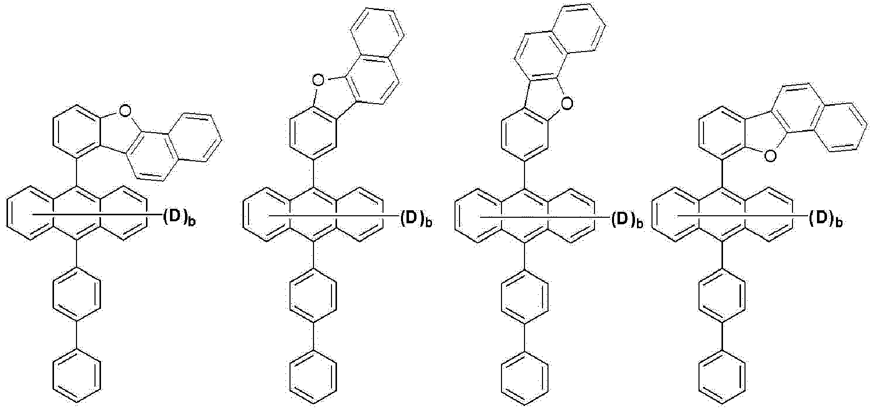

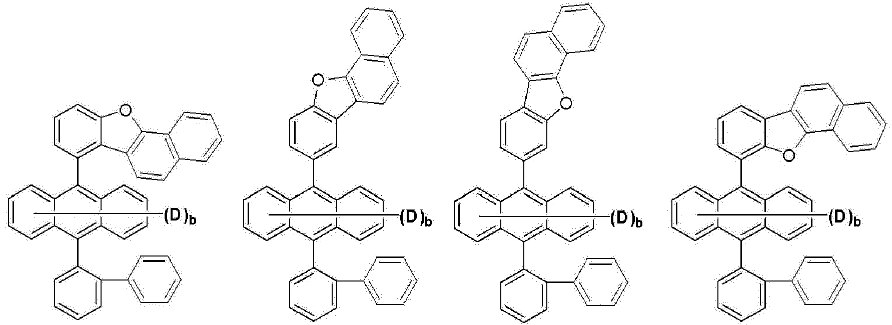

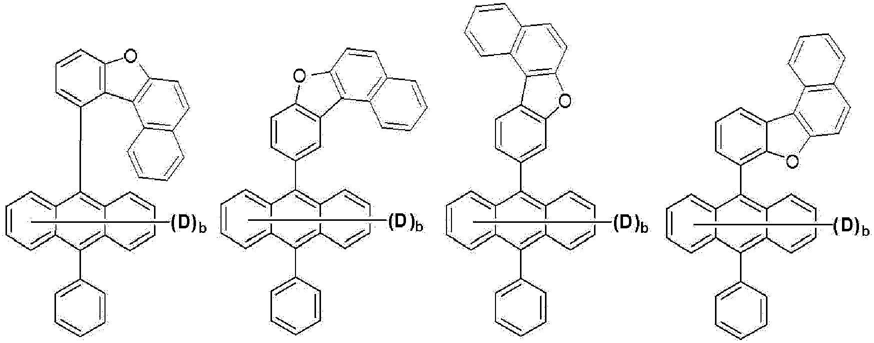

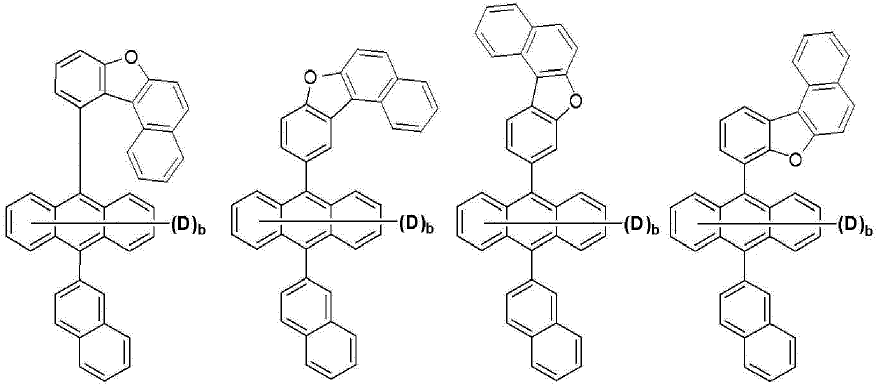

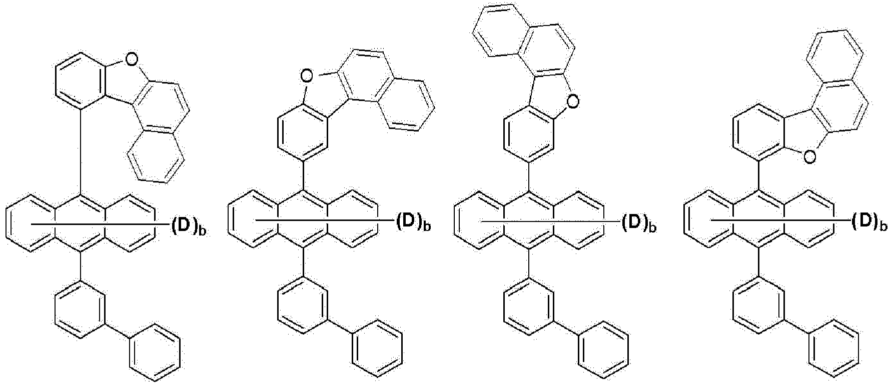

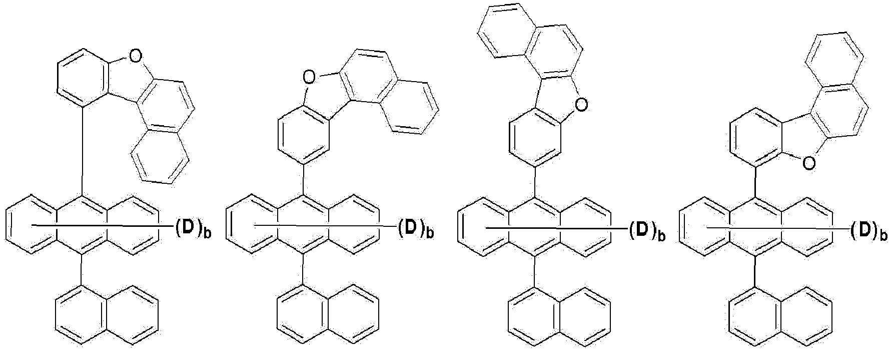

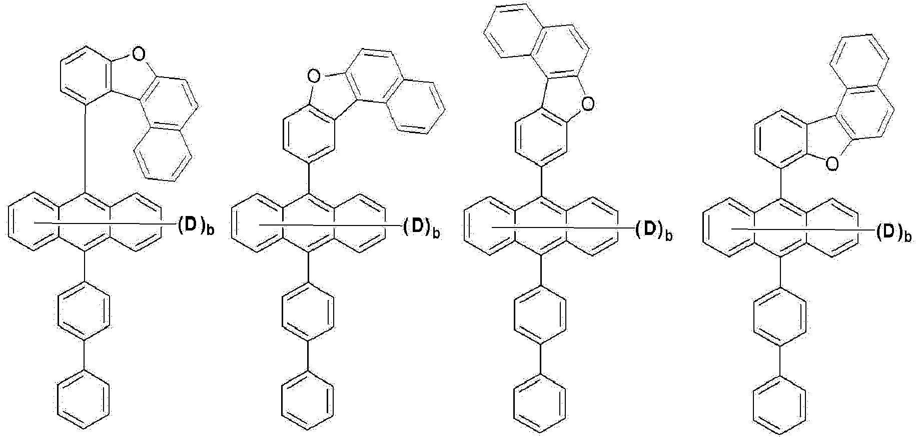

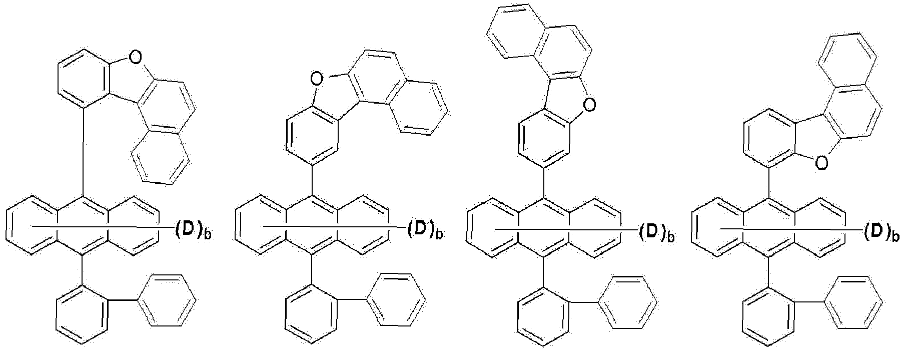

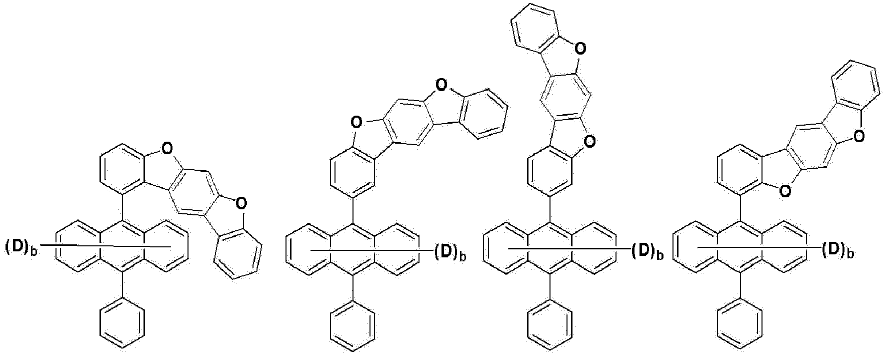

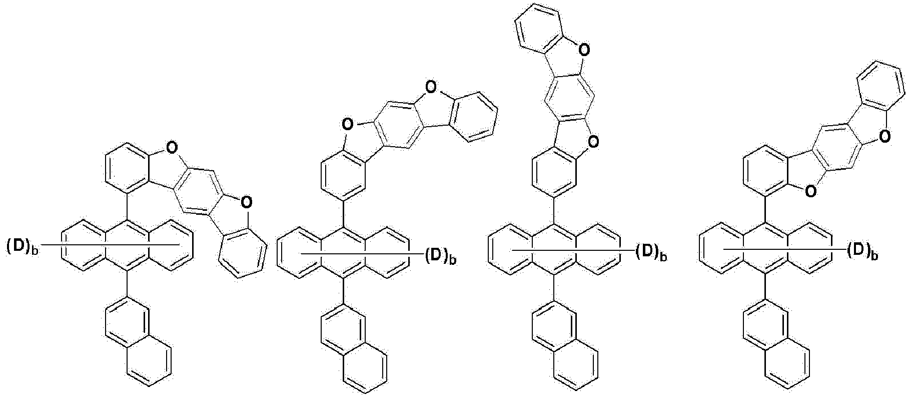

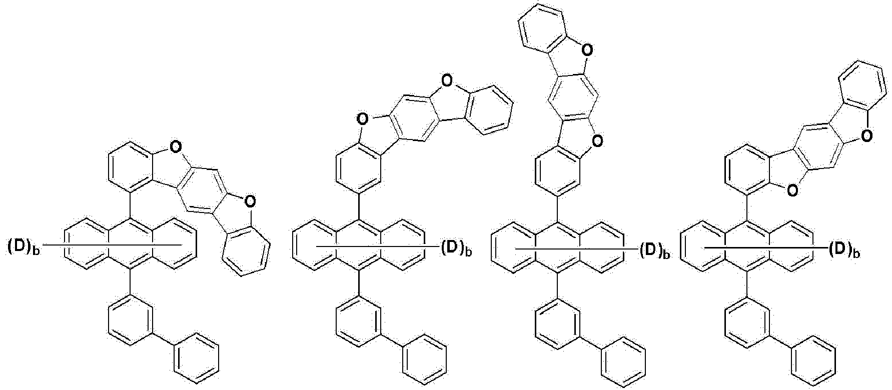

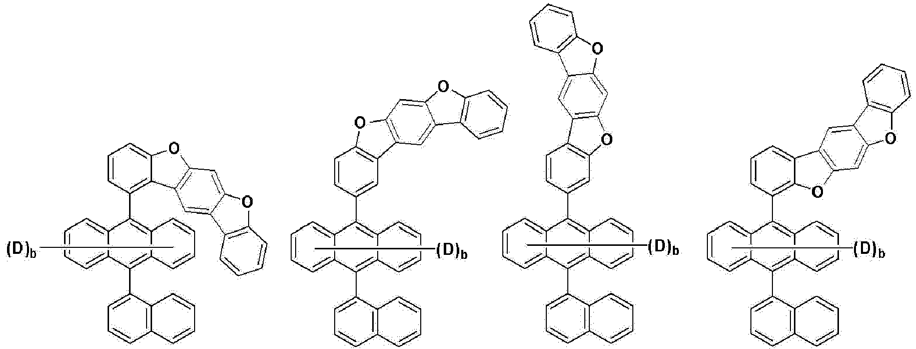

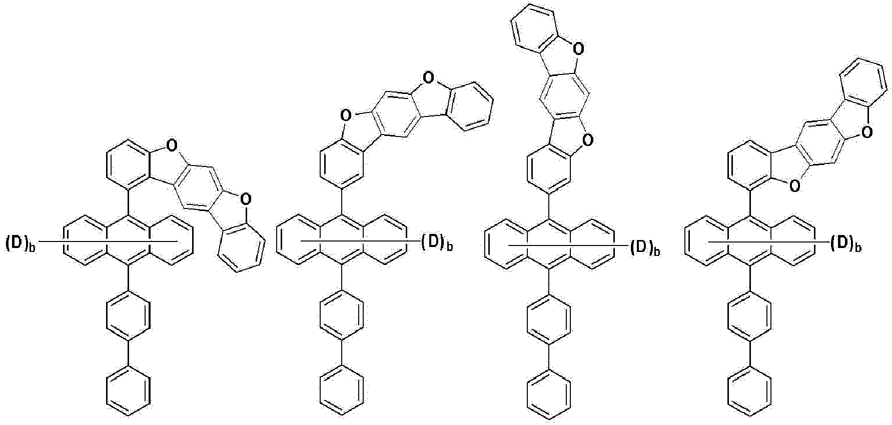

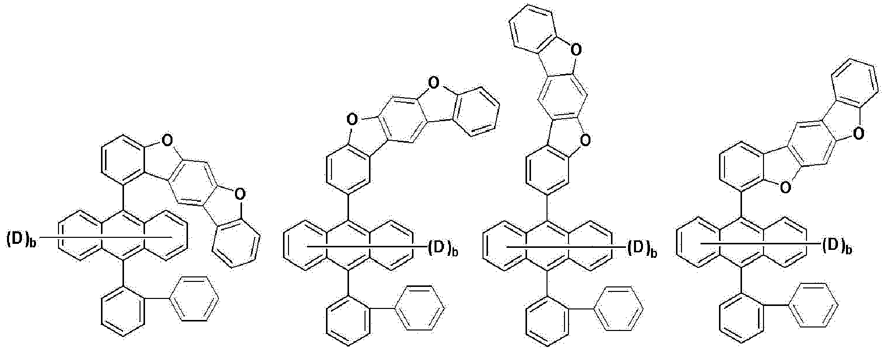

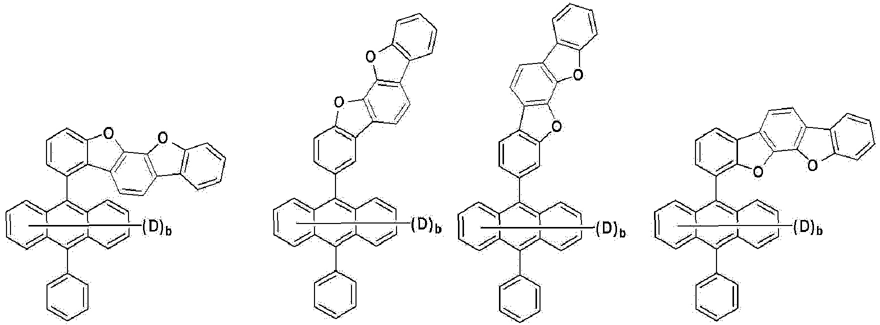

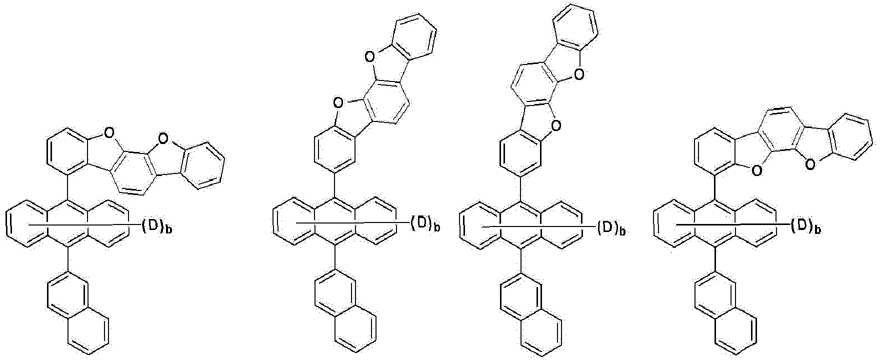

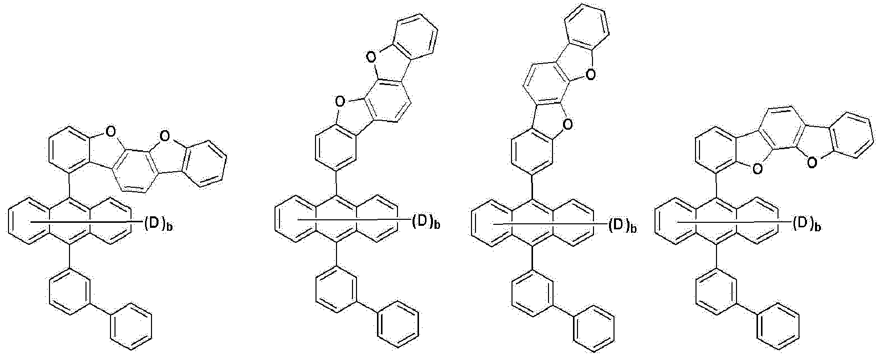

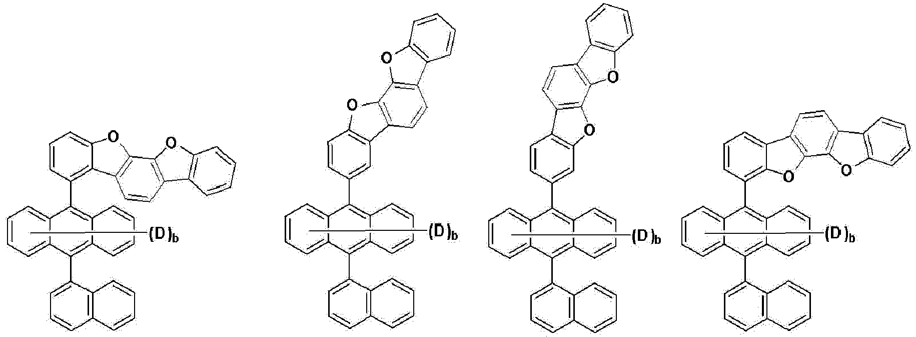

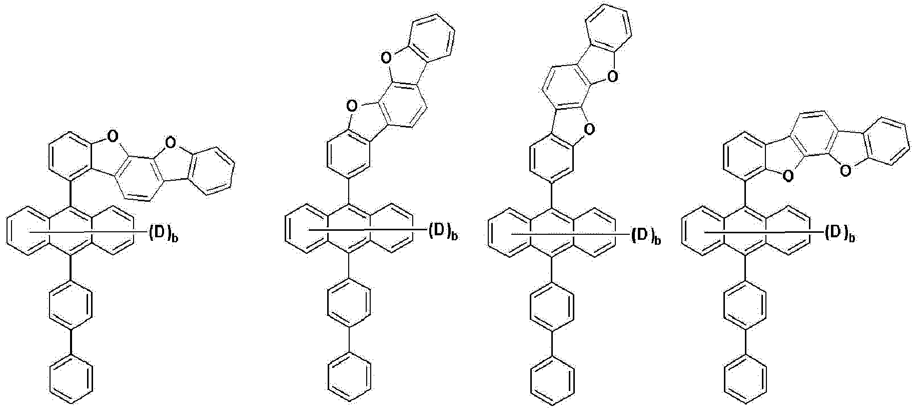

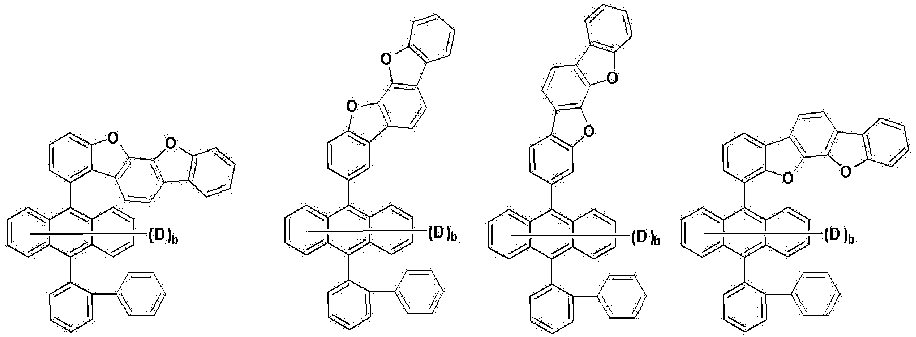

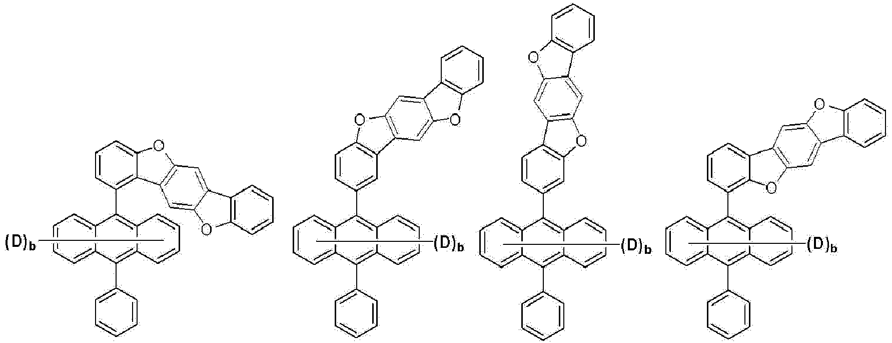

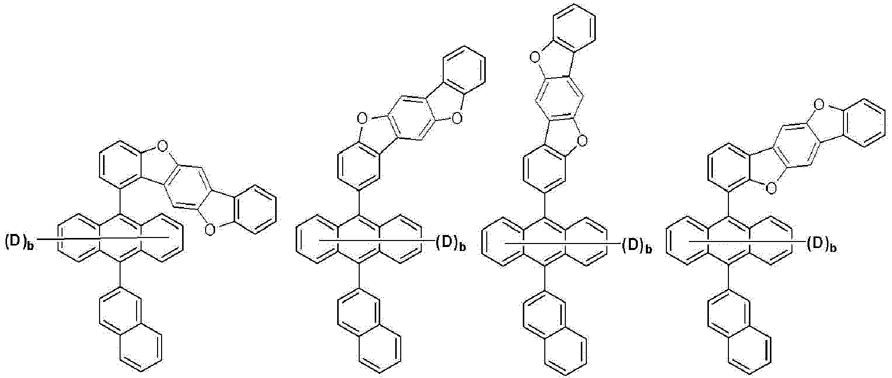

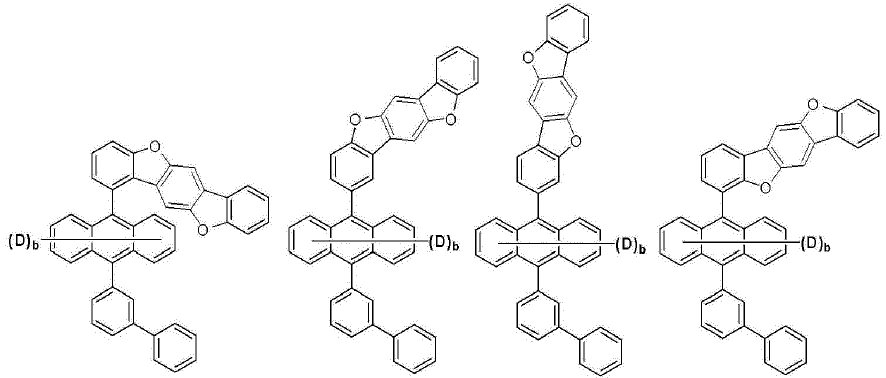

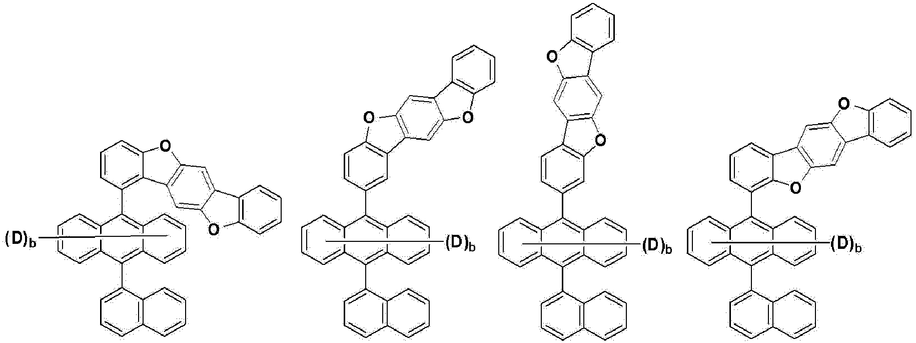

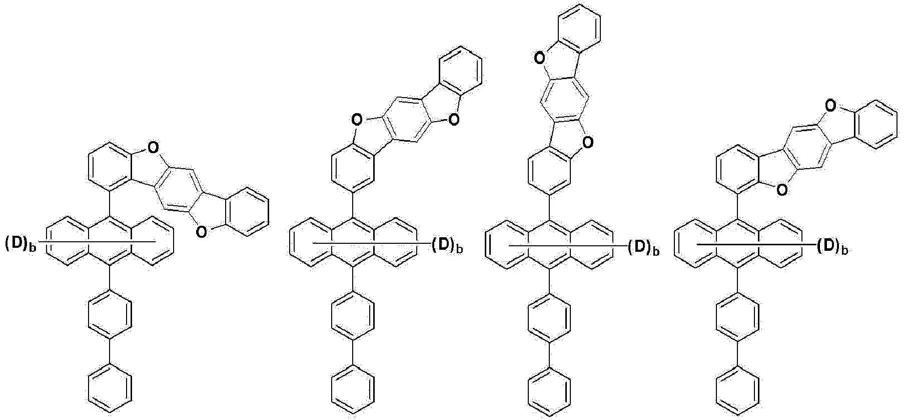

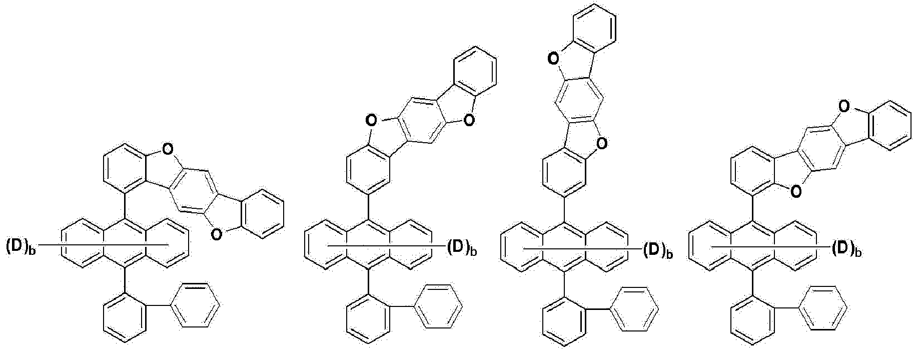

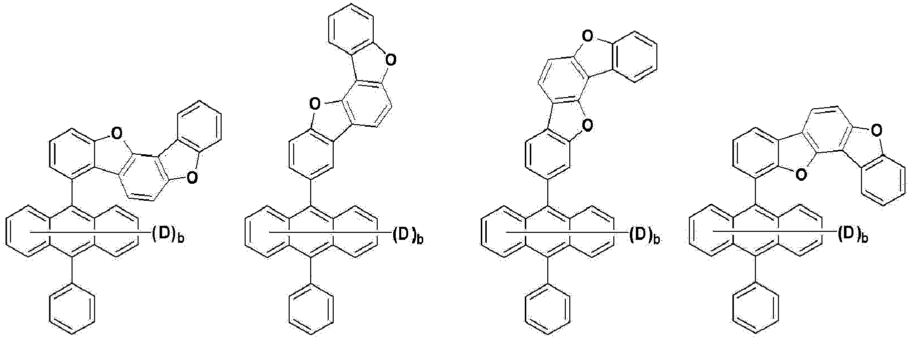

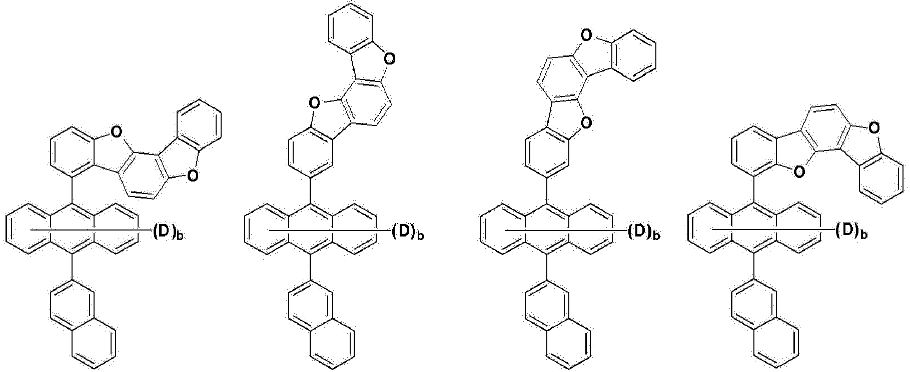

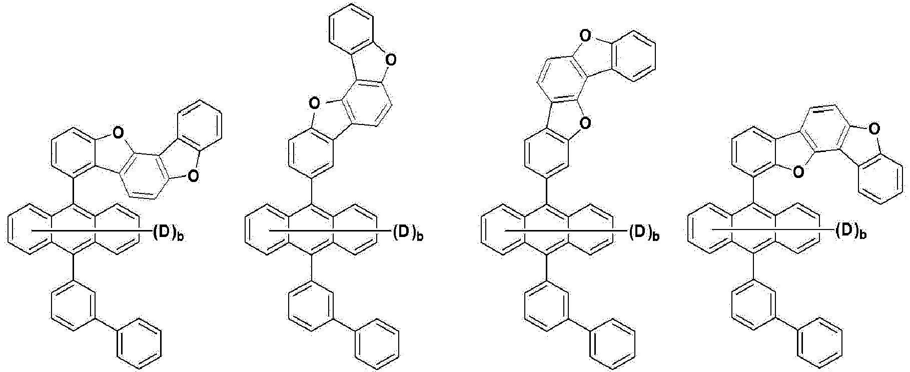

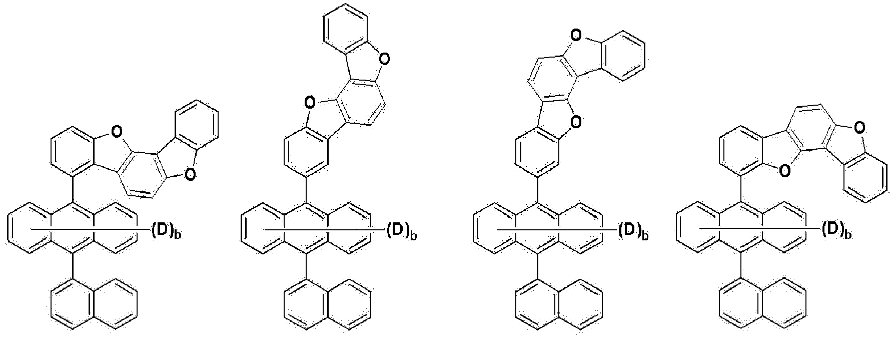

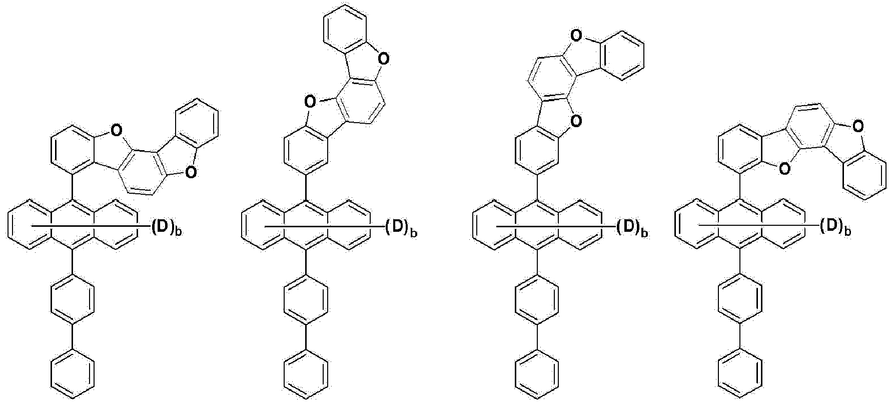

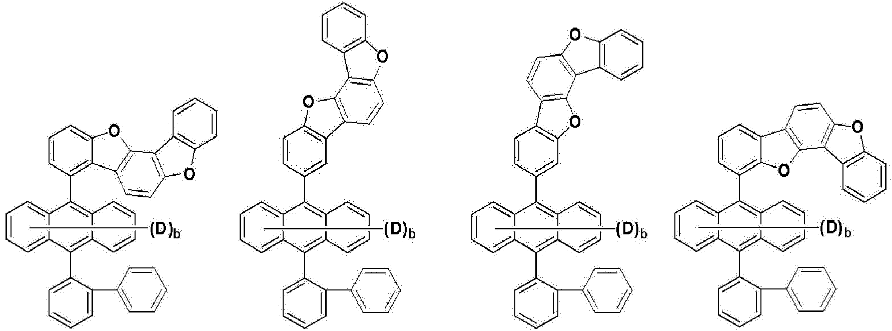

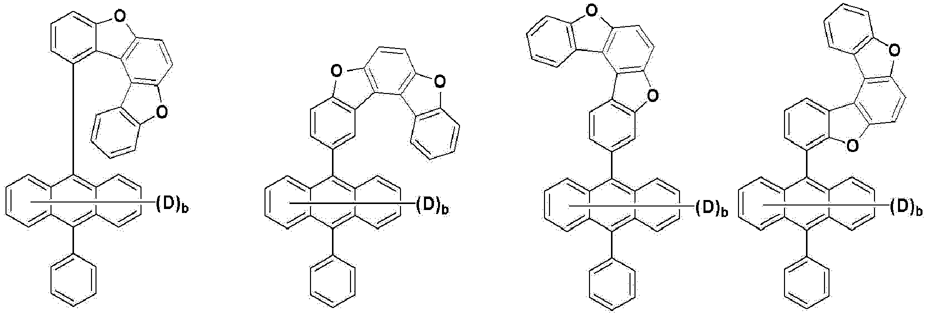

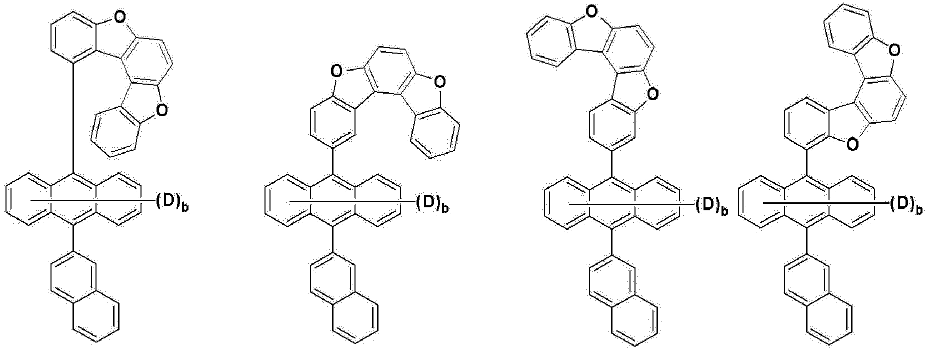

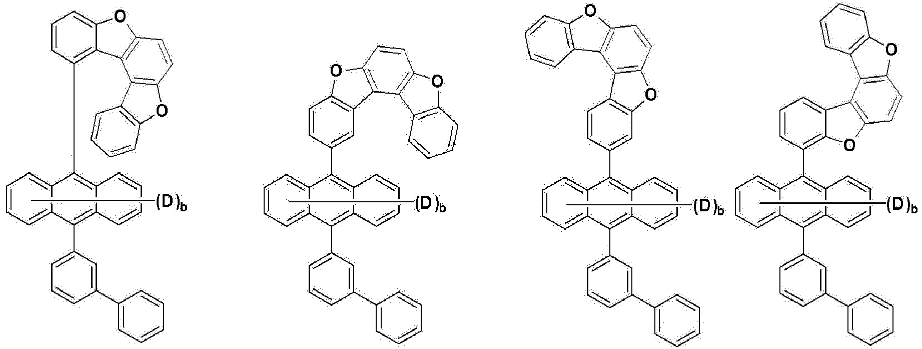

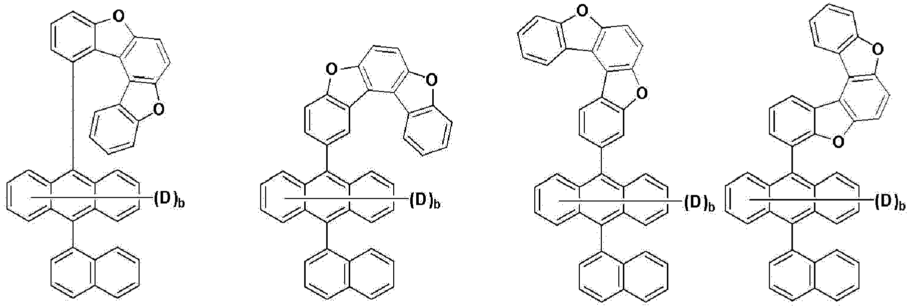

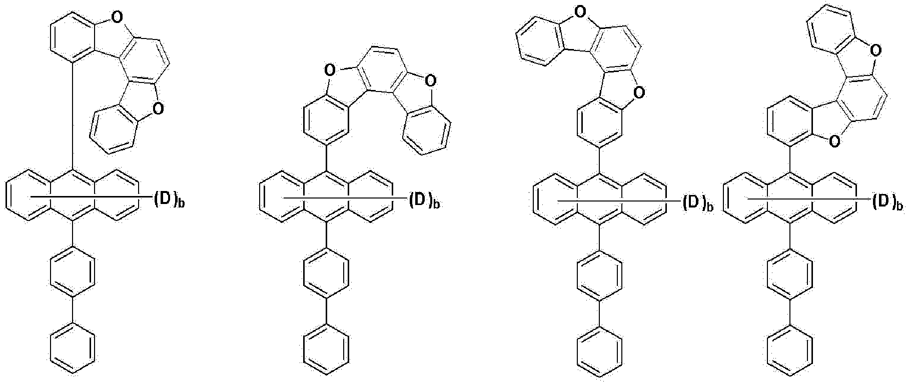

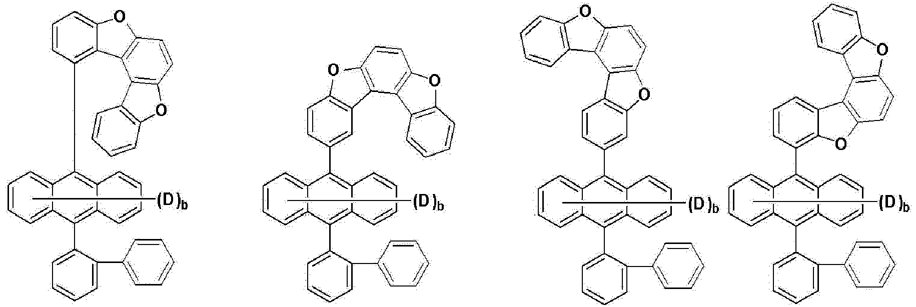

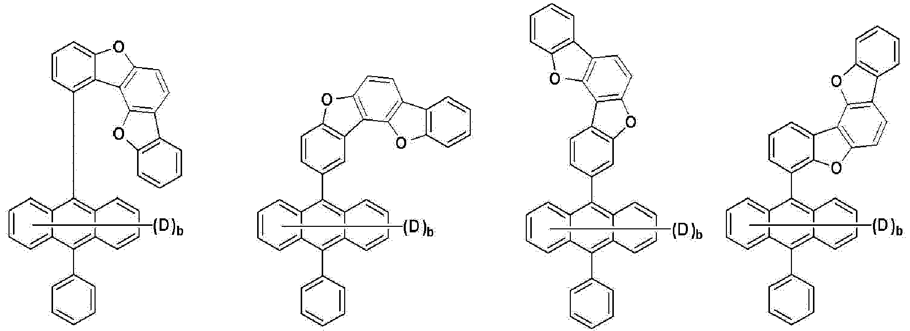

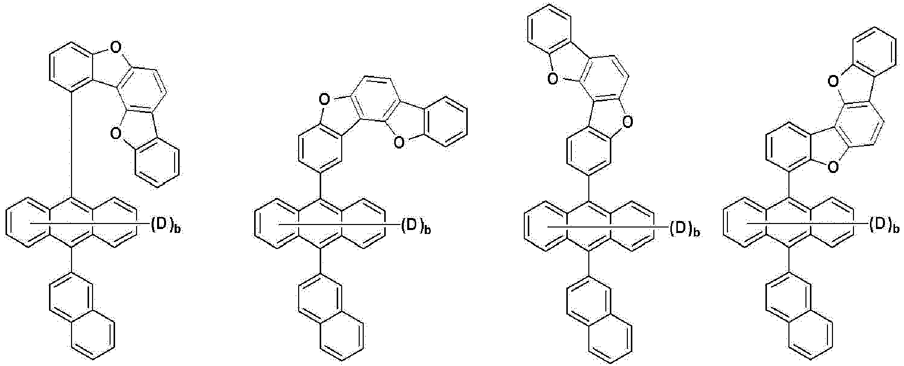

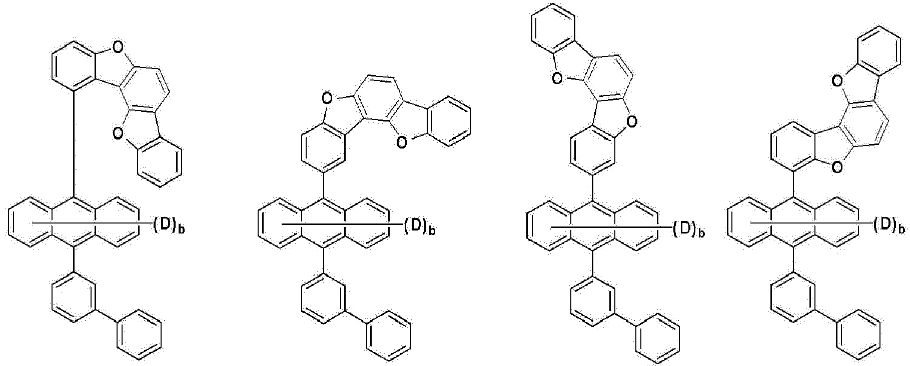

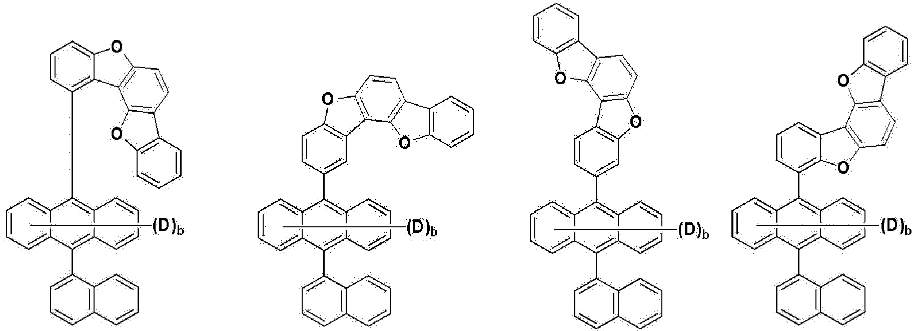

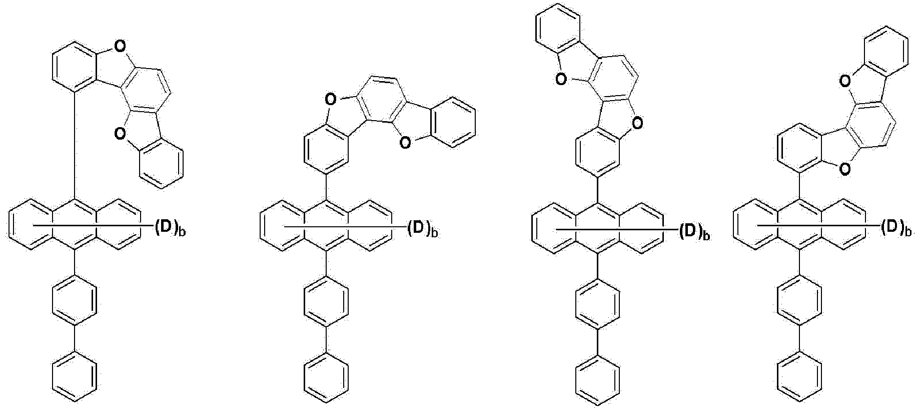

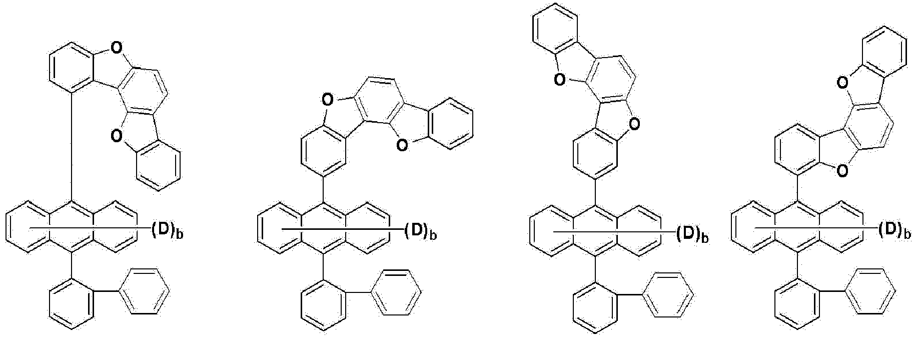

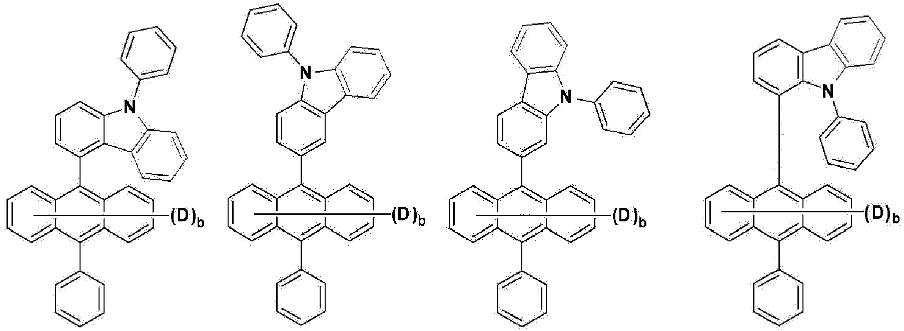

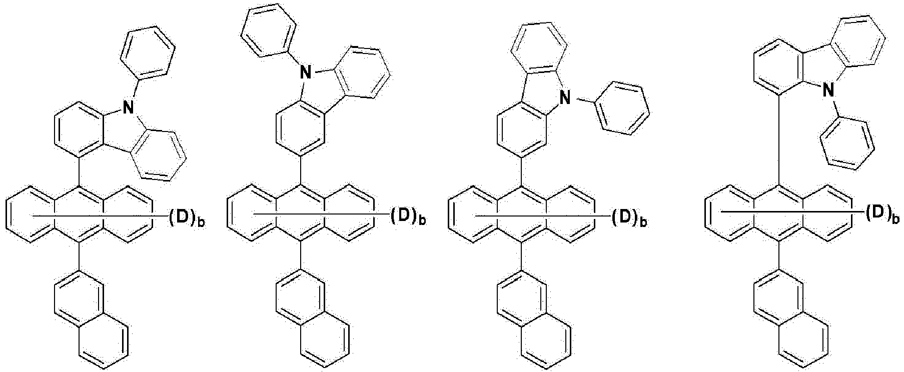

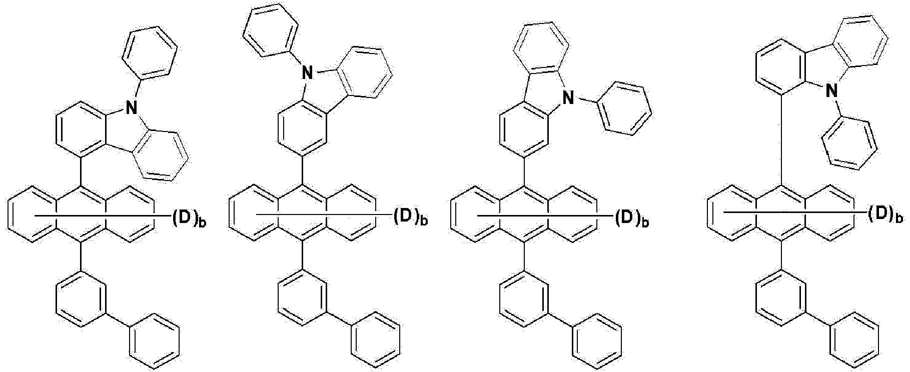

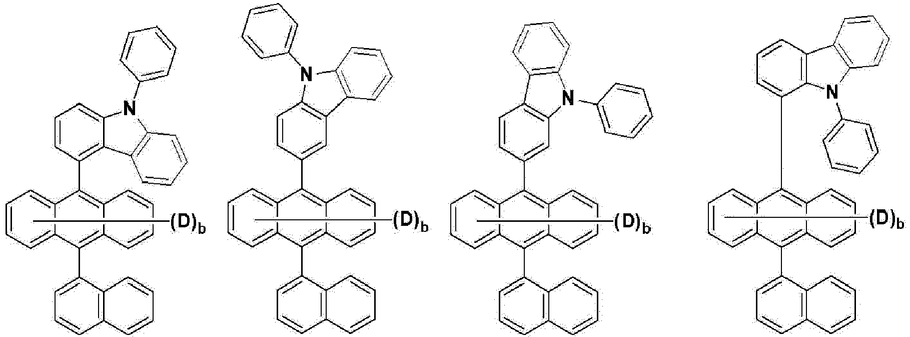

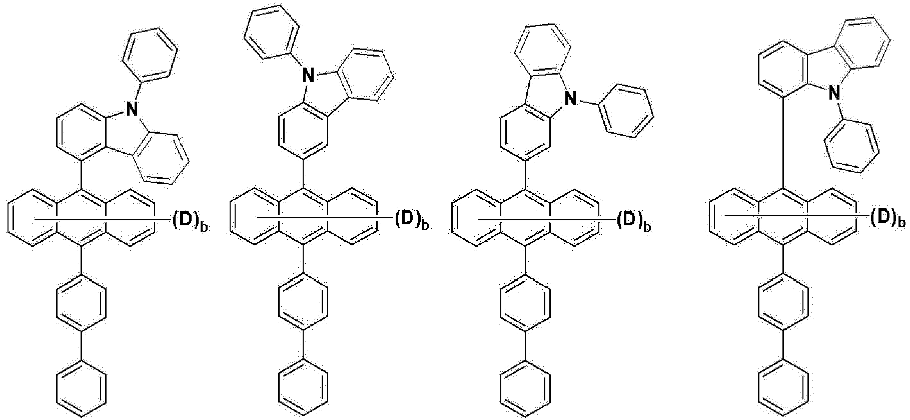

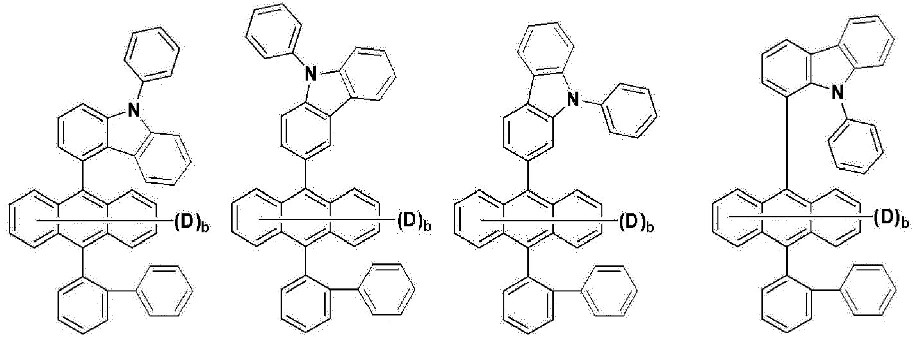

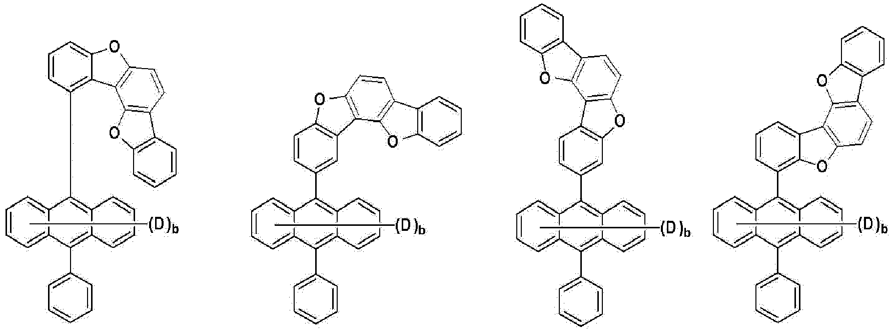

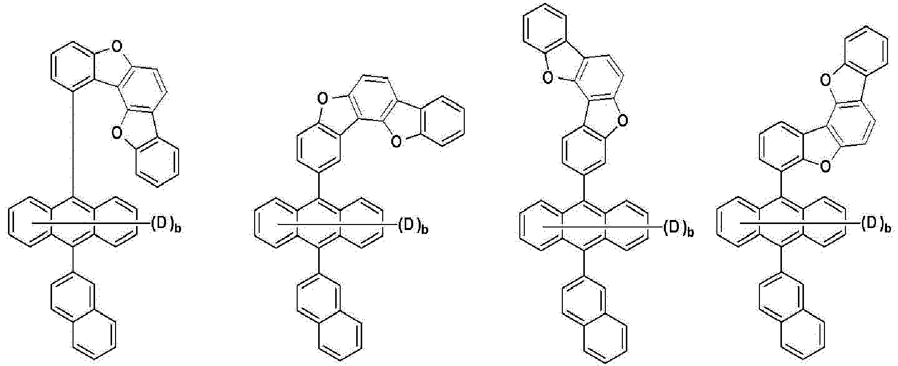

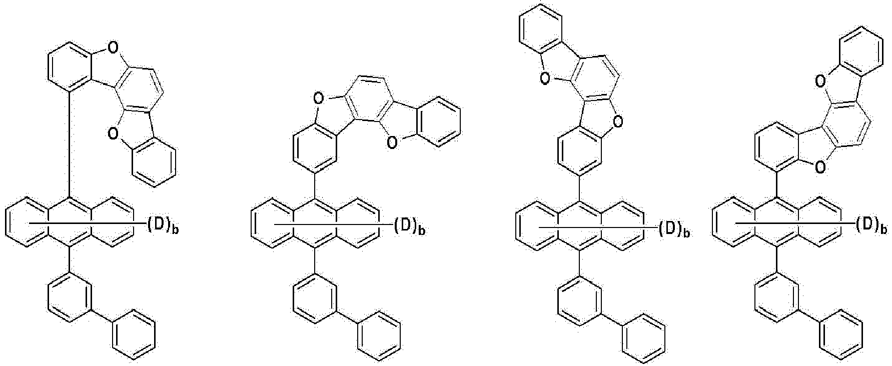

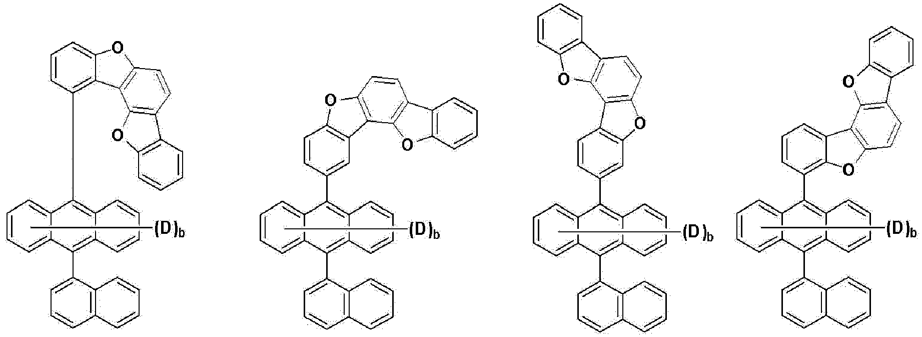

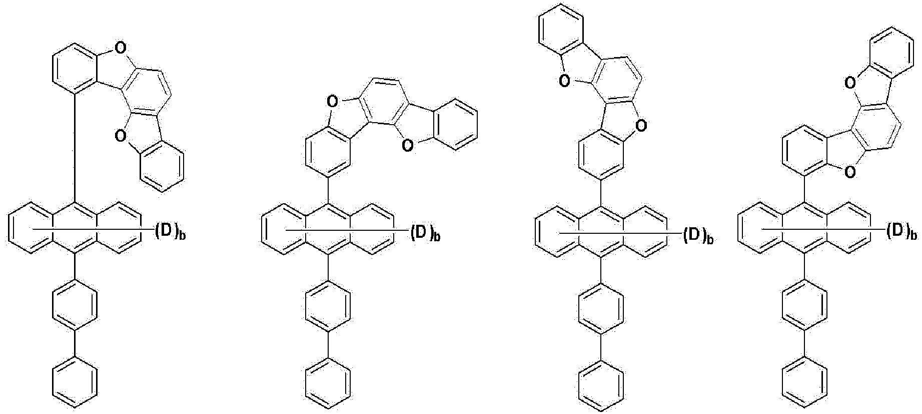

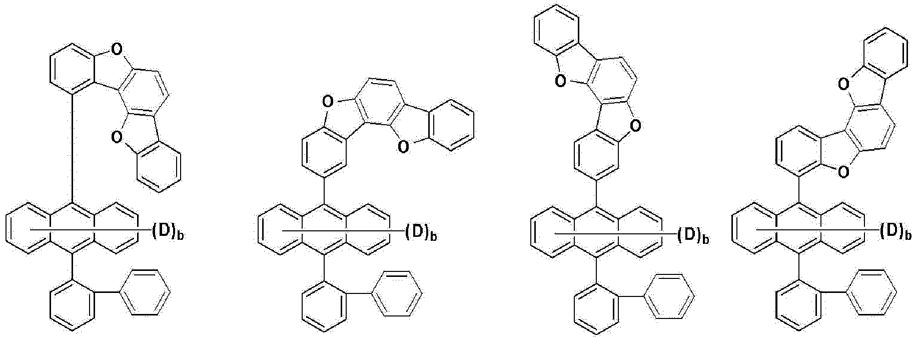

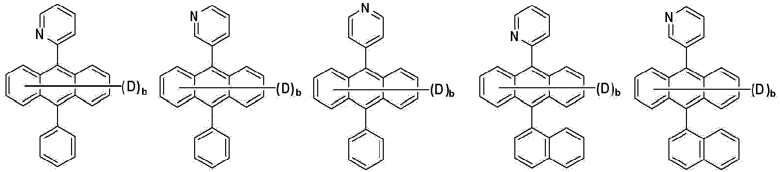

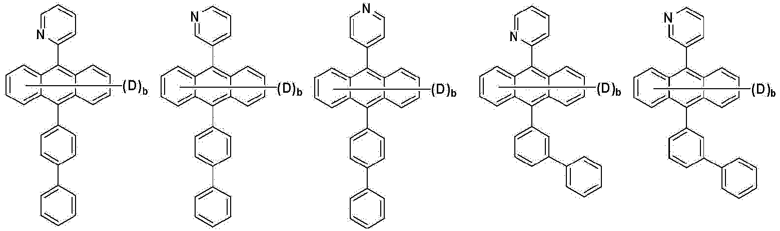

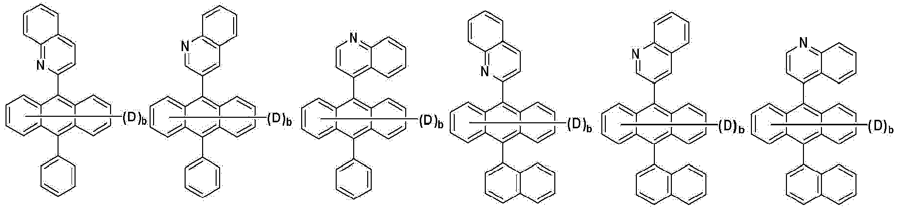

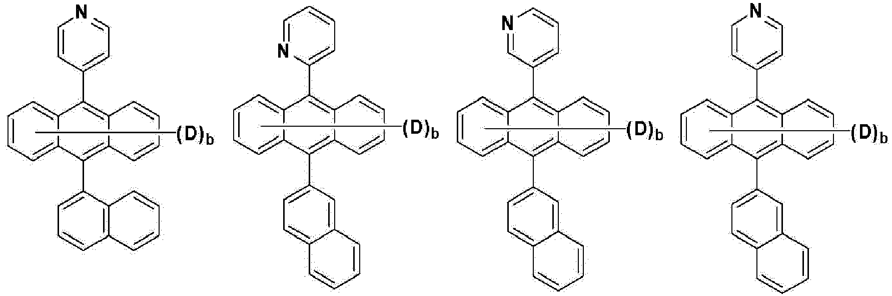

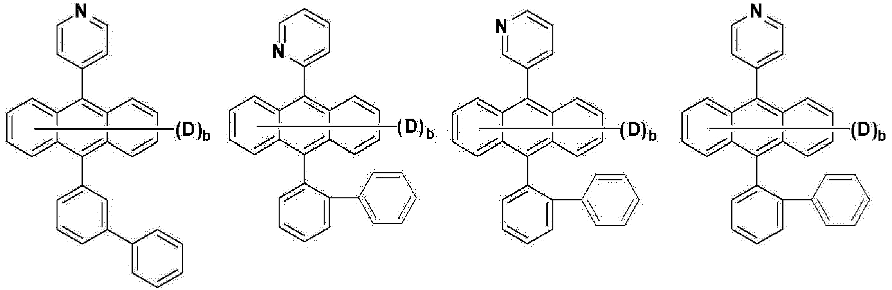

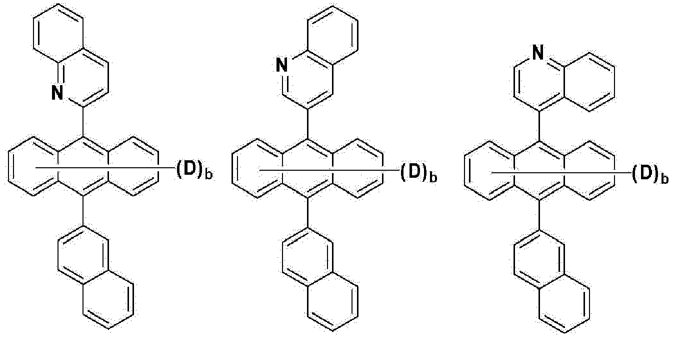

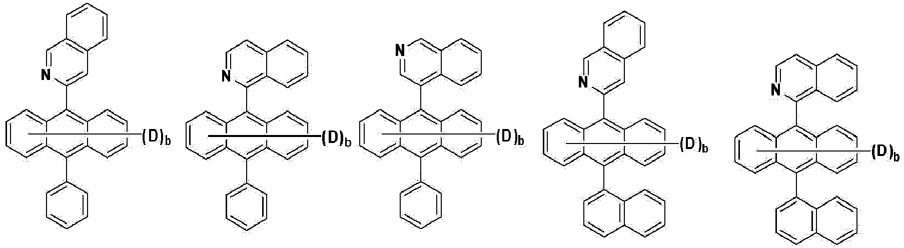

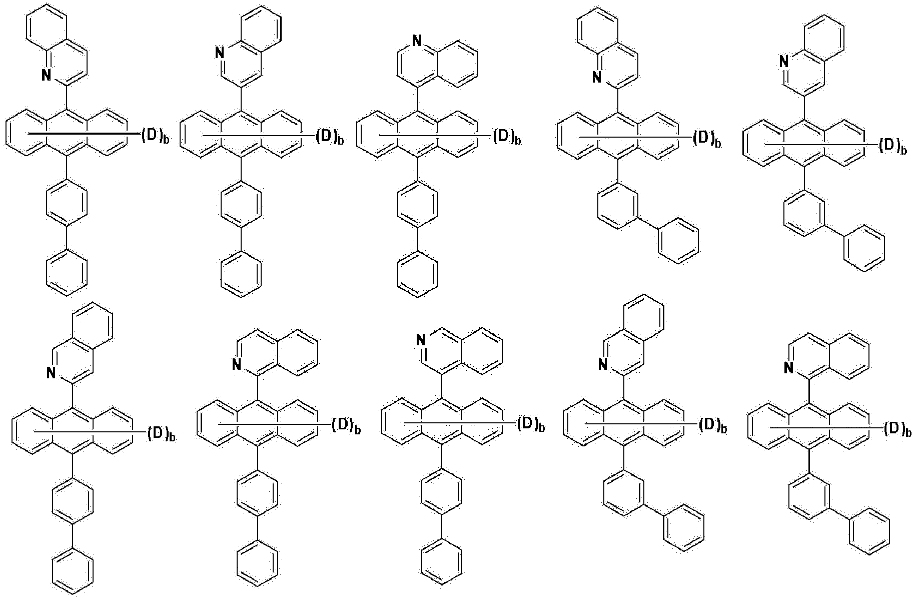

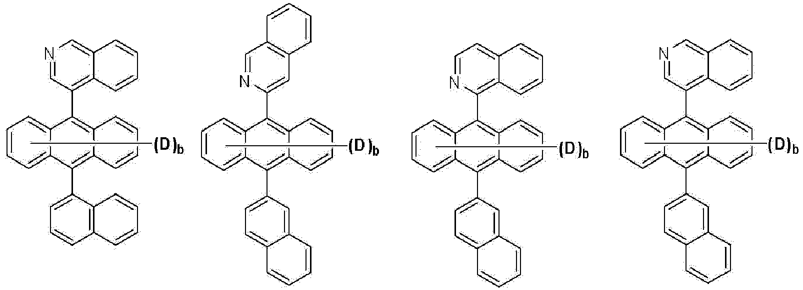

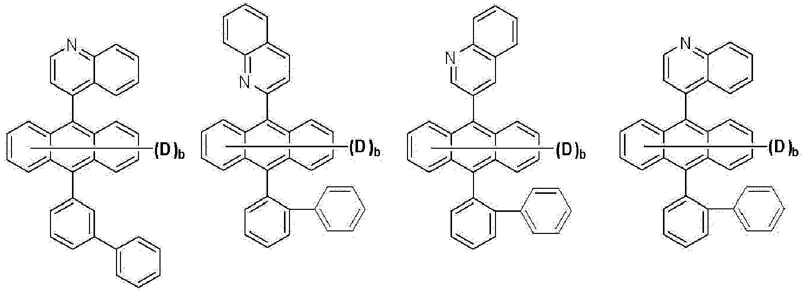

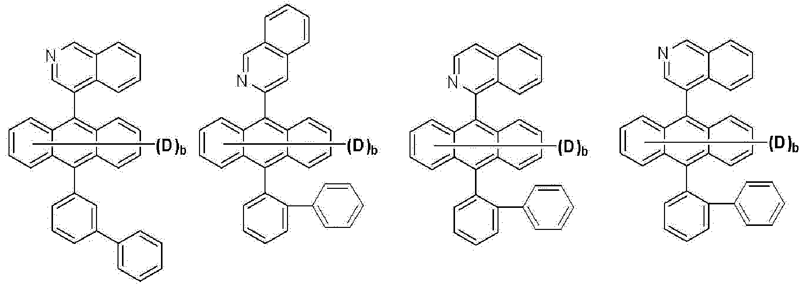

In the structures above, b is the number of deuterium substitutions, and is an integer from 1 to 8. b is determined by the deuterium conversion rate of deuterium substituted according to the synthesis method and conditions, the number of deuteriums substituted with an individual compound, and the substitution position of the deuterium substituted with the individual compound. Even though compounds having the same backbone are prepared, compounds having different numbers and substitution positions of deuteriums can be present in the individual compound.

In an exemplary embodiment of the present specification, a halogen supplying agent for synthesizing the compound of Chemical Formula 3 is not particularly limited as long as the halogen supplying agent can supply a halogen to the No. 10 position of the compound of Chemical Formula 2. For example, the halogen supplying agent can be selected from the group consisting of N-bromosuccinimide, bromine and hydrogen bromide.

In an exemplary embodiment of the present specification, the method for preparing a deuterated anthracene compound can further include synthesizing halogenated benzene-d5 using benzene-d6 and a halogen supplying agent before synthesizing of the compound of Chemical Formula 2.

In an exemplary embodiment of the present specification, the halogen supplying agent for synthesizing halogenated benzene-d5 is not particularly limited as long as the halogen supplying agent can substitute one deuterium in benzene-d6 with a halogen. For example, the halogen supplying agent can be selected from the group consisting of KBrO₃, bromine and hydrogen bromide.

The present specification provides a reaction composition including a halogenated benzene having at least one deuterium and the compound of Chemical Formula 1.

The description on the above-described method for preparing a deuterated anthracene compound can be cited for the reaction composition.

The present specification provides a deuterated anthracene compound prepared by the above-described preparation method.

In an exemplary embodiment of the present specification, the deuterated anthracene compound can include a compound represented by the following Chemical Formula A.

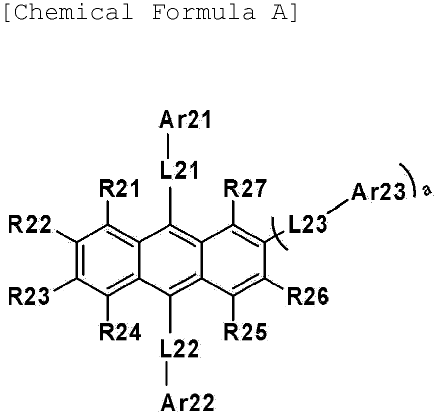

In Chemical Formula A,
L21 to L23 are the same as or different from each other, and are each independently a direct bond; or a substituted or unsubstituted arylene group; or a substituted or unsubstituted heteroarylene group,
R21 to R27 are the same as or different from each other, and are each independently hydrogen; a substituted or unsubstituted alkyl group; a substituted or unsubstituted cycloalkyl group; a substituted or unsubstituted silyl group; a substituted or unsubstituted aryl group; or a substituted or unsubstituted heteroaryl group,
Ar21 to Ar23 are the same as or different from each other, and are each independently deuterium; a substituted or unsubstituted aryl group; or a substituted or unsubstituted heteroaryl group, and
a is 0 or 1.

The present specification provides a deuterated anthracene compound of the following Chemical Formula 2.

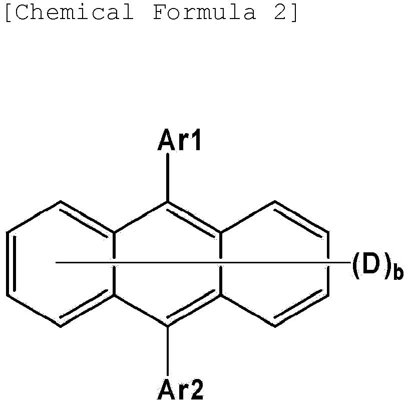

In Chemical Formula 2,
Ar1 is deuterium, a substituted or unsubstituted aryl group, or a substituted or unsubstituted heterocyclic group,
Ar2 is hydrogen, deuterium, a substituted or unsubstituted aryl group, or a substituted or unsubstituted heterocyclic group, and
b is an integer from 1 to 8.

The present specification provides an intermediate of the following Chemical Formula 5.

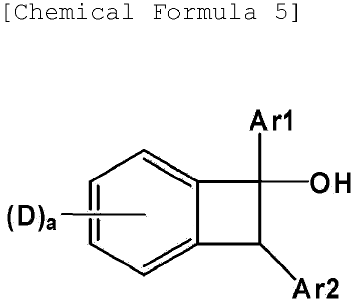

In Chemical Formula 5,
Ar1 is deuterium, a substituted or unsubstituted aryl group, or a substituted or unsubstituted heterocyclic group,
Ar2 is hydrogen, deuterium, a substituted or unsubstituted aryl group, or a substituted or unsubstituted heterocyclic group, and
a is an integer from 1 to 4.

The present specification provides a composition including an intermediate of the following Chemical Formula 5.

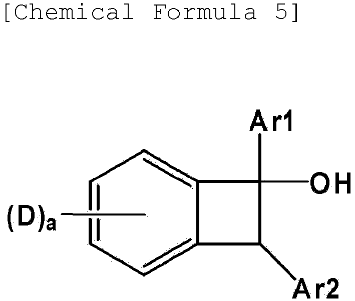

In Chemical Formula 5,
Ar1 is deuterium, a substituted or unsubstituted aryl group, or a substituted or unsubstituted heterocyclic group,
Ar2 is hydrogen, deuterium, a substituted or unsubstituted aryl group, or a substituted or unsubstituted heterocyclic group, and
a is an integer from 1 to 4.

In Chemical Formula 5, a is the number of deuteriums, and can be 1 to 8 depending on the number of deuteriums to be substituted.

In Chemical Formula 5, a is 1.

In Chemical Formula 5, a is 2.

In Chemical Formula 5, a is 3.

In Chemical Formula 5, a is 4. When a is 4, Chemical Formula 5 can be the following Chemical Formula 5-1.

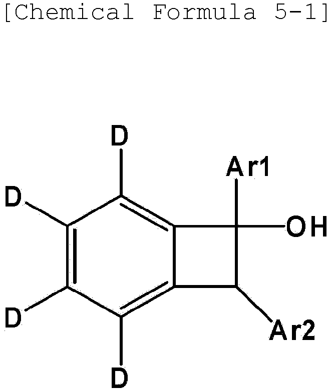

In Chemical Formula 5-1, Ar1 and Ar2 are the same as those defined in Chemical Formula 5.

In an exemplary embodiment of the present specification, the composition can further include a compound of the following Chemical Formula 2.

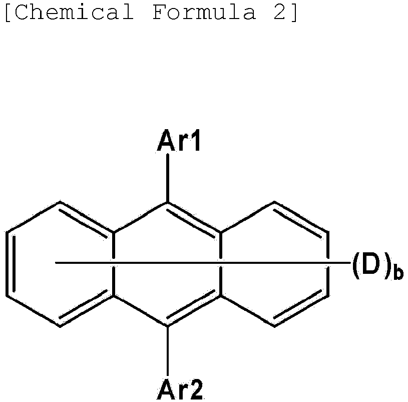

In Chemical Formula 2,

Ar1 is deuterium, a substituted or unsubstituted aryl group, or a substituted or unsubstituted heterocyclic group,
Ar2 is hydrogen, deuterium, a substituted or unsubstituted aryl group, or a substituted or unsubstituted heterocyclic group, and
b is an integer from 1 to 8.

In an exemplary embodiment of the present specification, a composition including the intermediate of Chemical Formula 5 can be a solution in the synthesizing of the deuterated anthracene compound by reacting a halogenated benzene having at least one deuterium with a compound of the following Chemical Formula 1.

In an exemplary embodiment of the present specification, the intermediate of Chemical Formula 5 is an intermediate formed by reacting a halogenated benzene having at least one deuterium with a compound of the following Chemical Formula 1 at 1:1 and is in a state of being synthesized into a deuterated anthracene compound which is a product, and the intermediate of Chemical Formula 5 is produced several times during a reaction of synthesizing the deuterated anthracene compound , and disappears while forming the deuterated anthracene compound. In addition, even after the synthesis of the deuterated anthracene compound is completed, intermediates that fail to be converted into the deuterated anthracene compound can remain.

In an exemplary embodiment of the present specification, the intermediate of Chemical Formula 5 can remain as an impurity even after the deuterated anthracene compound is obtained by purification.

Therefore, the composition including the intermediate of Chemical Formula 5 can mean a solution in which the synthetic reaction according to the preparation method of the present specification is being conducted, a solution in which the synthetic reaction according to the preparation method of the present specification is completed, or a deuterated anthracene compound obtained after the synthetic reaction according to the preparation method of the present specification is completed.

In this case, the deuterated anthracene compound obtained after the synthetic reaction is completed can include two or more isotopes having different molecular weights according to the number of substituted deuteriums, and thus can be expressed as a composition including two or more compounds having different numbers of substituted deuteriums.

In an exemplary embodiment of the present specification, for the deuterated anthracene compound obtained after the synthetic reaction is completed, even though the hydrogens of the substituents of Ar1 and Ar2 are not substituted with deuteriums, HOMO and LUMO are distributed in anthracene in the case of an anthracene derivative, so that an effect of improving the service life, which is similar to that of an anthracene compound having a deuterium substitution rate at a predetermined level or more (80% or more), is exhibited even though an additional deuterium substitution reaction is not performed.

Since no additional deuterated substitution reaction is required, an excessive amount of deuterium source for the deuterated substitution reaction is not required, which is economically advantageous, so that the deuterated anthracene compound can be prepared at a low cost.

In an exemplary embodiment of the present specification, in the deuterated anthracene compound of the present specification, most of the positions, which are not 'a substituted or unsubstituted aryl group or a substituted or unsubstituted hetero ring' in the Nos. 1 to 10 positions of the anthracene are substituted with deuterium.

In an exemplary embodiment of the present specification, in the deuterated anthracene compound of the present specification, the deuterium substitution rate for the positions which are not 'a substituted or unsubstituted aryl group or a substituted or unsubstituted hetero ring' among the Nos. 1 to 10 positions of the anthracene can be 50% or more, 60% or more, 70% or more, 80% or more, 90% or more, 95% or more, 96% or more, 97% or more, 98% or more, or 99% or more.

In an exemplary embodiment of the present specification, in the case of a deuterated anthracene compound in which 'a substituted or unsubstituted aryl group or a substituted or unsubstituted hetero ring' is substituted only at the Nos. 9 and 10 positions, the number of deuterium substitutions of the deuterated anthracene compound can be 5 or more, 6 or more, 7 or more, or 8.

In an exemplary embodiment of the present specification, in the case of a deuterated anthracene compound in which 'a substituted or unsubstituted aryl group or a substituted or unsubstituted hetero ring' is substituted only at the No. 9 position, the number of deuterium substitutions of the deuterated anthracene compound can be 5 or more, 6 or more, 7 or more, 8 or more, or 9.

In an exemplary embodiment of the present specification, in the case of a deuterated anthracene compound in which 'a substituted or unsubstituted aryl group or a substituted or unsubstituted hetero ring' is substituted only at the Nos. 2, 9 and 10 positions, the number of deuterium substitutions of the deuterated anthracene compound can be 5 or more, 6 or more, or 7.

In an exemplary embodiment of the present specification, in the case of a deuterated anthracene compound which does not have a position substituted with 'a substituted or unsubstituted aryl group or a substituted or unsubstituted hetero ring' at the Nos. 1 to 10 positions, the number of deuterium substitutions of the deuterated anthracene compound can be 5 or more, 6 or more, 7 or more, 8 or more, 9 or more, or 10.

Examples of the substituents in the present specification will be described below, but are not limited thereto.

The term "substitution" means that a hydrogen atom bonded to a carbon atom of a compound is changed into another substituent, and a position to be substituted is not limited as long as the position is a position at which the hydrogen atom is substituted, that is, a position at which the substituent can be substituted, and when two or more are substituted, the two or more substituents can be the same as or different from each other.

In the present specification, the term "substituted or unsubstituted" means being substituted with one or two or more substituents selected from the group consisting of deuterium; a halogen group; a nitrile group; a nitro group; a hydroxyl group; an amine group; a silyl group; a boron group; an alkoxy group; an alkyl group; a cycloalkyl group; an aryl group; and a heterocyclic group, being substituted with a substituent to which two or more substituents among the above-exemplified substituents are linked, or having no substituent. For example, "the substituent to which two or more substituents are linked" can be a biphenyl group. That is, the biphenyl group can also be an aryl group, and can be interpreted as a substituent to which two phenyl groups are linked.

In the present specification, examples of a halogen group include fluorine (-F), chlorine (-Cl), bromine (-Br) or iodine (-I).

In the present specification, a silyl group can be a chemical formula of -SiYₐY_{b}Y_{c}, and the Yₐ, Y_{b}, and Y_{c} can be each hydrogen, a substituted or unsubstituted alkyl group, or a substituted or unsubstituted aryl group. Specific examples of the silyl group include a trimethylsilyl group, a triethylsilyl group, a tert-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, a boron group can be a chemical formula of -BY_{d}Yₑ, and the Y_{d} and Yₑ can be each hydrogen, a substituted or unsubstituted alkyl group, or a substituted or unsubstituted aryl group. Specific examples of the boron group include a trimethylboron group, a triethylboron group, a tert-butyldimethylboron group, a triphenylboron group, a phenylboron group, and the like, but are not limited thereto.

In the present specification, the alkyl group can be straight-chained or branched, and the number of carbon atoms thereof is not particularly limited, but is preferably 1 to 60. According to an exemplary embodiment, the number of carbon atoms of the alkyl group is 1 to 30. According to another exemplary embodiment, the number of carbon atoms of the alkyl group is 1 to 20. According to still another exemplary embodiment, the number of carbon atoms of the alkyl group is 1 to 10. Specific examples of the alkyl group include a methyl group, an ethyl group, a propyl group, an n-propyl group, an isopropyl group, a butyl group, an n-butyl group, an isobutyl group, a tert-butyl group, a pentyl group, an n-pentyl group, a hexyl group, an n-hexyl group, a heptyl group, an n-heptyl group, an octyl group, an n-octyl group, and the like, but are not limited thereto.

In the present specification, the alkoxy group can be straight-chained, branched, or cyclic. The number of carbon atoms of the alkoxy group is not particularly limited, but is preferably 1 to 20. Specific examples thereof include methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, and the like, but are not limited thereto.

Substituents including an alkyl group, an alkoxy group, and other alkyl group moieties described in the present specification include both a straight-chained form and a branched form.

In the present specification, a cycloalkyl group is not particularly limited, but has preferably 3 to 60 carbon atoms, and according to an exemplary embodiment, the number of carbon atoms of the cycloalkyl group is 3 to 30. According to another exemplary embodiment, the number of carbon atoms of the cycloalkyl group is 3 to 20. According to still another exemplary embodiment, the number of carbon atoms of the cycloalkyl group is 3 to 6. Specific examples thereof include a cyclopropyl group, a cyclobutyl group, a cyclopentyl group, a cyclohexyl group, a cycloheptyl group, a cyclooctyl group, and the like, but are not limited thereto.

In the present specification, an aryl group is not particularly limited, but has preferably 6 to 60 carbon atoms, and can be a monocyclic aryl group or a polycyclic aryl group. According to an exemplary embodiment, the number of carbon atoms of the aryl group is 6 to 39. According to an exemplary embodiment, the number of carbon atoms of the aryl group is 6 to 30. Examples of the monocyclic aryl group include a phenyl group, a biphenyl group, a terphenyl group, a quarterphenyl group, and the like, but are not limited thereto. Examples of the polycyclic aryl group include a naphthyl group, an anthracenyl group, a phenanthrenyl group, a pyrenyl group, a perylenyl group, a triphenyl group, a chrysenyl group, a fluorenyl group, a triphenylenyl group, and the like, but are not limited thereto.

In the present specification, a fluorene group can be substituted, and two substituents can be bonded to each other to form a spiro structure.

When the fluorene group is substituted, the fluorene group can be a spirofluorene group such as 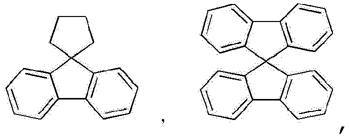 and a substituted fluorene group such as 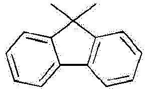 (a 9,9-dimethylfluorene group) and 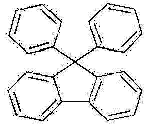 (a 9,9-diphenylfluorene group). However, the substituent is not limited thereto.

In the present specification, a heterocyclic group is a cyclic group including one or more of N, O, P, S, Si, and Se as a heteroatom, and the number of carbon atoms thereof is not particularly limited, but is preferably 2 to 60. According to an exemplary embodiment, the number of carbon atoms of the heterocyclic group is 2 to 36. Examples of the heterocyclic group include a pyridine group, a pyrrole group, a pyrimidine group, a quinoline group, a pyridazine group, a furan group, a thiophene group, an imidazole group, a pyrazole group, a dibenzofuran group, a dibenzothiophene group, a carbazole group, a benzocarbazole group, a benzonaphthofuran group, a benzonaphthothiophene group, an indenocarbazole group, an indolocarbazole group, and the like, but are not limited thereto.

In the present specification, the above-described description on the heterocyclic group can be applied to a heteroaryl group except for an aromatic heteroaryl group.

In the present specification, an amine group can be selected from the group consisting of -NH₂; an alkylamine group; an N-alkylarylamine group; an arylamine group; an N-arylheteroarylamine group; an N-alkylheteroarylamine group; and a heteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, an anthracenylamine group, a 9-methyl-anthracenylamine group, a diphenylamine group, an N-phenylnaphthylamine group, a ditolylamine group, an N-phenyltolylamine group, a triphenylamine group, an N-phenylbiphenylamine group, an N-phenylnaphthylamine group, an N-biphenylnaphthylamine group, an N-naphthylfluorenylamine group, an N-phenylphenanthrenylamine group, an N-biphenylphenanthrenylamine group, an N-phenylfluorenylamine group, an N-phenyl terphenylamine group, an N-phenanthrenylfluorenylamine group, an N-biphenylfluorenylamine group, and the like, but are not limited thereto.

In the present specification, an N-alkylarylamine group means an amine group in which an alkyl group and an aryl group are substituted with N of the amine group.

In the present specification, an N-arylheteroarylamine group means an amine group in which an aryl group and a heteroaryl group are substituted with N of the amine group.

In the present specification, an N-alkylheteroarylamine group means an amine group in which an alkyl group and a heteroaryl group are substituted with N of the amine group.

In the present specification, an alkyl group, an aryl group, and a heteroaryl group in an alkylamine group; an N-alkylarylamine group; an arylamine group; an N-arylheteroarylamine group; an N-alkylheteroarylamine group, and a heteroarylamine group are each the same as the above-described examples of the alkyl group, the aryl group, and the heterocyclic group.

In an exemplary embodiment of the present specification, the deuterated anthracene compound can be any one of the following structures.

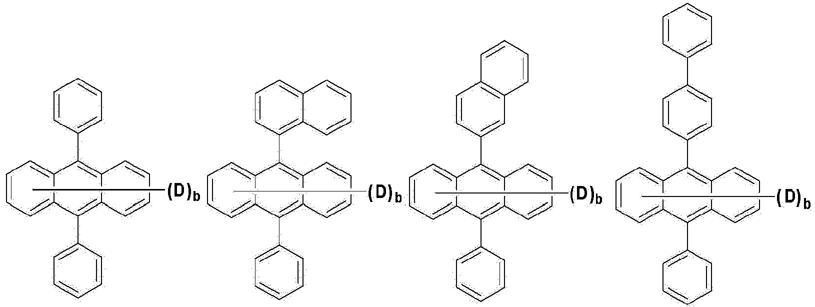

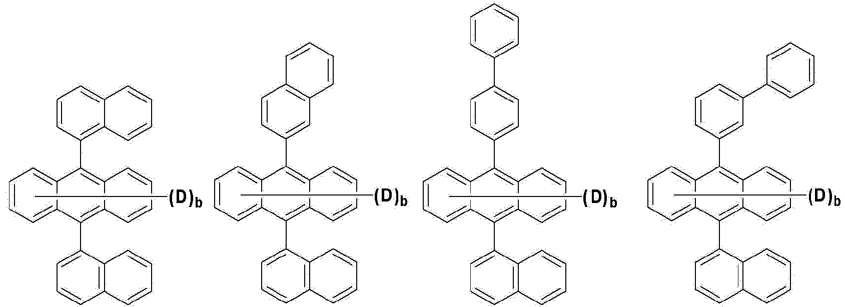

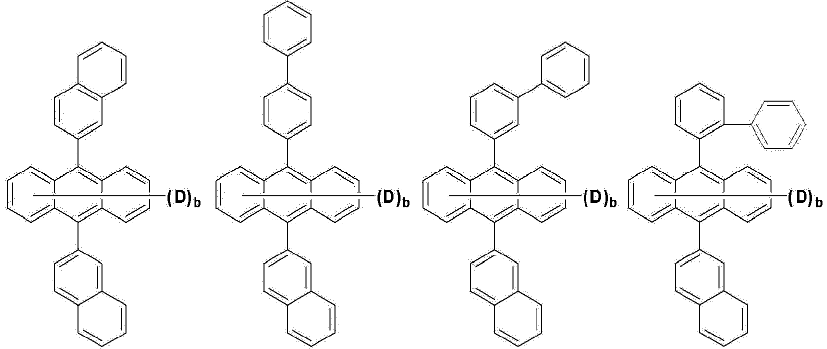

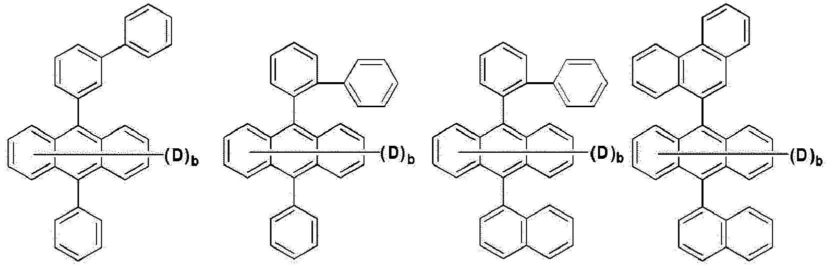

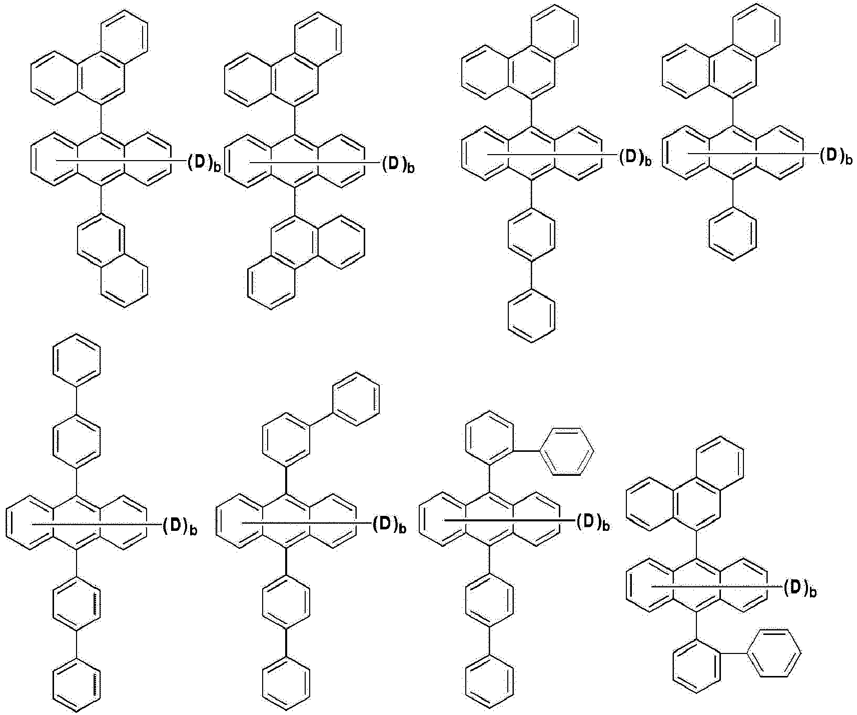

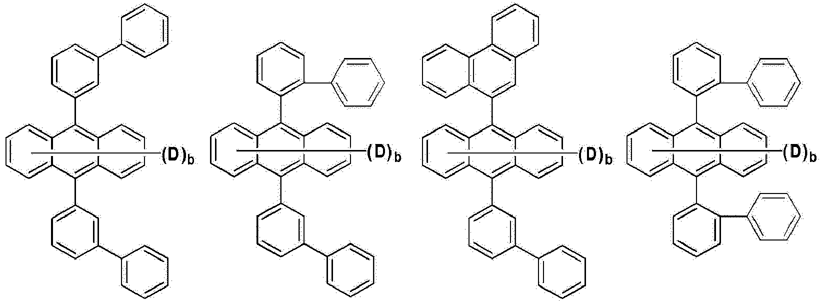

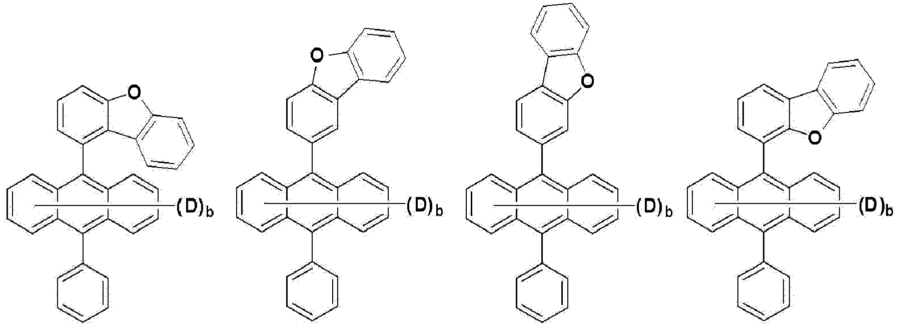

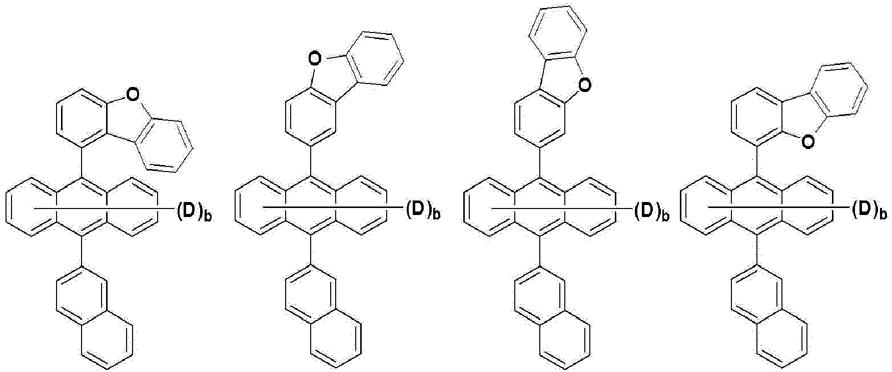

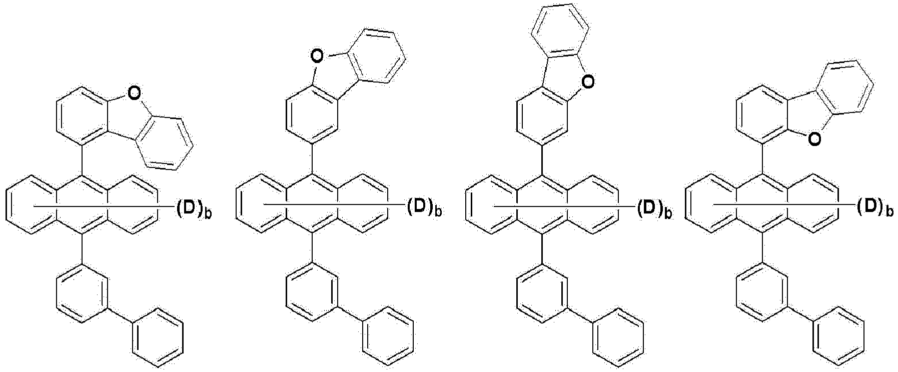

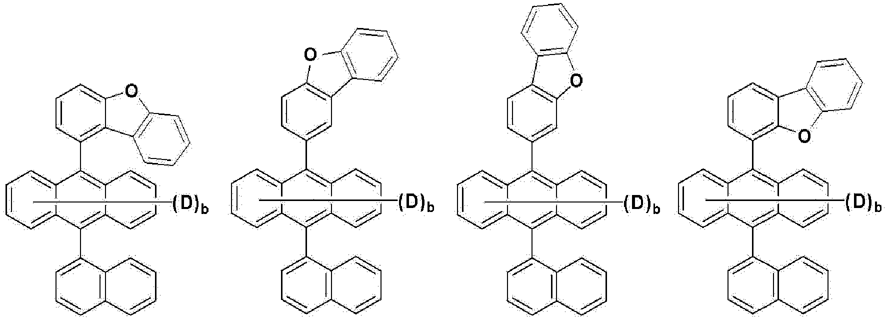

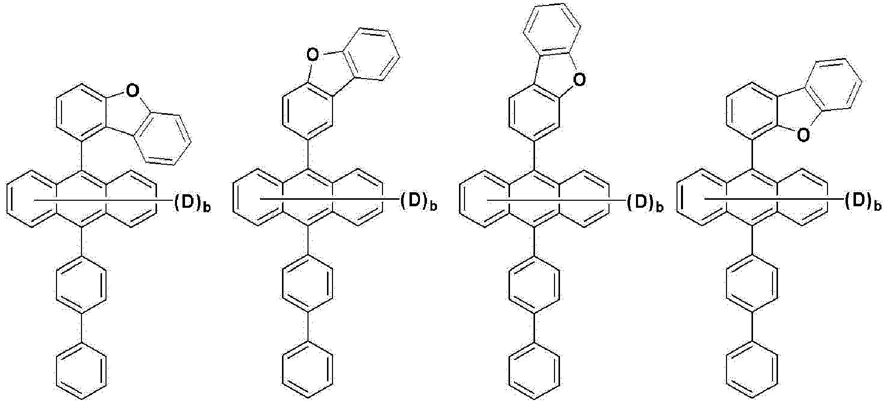

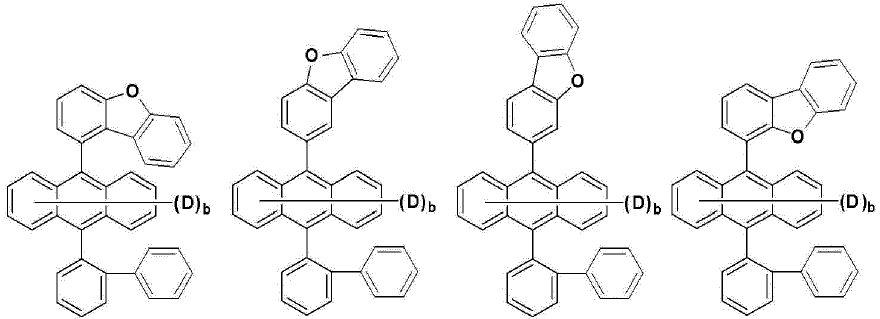

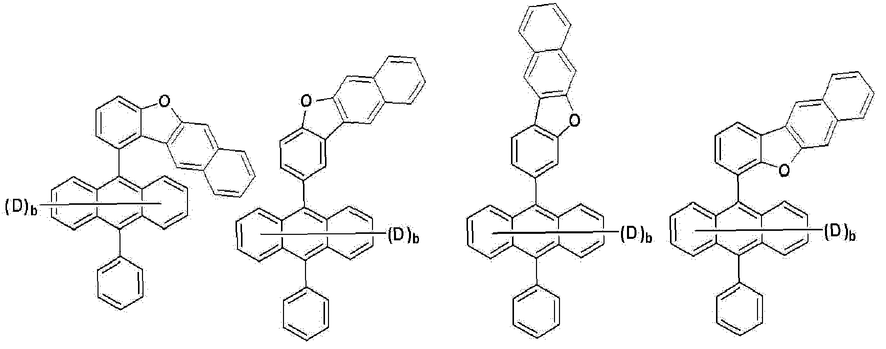

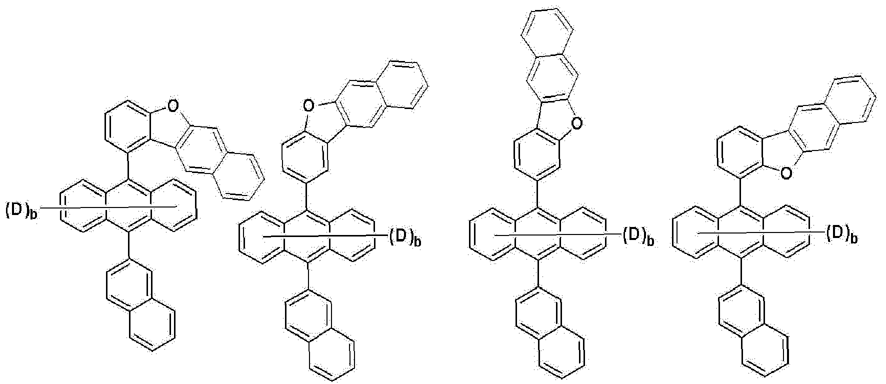

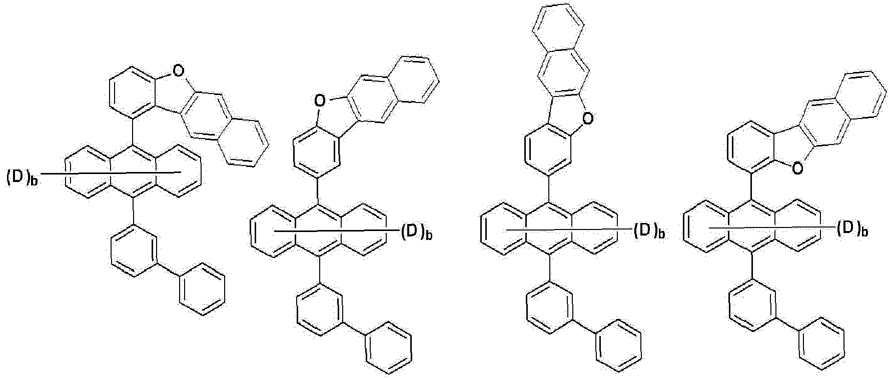

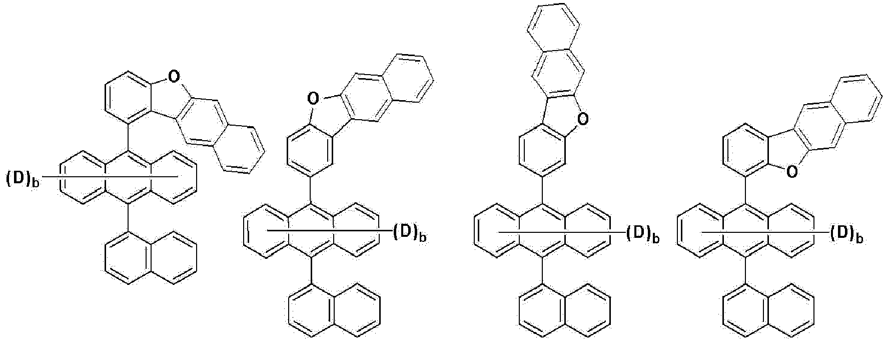

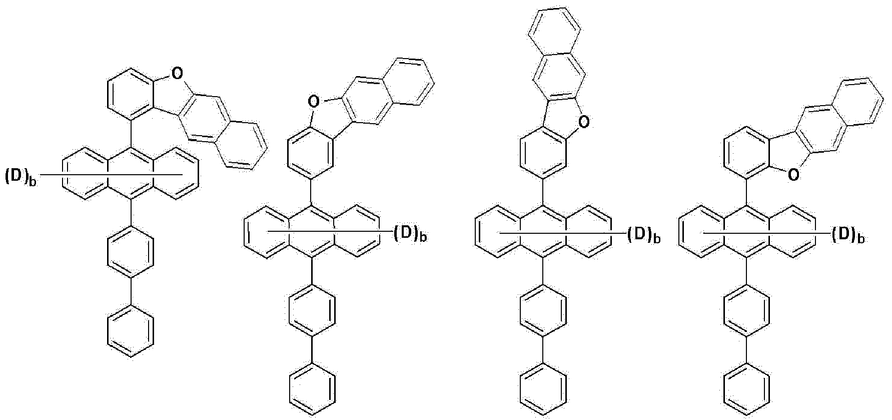

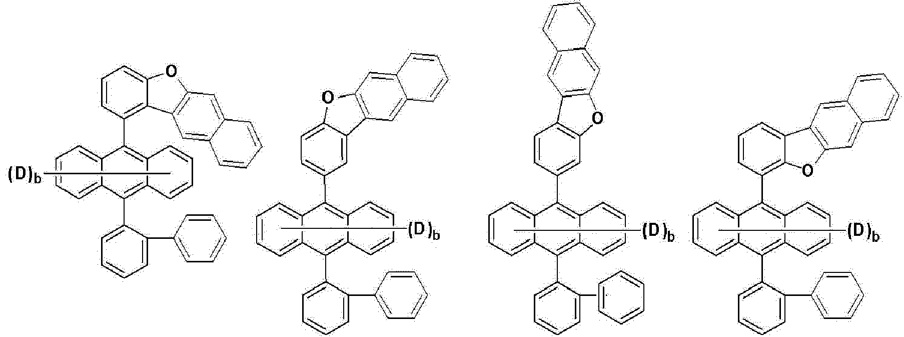

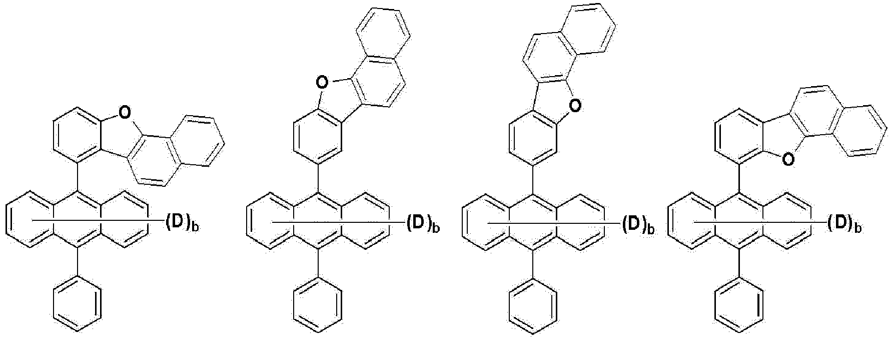

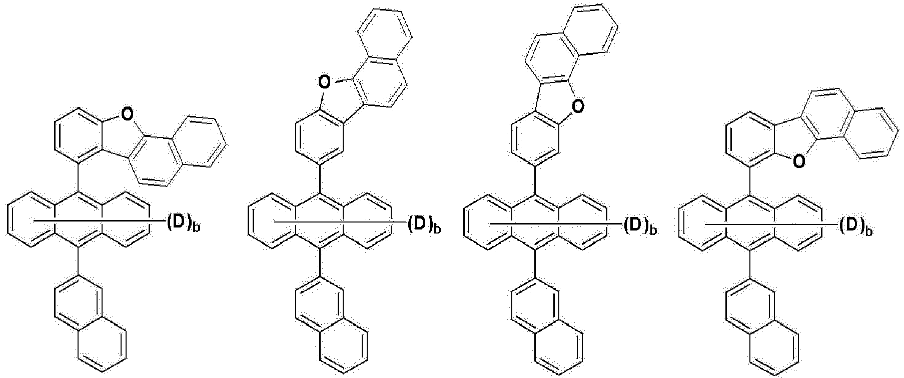

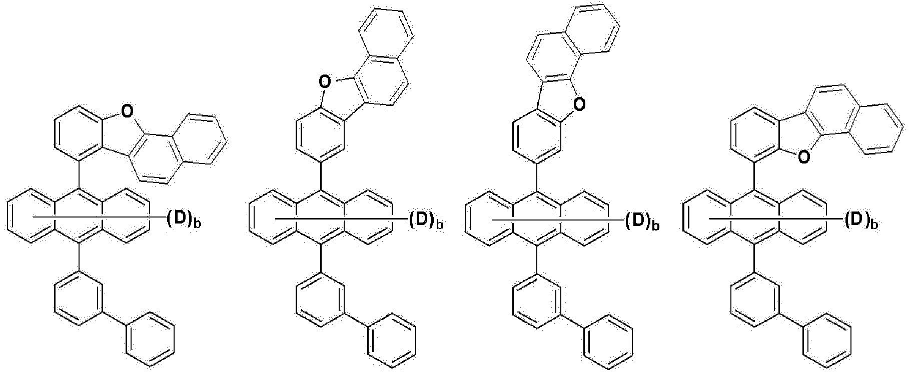

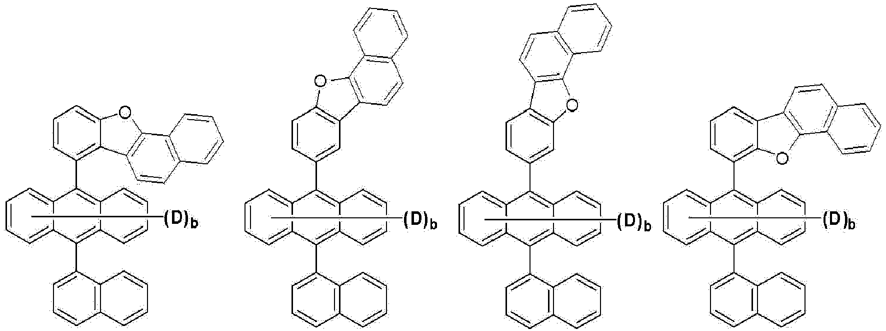

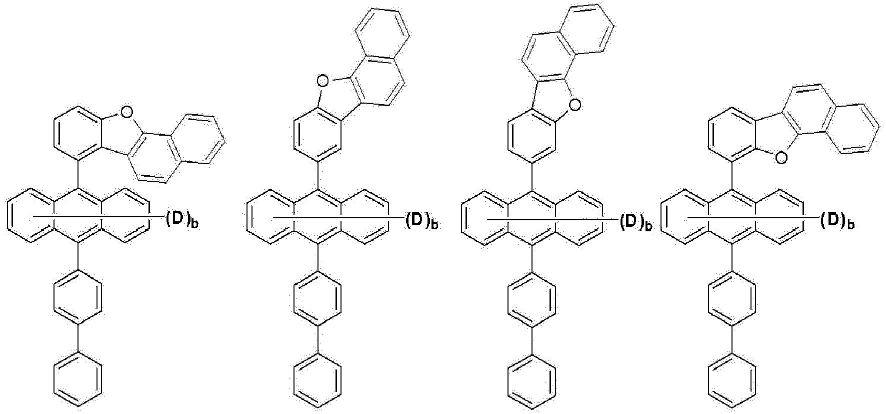

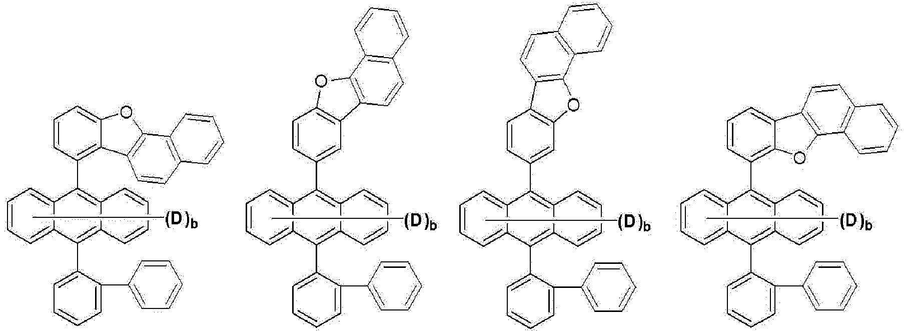

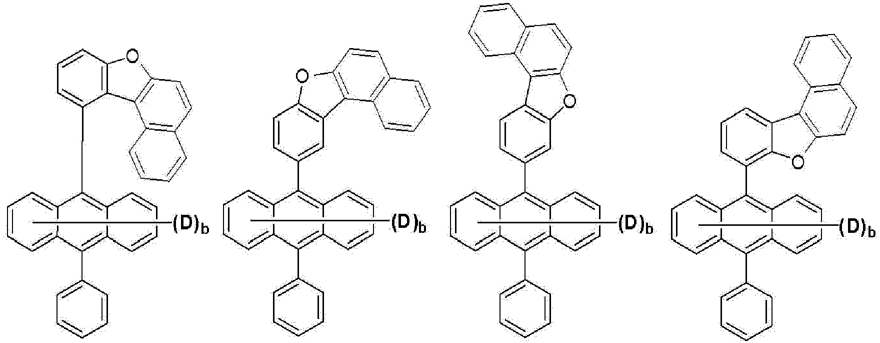

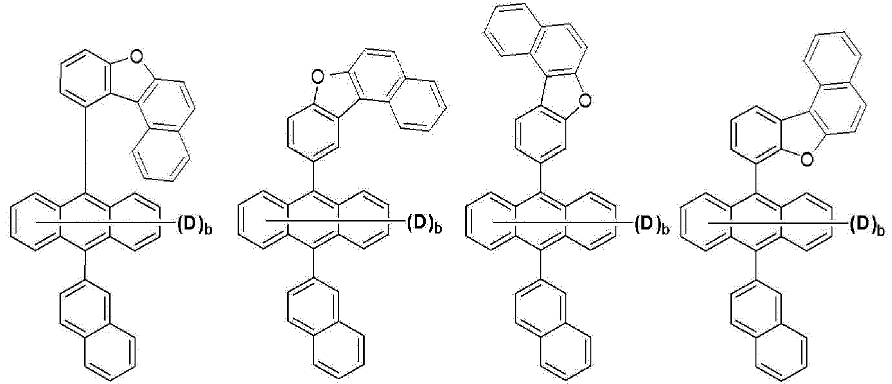

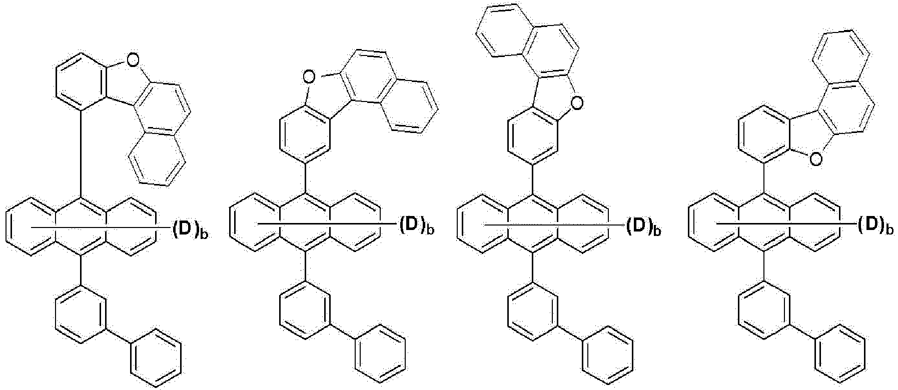

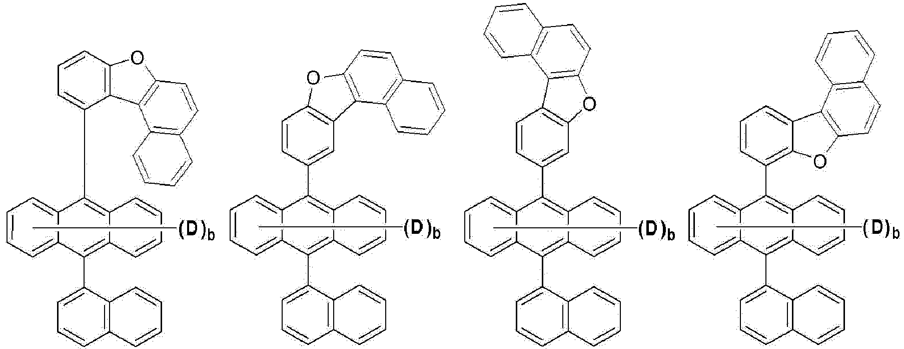

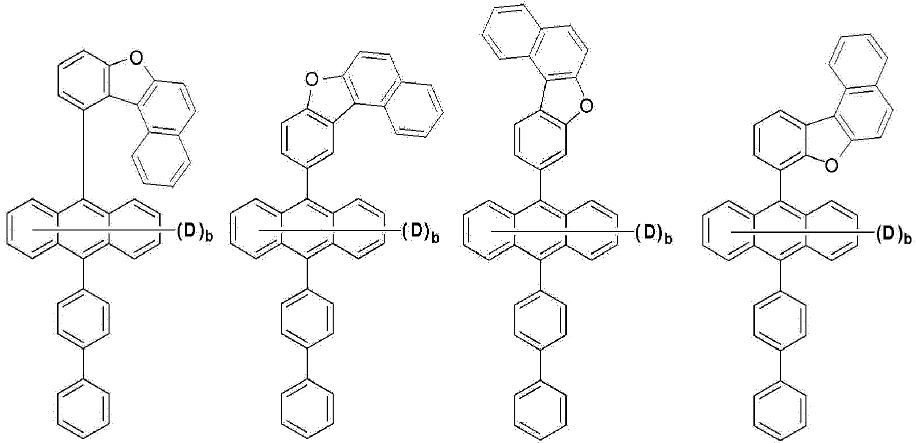

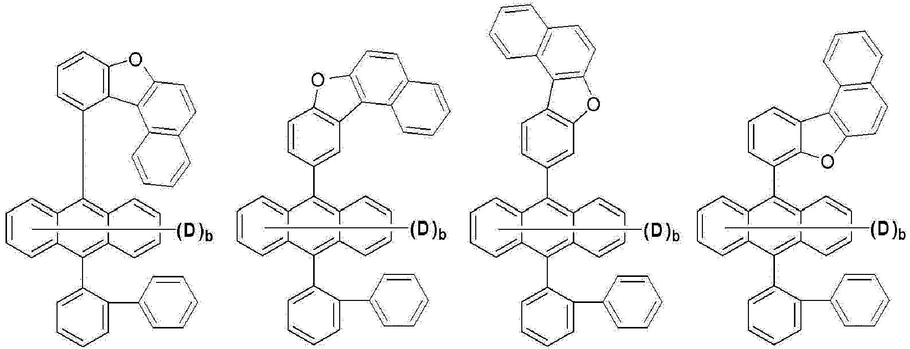

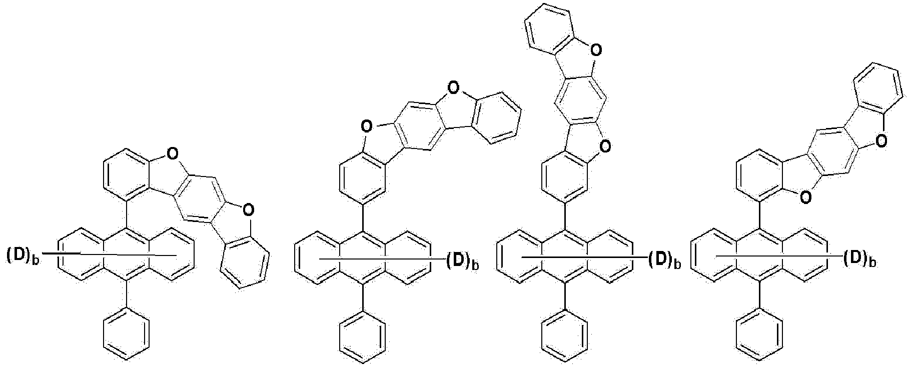

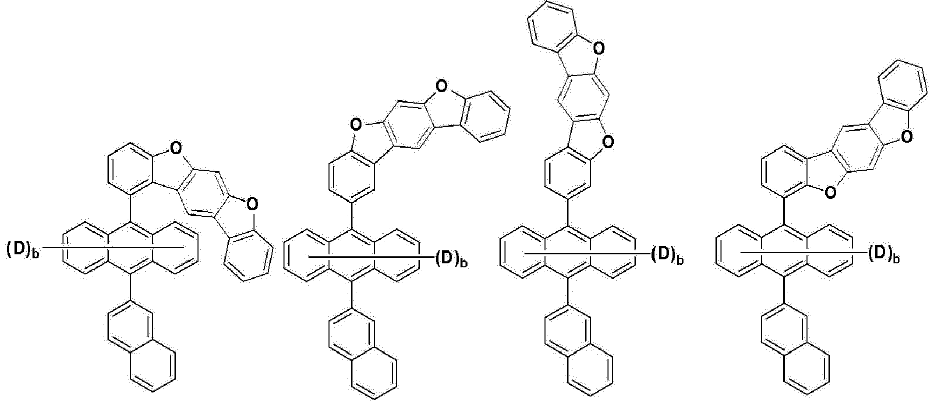

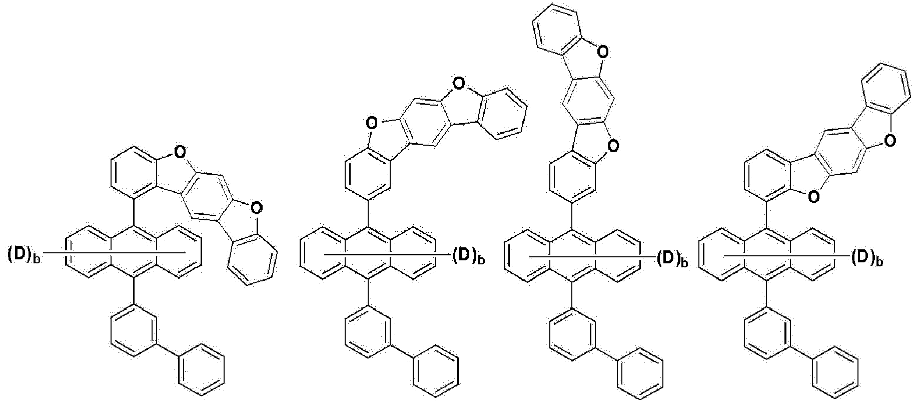

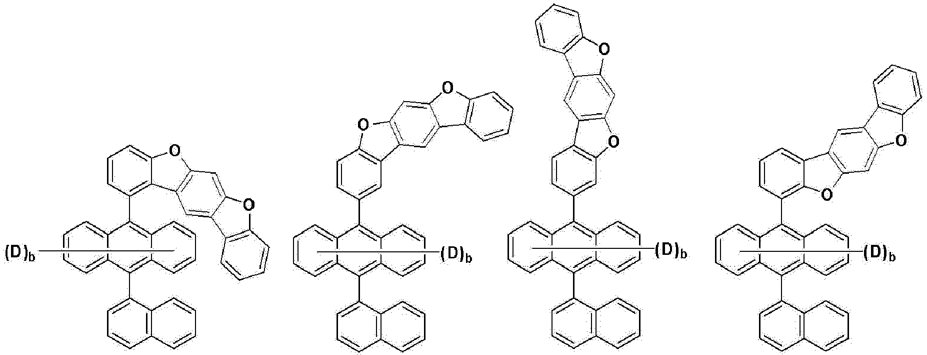

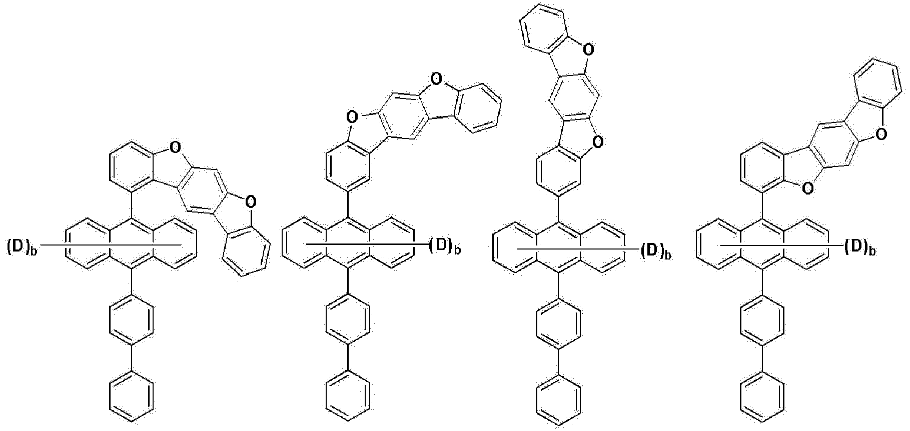

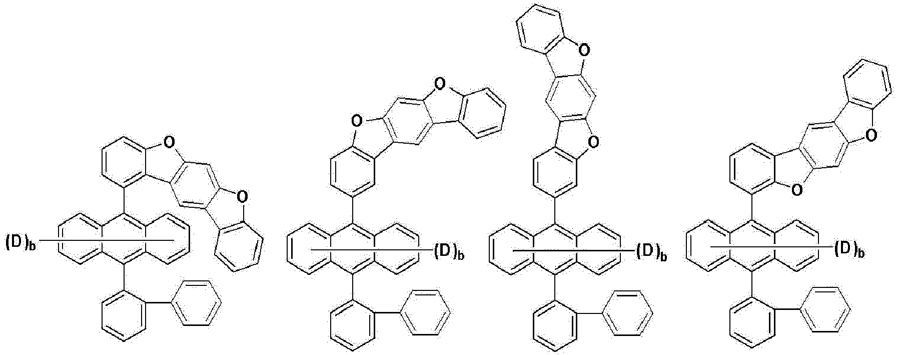

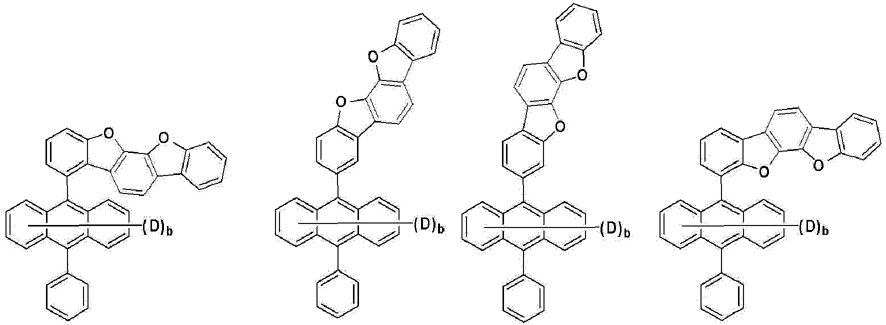

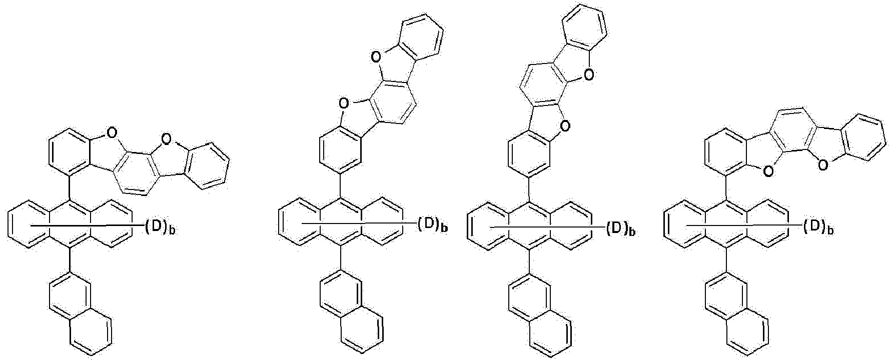

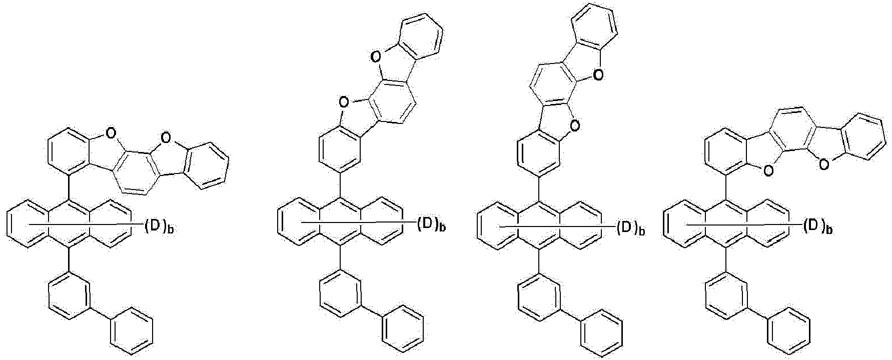

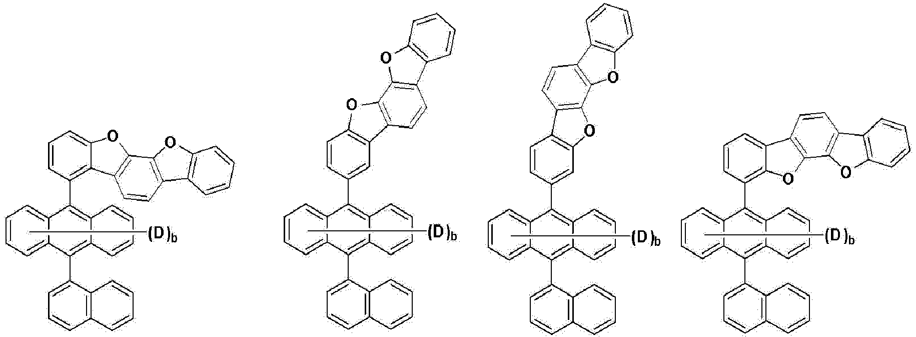

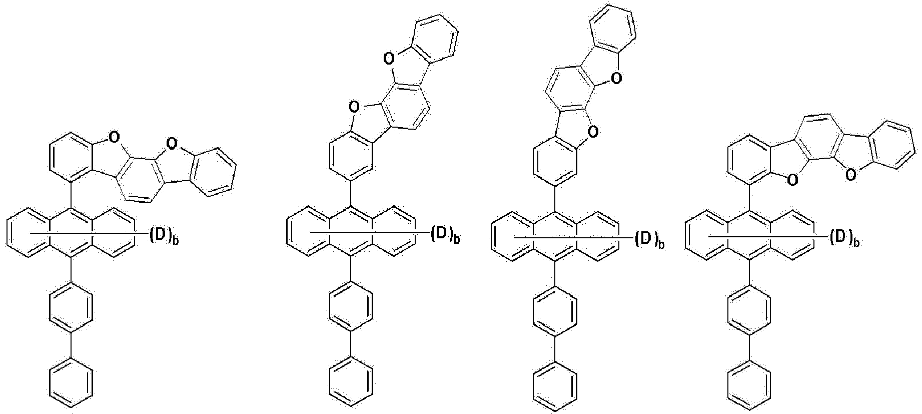

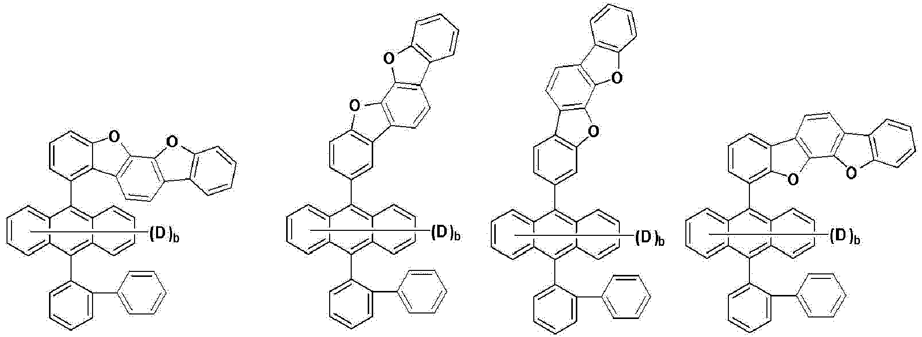

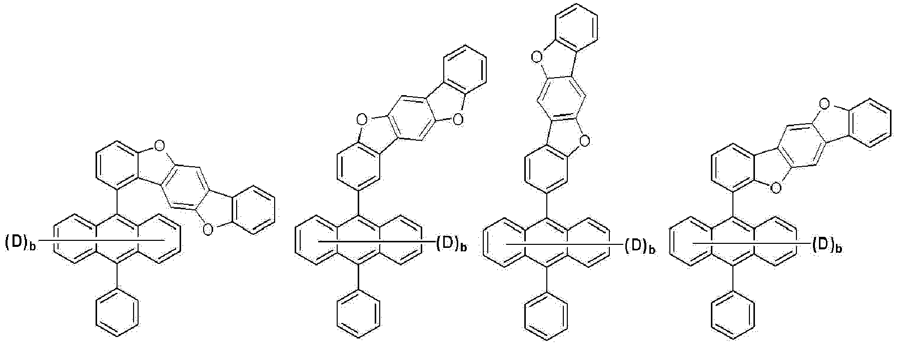

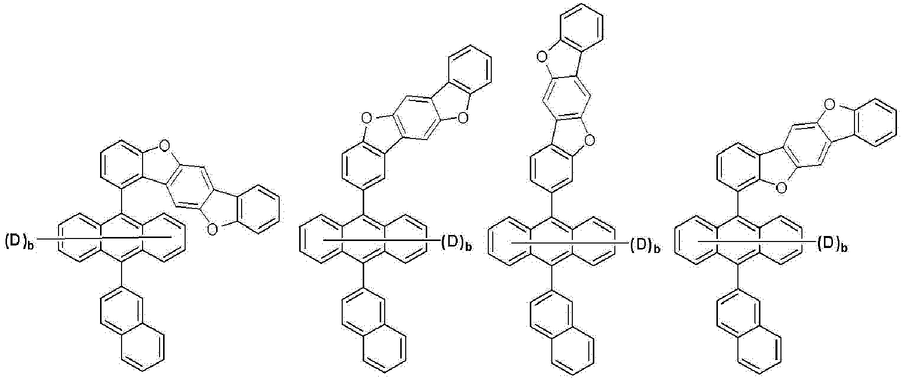

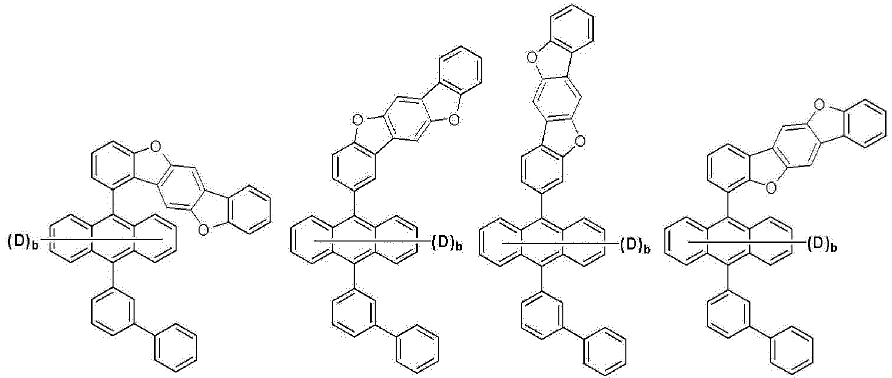

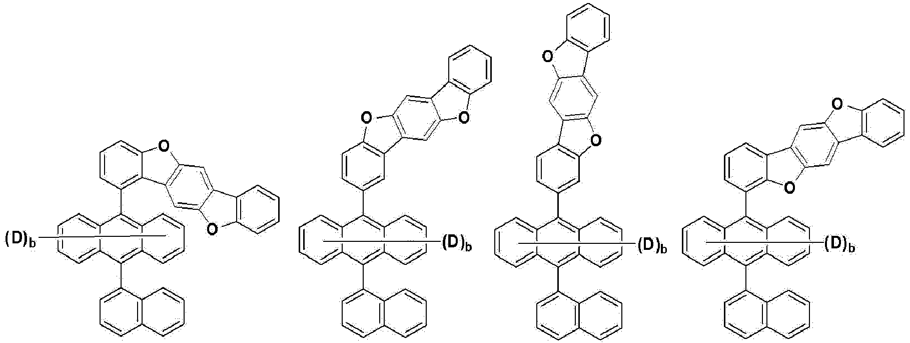

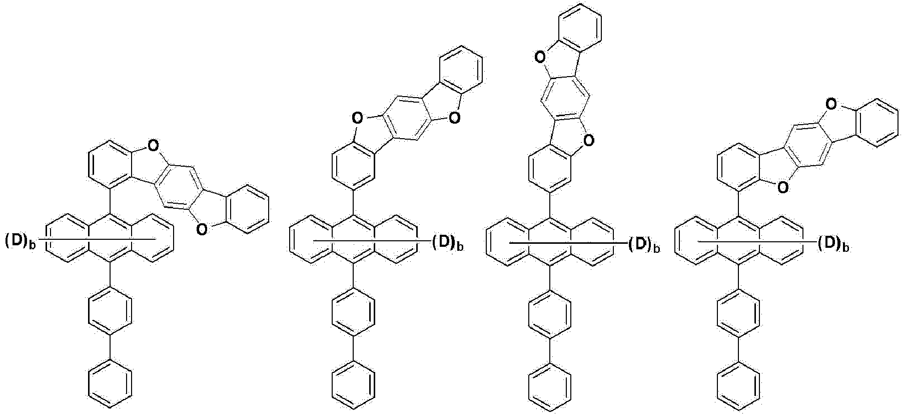

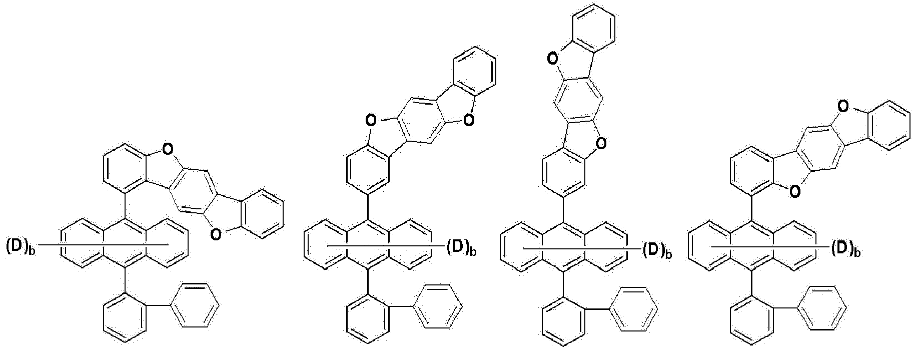

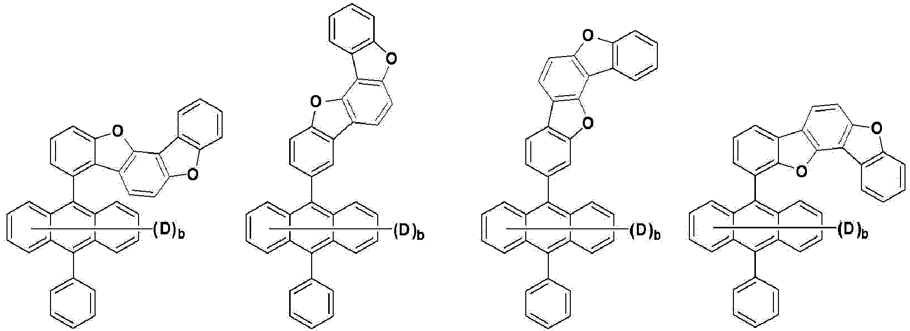

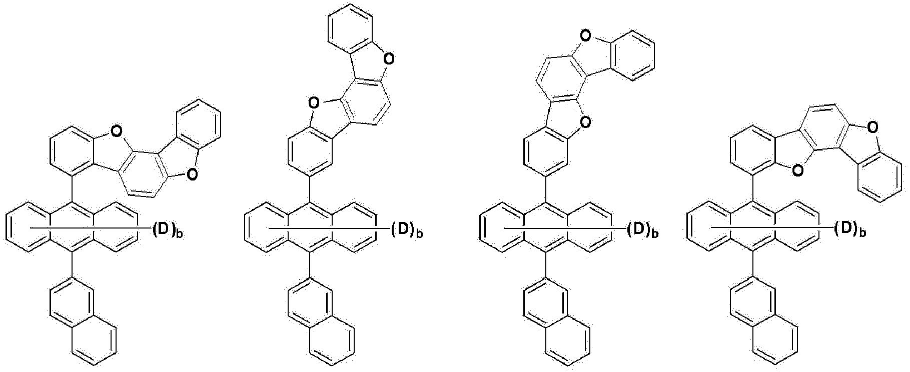

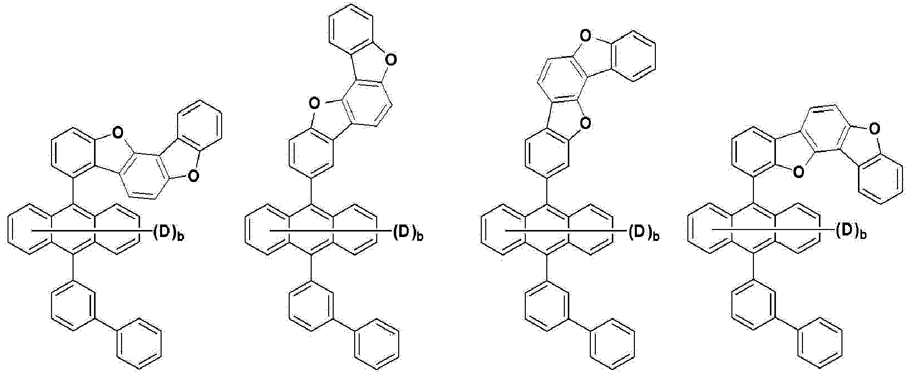

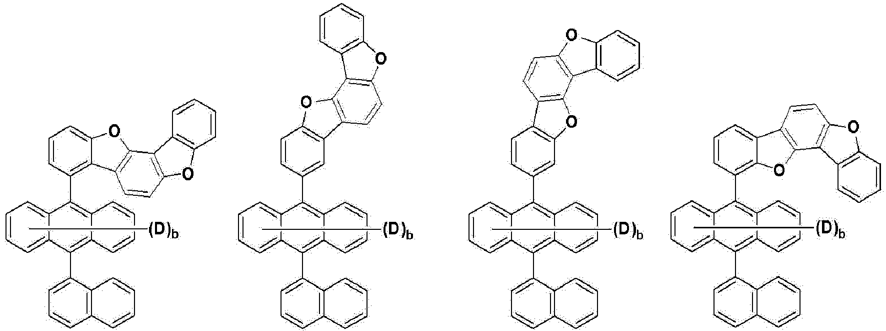

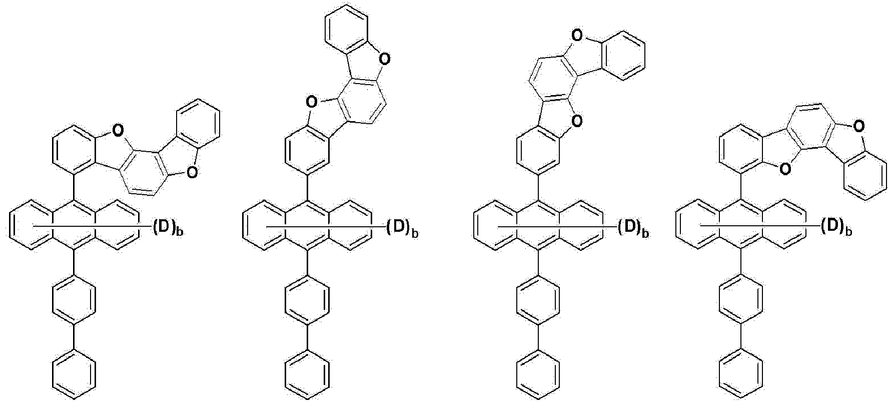

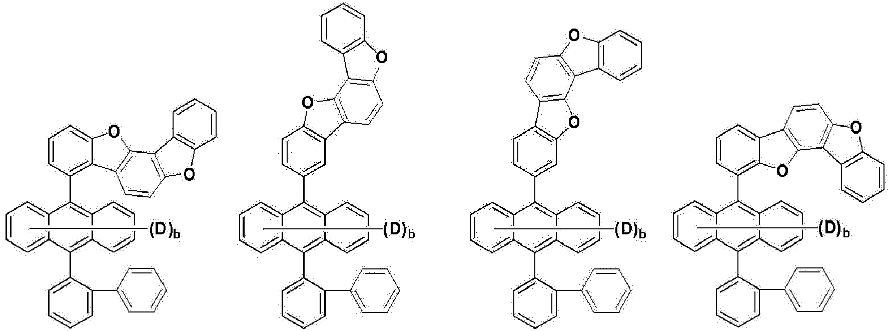

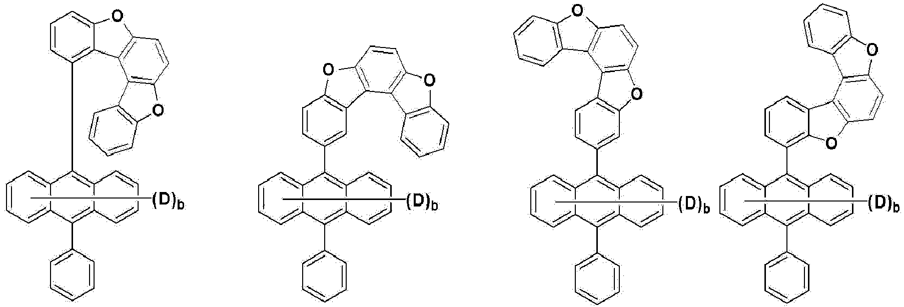

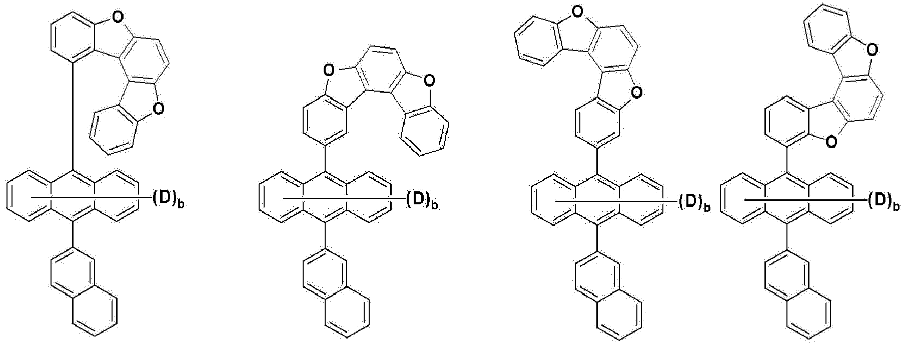

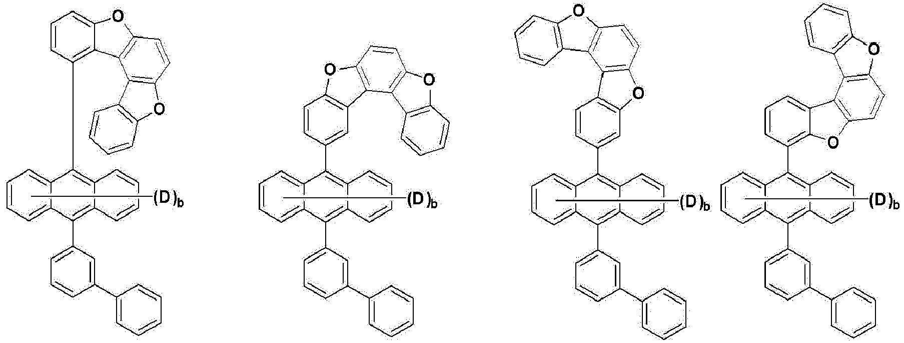

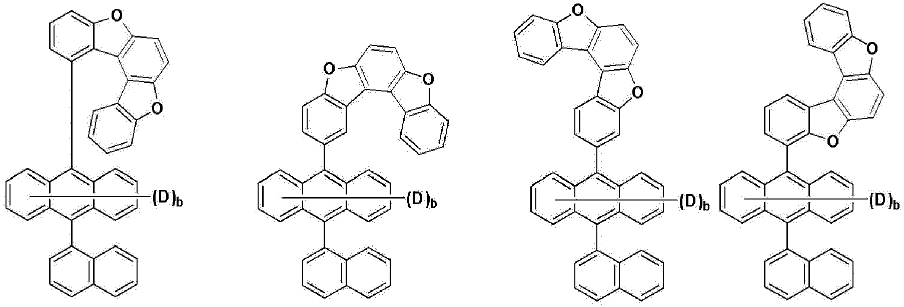

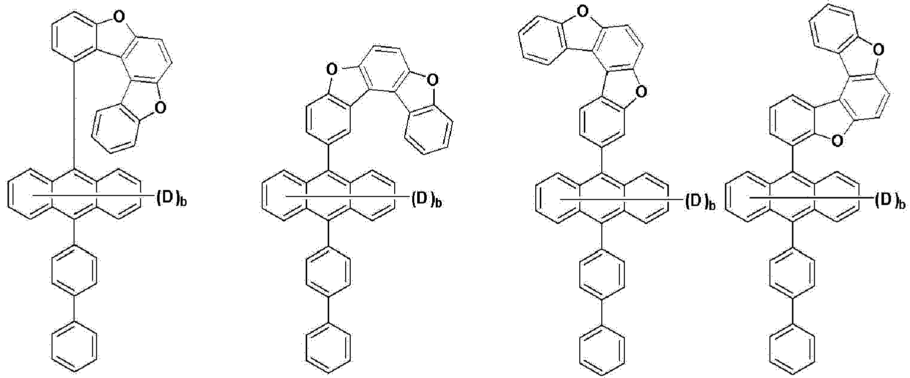

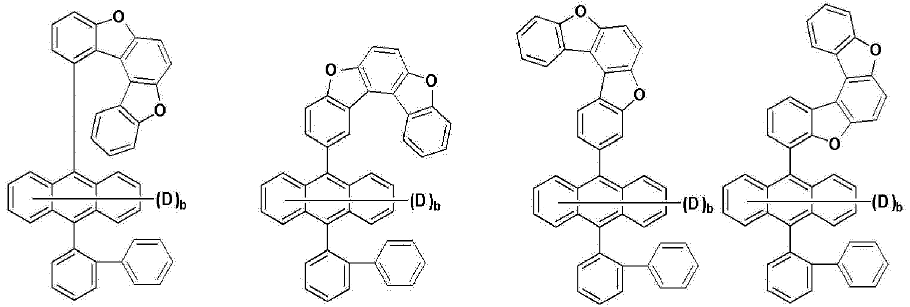

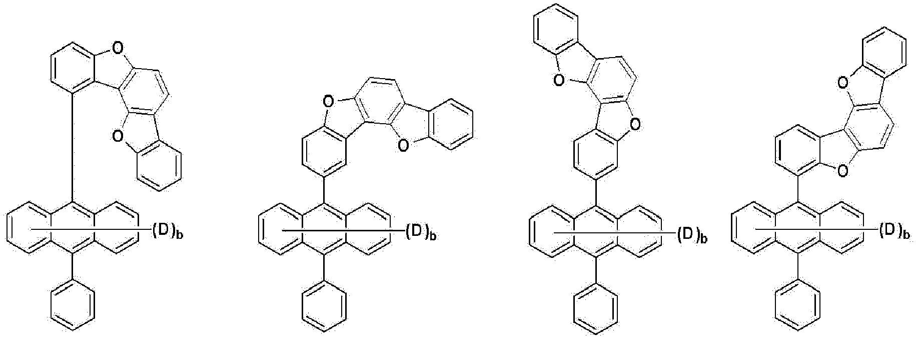

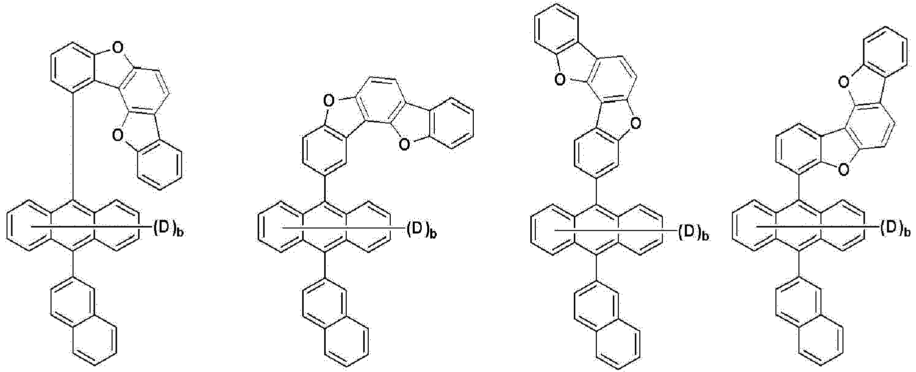

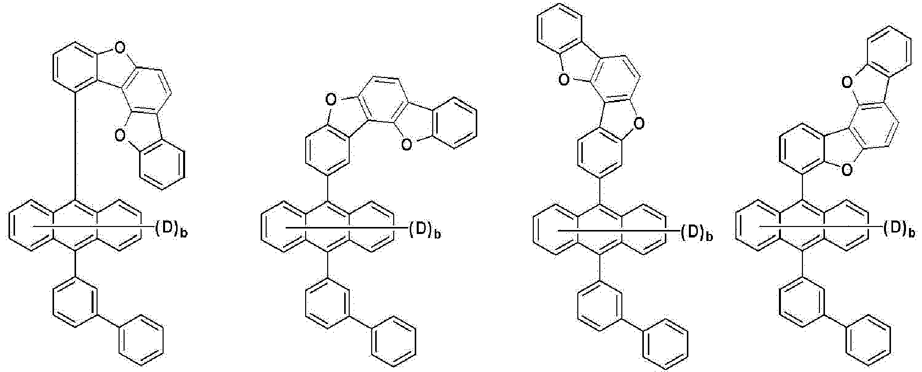

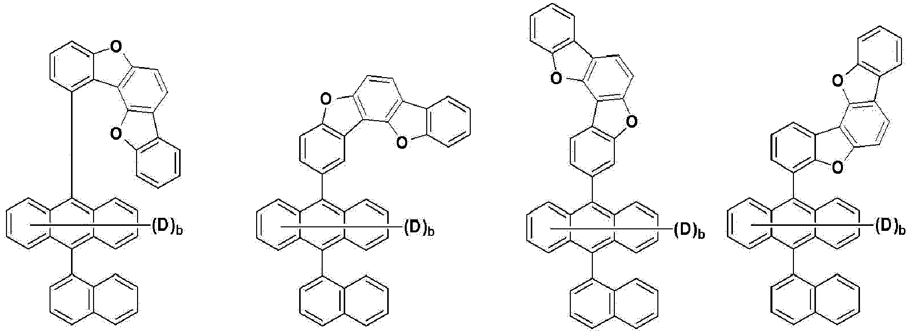

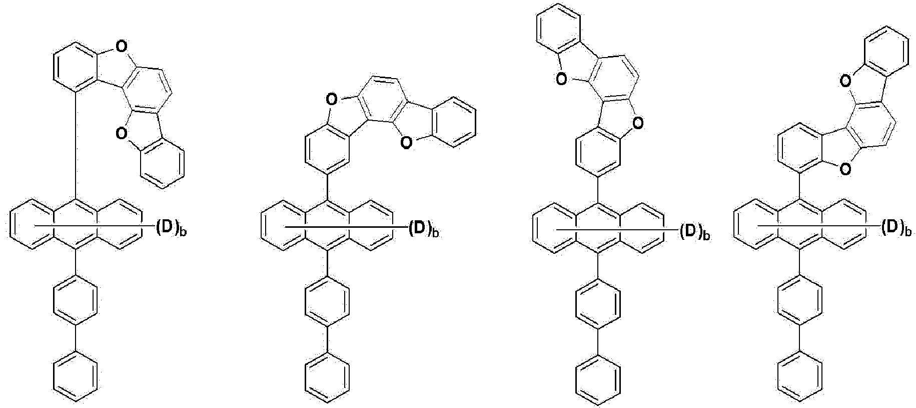

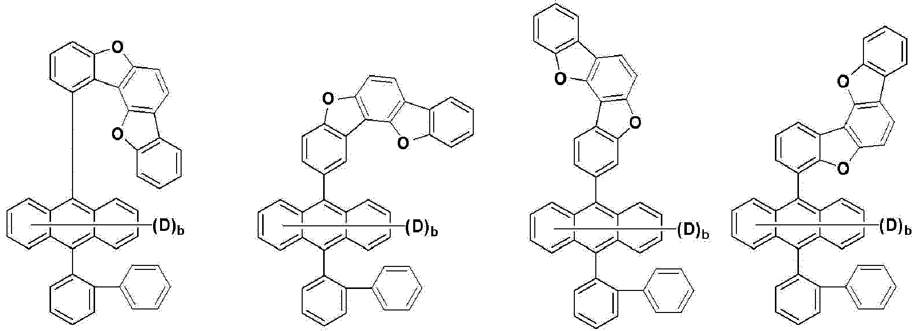

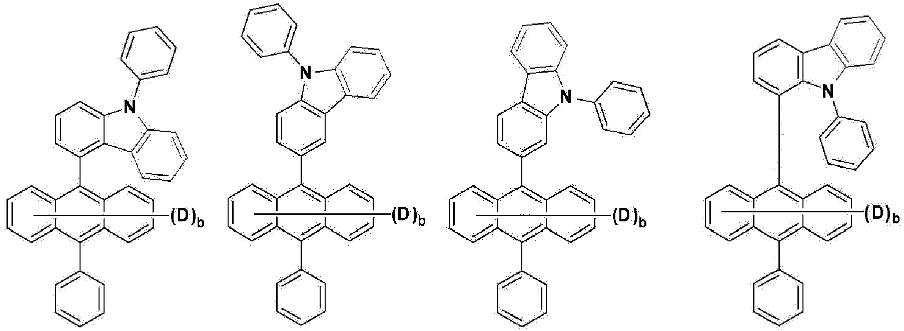

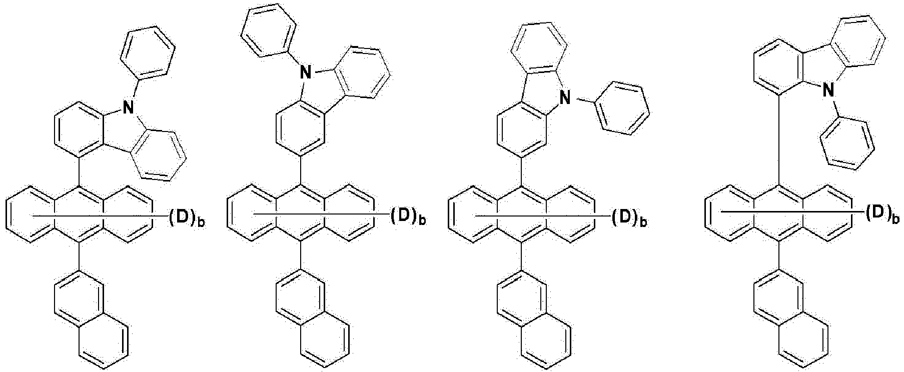

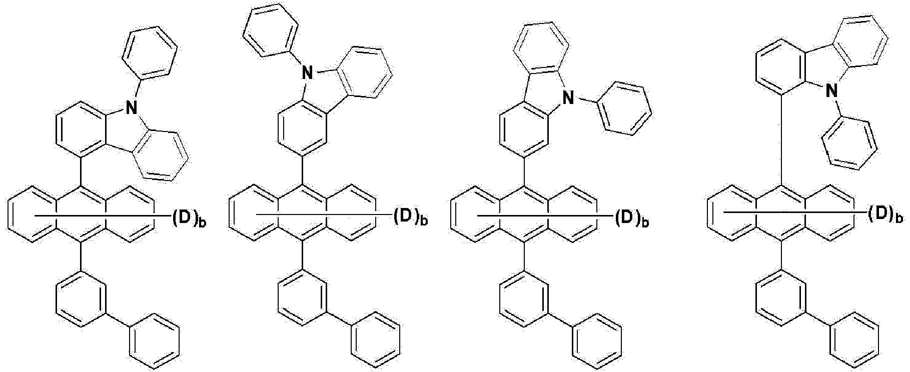

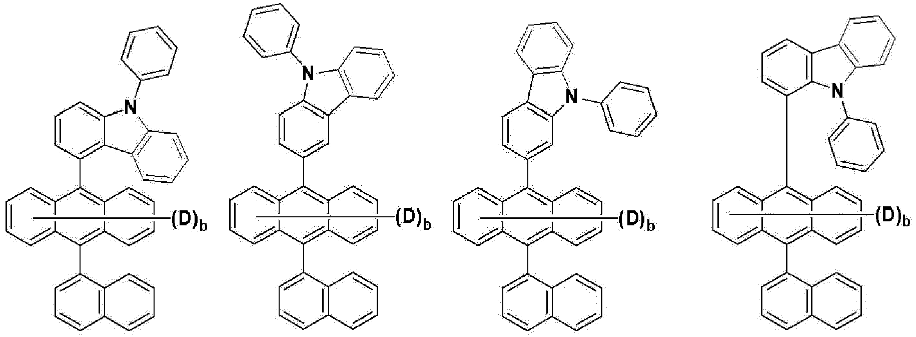

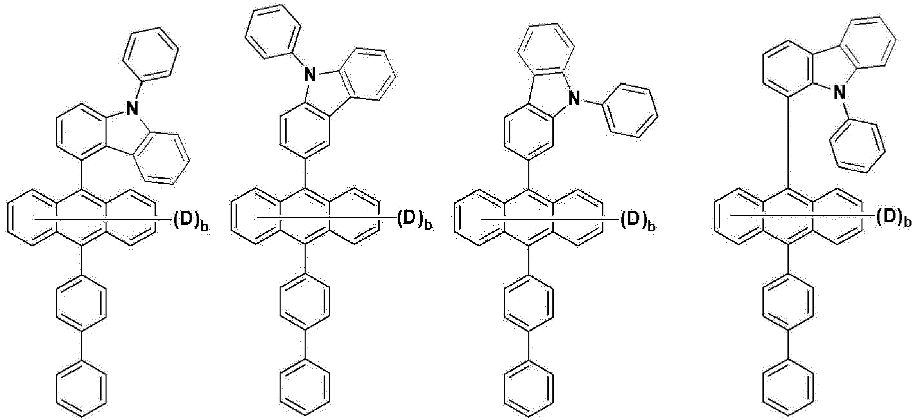

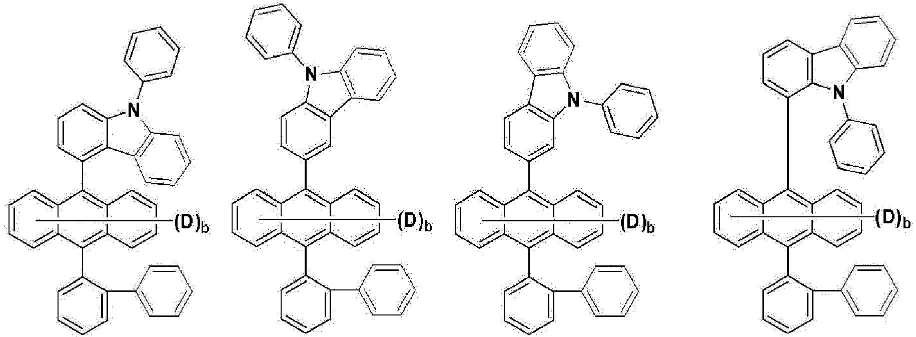

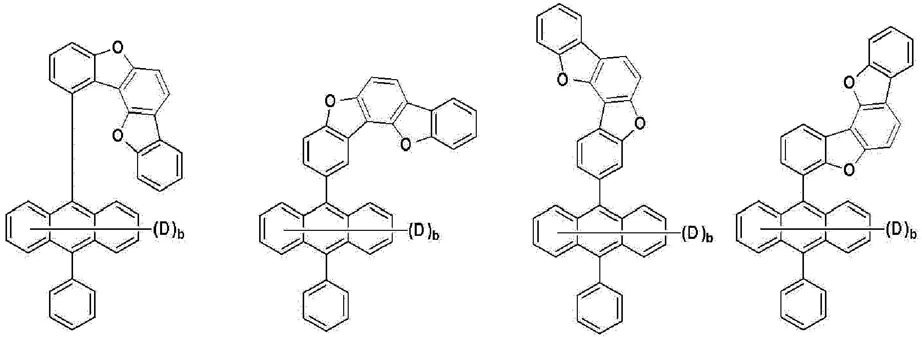

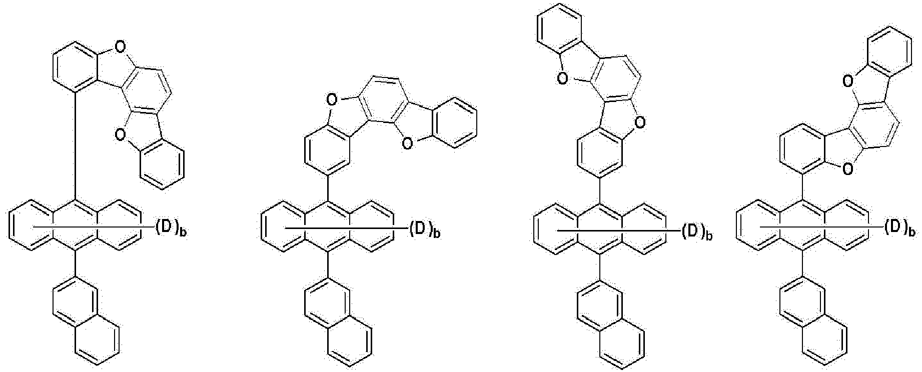

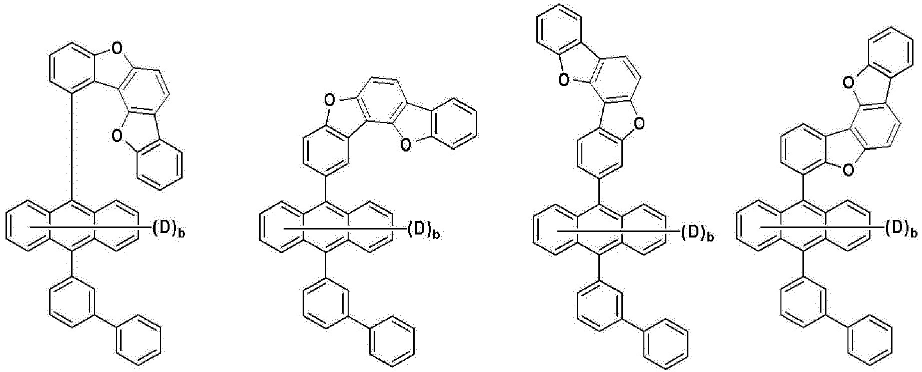

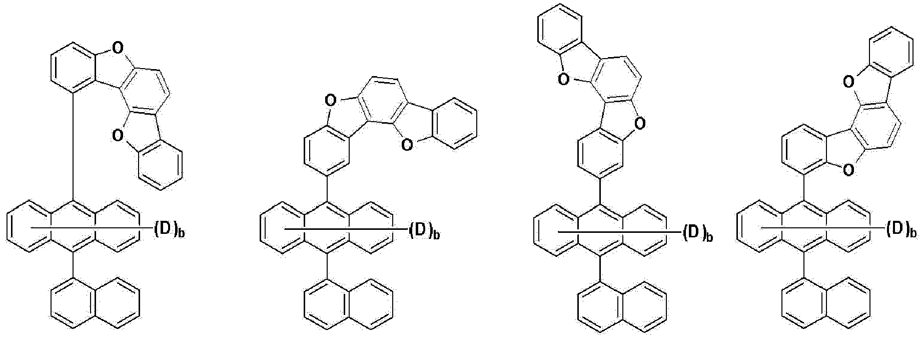

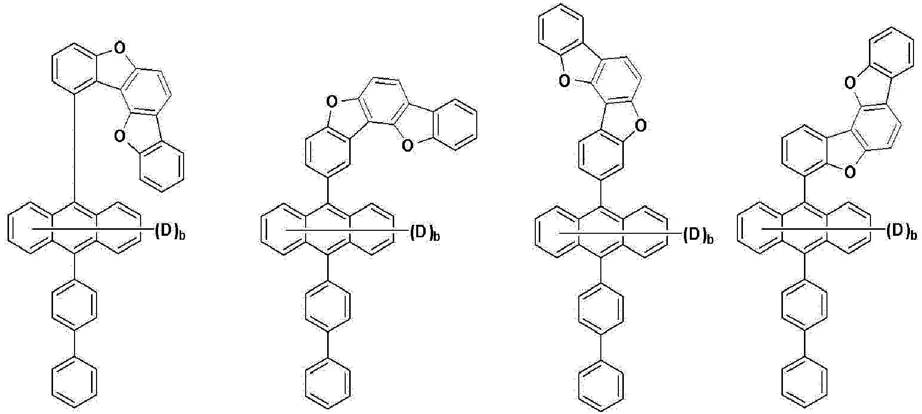

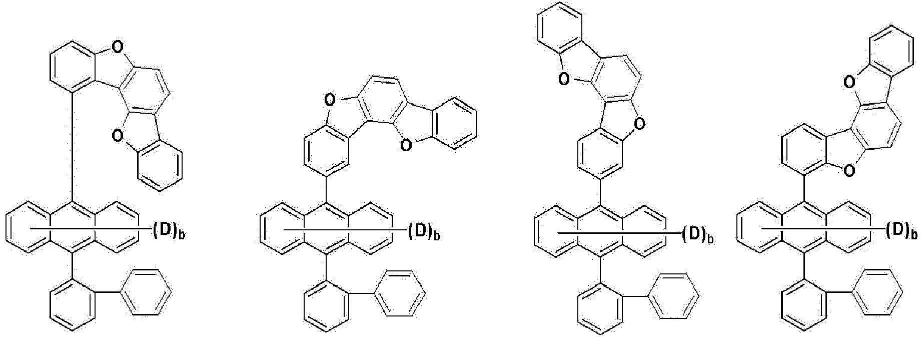

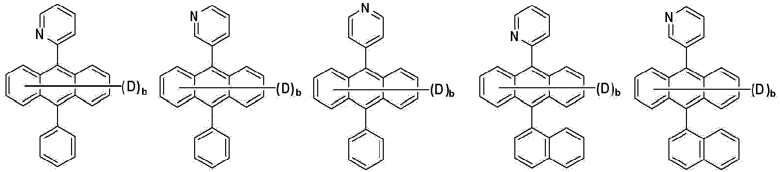

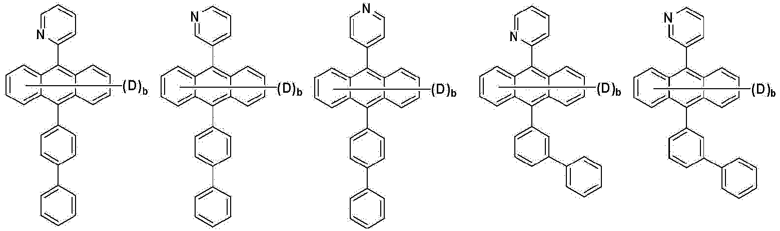

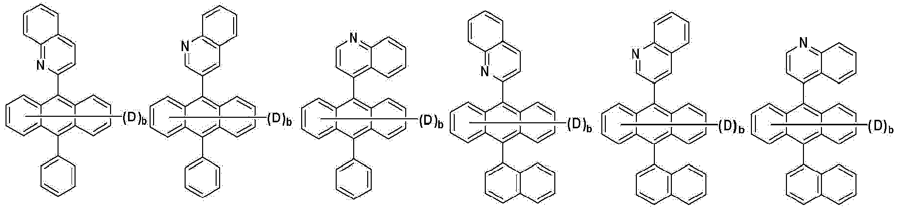

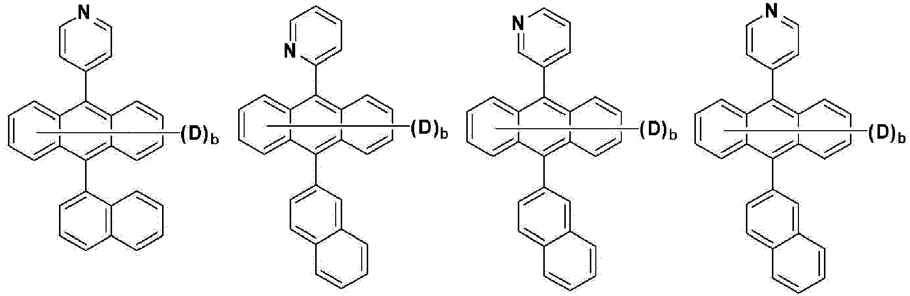

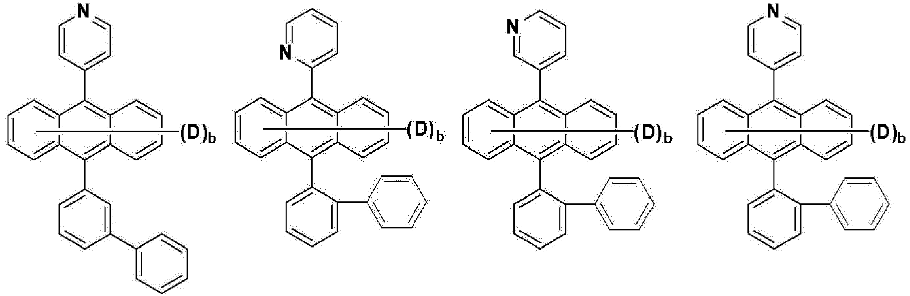

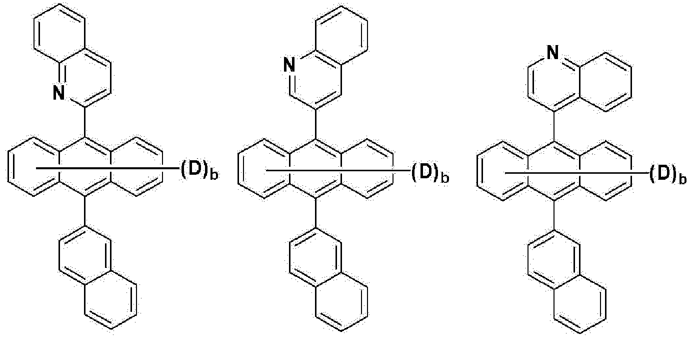

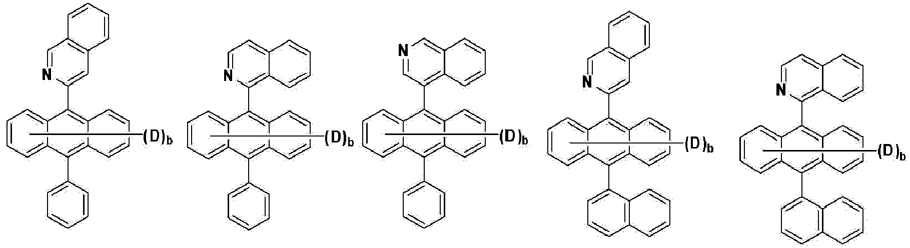

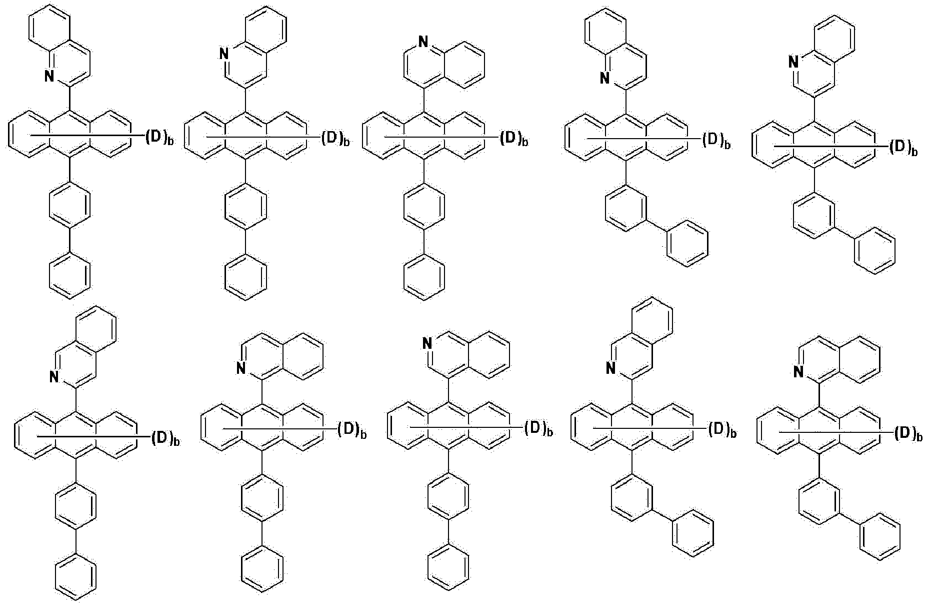

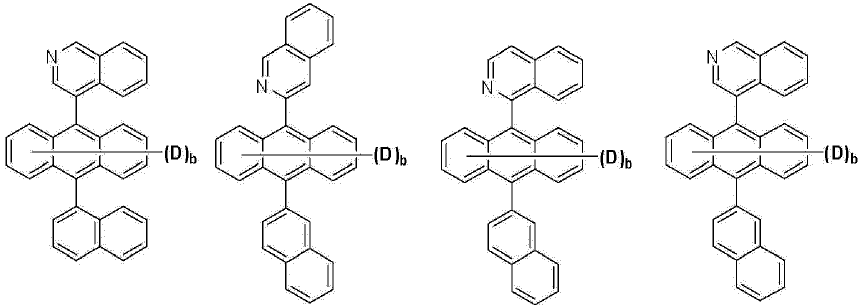

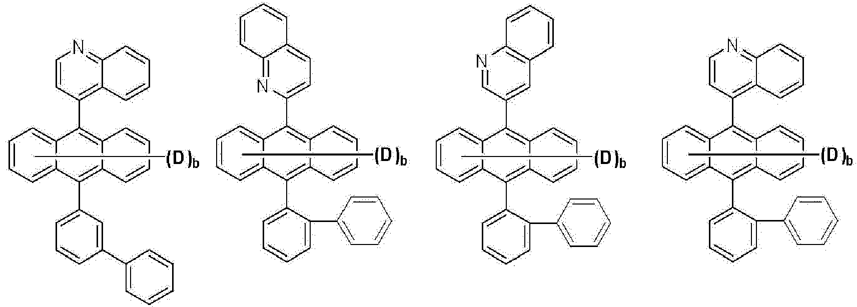

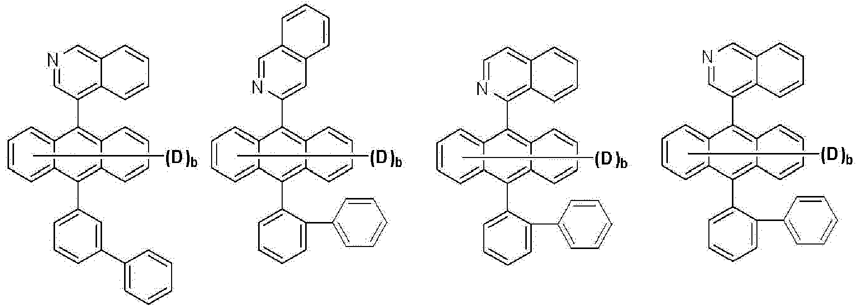

In the structures above, b is an integer from 1 to 8.

Theoretically, when all the hydrogens in the deuterated compound are substituted with deuterium, that is, when the deuterium substitution rate is 100%, the service life characteristics are most ideally improved. However, there are problems such as the need for extreme conditions due to steric hindrance and the destruction of compound before the compound is deuterated due to side reactions, and in reality, it is difficult to obtain all the hydrogen of a compound at a deuterium substitution rate of 100%, and even when a deuterated substitution rate of nearly 100% is obtained, the efficiency compared to investment is not good in consideration of process time, cost, and the like.

In the present specification, since a deuterated compound produced by a deuterated reaction and having one or more deuteriums is produced as a composition having two or more isotopes having different molecular weights depending on the number of substituted deuteriums, the position where deuterium is substituted in the structure will be omitted.

In the compound having the structure, at least one of the positions which are indicated by hydrogen or in which substituted hydrogen is omitted can be substituted with deuterium.

The present specification provides an electronic device including a deuterated anthracene compound prepared by the above-described preparation method.

The present specification provides an electronic device including the above-described deuterated anthracene compound.

The present specification provides a method for manufacturing an electronic device, the method including: manufacturing an electronic device using the above-described deuterated anthracene compound.

For the electronic device and the method for manufacturing an electronic device, an applicable description in the above-described description can be cited, and the repeated description will be omitted.

The electronic device is not particularly limited as long as the electronic device can use the above-described deuterated anthracene compound, and can be, for example, an organic light emitting device, an organic phosphorescent device, an organic solar cell, an organic photo conductor, an organic transistor, or the like.

The electronic device includes: a first electrode; a second electrode; and an organic material layer having one or more layers provided between the first electrode and the second electrode, and one or more layers of the organic material layer can include the above-described deuterated anthracene compound.

The present specification provides an organic light emitting device including the above-described deuterated anthracene compound.

The present specification provides an organic light emitting device including a deuterated anthracene compound prepared by the above-described preparation method.

In an exemplary embodiment of the present specification, the organic light emitting device includes: a first electrode; a second electrode; and an organic material layer provided between the first electrode and the second electrode, in which the organic material layer includes the deuterated anthracene compound.

In an exemplary embodiment of the present specification, the organic material layer includes a light emitting layer including the deuterated anthracene compound.

The organic material layer of the organic light emitting device of the present specification can also be composed of a single-layered structure, but can be composed of a multi-layered structure in which two or more organic material layers are stacked. For example, the organic material layer of the present specification can be composed of one to three layers. Further, the organic light emitting device of the present specification can have a structure including a hole injection layer, a hole transport layer, a light emitting layer, an electron transport layer, an electron injection layer, and the like as organic material layers. However, the structure of the organic light emitting device is not limited thereto, and can include a fewer number of organic layers.

When the organic light emitting device includes a plurality of organic material layers, the organic material layers can be formed of the same material or different materials.

For example, the organic light emitting device of the present specification can be manufactured by sequentially stacking a positive electrode, an organic material layer, and a negative electrode on a substrate. In this case, the organic light emitting device can be manufactured by depositing a metal or a metal oxide having conductivity, or an alloy thereof on a substrate to form a positive electrode, forming an organic material layer including a hole injection layer, a hole transport layer, a light emitting layer, and an electron transport layer thereon, and then depositing a material, which can be used as a negative electrode, thereon, by using a physical vapor deposition (PVD) method such as sputtering or e-beam evaporation. In addition to the method described above, an organic light emitting device can be made by sequentially depositing a negative electrode material, an organic material layer, and a positive electrode material on a substrate.

In addition, the material in each layer can be formed as an organic material layer by not only a vacuum deposition method, but also a solution application method when an organic light emitting device is manufactured. Here, the solution application method means spin coating, dip coating, doctor blading, inkjet printing, screen printing, a spray method, roll coating, and the like, but is not limited thereto.

In an exemplary embodiment of the present specification, the first electrode is a positive electrode, and the second electrode is a negative electrode.

According to another exemplary embodiment, the first electrode is a negative electrode, and the second electrode is a positive electrode.

In another exemplary embodiment, the organic light emitting device can be a normal type organic light emitting device in which a positive electrode, an organic material layer having one or more layers, and a negative electrode are sequentially stacked on a substrate.

In still another exemplary embodiment, the organic light emitting device can be an inverted type organic light emitting device in which a negative electrode, an organic material layer having one or more layers, and a positive electrode are sequentially stacked on a substrate.

In the present specification, the materials for the negative electrode, the organic material layer and the positive electrode are not particularly limited except for including an anthracene compound deuterated in at least one layer of the organic material layer, and a material known in the art can be used.

In the present specification, the above-described deuterated anthracene compound can be used by a principle which is similar to the principle applied to an organic light emitting device, even in an electronic device including an organic phosphorescent device, an organic solar cell, an organic photo conductor, an organic transistor, and the like. For example, the organic solar cell can have a structure including a negative electrode, a positive electrode, and a photoactive layer provided between the negative electrode and the positive electrode, and the photoactive layer can include the selected deuterated anthracene compound.

### Mode for Invention

Hereinafter, the present specification will be described in more detail through Examples. However, the following Examples are provided only for exemplifying the present specification, but are not intended to limit the present specification. Throughout the example, (D)_{b}, (D)_{c}, (D)_{d}, (D)ₑ, (D)_{f} and (D)_{g} mean the number of deuteriums to be substituted, respectively, and since the individual compounds of acquired compounds and obtained compounds can have different numbers and positions of deuterium substitutions according to the deuterium conversion rate of each reaction, b to g, which are the numbers of deuterium substitutions, and substitution positions will not be specified. In this case, b is an integer from 1 to 8, c is an integer from 1 to 6, d is an integer from 1 to 5, e is an integer from 1 to 4, f is an integer from 1 to 7, and g is an integer from 1 to 7.

### [Measurement of deuterium conversion rate and anthracene deuterium conversion rate]

In the present specification, a deuterium conversion rate and an anthracene deuterium conversion rate were measured by nuclear magnetic resonance (NMR) analysis. The detailed NMR analysis method is as follows.

After N-NMR analysis of a compound which was not substituted with deuterium, H-NMR analysis was performed by mixing a material having the same structure as the compound analyzed by N-NMR, in which hydrogen was substituted with deuterium, with an internal standard material. In this case, when hydrogen is 100% substituted with deuterium, no hydrogen peak at a position substituted with deuterium can be observed in the H-NMR, so that through the H-NMR analysis results of products that are not substituted with deuterium and the H-NMR analysis results of deuterium substitution materials, the deuterium substitution rate at each position can be measured.

However, in Preparation Examples 1 to 12 to be described below, the hydrogen position of anthracene and the hydrogen position of the substituent in H-NMR can overlap each other depending on the substituent. In the present specification, when a substituent was introduced into 9-bromo-10-(1-naphthyl)anthracene-d8, the anthracene deuterium substitution rate was replaced with the deuterium substitution rate of 9-bromo-10-(1-naphthyl)anthracene-d8.

### [Preparation Example 1] Synthesis of bromobenzene-d5

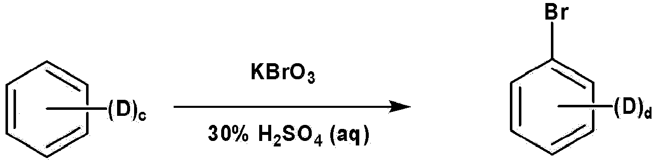

After benzene-d6 [deuterium conversion rate 99%] (100g, 1.18 mol) was put into 30 wt% H₂SO₄ (240 ml, 7.34 mol), the resulting mixture was stirred at 40°C. After KBrO₃ was added thereto in 5 batches, reaction monitoring was performed using high performance liquid chromatography (HPLC). After the reaction was completed, 1-bromobenzene-2,3,4,5,6-d5 (140 g, 73%, deuterium conversion rate 99%) was obtained by performing layer separation.

### [Preparation Example 2] Synthesis of 9-aryl substituted anthracene

### A. Synthesis of 9-(naphthalen-1-yl)anthracene-1,2,3,4,5,6,7,8-d8

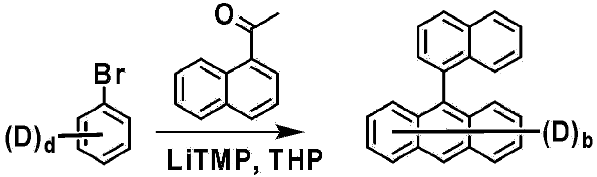

After 2,2,6,6-tetramethylpiperidine (TMP) (26.2 g, 0.185 mol) was put into anhydrous tetrahydropyran (THP) (100 mL) under nitrogen, the resulting mixture was stirred at 0°C. 1-(naphthalen-1-yl)ethan-1-one (10.52 g, 0.062 mol), 2.5 M n-BuLi (98.7 mL, 0.247 mol) and bromobenzene-d5 (20 g, 0.123 mol) were sequentially added thereto while maintaining the temperature, and the resulting mixture was refluxed by increasing the temperature. After 30 minutes, the mixture was cooled, and then quenched by adding 100 mL of water thereto, and then the aqueous layer was removed with a separatory funnel. Moisture was removed from the organic layer using MgSO₄ and the organic layer was filtered through a silica pad. The filtrate was purified by column chromatography to obtain 9-(naphthalen-1-yl)anthracene-1,2,3,4,5,6,7,8-d8 (7.8 g, 0.025 mol, yield 40%, and deuterium conversion rate of anthracene 99%).

### B. Synthesis of 9-(naphthalen-2-yl)anthracene-1,2,3,4,5,6,7,8-d8

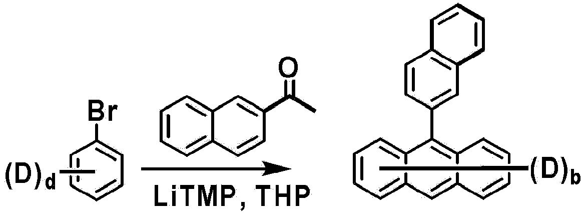

After 2,2,6,6-tetramethylpiperidine (26.2 g, 0.185 mol) was put into anhydrous tetrahydropyran (100 mL) under nitrogen, the resulting mixture was stirred at 0°C. 1-(naphthalen-2-yl)ethan-1-one (10.52 g, 0.062 mol), 2.5 M *n*-BuLi (98.7 mL, 0.247 mol) and bromobenzene-d5 (20 g, 0.123 mol) were sequentially added thereto while maintaining the temperature, and the resulting mixture was refluxed by increasing the temperature. After 30 minutes, the mixture was cooled, and then quenched by adding 100 mL of water thereto, and then the aqueous layer was removed with a separatory funnel. Moisture was removed from the organic layer using MgSO₄ and the organic layer was filtered through a silica pad. The filtrate was purified by column chromatography to obtain 9-(naphthalen-2-yl)anthracene-1,2,3,4,5,6,7,8-d8 (7.8 g, 0.025 mol, yield 40%, and deuterium conversion rate of anthracene 99%).

### C. Synthesis of 2-(anthracen-9-yl-1,2,3,4,5,6,7,8-d8)dibenzo[b,d]furan

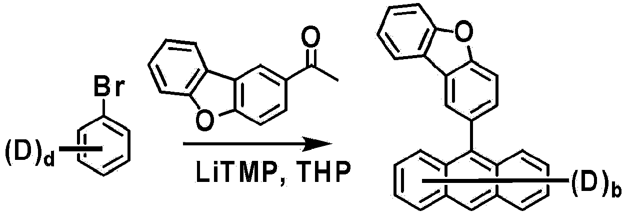

After 2,2,6,6-tetramethylpiperidine (26.2 g, 0.185 mol) was put into anhydrous tetrahydropyran (100 mL) under nitrogen, the resulting mixture was stirred at 0°C. 1-(dibenzo[b,d]furan-2-yl)ethan-1-one (13.02 g, 0.062 mol), 2.5 M *n*-BuLi (98.7 mL, 0.247 mol) and bromobenzene-d5 (20 g, 0.123 mol) were sequentially added thereto while maintaining the temperature, and the resulting mixture was refluxed by increasing the temperature. After 30 minutes, the mixture was cooled, and then quenched by adding 100 mL of water thereto, and then the aqueous layer was removed with a separatory funnel. Moisture was removed from the organic layer using MgSO₄ and the organic layer was filtered through a silica pad. The filtrate was purified by column chromatography to obtain 2-(anthracen-9-yl-1,2,3,4,5,6,7,8-d8)dibenzo[b,d]furan (8.09 g, 0.023 mol, yield 37%, and deuterium conversion rate of anthracene 99%) .

### D. Synthesis of 4-(anthracen-9-yl-1,2,3,4,5,6,7,8-d8)dibenzo[b,d]furan

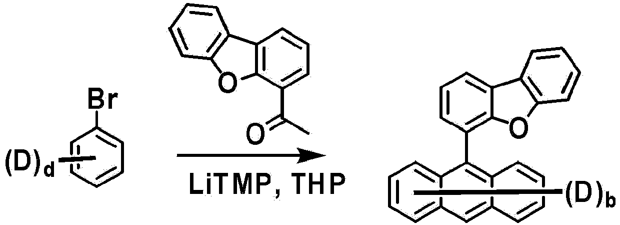

After 2,2,6,6-tetramethylpiperidine (26.2 g, 0.185 mol) was put into anhydrous tetrahydropyran (100 mL) under nitrogen, the resulting mixture was stirred at 0°C. 1-(dibenzo[b,d]furan-4-yl)ethan-1-one (10.52 g, 0.062 mol), 2.5 M *n*-BuLi (98.7 mL, 0.247 mol) and bromobenzene-d5 (20 g, 0.123 mol) were sequentially added thereto while maintaining the temperature, and the resulting mixture was refluxed by increasing the temperature. After 30 minutes, the mixture was cooled, and then quenched by adding 100 mL of water thereto, and then the aqueous layer was removed with a separatory funnel. Moisture was removed from the organic layer using MgSO₄ and the organic layer was filtered through a silica pad. The filtrate was purified by column chromatography to obtain 4-(anthracen-9-yl-1,2,3,4,5,6,7,8-d8)dibenzo[b,d]furan (8.09 g, 0.023 mol, yield 37%, and deuterium conversion rate of anthracene 99%) .

### E. Synthesis of 1-(anthracen-9-yl-1,2,3,4,5,6,7,8-d8)-9-phenyl-9H-carbazole

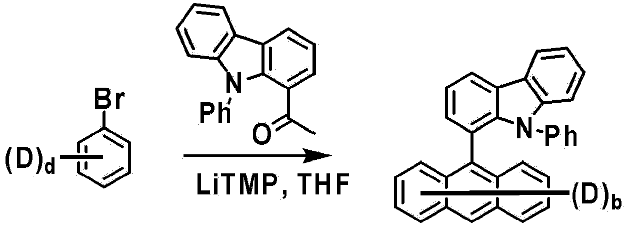

After 2,2,6,6-tetramethylpiperidine (26.2 g, 0.185 mol) was put into anhydrous tetrahydropyran (100 mL) under nitrogen, the resulting mixture was stirred at 0°C. 1-(9-phenyl-9H-carbazol-1-yl)ethan-1-one (17.67 g, 0.062 mol), 2.5 M *n*-BuLi (98.7 mL, 0.247 mol) and bromobenzene-d5 (20 g, 0.123 mol) were sequentially added thereto while maintaining the temperature, and the resulting mixture was refluxed by increasing the temperature. After 30 minutes, the mixture was cooled, and then quenched by adding 100 mL of water thereto, and then the aqueous layer was removed with a separatory funnel. Moisture was removed from the organic layer using MgSO₄ and the organic layer was filtered through a silica pad. The filtrate was purified by column chromatography to obtain 1-(anthracen-9-yl-1,2,3,4,5,6,7,8-d8)-9-phenyl-9H-carbazole (9.8 g, 0.023 mol, yield 37%, and deuterium conversion rate of anthracene 99%).

### [Preparation Example 3] Synthesis of 9-aryl-10-Bromoanthracene

### A. Synthesis of 9-bromo-10-(1-naphthyl)anthracene-d8

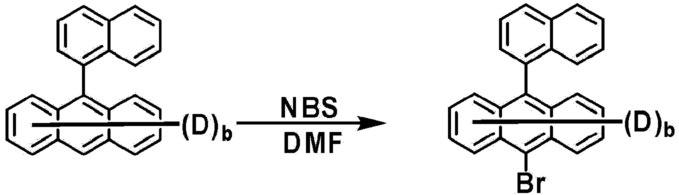

While 9-(1-naphthyl)anthracene (5 g, 0.016 mol) and N,N-dimethylformamide (DMF) (30 mL) were stirred, N-bromosuccinimide (NBS) (2.8 g, 0.016 mol) was added thereto. After 5 hours, obtained crystals were filtered by adding water (100 mL) thereto. After the filtered solid was dissolved in chloroform (50 mL), the resulting solution was washed with water (15 mL), dried using MgSO₄, and then filtered. After the filtrate was distilled under reduced pressure, the residue was purified by column chromatography to obtain 9-bromo-10-(1-naphthyl)anthracene (5.64 g, yield 90%, deuterium conversion rate of anthracene 99%, and m/s (M+H) 320).

### [Preparation Example 4] Synthesis of BH1

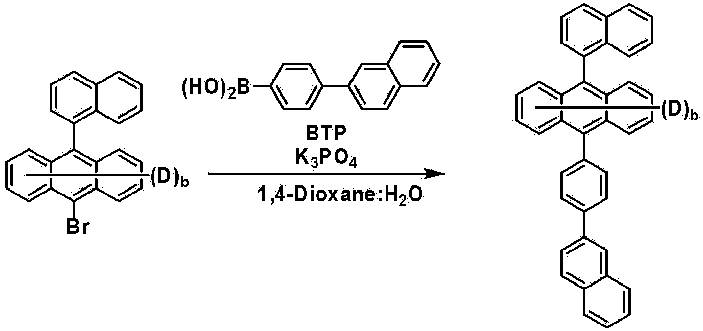

After 9-bromo-10-(1-naphthyl)anthracene-d8 (10 g, 0.026 mol), (4-(naphthalen-2-yl)phenyl)boronic acid (7.6 g, 0.031 mol), 1,4-dioxane (200 mL), potassium phosphate (10.8 g, 0.052 mol), H₂O (32.4 mL) and bis(tri-tert-butylphosphine)palladium(0) (BTP) (0.13 g, 0.0026 mol) were put into a round bottom flask, the resulting mixture was refluxed by heating. After 5 hours, the reaction solution was cooled, and then the aqueous layer was removed by a separatory funnel. The organic layer was dried using MgSO₄, and then filtered through a silica pad. After the filtrate was distilled under reduced pressure, the residue was purified by column chromatography to obtain BH1 (8.5 g, yield 65%, deuterium conversion rate of anthracene 99%, and m/s (M+H) 515).

### [Preparation Example 5] Synthesis of BH2

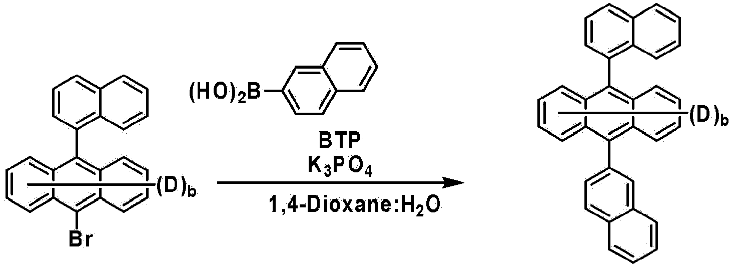

BH2 was synthesized in the same manner as in Preparation Example 4, except that a reactant having a different structure was used in preparation Example 5. (obtained amount 8.1 g, yield 73%, deuterium conversion rate of anthracene 99%, and m/s (M+H) 439)

### [Preparation Example 6] Synthesis of BH3

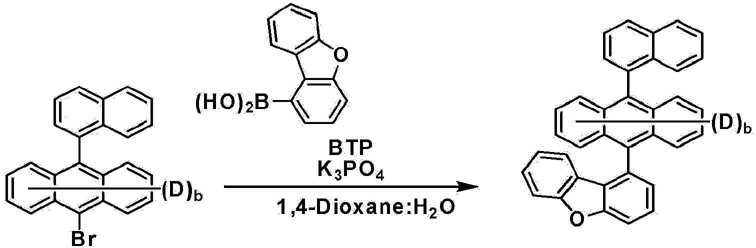

BH3 was synthesized in the same manner as in Preparation Example 4, except that a reactant having a different structure was used in Preparation Example 6. (obtained amount 9.3 g, yield 70%, deuterium conversion rate of anthracene 99%, and m/s (M+H) 479)

### [Preparation Example 7] Synthesis of BH4

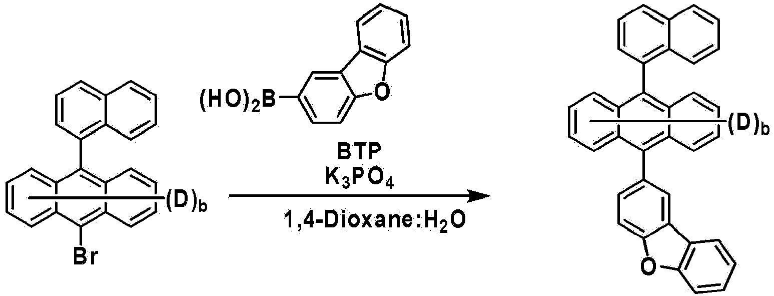

BH4 was synthesized in the same manner as in Preparation Example 4, except that a reactant having a different structure was used in Preparation Example 7. (obtained amount 9.1 g, yield 75%, deuterium conversion rate of anthracene 99%, and m/s (M+H) 479)

### [Preparation Example 8] Synthesis of BH5

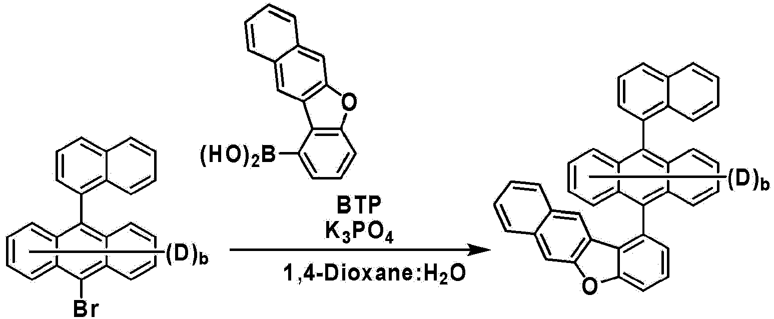

BH5 was synthesized in the same manner as in Preparation Example 4, except that a reactant having a different structure was used in Preparation Example 8. (obtained amount 8.7 g, yield 65%, deuterium conversion rate of anthracene 99%, and m/s (M+H) 529)

### [Preparation Example 9] Synthesis of BH6

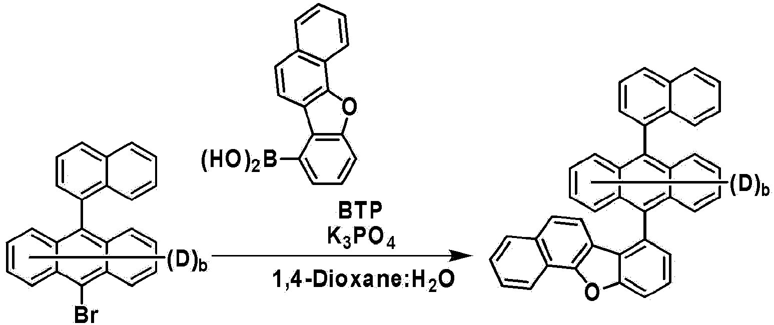

BH6 was synthesized in the same manner as in Preparation Example 4, except that a reactant having a different structure was used in Preparation Example 9. (obtained amount 9.4 g, yield 70%, deuterium conversion rate of anthracene 99%, and m/s (M+H) 529)

### [Preparation Example 10] Synthesis of BH7

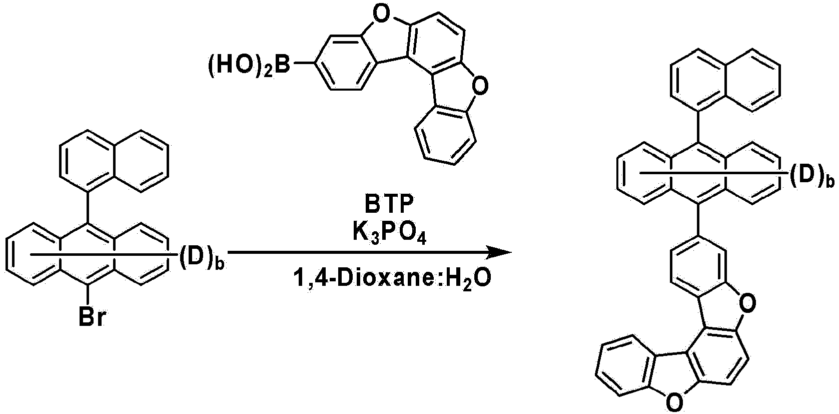

BH7 was synthesized in the same manner as in Preparation Example 4, except that a reactant having a different structure was used in Preparation Example 10. (obtained amount 11.5 g, yield 80%, deuterium conversion rate of anthracene 99%, and m/s (M+H) 569)

### [Preparation Example 11] Synthesis of BH8

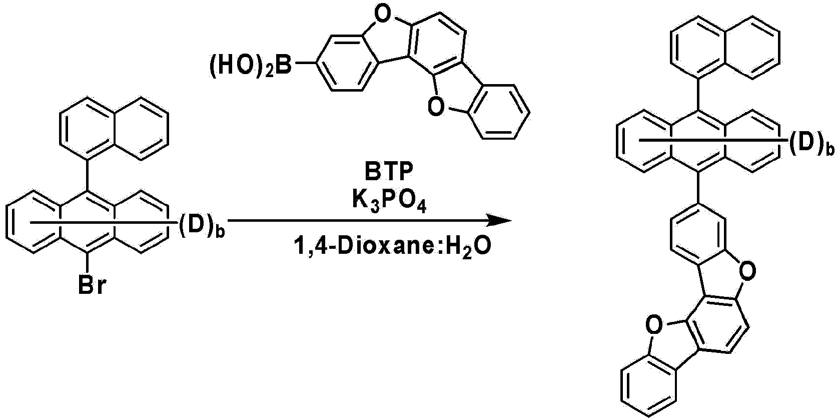

BH8 was synthesized in the same manner as in Preparation Example 4, except that a reactant having a different structure was used in Preparation Example 11. (obtained amount 10.1 g, yield 70%, deuterium conversion rate of anthracene 99%, and m/s (M+H) 569)

### [Preparation Example 12] Synthesis of BH9

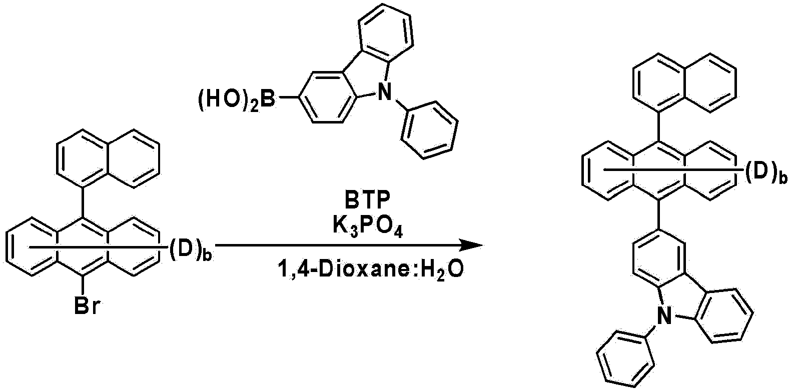

BH9 was synthesized in the same manner as in Preparation Example 4, except that a reactant having a different structure was used in Preparation Example 12. (obtained amount 8.9 g, yield 65%, deuterium conversion rate of anthracene 99%, and m/s (M+H) 534)

### [Comparative Synthesis Example 1] Synthesis of BH10

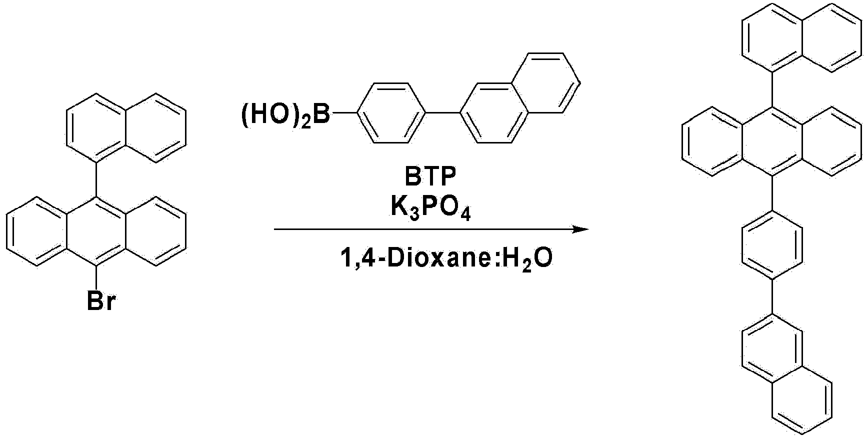

After 9-bromo-10-(1-naphthyl)anthracene (10 g, 0.026 mol), (4-(naphthalen-2-yl)phenyl)boronic acid (7.6 g, 0.031 mol), 1,4-dioxane (200 mL), potassium phosphate (10.8 g, 0.052 mol), H₂O (32.4 mL), and bis(tri-tert-butylphosphine)palladium(0) (0.13 g, 0.0026 mol) were put into a round bottom flask, the resulting mixture was refluxed by heating. After 5 hours, the reaction solution was cooled, and then the aqueous layer was removed by a separatory funnel. The organic layer was dried using MgSO₄, and then filtered through a silica pad. After the filtrate was distilled under reduced pressure, the residue was purified by column chromatography to obtain BH10 (10.2 g, yield 65%, and m/s (M+H) 506).

### [Comparative Synthesis Example 2] Synthesis of BH11

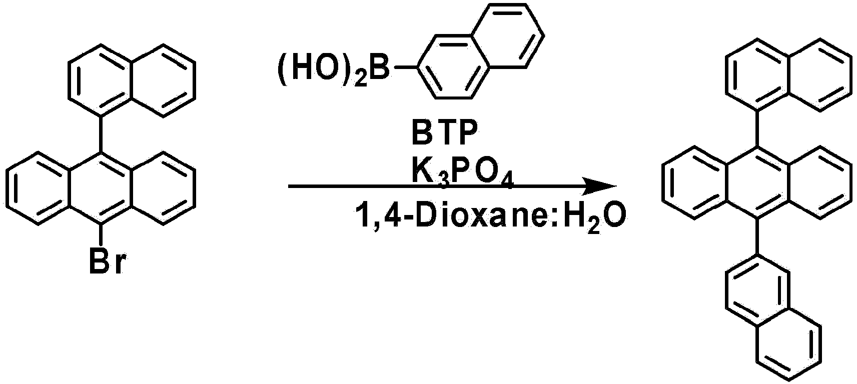

BH11 was synthesized in the same manner as in Comparative Synthesis Example 1, except that a reactant having a different structure was used in Comparative Synthesis Example 2. (obtained amount 8.4 g, yield 75%, and m/s (M+H) 430)

### [Comparative Synthesis Example 3] Synthesis of BH12

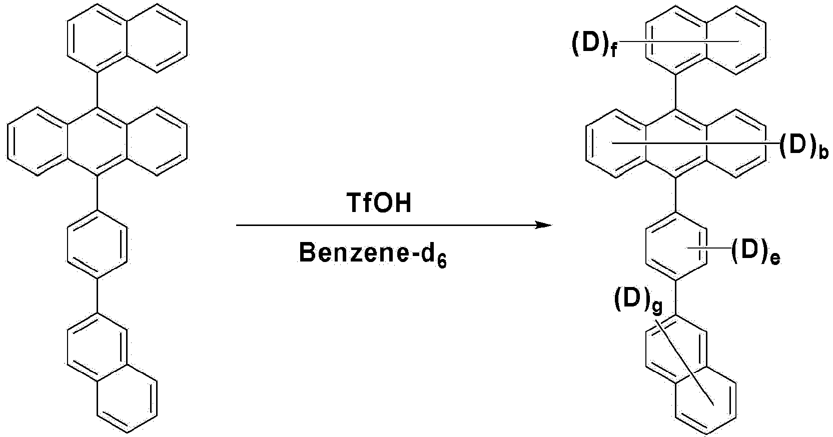

After BH10 (10 g, 0.020 mol), benzene-d6 (475 g, 5.65 mol) and trifluoromethanesulfonic acid (TfOH) (13.5 g, 0.09 mol) was put into a round bottom flask, the resulting mixture was heated to 70°C. After 2 hours, the reaction solution was cooled, and then neutralized by adding 150 ml of 2 M K₃PO₄ (aq) , and then the aqueous layer was removed by a separatory funnel. The organic layer was dried using MgSO₄, and then filtered through a silica pad. After the filtrate was distilled under reduced pressure, the residue was purified by column chromatography to obtain BH12 (9.4 g, yield 90%, deuterium conversion rate 87%, and deuterium conversion rate of anthracene 91%). Here, the molecular weight of BH12 was confirmed to be distributed. [cal. m/s: 532, exp. m/s (M+) 524-533]

### [Comparative Synthesis Example 4] Synthesis of BH13

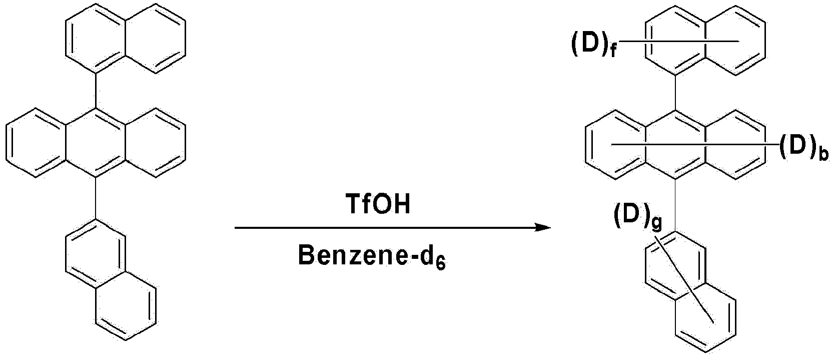

BH13 was synthesized in the same manner as in Comparative Synthesis Example 3, except that BH11 was used instead of BH10 in Comparative Synthesis Example 3. (obtained amount 8.1 g, yield 78%, deuterium conversion rate 85%, deuterium conversion rate of anthracene 87.5%) Here, the molecular weight of BH13 was confirmed to be distributed. [cal. m/s: 452, exp. m/s (M+) 443-452]

### [Evaluation Example] Manufacture of OLED device

A glass substrate thinly coated with indium tin oxide (ITO) to have a thickness of 150 nm was put into distilled water in which a detergent was dissolved, and ultrasonically washed. In this case, a product manufactured by the Fischer Co., was used as the detergent, and distilled water twice filtered using a filter manufactured by Millipore Co., was used as the distilled water. After the ITO was ultrasonically washed for 30 minutes, ultrasonic washing was repeated twice by using distilled water for 10 minutes. After the ultrasonic washing using distilled water was completed, ultrasonic washing was conducted by using isopropyl alcohol, acetone, and methanol solvents, and the resulting product was dried and then transported to a plasma washing machine. The substrate was treated using nitrogen plasma for 5 minutes, and then was transported to a vacuum deposition machine. The following compound HAT-CN compound was thermally vacuum-deposited to have a thickness of 5 nm on the ITO transparent electrode thus prepared, thereby forming a hole injection layer. Subsequently, the following compound HT1 was thermally vacuum-deposited to have a thickness of 100 nm, and then the following compound HT2 was thermally vacuum-deposited to have a thickness of 10 nm, thereby forming a hole transport layer. Subsequently, any one of the following compounds BH1 to BH11 as a host and the following compound BD (weight ratio 97:3) as a dopant were simultaneously vacuum-deposited, thereby forming a light emitting layer having a thickness of 20 nm. Subsequently, the following compound ET was vacuum-deposited to have a thickness of 20 nm, thereby forming an electron transport layer. Subsequently, LiF was vacuum-deposited to have a thickness of 0.5 nm, thereby forming an electron injection layer. Subsequently, aluminum was deposited to have a thickness of 100 nm to form a negative electrode, thereby manufacturing an organic light emitting device.

The structures of the compounds used in the examples are as follows.

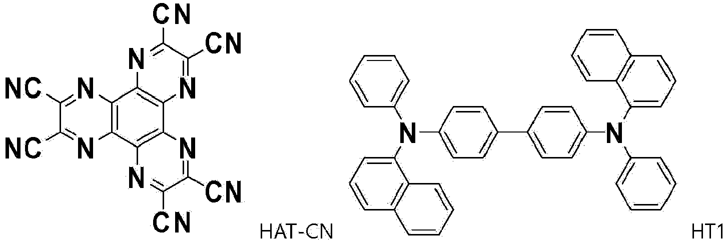

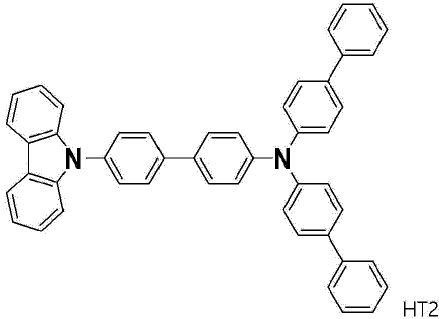

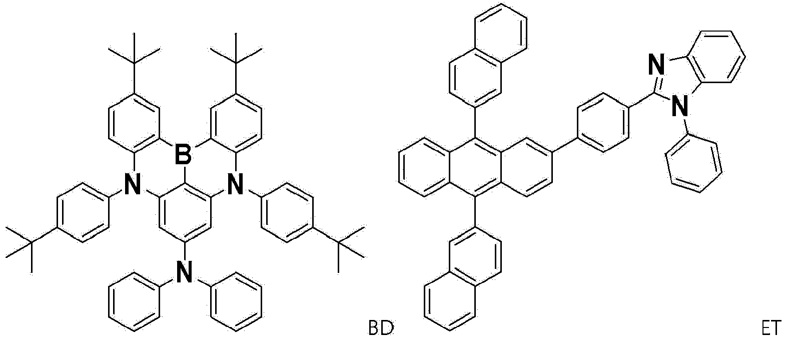

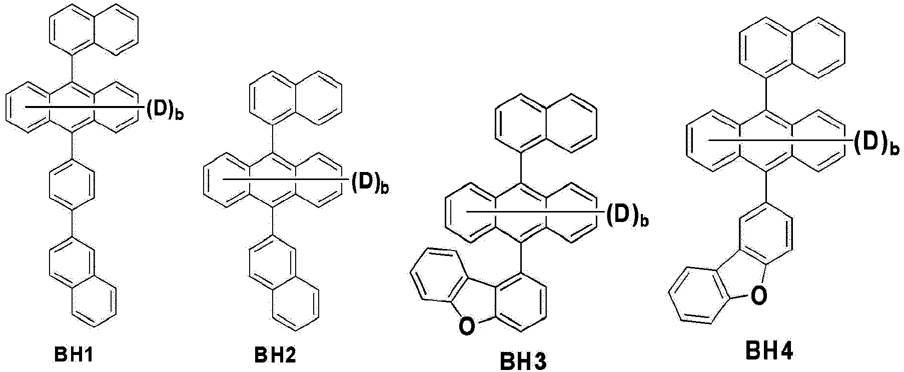

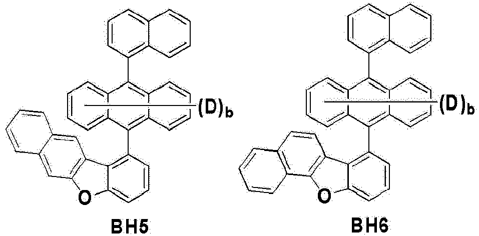

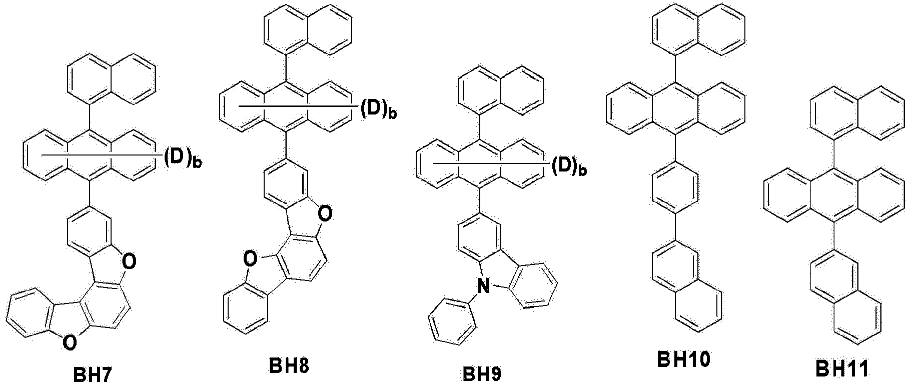

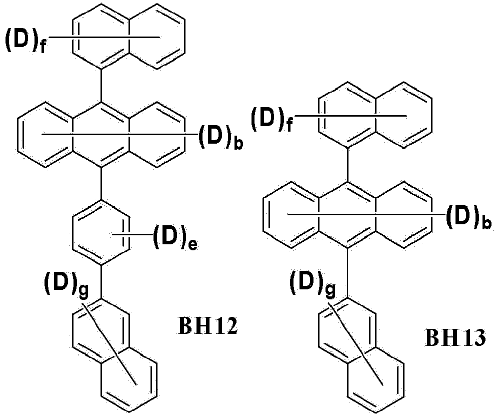

In the organic light emitting devices manufactured in Examples 1 to 9 and Comparative Examples 1 to 4, the driving voltage and the light emitting efficiency were measured at a current density of 10 mA/cm², and a time (LT) for reaching a 95% value compared to the initial luminance was measured at a current density of 20 mA/cm², and the results are shown in the following Table 1.

**[Table 1]**

| | Compound | Value measured at 10 mA/cm² | | LT (T95, h) |
|---|---|---|---|---|
| | (Light emitting layer host) | Driving voltage (Vop) | Light emitting efficiency (Cd/A) | |
| Example 1 | BH1 | 4.21 | 6.65 | 175 |
| Example 2 | BH2 | 4.31 | 6.60 | 186 |
| Example 3 | BH3 | 4.03 | 6.31 | 120 |
| Example 4 | BH4 | 4.14 | 6.62 | 152 |
| Example 5 | BH5 | 3.90 | 6.49 | 137 |
| Example 6 | BH6 | 3.97 | 6.52 | 129 |
| Example 7 | BH7 | 3.88 | 6.45 | 114 |
| Example 8 | BH8 | 3.90 | 6.23 | 123 |
| Example 9 | BH9 | 3.99 | 6.25 | 118 |
| Comparative Example 1 | BH10 | 4.21 | 6.65 | 125 |
| Comparative Example 2 | BH11 | 4.32 | 6.60 | 131 |
| Comparative Example 3 | BH12 | 4.21 | 6.65 | 185 |
| Comparative Example 4 | BH13 | 4.32 | 6.60 | 194 |

According to Table 1, it can be confirmed that Examples 1 to 9 where a compound in which deuterium is substituted is used have lower driving voltages, higher light emitting efficiencies, or longer service lives than Comparative Examples 1 and 2 where a compound in which deuterium is not substituted is used.

In particular, when Example 1 where compound BH1 having the same backbone structure is used is compared to Comparative Example 1 where compound BH10 is used, it can be confirmed that the service life of Example 1 where deuterium is substituted is remarkably lengthened compared to Comparative Example 1. Further, even though Example 2 where compound BH2 having the same backbone structure is used is compared to Comparative Example 2 where compound BH11 is used, it can be confirmed that the service life of Example 2 where deuterium is substituted is remarkably lengthened compared to Comparative Example 2.

Compound BH1 used in Example 1 and compound BH12 used in Comparative Example 3 have the same backbone structure as each other. In this case, for compound BH1, when anthracene was synthesized using benzene-d6, which had been deuterated by a bottom-up method, deuterium had already been substituted, and then an additional substituent was introduced. Deuterium was substituted at 99% for 8 substitution positions based on the anthracene, and deuterium was substituted at 30.7% for 26 substitution positions based on the compound BH10 entire compound. In contrast, for compound BH12, compound BH10 was synthesized by Comparative Synthesis Example 1, and then deuterium was substituted with compound BH10 through a deuteration process by Comparative Synthesis Example 3. As a result, deuterium was substituted at 910 for 8 substitution positions based on the anthracene, and deuterium was substituted at 87% for 26 substitution positions based on the compound BH10 entire compound. Consequently, even though the service life of Comparative Example 3 where compound BH12 having a high deuterium conversion rate based on the entire compound is used has a longer service life than Example 1, it can be confirmed that even though Example 1 has a deuterium conversion rate of 30.7% based on the entire compound, Example 1 exhibits a service life effect of 95% ((175/185)×100) of the service life of Comparative Example 3 having a deuterium conversion rate of 87% based on the entire compound. Furthermore, since compound BH1 of Example 1 directly synthesizes anthracene using benzene-d6 which has already been deuterated, a separate deuteration process is not performed, so that the efficiency in which the service life is improved through deuterated anthracene is high while the process is simplified.

Such an effect can also be confirmed by comparing compound BH2 used in Example 2 with compound BH13 used in Comparative Example 4. Specifically, it can be confirmed that even though Example 2 has a deuterium conversion rate of 36% based on the entire compound, Example 2 exhibits a service life effect of 96% ((186/194)×100) of the service life of Comparative Example 3 having a deuterium conversion rate of 85% based on the entire compound.

## Claims

1. A method for preparing a deuterated anthracene compound, the method comprising:
synthesizing a deuterated anthracene compound by reacting a halogenated benzene having at least one deuterium with a compound of Chemical Formula 1: 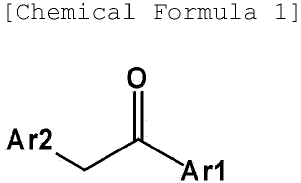
wherein in Chemical Formula 1:
Ar1 is deuterium, a substituted or unsubstituted aryl group, or a substituted or unsubstituted heterocyclic groupand
Ar2 is hydrogen, deuterium, a substituted or unsubstituted aryl group, or a substituted or unsubstituted heterocyclic group.

2. A method for preparing a deuterated anthracene compound, the method comprising:
synthesizing a compound of the following Chemical Formula 2 by reacting halogenated benzene-d5 with a compound of the following Chemical Formula 1: 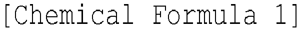 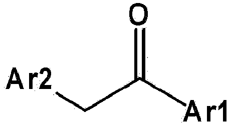 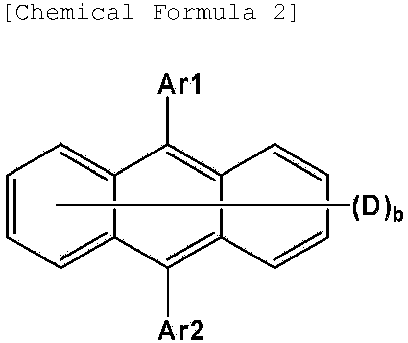
in Chemical Formulae 1 and 2,
Ar1 is deuterium, a substituted or unsubstituted aryl group, or a substituted or unsubstituted heterocyclic group,
Ar2 is hydrogen, deuterium, a substituted or unsubstituted aryl group, or a substituted or unsubstituted heterocyclic group, and
b is an integer from 1 to 8.

3. The method of claim 1, wherein the halogenated benzene having at least one deuterium is halogenated benzene-d5.

4. The method of claim 1, wherein the deuterated anthracene compound is a compound of Chemical Formula 2: 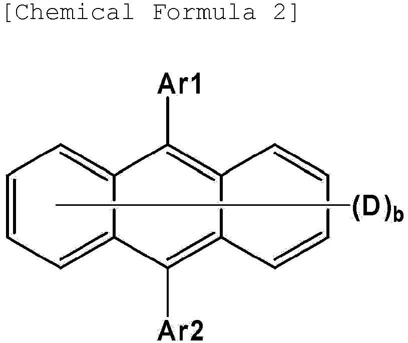 wherein in Chemical Formula 2:
Ar1 is deuterium, a substituted or unsubstituted aryl group, or a substituted or unsubstituted heterocyclic group;
Ar2 is hydrogen, deuterium, a substituted or unsubstituted aryl group, or a substituted or unsubstituted heterocyclic group; and
b is an integer from 1 to 8.

5. The method of claim 2 or 4, wherein the method comprises synthesizing a compound of the following Chemical Formula 3 using a solution comprising a compound in which Ar2 is hydrogen among the compounds of Chemical Formula 2 and a halogen supplying agent; and
synthesizing a compound of the following Chemical Formula 4 by reacting the compound of Chemical Formula 3 with L-Ar2: 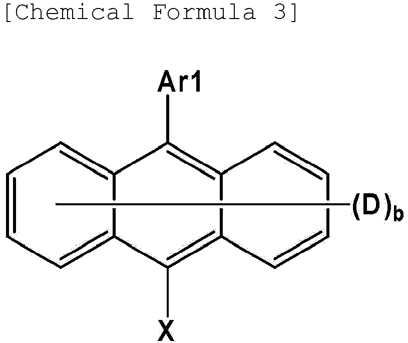 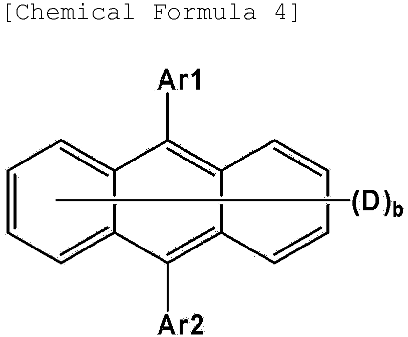
wherein in Chemical Formulae 3 and 4:
Ar1 and Ar2 are the same as or different from each other, and are each independently deuterium, a substituted or unsubstituted aryl group, or a substituted or unsubstituted heterocyclic group;
X is a halogen group;
L is a leaving group; and
b is an integer from 1 to 8.

6. The method of claim 1, further comprising synthesizing a halogen benzene having at least one deuterium from benzene-d6 using a halogen supplying agent before the synthesizing of the deuterated anthracene compound.

7. The method of claim 1 or 2, wherein Chemical Formula 1 is any one of the following Chemical Formulae 1-1 to 1-3: 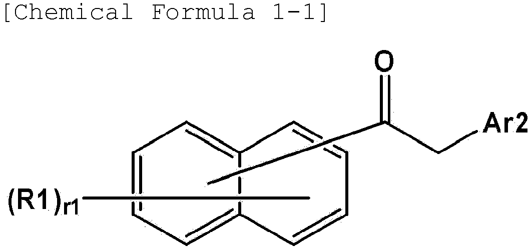 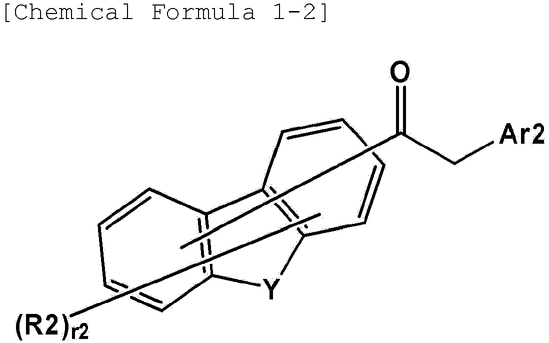 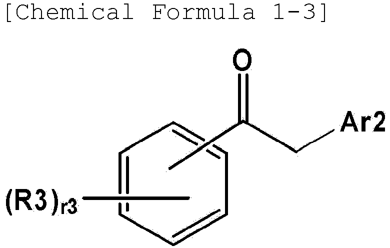 wherein in Chemical Formulae 1-1 to 1-3:
Y is CRaRb, NRc, O or S;
Ar2 is hydrogen, deuterium, a substituted or unsubstituted aryl group, or a substituted or unsubstituted heterocyclic group;
Ra, Rb, Rc and R1 to R3 are the same as or different from each other, and are each independently hydrogen, deuterium, a substituted or unsubstituted alkyl group, a substituted or unsubstituted aryl group, or a substituted or unsubstituted heterocyclic group, or are bonded to an adjacent group to form a substituted or unsubstituted ring;
r1 and r2 are each an integer from 1 to 7, and r3 is an integer from 1 to 5,
when r1 is 2 or higher, the R1s are the same as or different from each other,
when r2 is 2 or higher, the R2s are the same as or different from each other, and
when r3 is 2 or higher, the R3s are the same as or different from each other.

8. The method of claim 1 or 2, wherein Chemical Formula 1 is any one of the following structures:

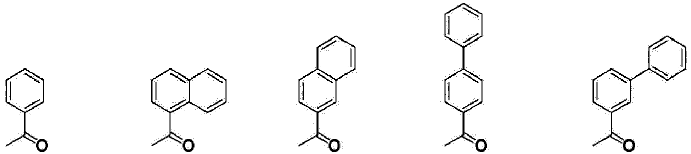

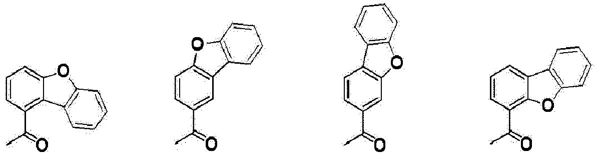

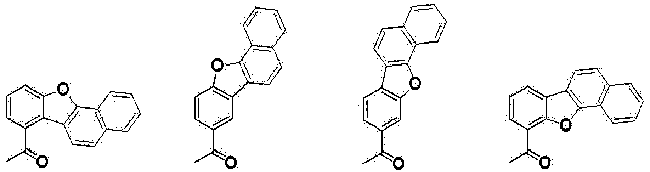

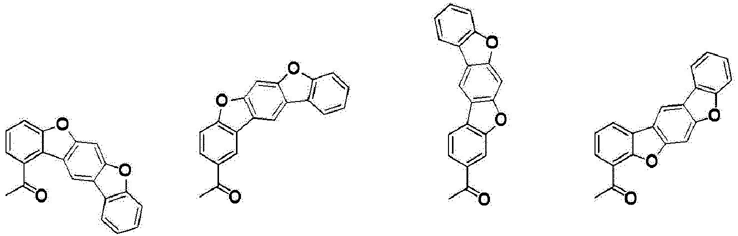

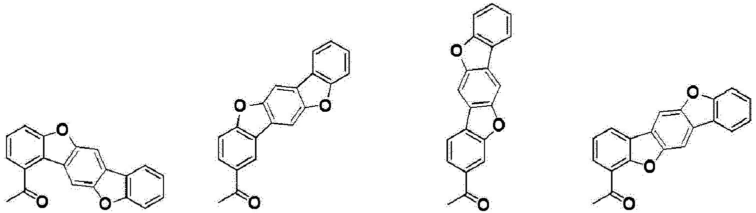

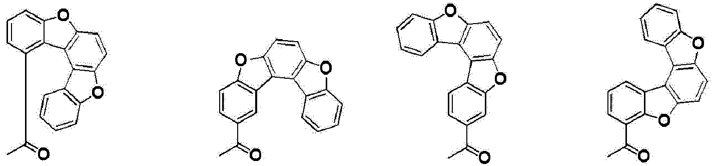

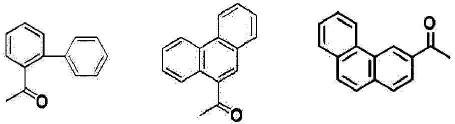

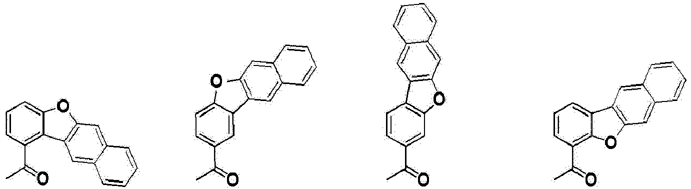

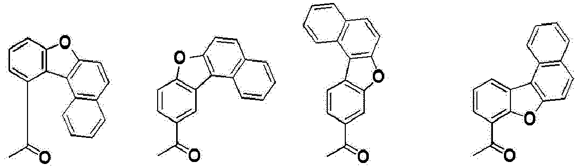

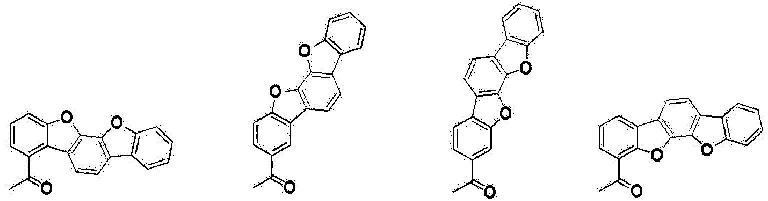

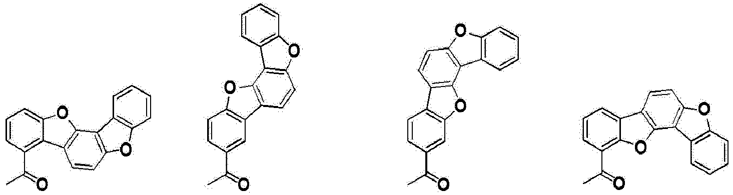

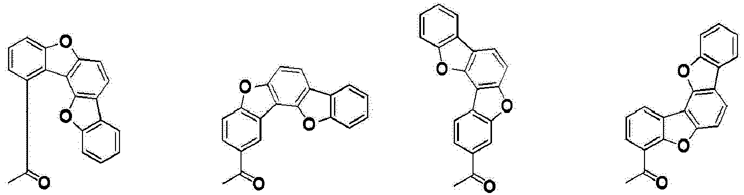

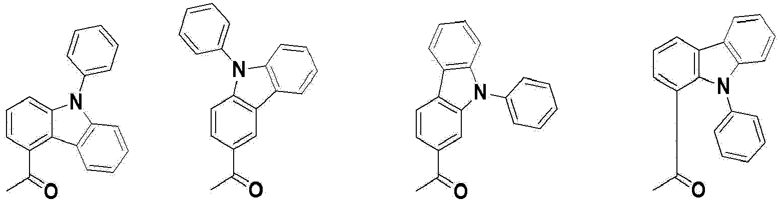

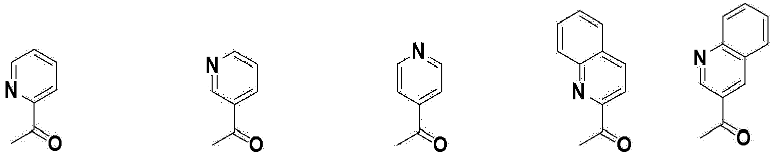

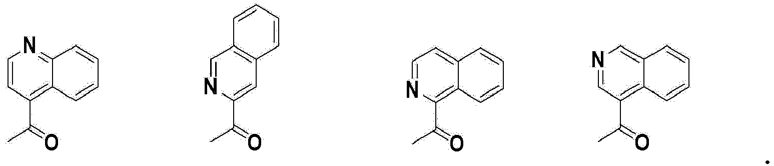

9. The method of claim 2 or 4, wherein the synthesizing of the compound of Chemical Formula 2 synthesizes a compound of Chemical Formula 2 using a solution comprising halogenated benzene-d5, the compound of Chemical Formula 1, 2,2,6,6-tetramethylpiperidine, an alkyllithium and an ether-based solvent.

10. The method of claim 2 or 4, wherein Chemical Formula 2 is any one of the following structures: 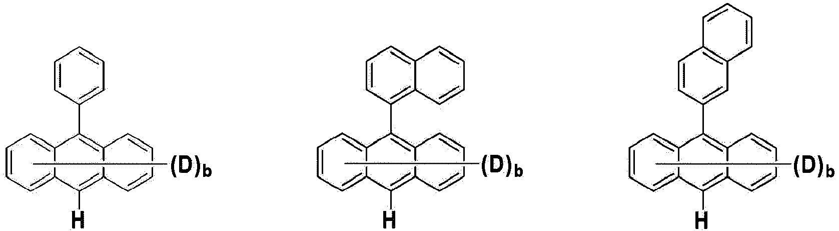 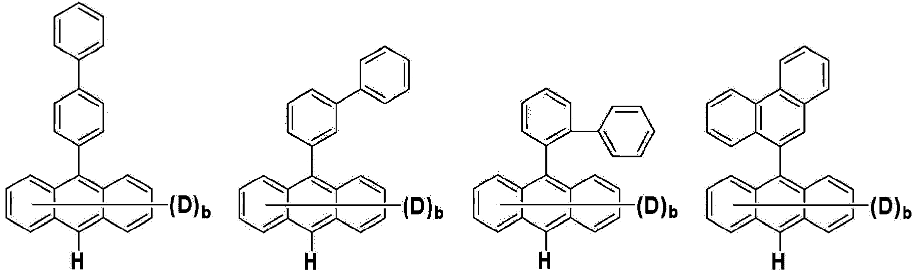 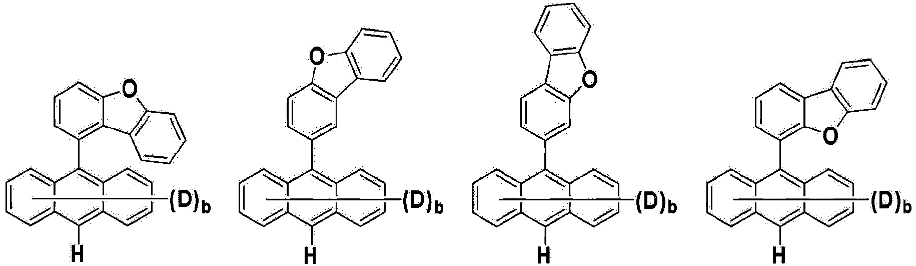 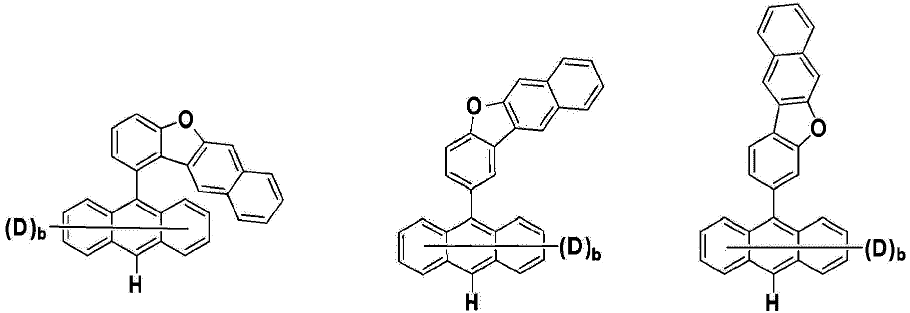 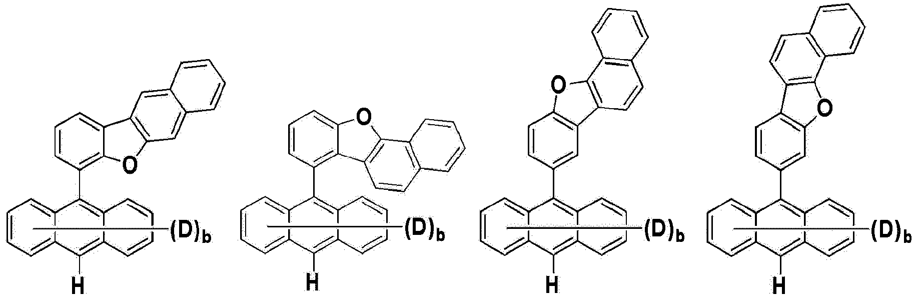 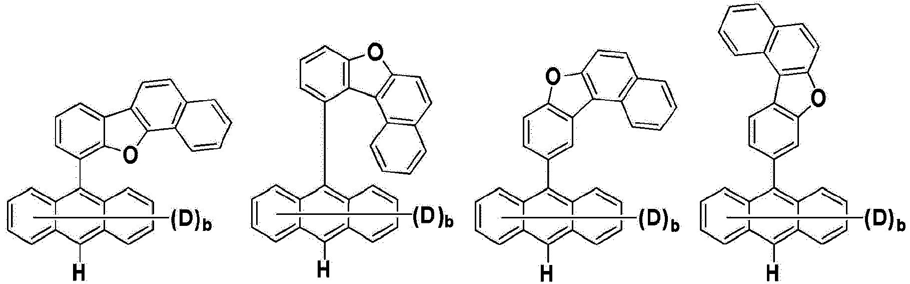 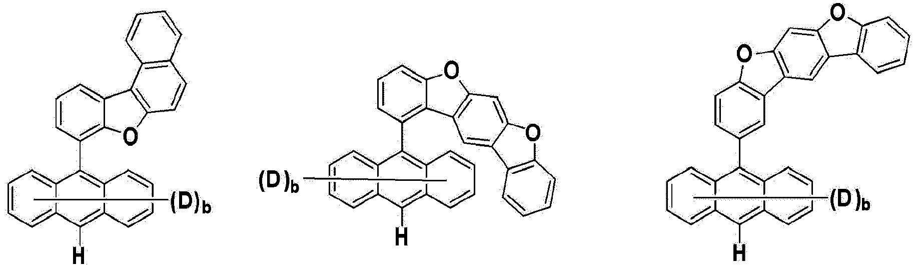 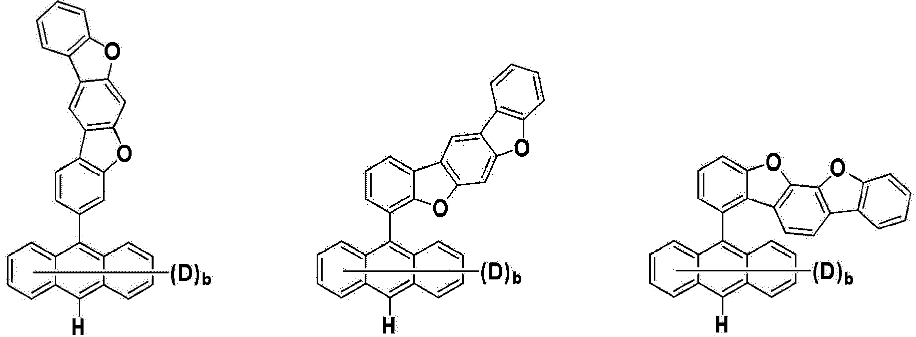 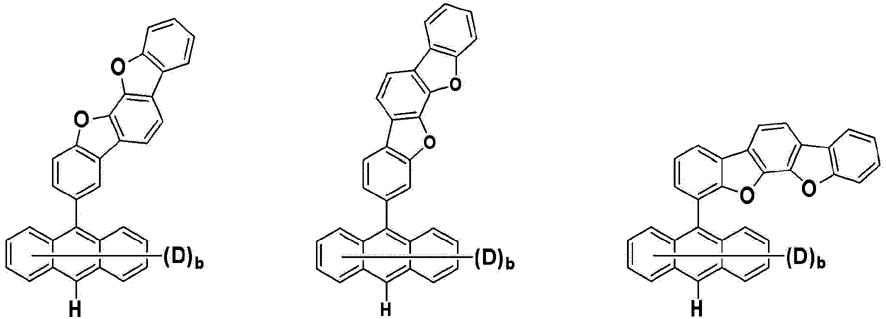 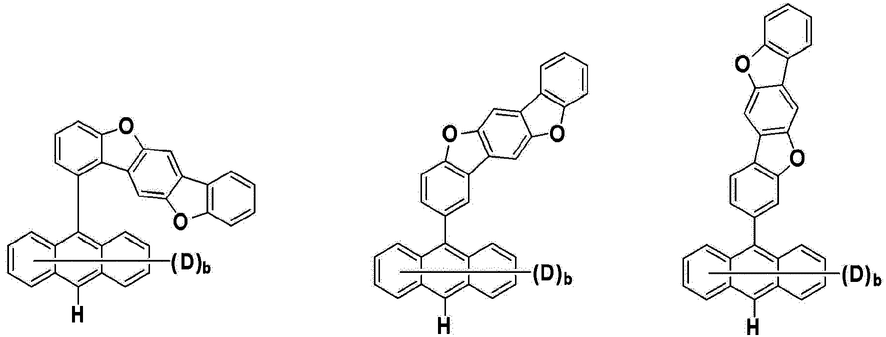 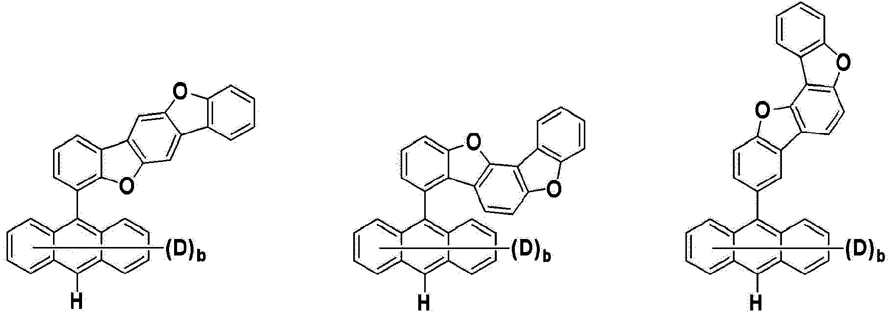 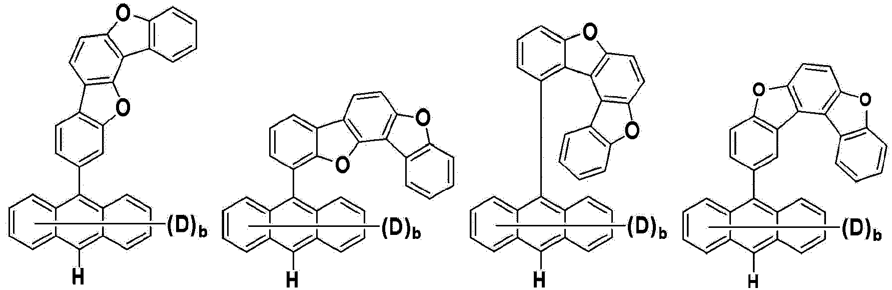 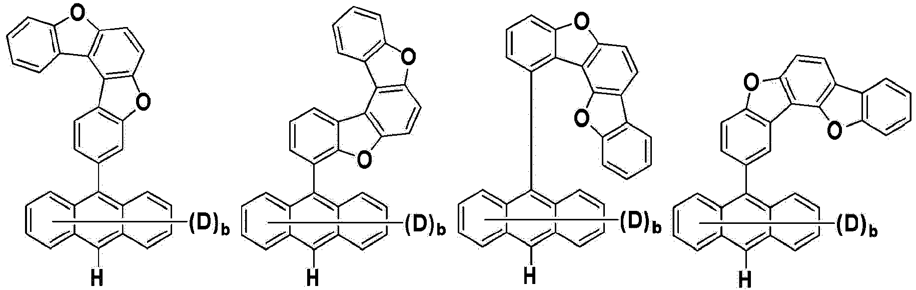 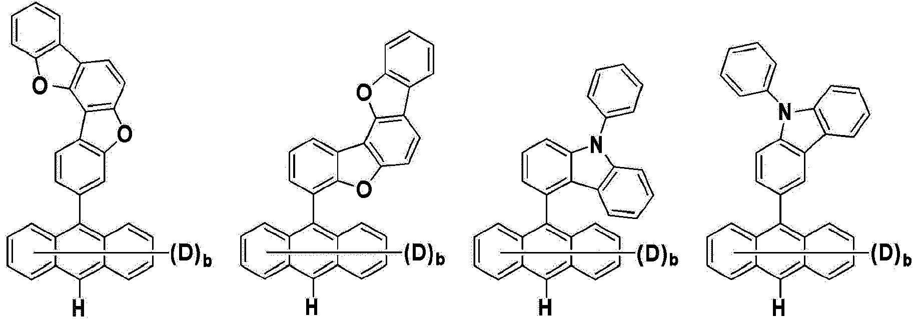 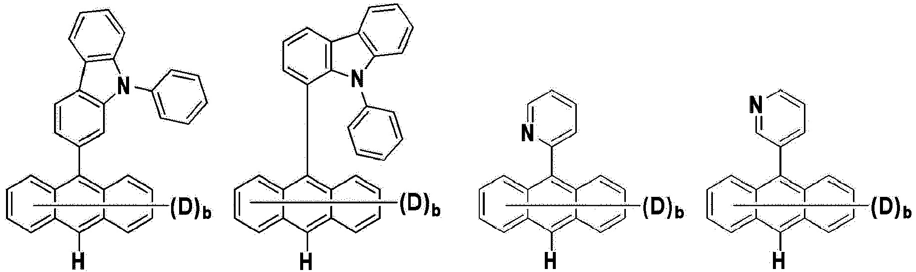 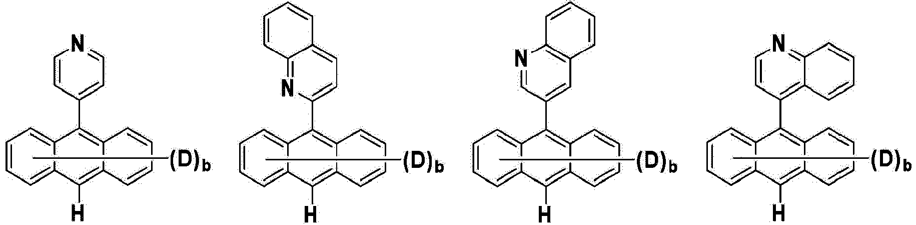 wherein b is an integer from 1 to 8.

11. The method of claim 5, wherein Chemical Formula 3 is any one of the following structures: 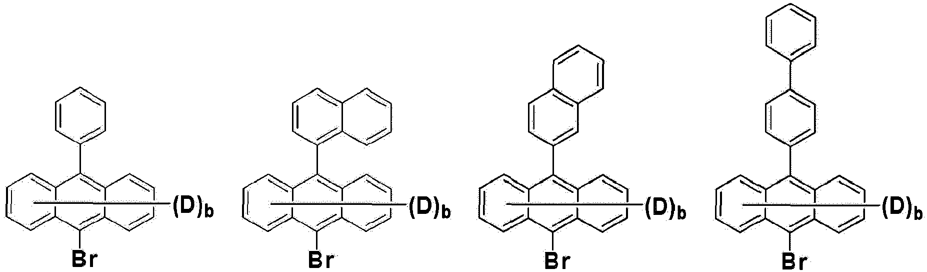 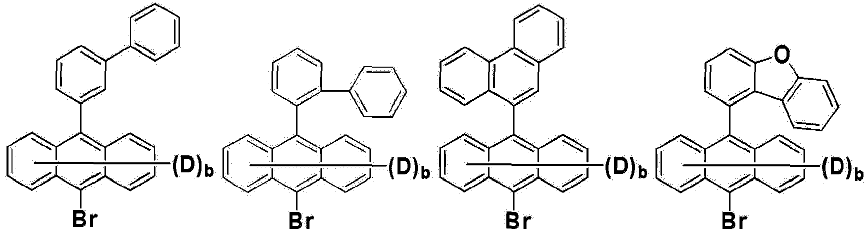 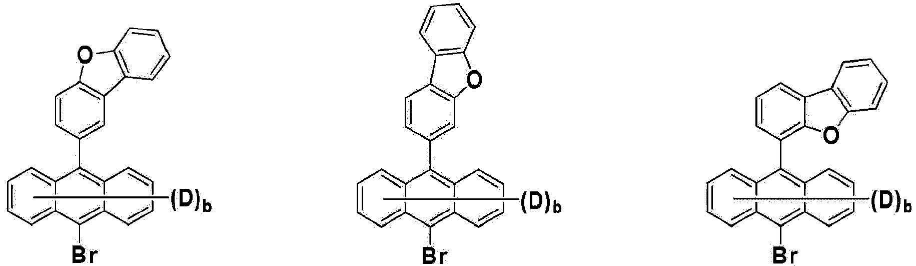 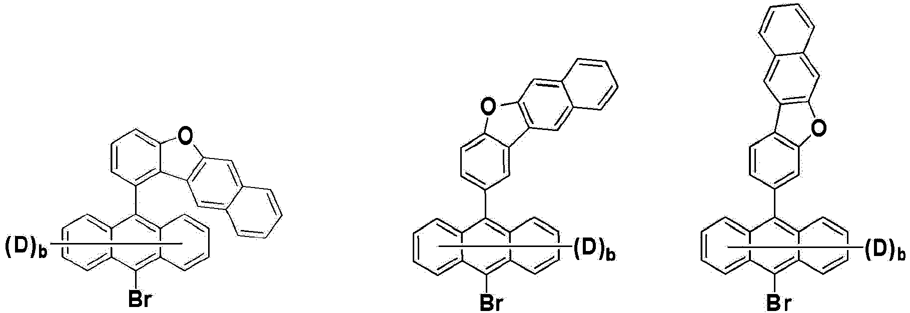 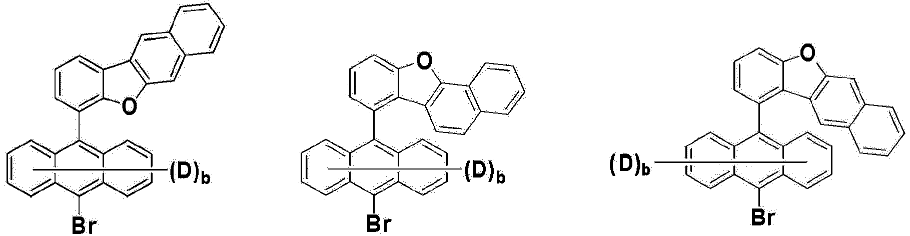 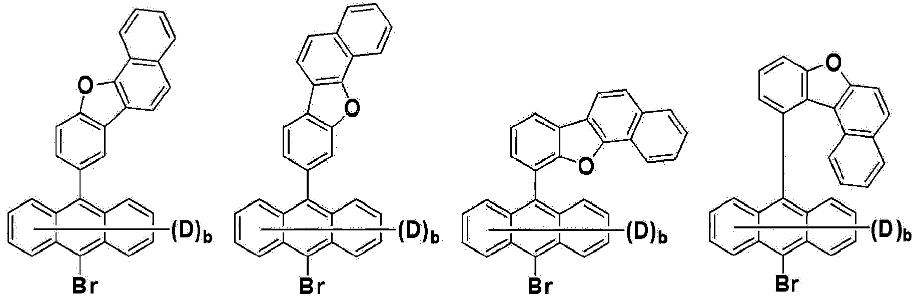 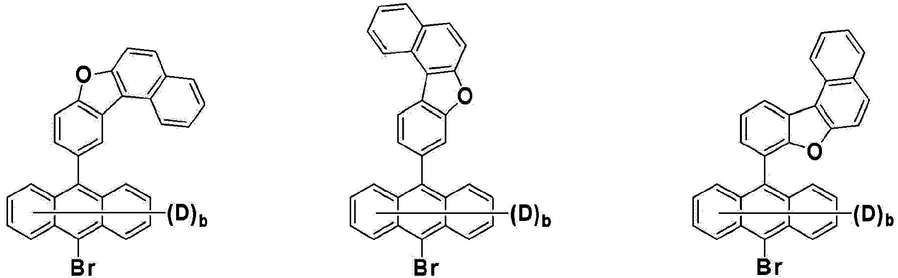 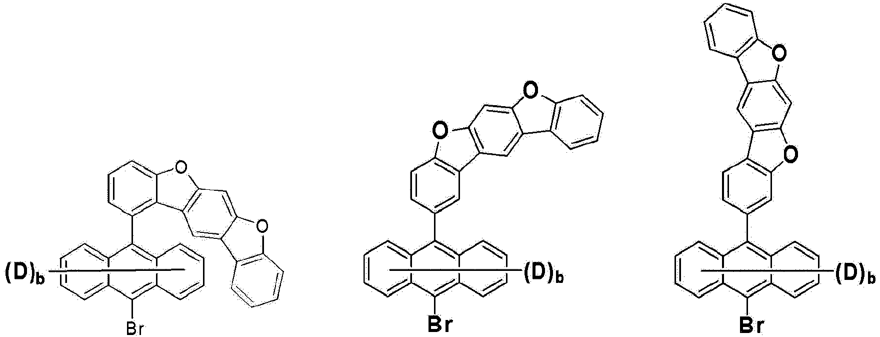 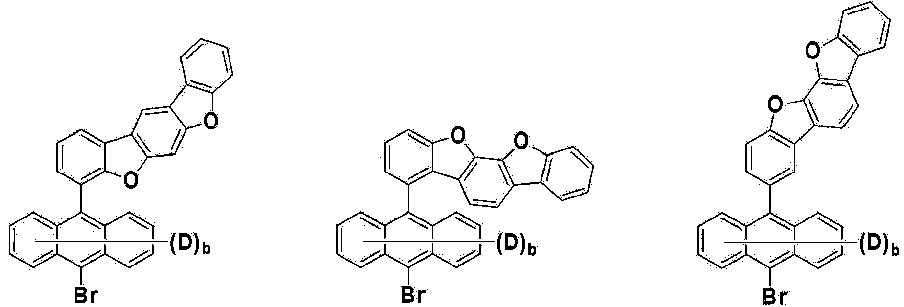 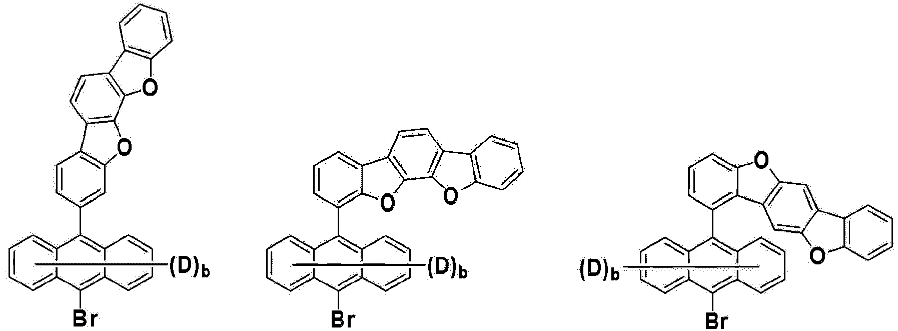 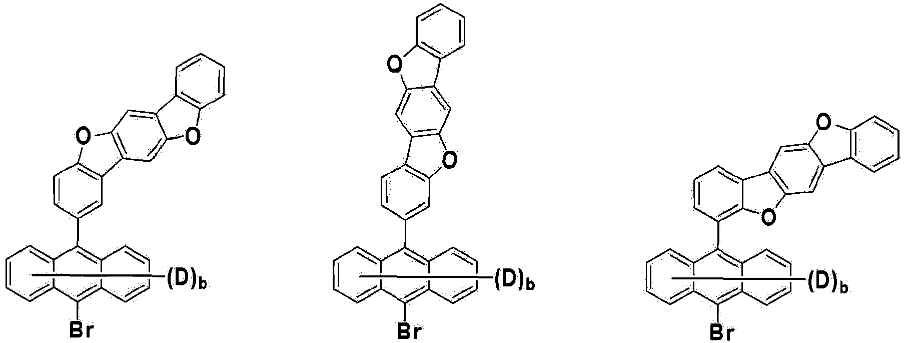 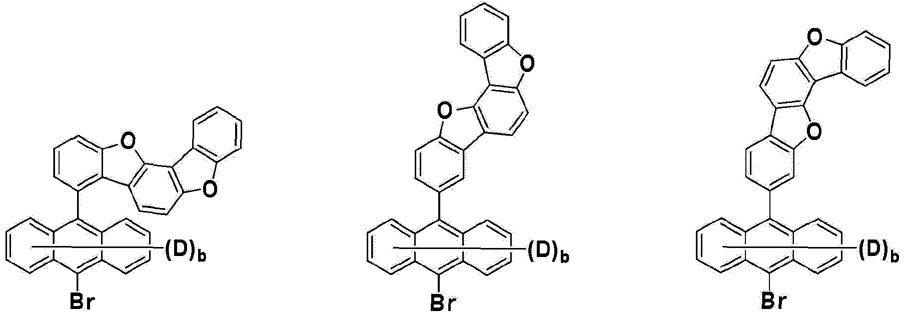 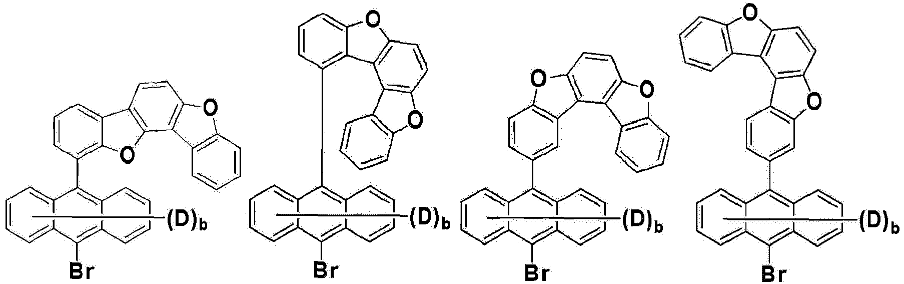 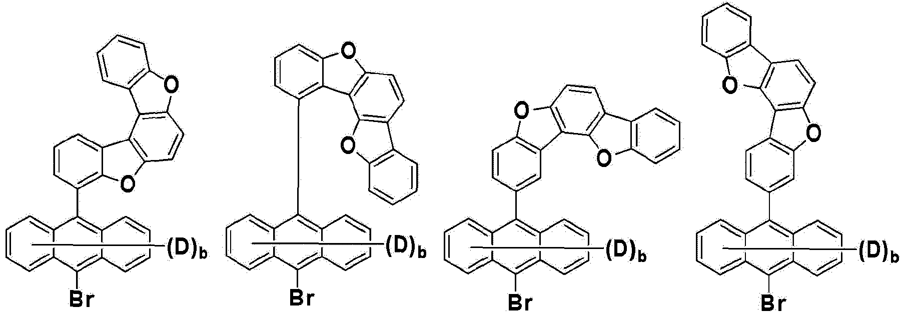 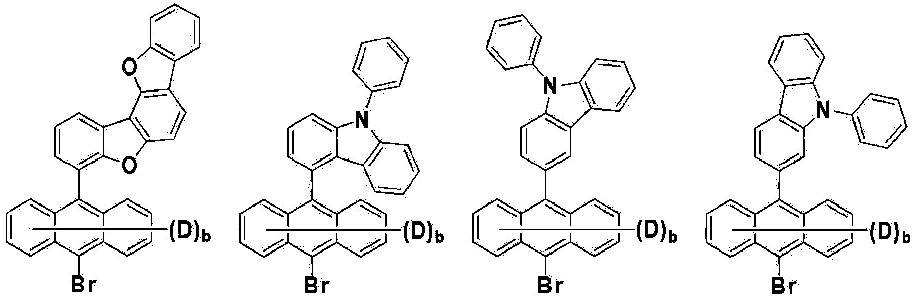 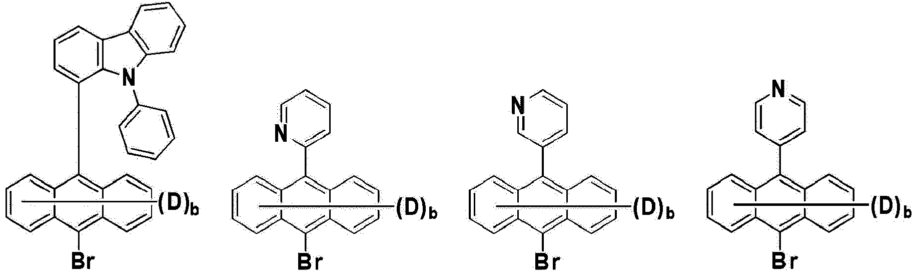 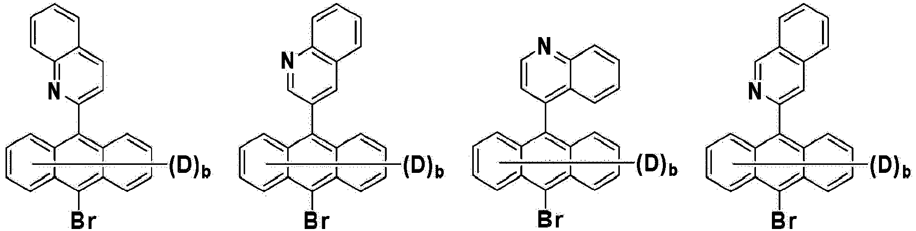 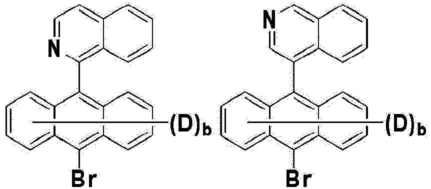 wherein b is an integer from 1 to 8.

12. The method of claim 5, wherein Chemical Formula 4 is any one of the following structures: 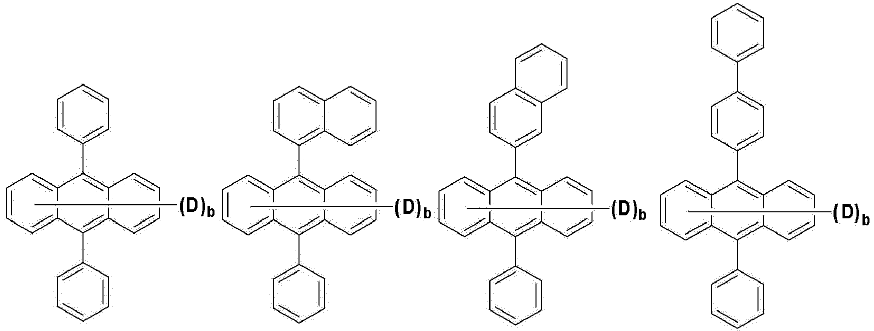 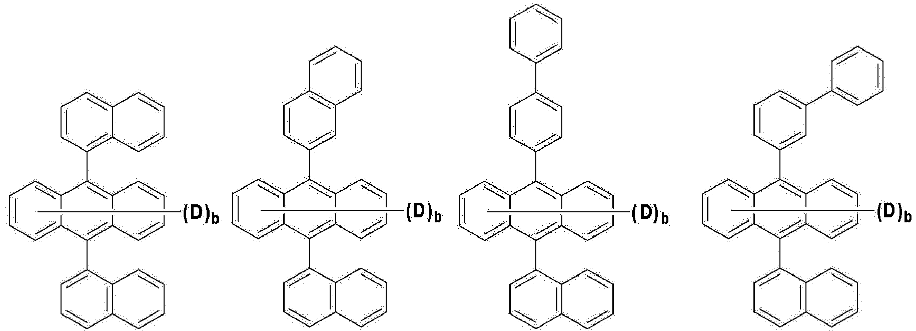 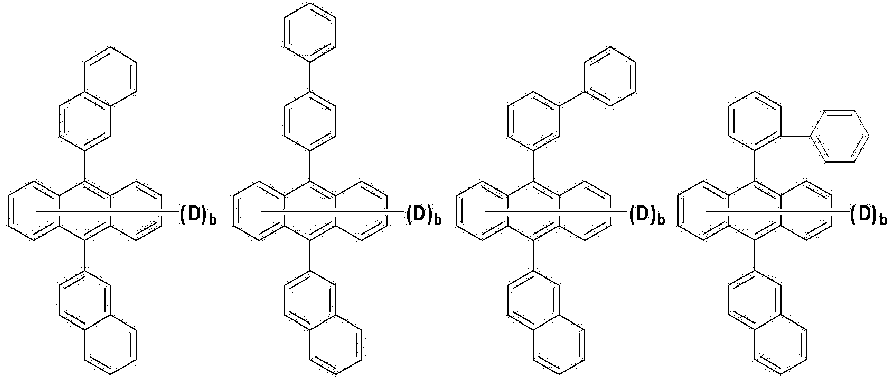 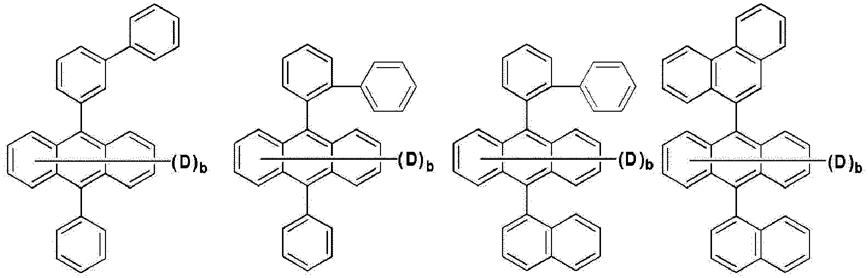 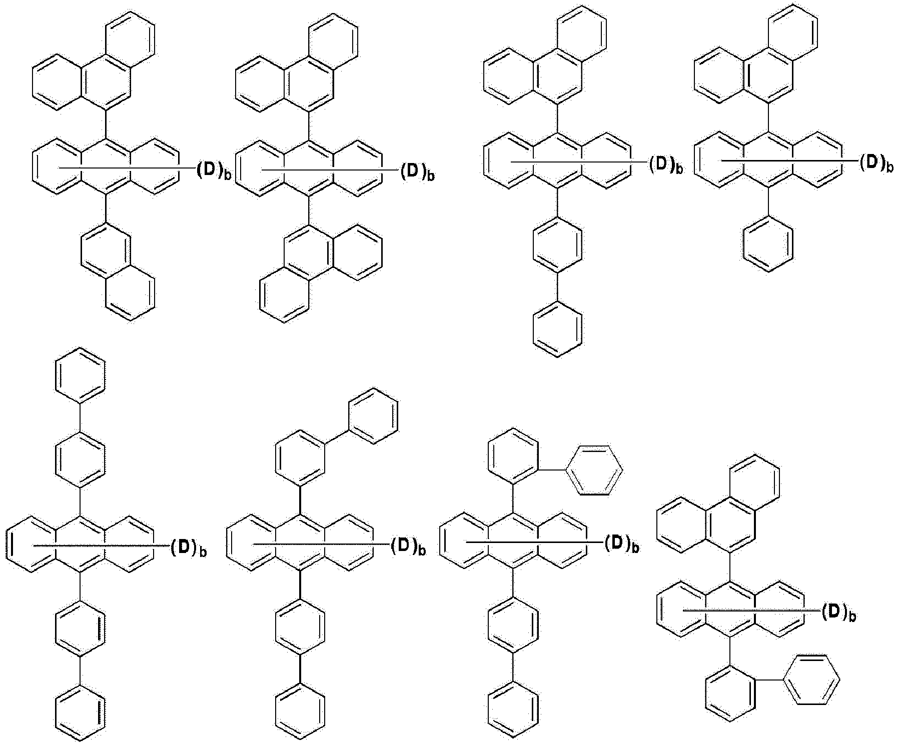 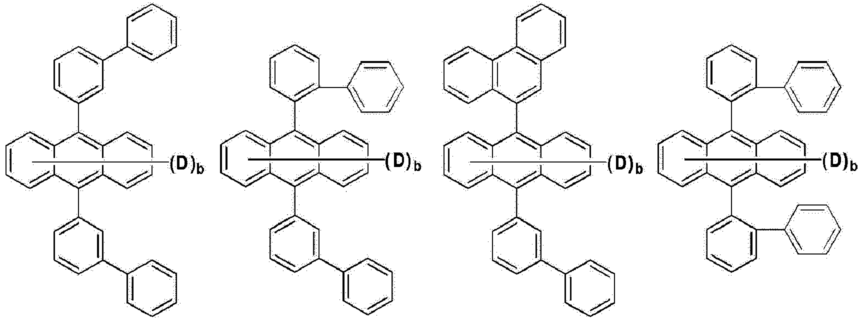 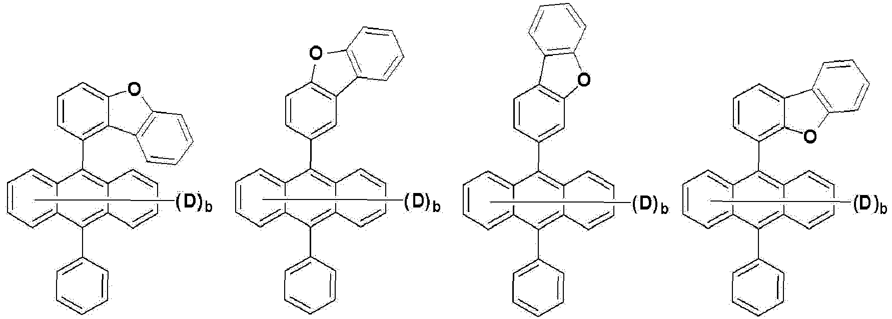 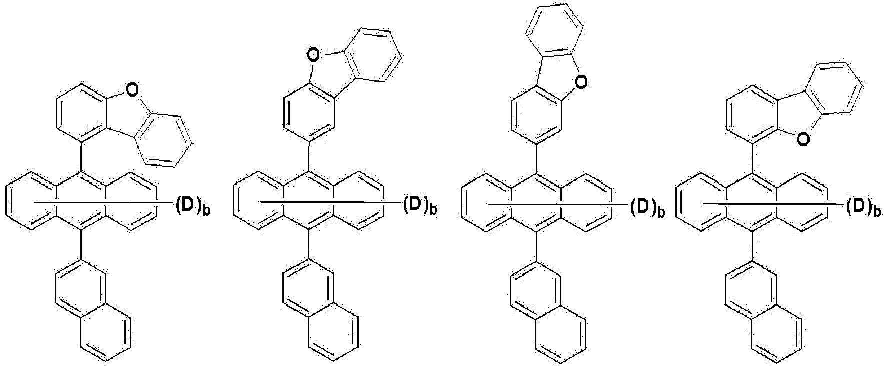 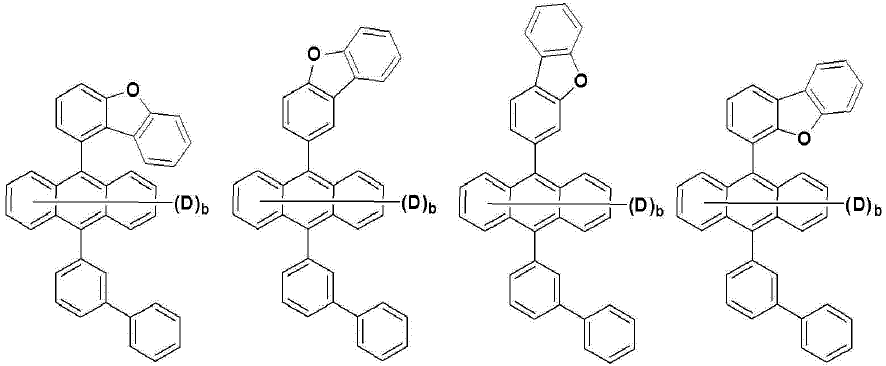 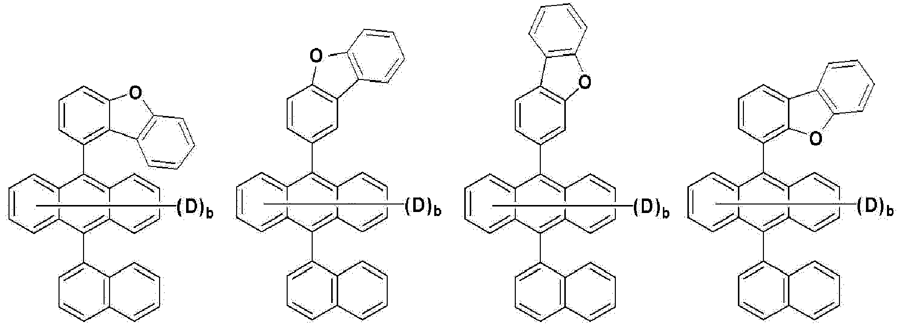 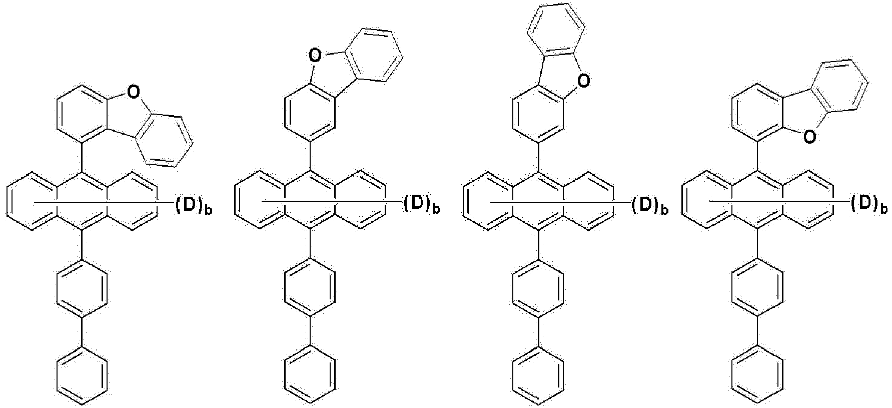 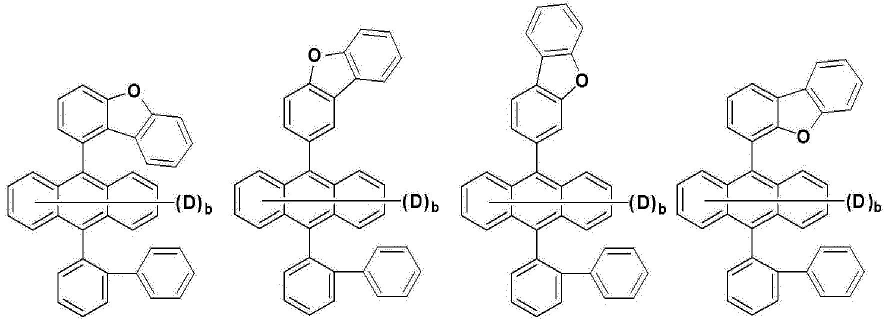 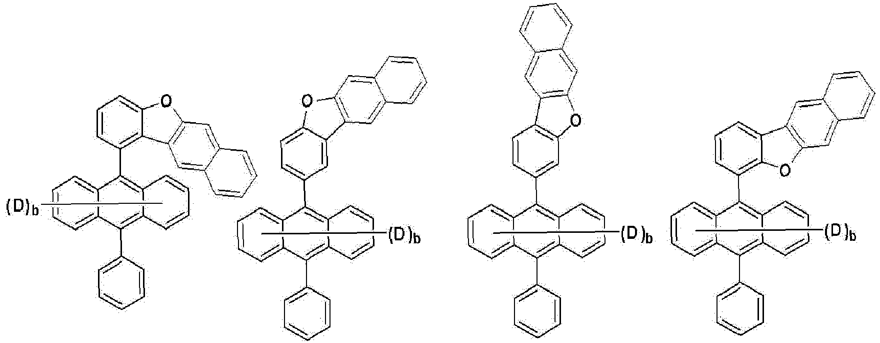 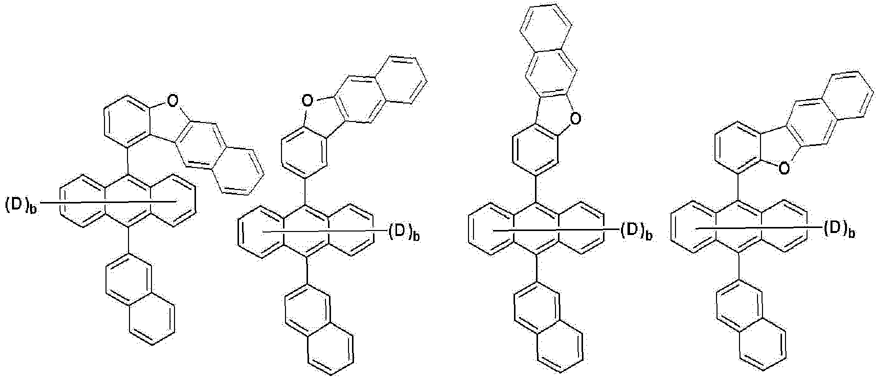 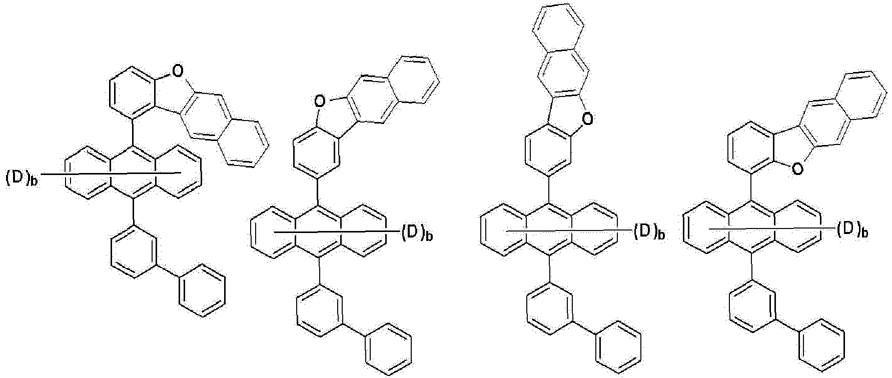 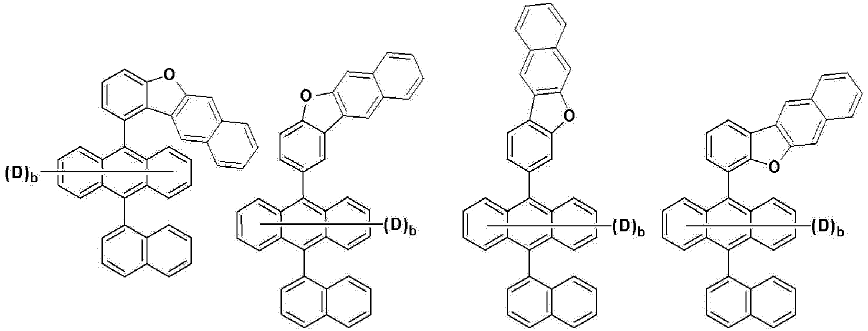 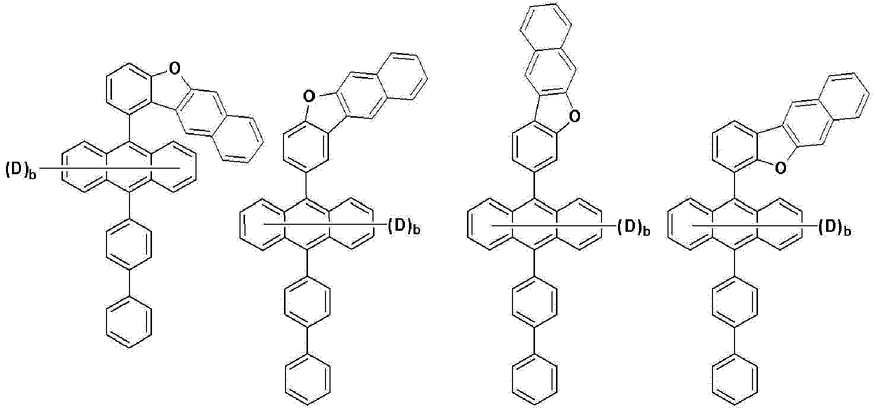 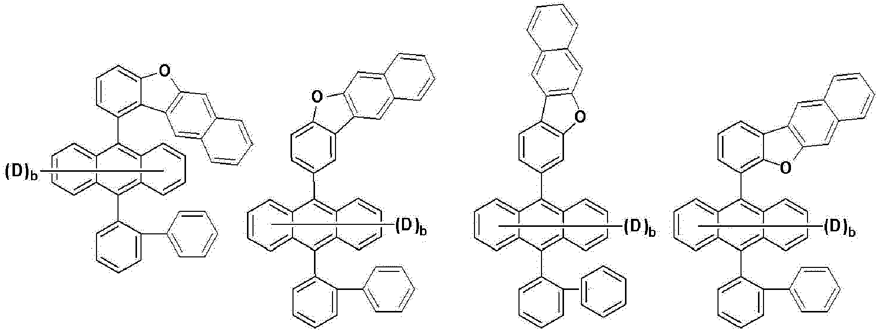 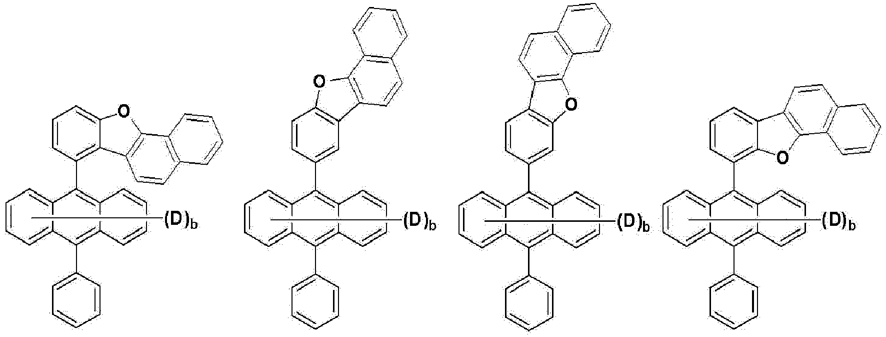 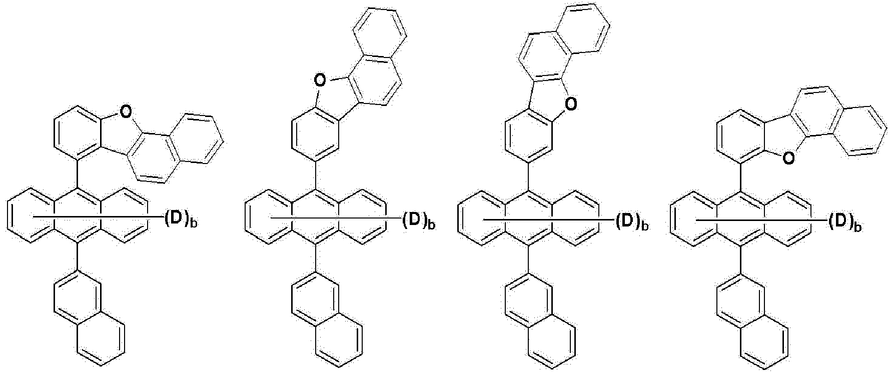 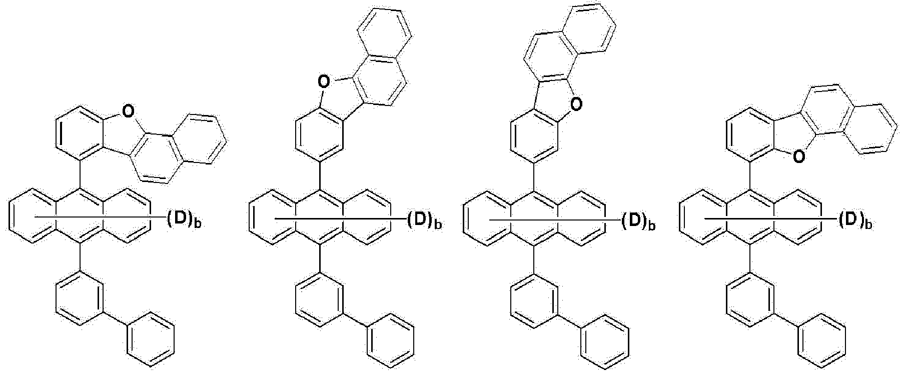 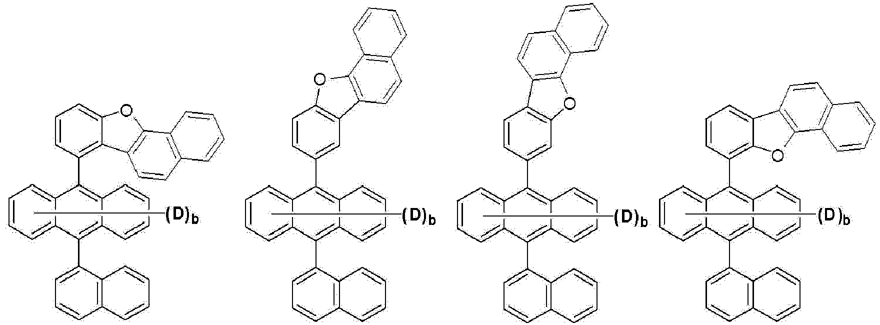 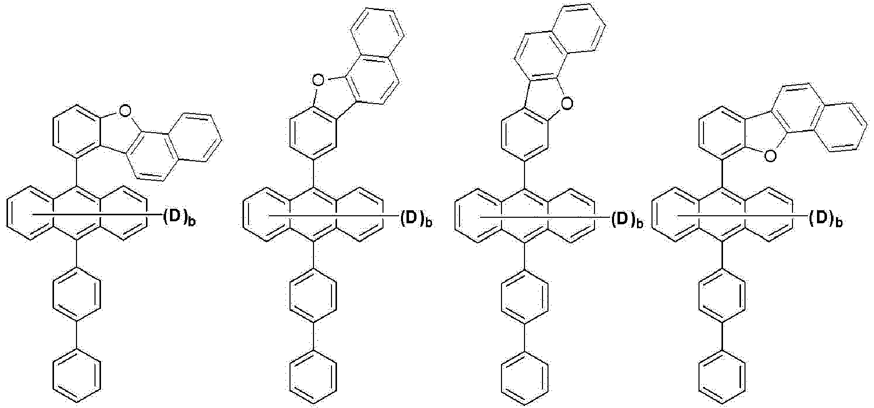 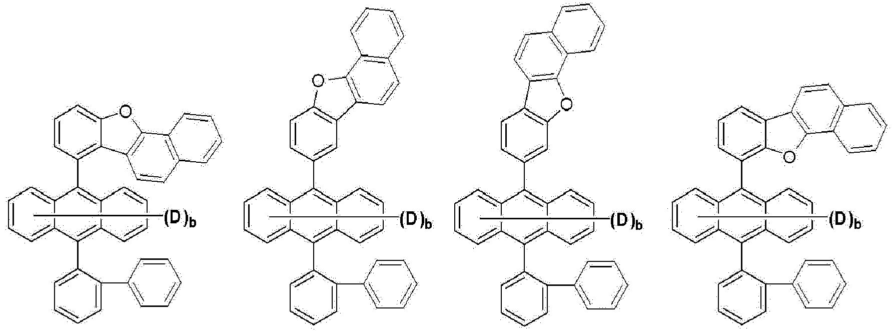 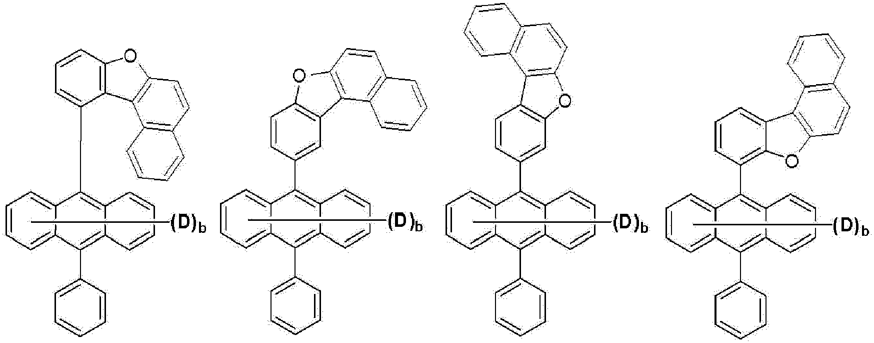 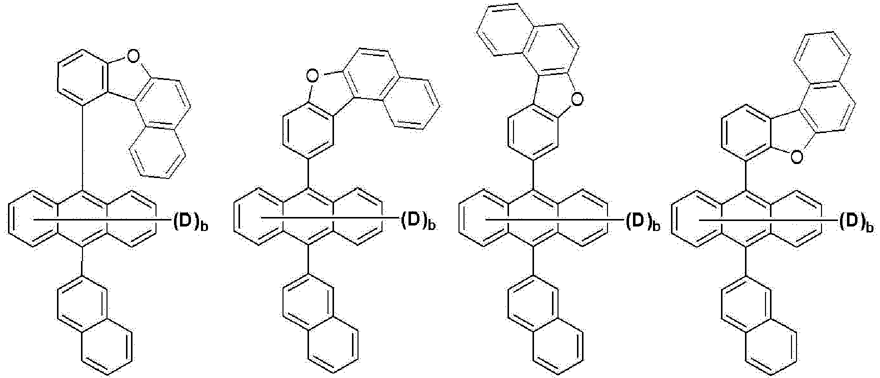 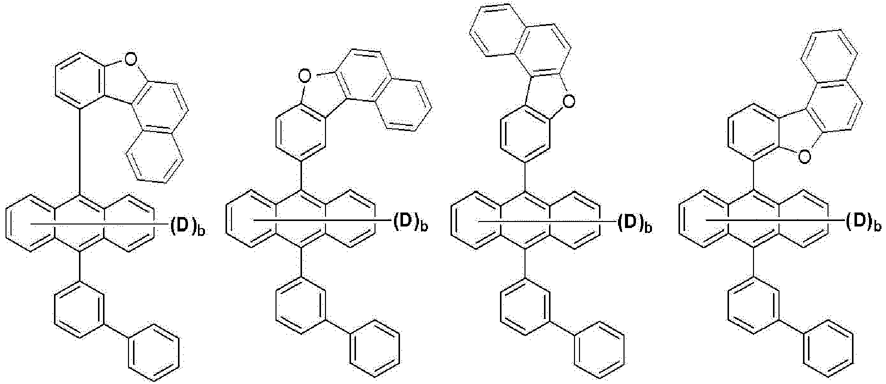 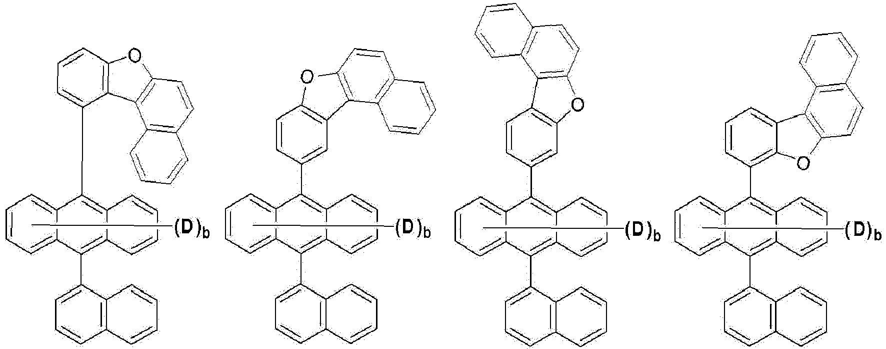 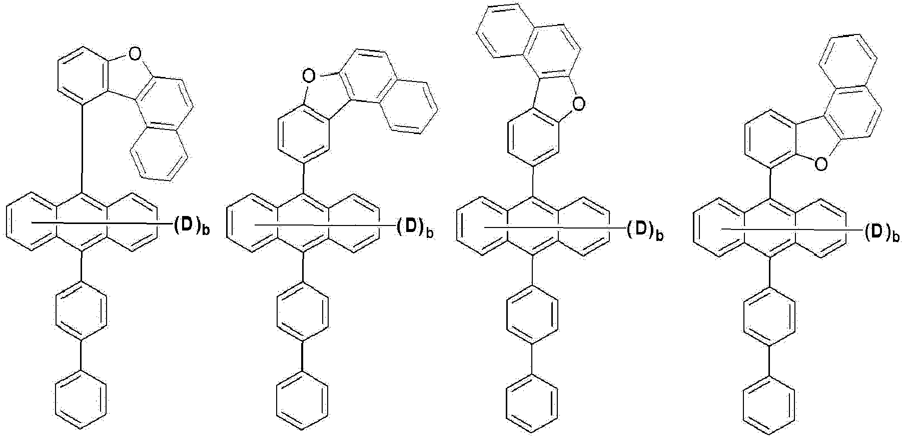 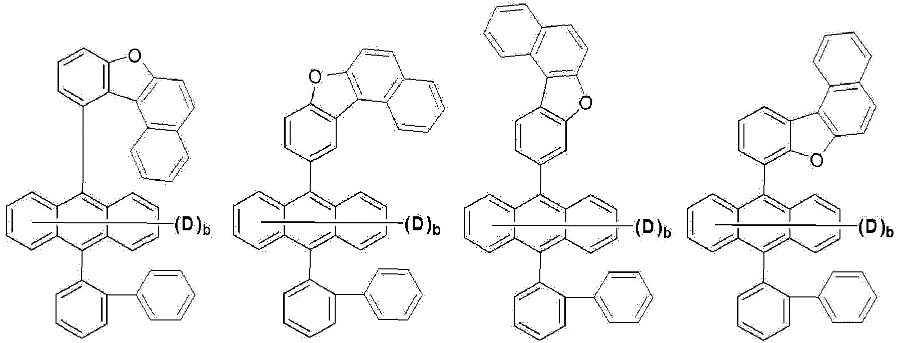 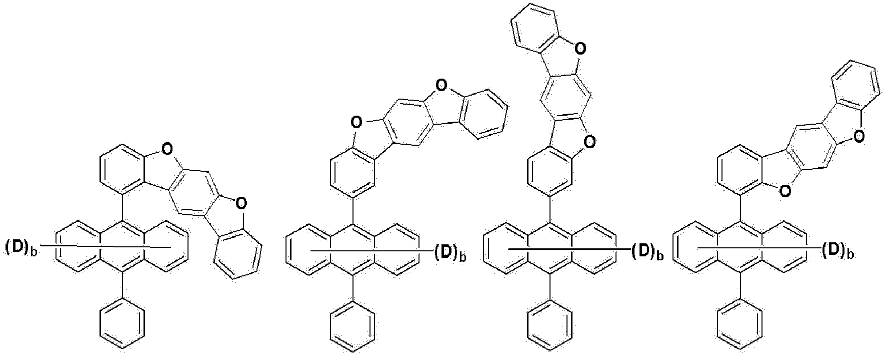 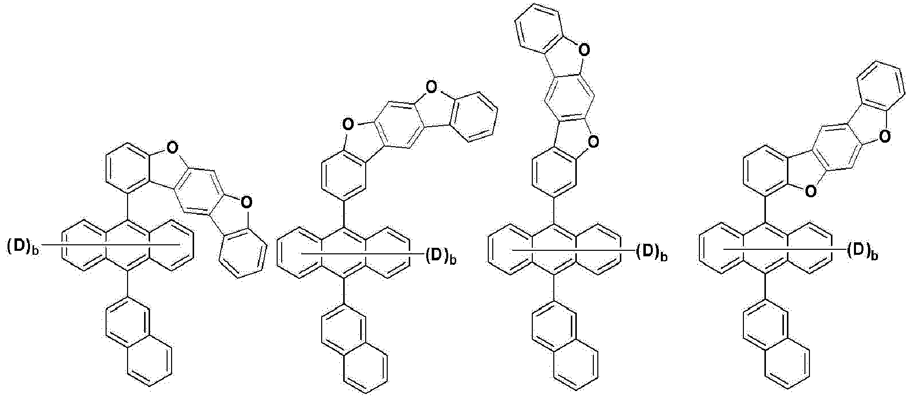 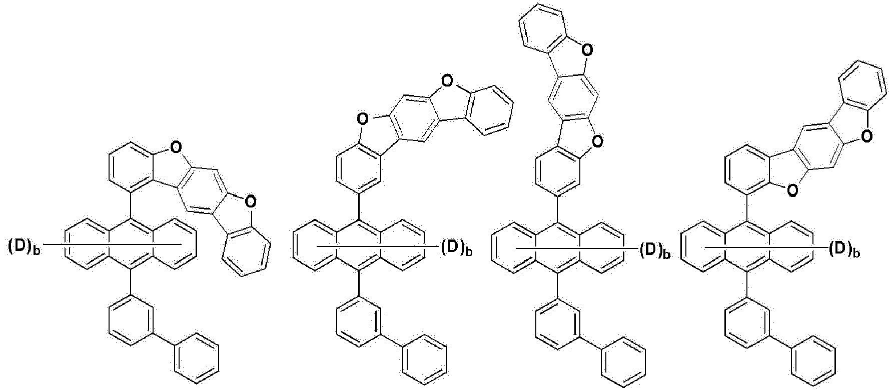 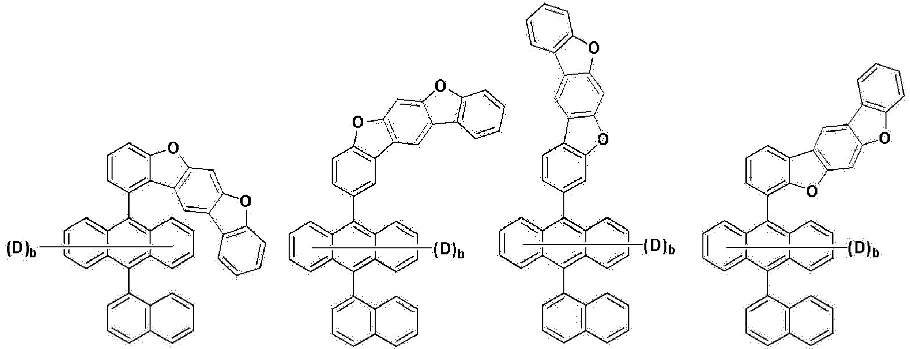 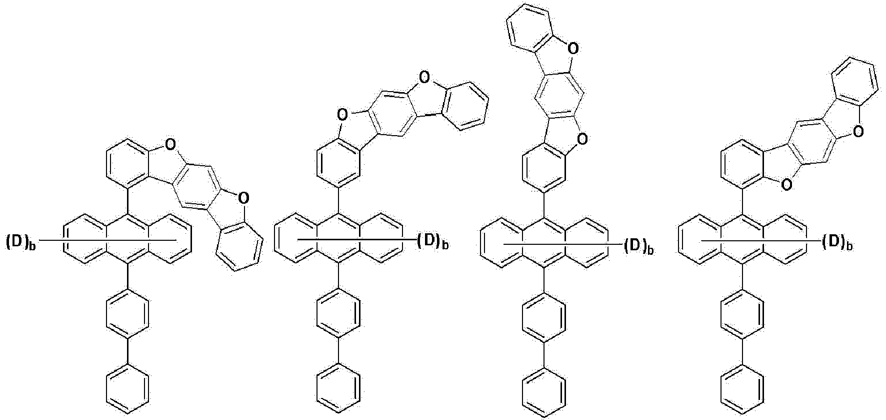 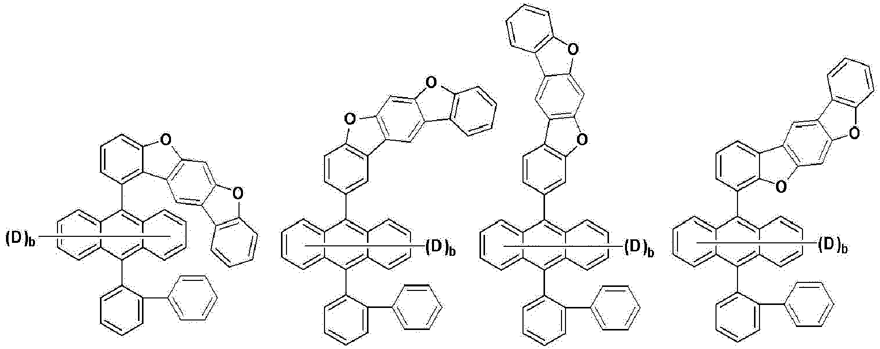 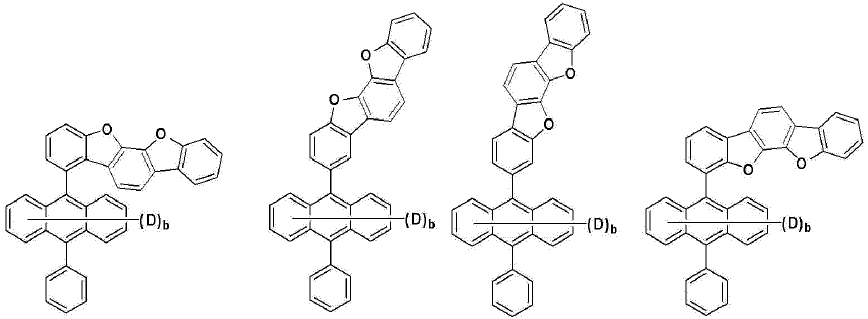 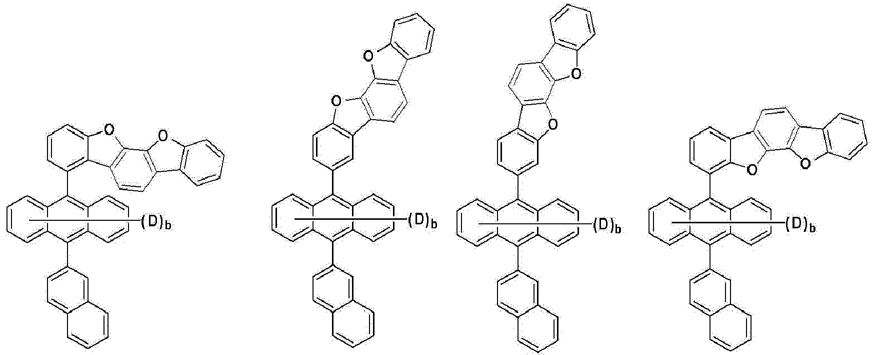 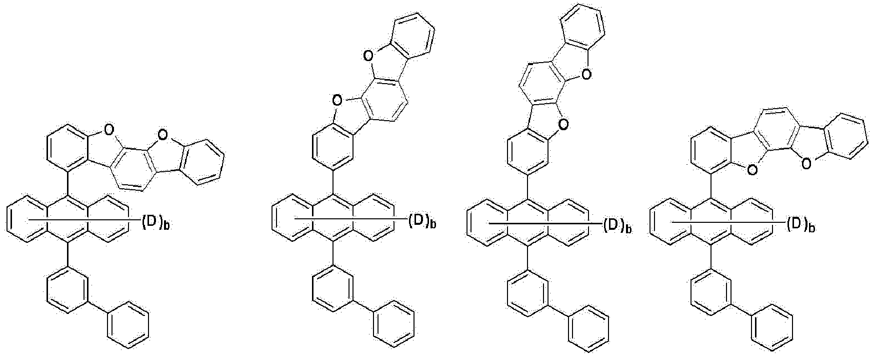 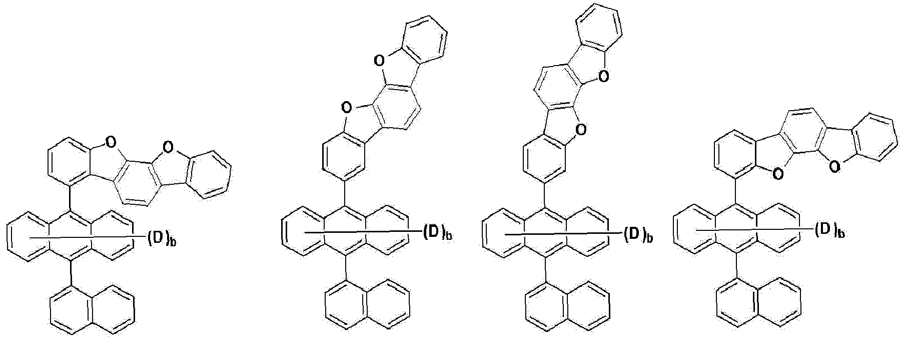 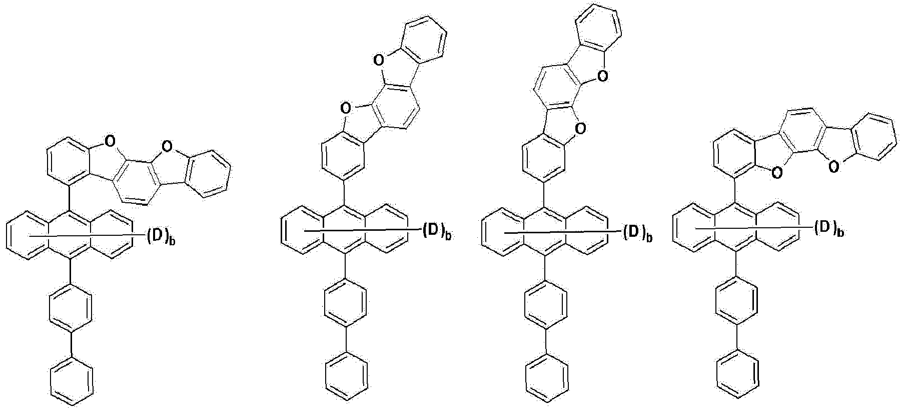 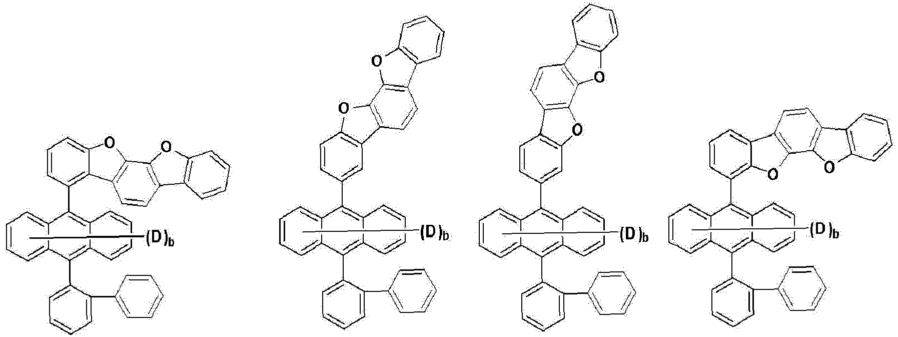 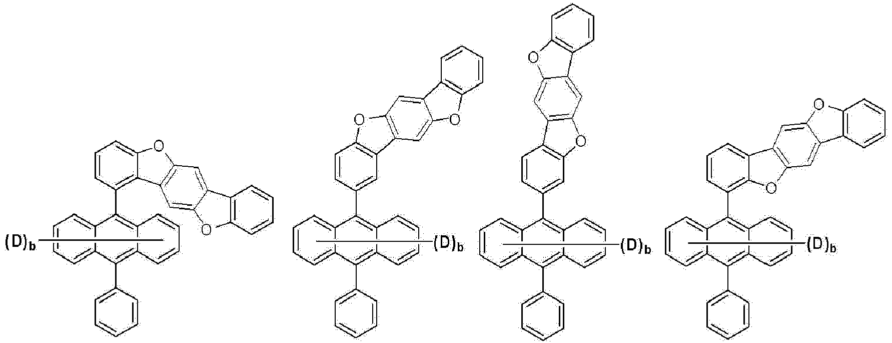 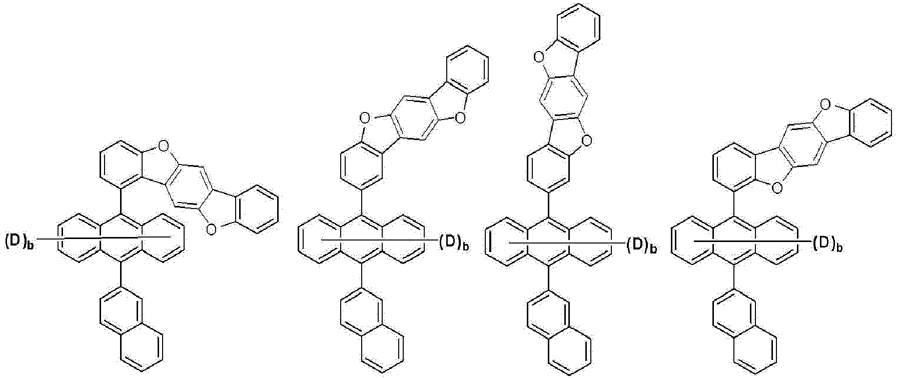 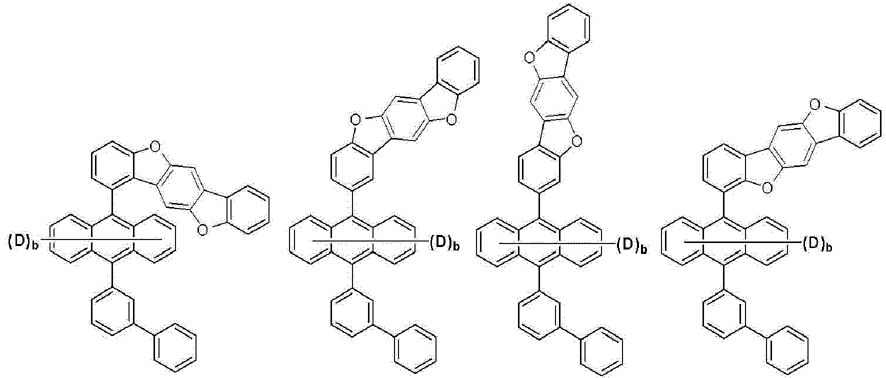 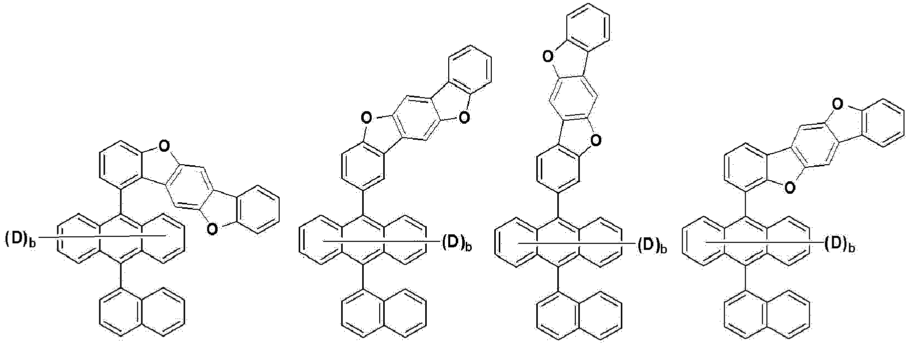 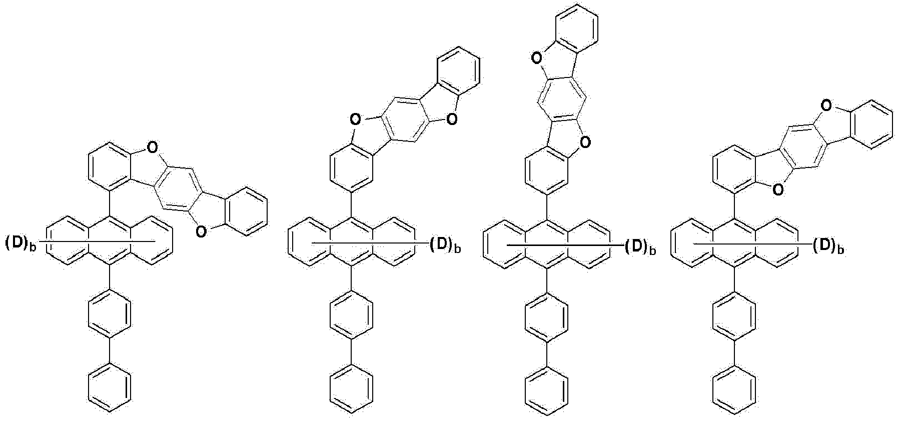 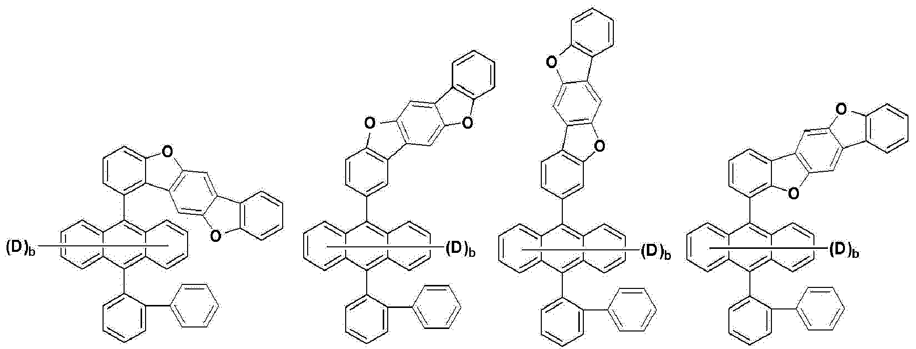 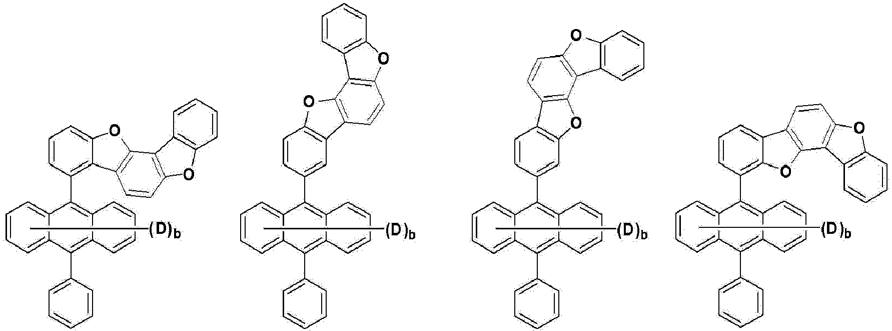 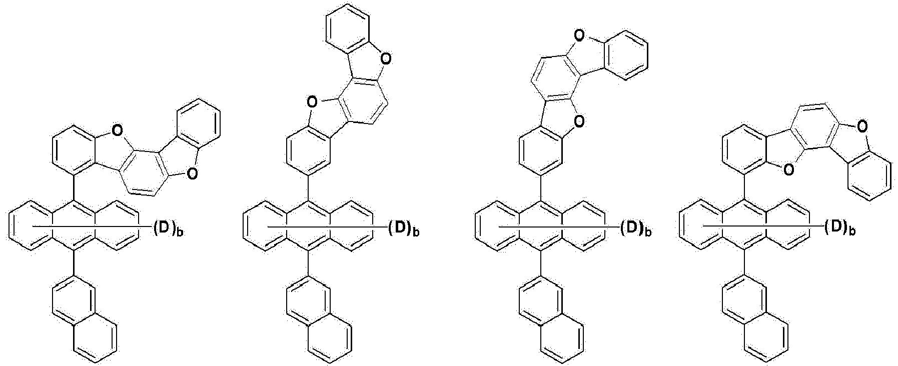 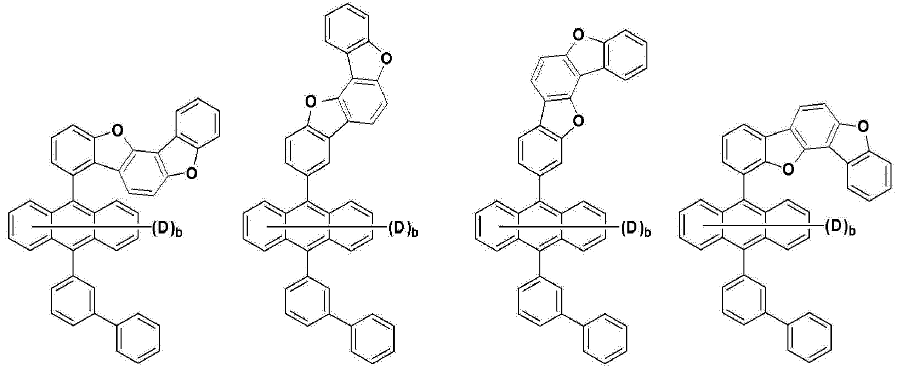 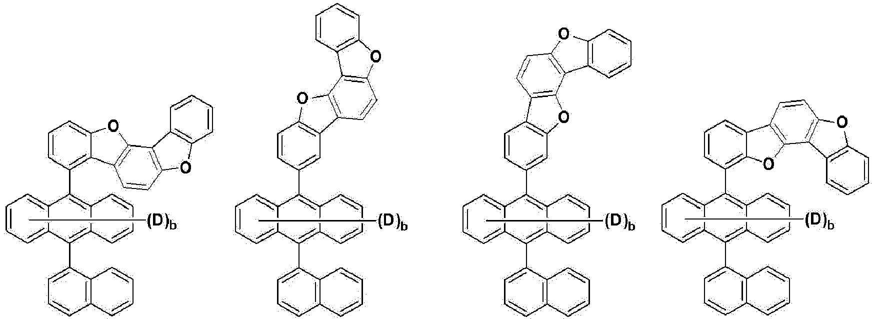 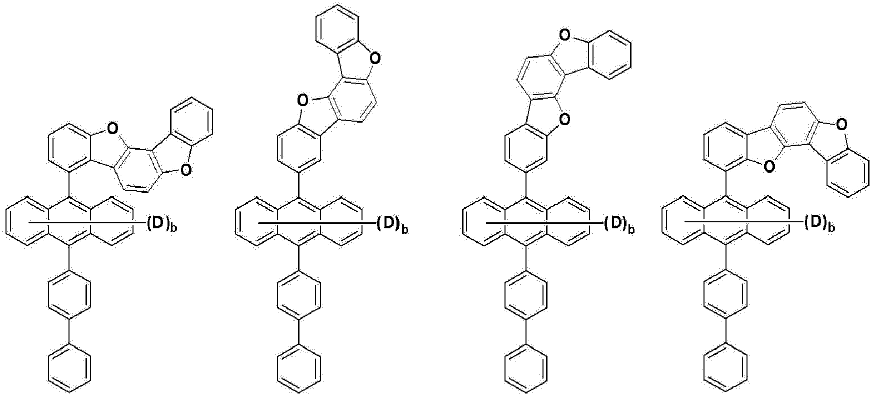 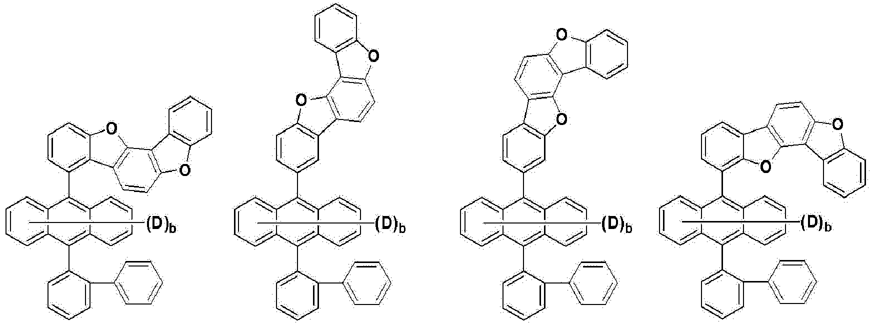 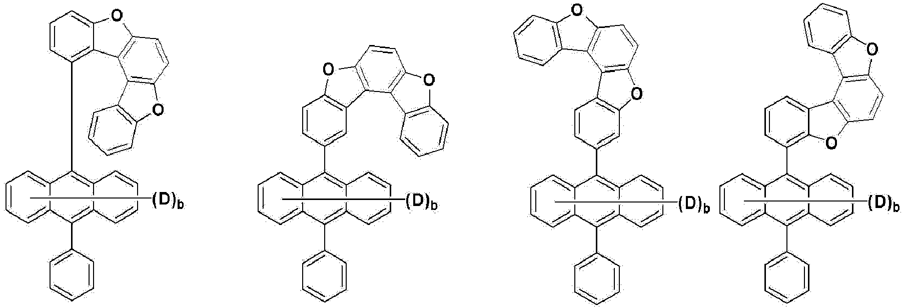 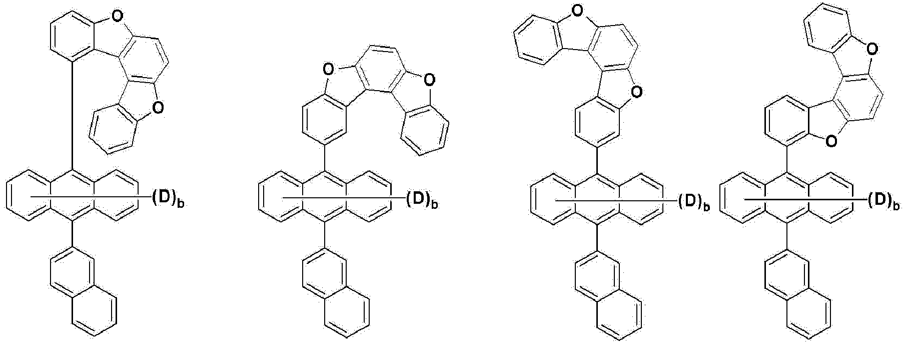 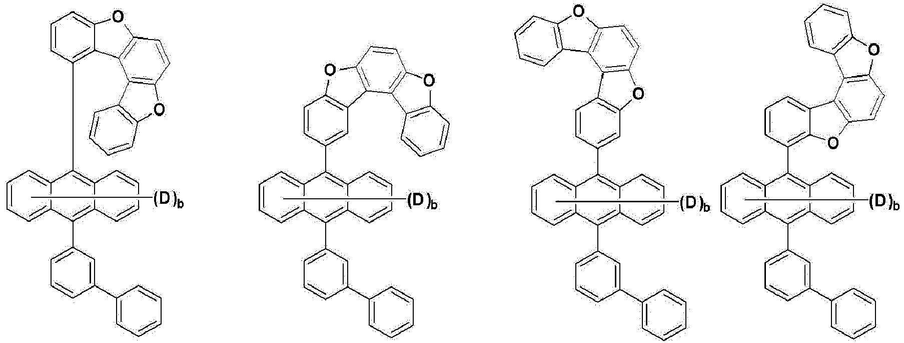 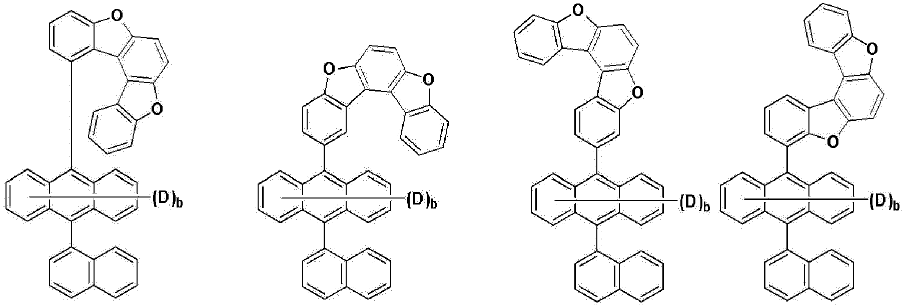 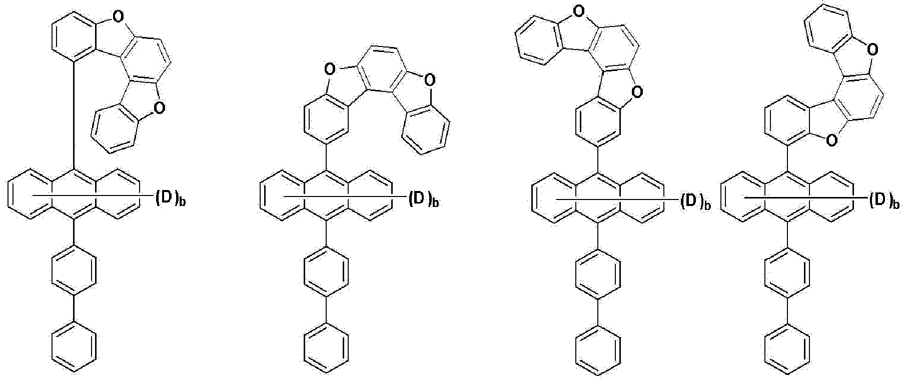 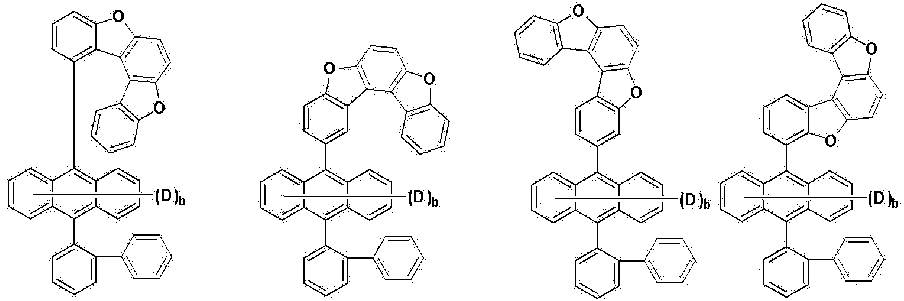 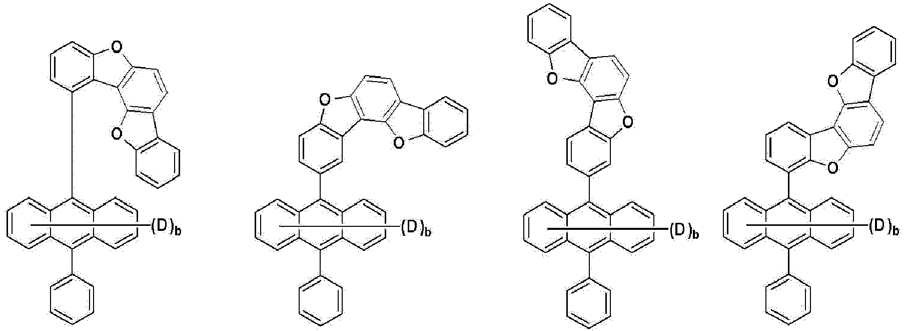 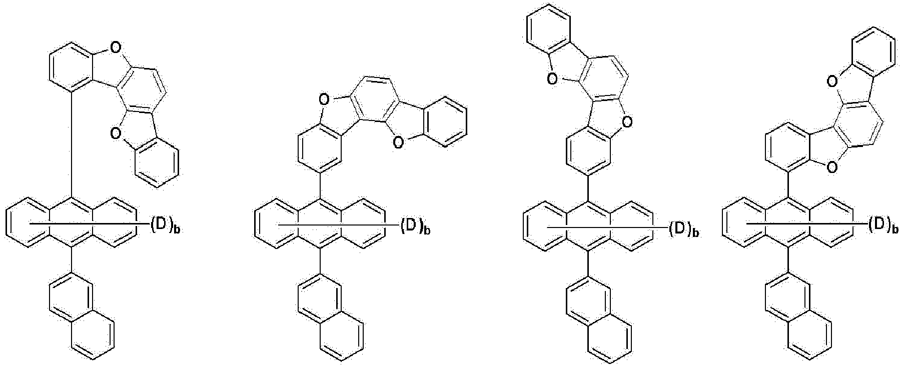 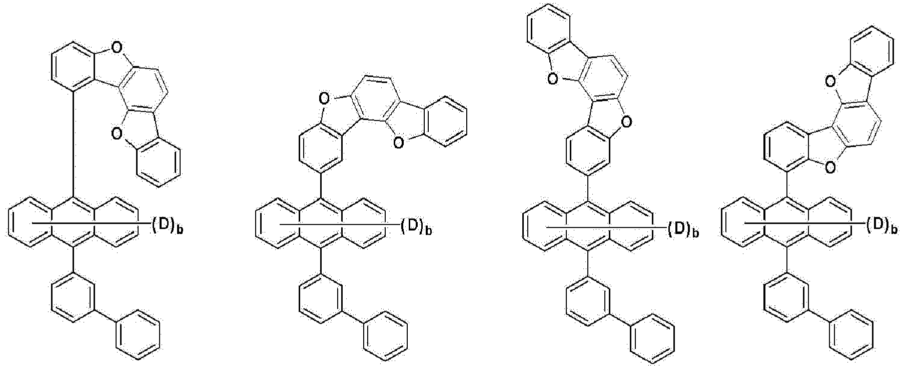 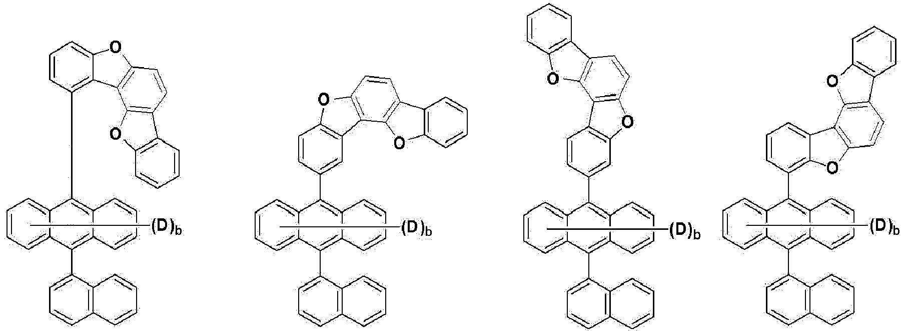 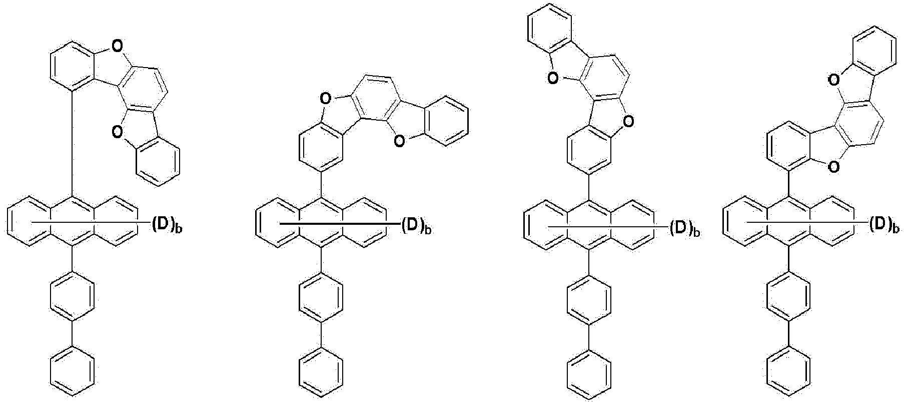 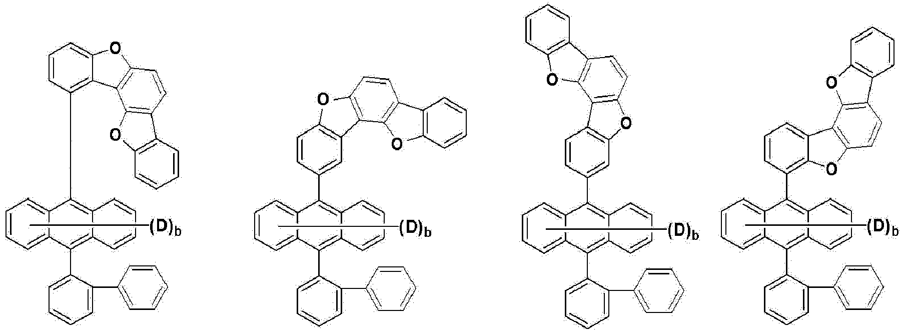 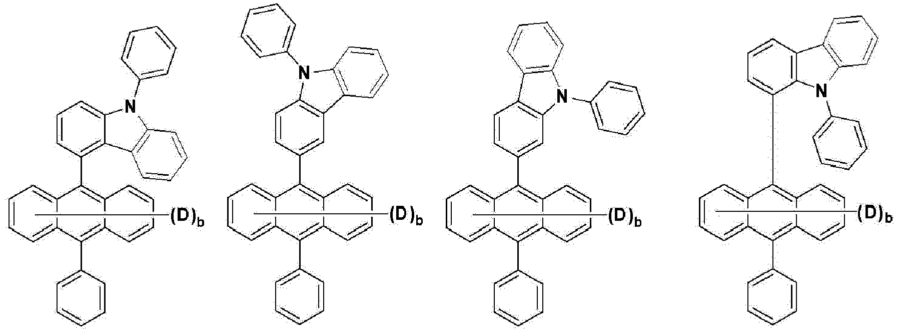 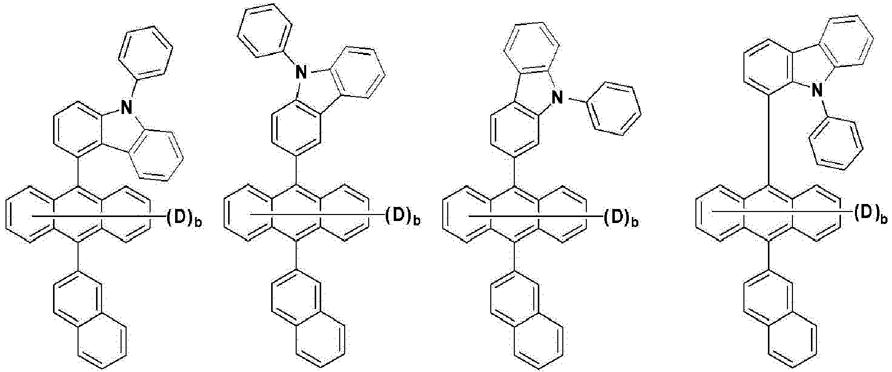 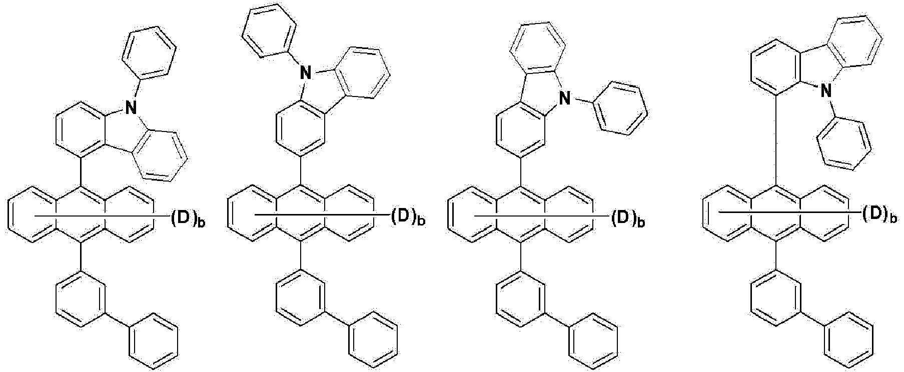 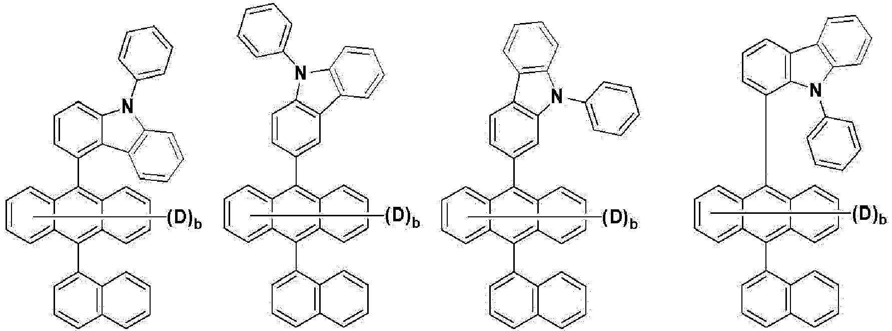 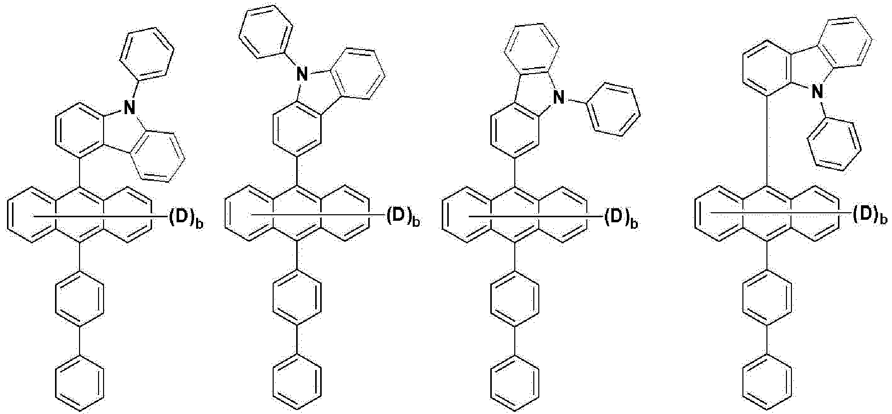 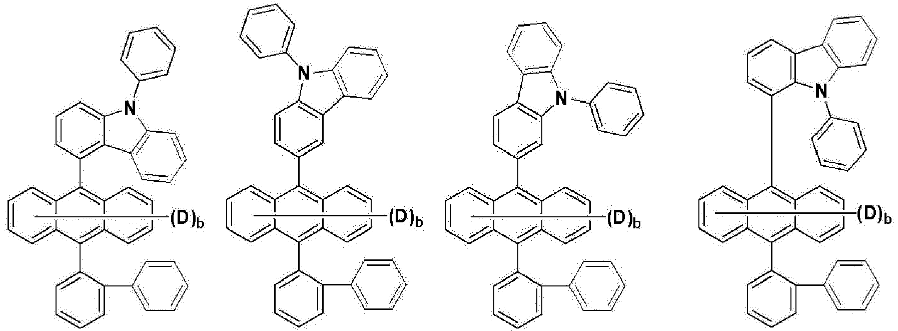 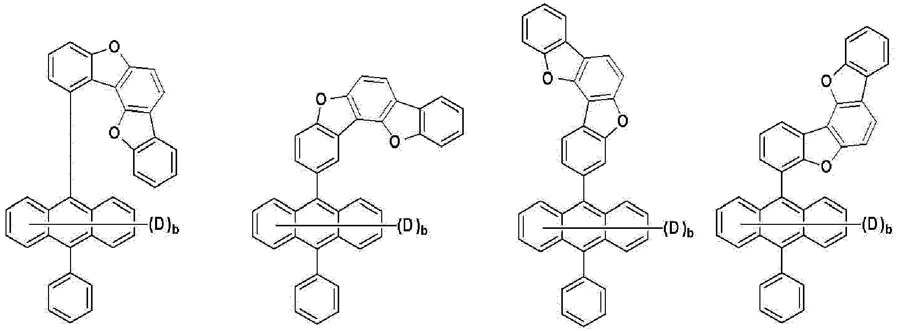 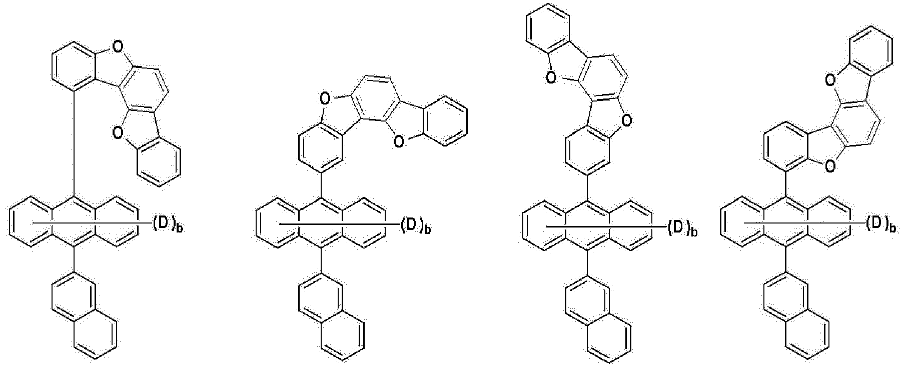 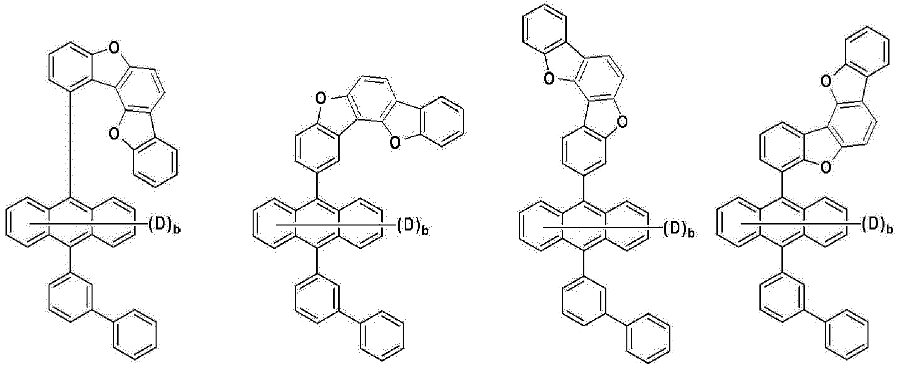 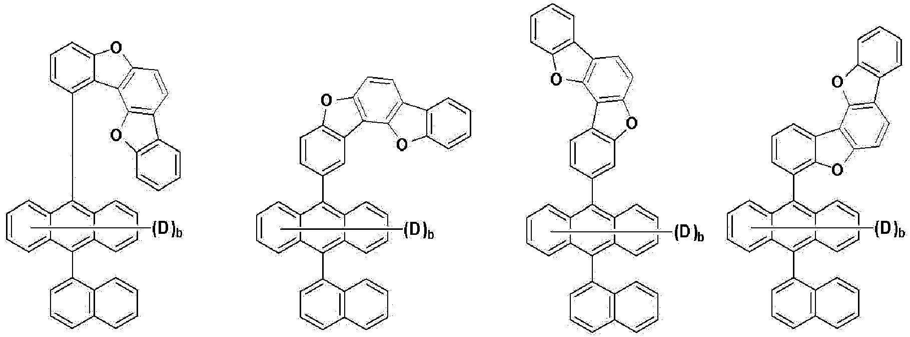 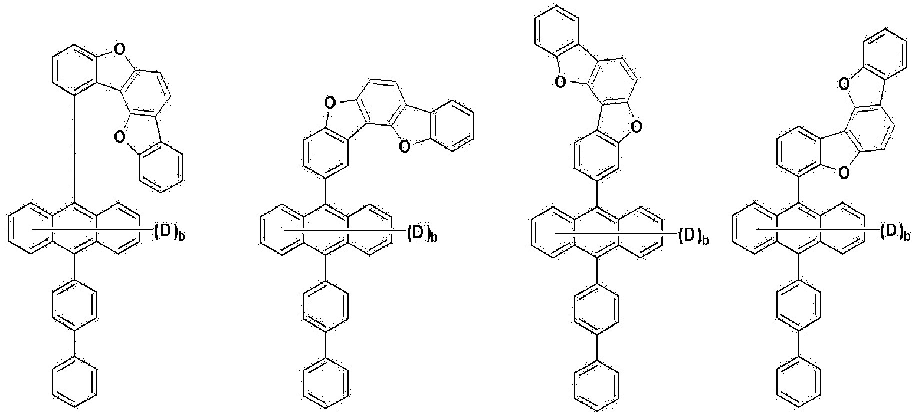 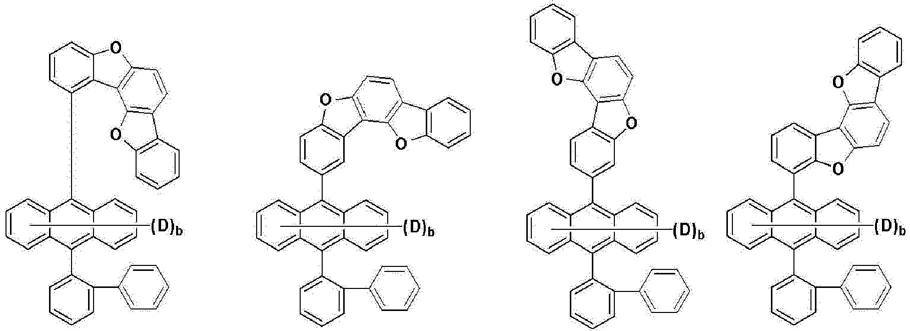 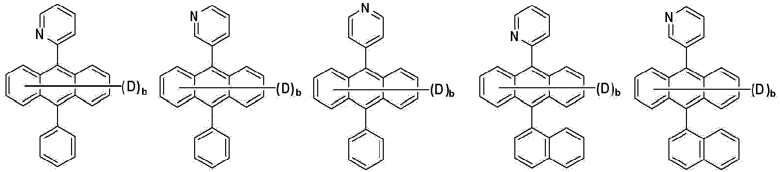 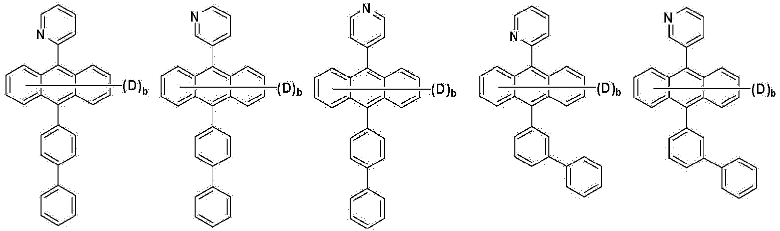 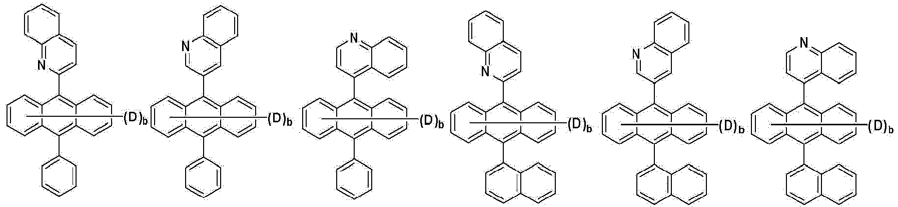 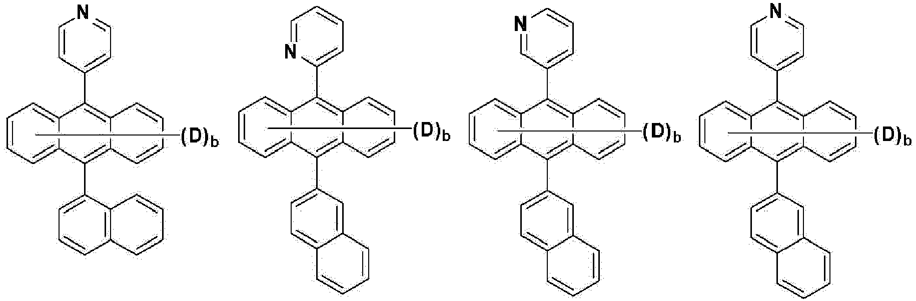 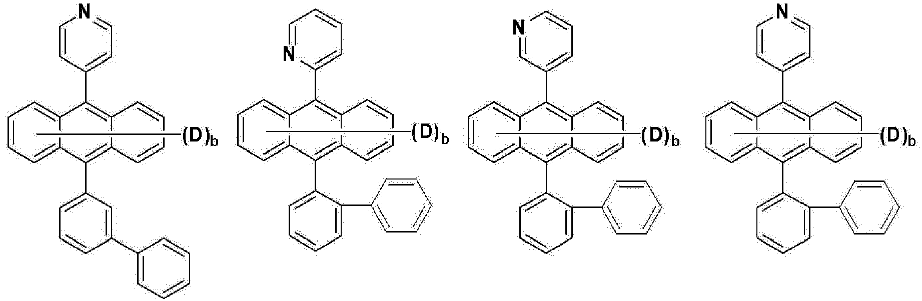 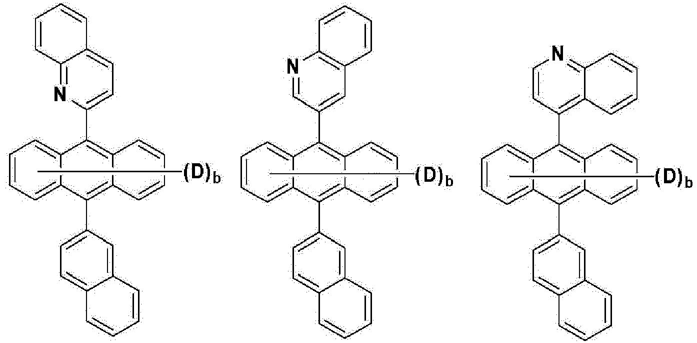 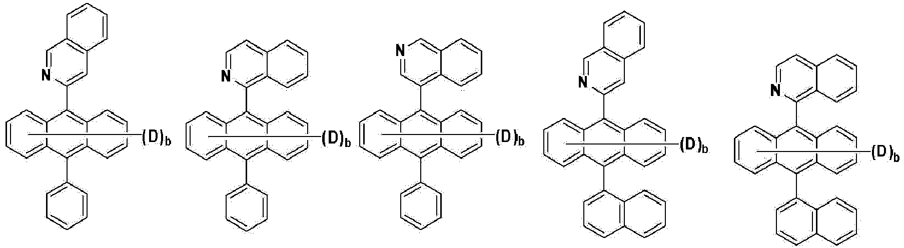 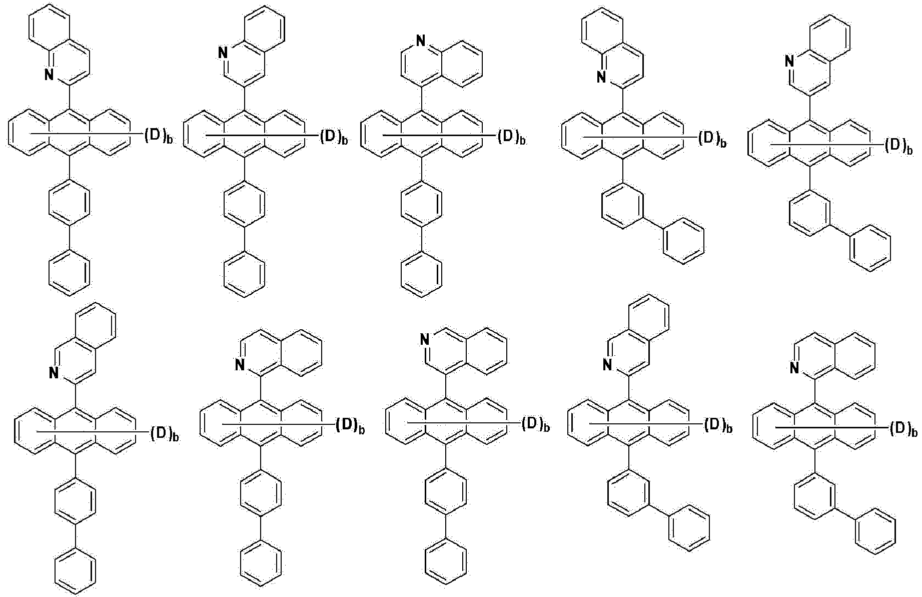 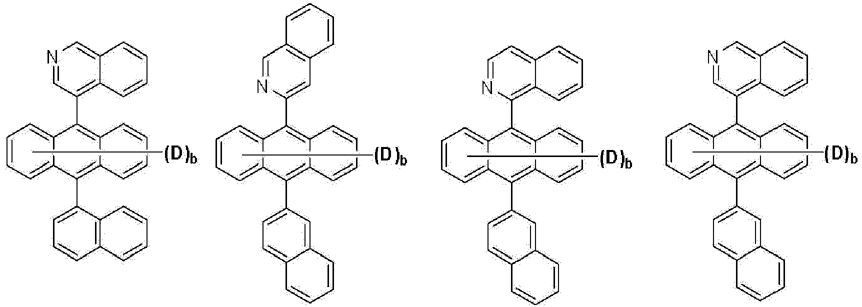 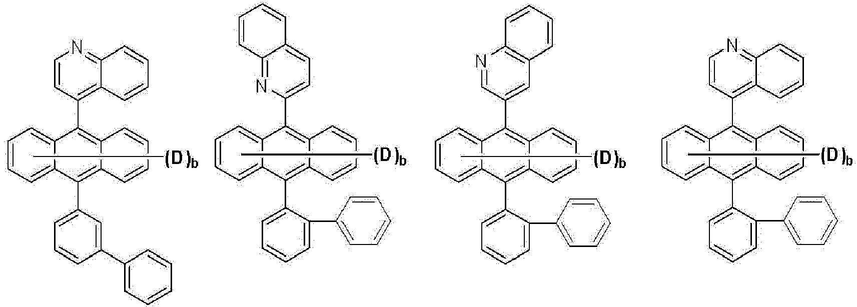 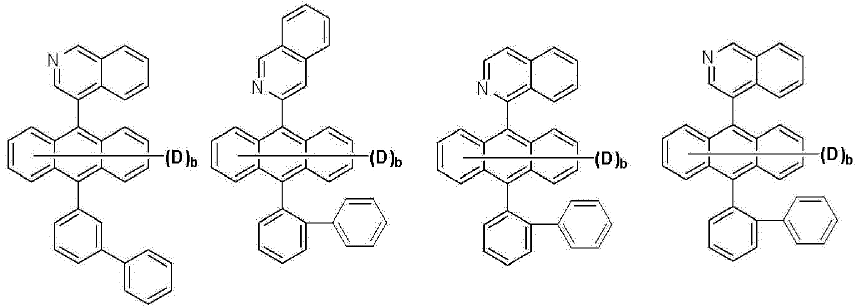 wherein b is an integer from 1 to 8.

13. A reaction composition, comprising:
a halogenated benzene having at least one deuterium; and
a compound of the following Chemical Formula 1: 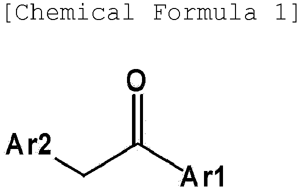
wherein in Chemical Formula 1:
Ar1 is deuterium, a substituted or unsubstituted aryl group, or a substituted or unsubstituted heterocyclic group; and
Ar2 is hydrogen, deuterium, a substituted or unsubstituted aryl group, or a substituted or unsubstituted heterocyclic group.

14. The reaction composition of claim 13, wherein the halogenated benzene having at least one deuterium is halogenated benzene-d5.

15. The reaction composition of claim 13, wherein Chemical Formula 1 is any one of the following Chemical Formulae 1-1 to 1-3: 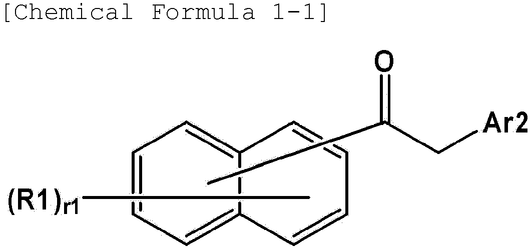 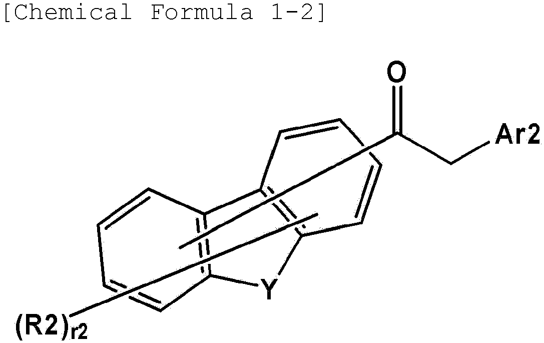 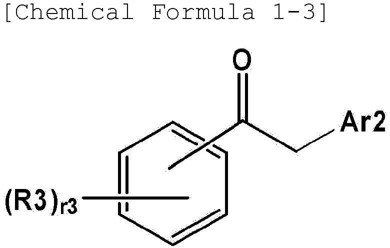 wherein in Formulae 1-1 to 1-3:
Y is CRaRb, NRc, O or S;
Ar2 is hydrogen, deuterium, a substituted or unsubstituted aryl group, or a substituted or unsubstituted heterocyclic group;
Ra, Rb, Rc and R1 to R3 are the same as or different from each other, and are each independently hydrogen, deuterium, a substituted or unsubstituted alkyl group, a substituted or unsubstituted aryl group, or a substituted or unsubstituted heterocyclic group, or are bonded to an adjacent group to form a substituted or unsubstituted ring;
r1 and r2 are each an integer from 1 to 7, and r3 is an integer from 1 to 5,
when r1 is 2 or higher, the R1s are the same as or different from each other,
when r2 is 2 or higher, the R2s are the same as or different from each other, and
when r3 is 2 or higher, the R3s are the same as or different from each other.

16. The reaction composition of claim 13, wherein Chemical Formula 1 is any one of the following structures:

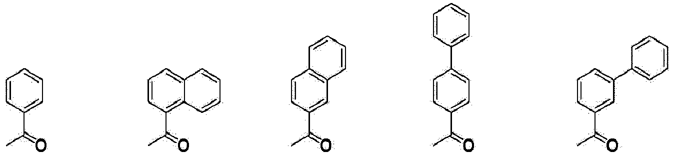

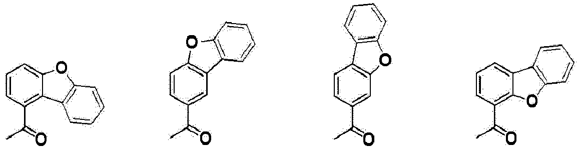

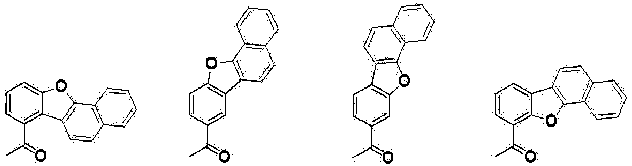

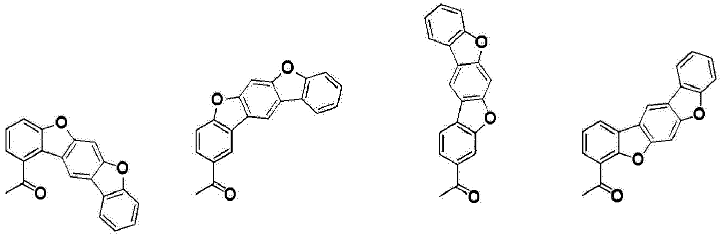

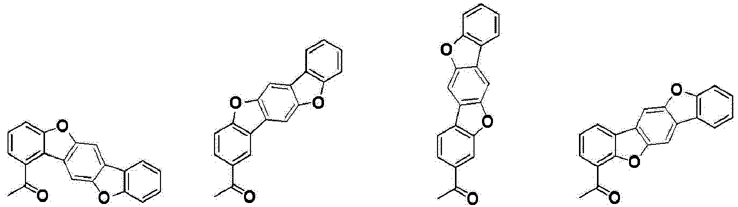

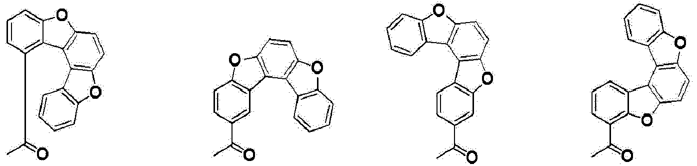

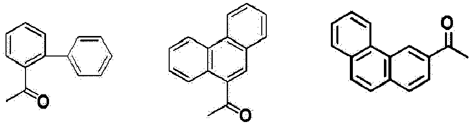

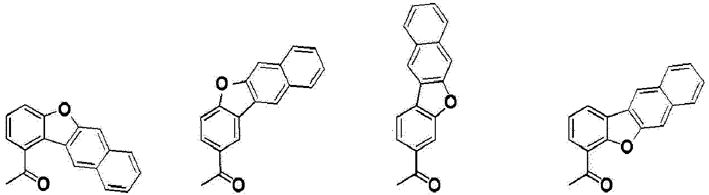

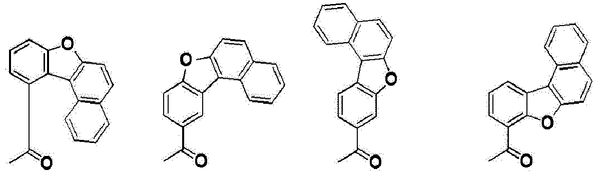

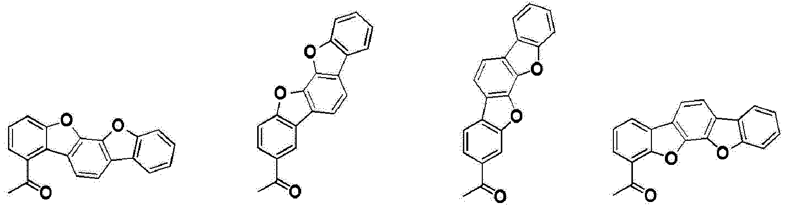

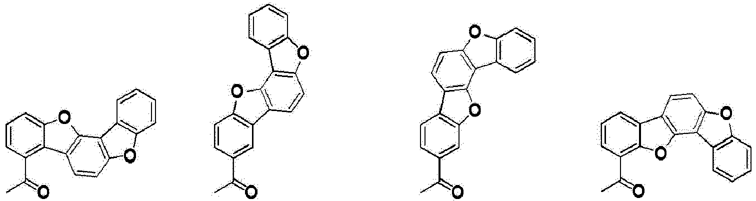

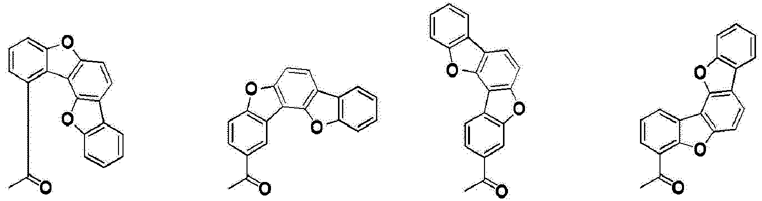

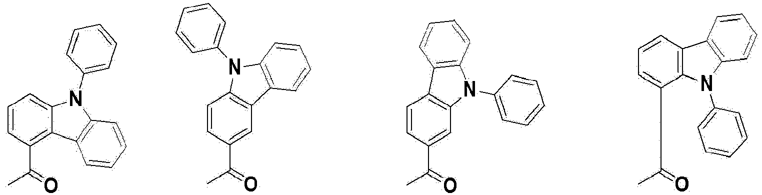

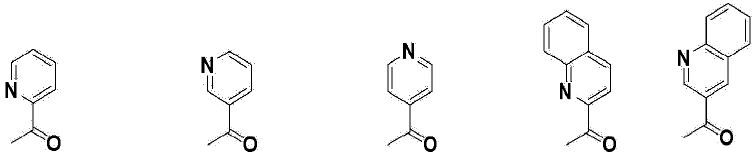

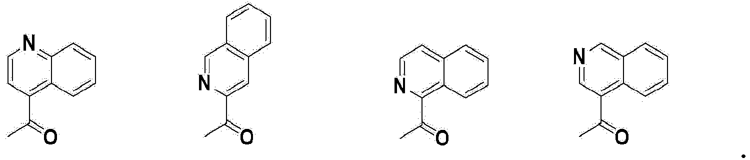

17. The reaction composition of claim 13, further comprising 2,2,6,6-tetramethylpiperidine, an alkyllithium and an ether-based solvent.

18. A deuterated anthracene compound prepared by the preparation method of any one of claims 1 to 4.

19. A composition, comprising an intermediate of the following Chemical Formula 5: 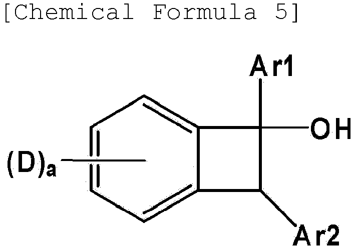 wherein in Chemical Formula 5:
Ar1 is deuterium, a substituted or unsubstituted aryl group, or a substituted or unsubstituted heterocyclic group;
Ar2 is hydrogen, deuterium, a substituted or unsubstituted aryl group, or a substituted or unsubstituted heterocyclic group; and
a is an integer from 1 to 4.

20. The composition of claim 19, further comprising a compound of Chemical Formula 2: 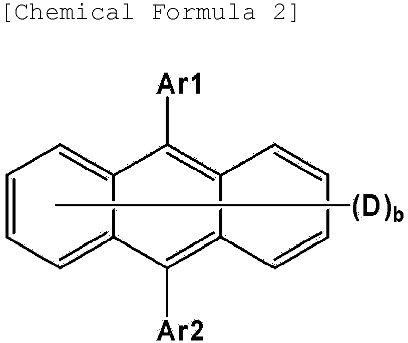 wherein in Chemical Formula 2:
Ar1 is deuterium, a substituted or unsubstituted aryl group, or a substituted or unsubstituted heterocyclic group;
Ar2 is hydrogen, deuterium, a substituted or unsubstituted aryl group, or a substituted or unsubstituted heterocyclic group; and
b is an integer from 1 to 8.
